# FASCICULE DE BREVET EUROPEEN

(11) **EP 0 214 029 B1**
(45) Date de publication et mention de la délivrance du brevet: **25.03.1992**
(21) Numéro de dépôt: 86401769.4
(22) Date de dépôt: 07.08.1986
(51) Int. Cl.: C07D 513/04, A61K 31/54

(54) **Dérivés de l'acide l-déthia 2-thia céphalosporanique, leur procédé de préparation, leur application comme médicaments et les compositions pharmaceutiques les renfermant**
1-Dethia-2-thiacephalosporancarbonsäurederivate, Verfahren zu deren Herstellung, ihre Verwendung als Arzneimittel und sie enthaltende pharmazeutische Zusammensetzungen
1-Dethia-2-thia cephalosporanic-acid derivatives, process for their preparation, their use as therapeutic agents and pharmaceutical compositions containing them

(30) Priorité: 09.08.1985 FR 8512218
(43) Date de publication de la demande: 11.03.1987
(73) Titulaire: ROUSSEL-UCLAF, 75007 Paris (FR)
(72) Inventeur: Teutsch, Jean Georges, F-93500 Pantin (FR); Bonnet, Alain, F-93190 Livry-Gargan (FR); Aszodi, Jozsef, F-94600 Choisy-Le-Roi (FR)
(74) Mandataire: Bourgouin, André

(56) Documents cités:
- EP-A- 0 153 229
- FR-A- 2 324 639
- FR-A- 2 505 840

## Description

Dans le brevet nα2 559 486 déposé le 13 Février 1984, la Société demanderesse a décrit de nouveaux dérivés de l'acide 1-déthia 2-thia céphalosporanique et leurs sels, leur procédé de préparation et leur application comme médicaments.

Les produits décrits dans ce brevet répondent à la formule générale: 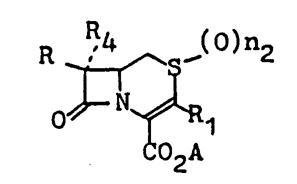 dans laquelle R représente soit un radical 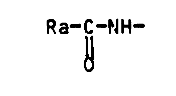 dans lequel Ra représente un radical organique, soit un radical 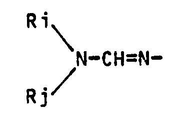 dans lequel Ri et Rj identiques ou différents représentent un atome d'hydrogène ou un radical hydrocarboné aliphatique ou aromatique, un radical hétérocyclique ou Ri et Rj forment avec l'atome d'azote auquel ils sont liés, un radical cyclique éventuellement substitué, soit un radical Rb-NH- dans lequel Rb représente un radical aryle carbocyclique ou hétérocyclique éventuellement substitué,

R₁ représente, soit :
a) un radical -Z-R₂, dans lequel R₂ est un radical alkyle, alkényle ou alkynyle éventuellement substitué ou interrompu par un hétéroatome et Z représente un atome de soufre éventuellement oxydé, de sélénium, d'oxygène ou -NH-,
b) un radical Zₐ-R₃, dans lequel R₃ représente un radical aryle carbocyclique ou hétérocyclique éventuellement substitué ou un ammonium quaternaire éventuellement substitué et Zₐ représente un radical méthylène, un atome de soufre, de sélénium, d'oxygène ou -NH-, soit Zₐ représente une simple liaison, soit Zₐ représente un radical -CH₂-S-c) un radical alkyle, alkényle ou alkynyle ayant de 2 à 8 atomes de carbone éventuellement substitué ou interrompu par un hétéroatome
d) un atome d'halogène, un radical nitrile, azido, thiocyanato ou isothiocyanato,
e) un radical azidométhyle, amino ou amino mono ou disubstitué méthyle, thiocyanato méthyle, isothiocyanatométhyle, carbamoyloxyméthyle R₄ représente un atome d'hydrogène ou un radical méthoxy, A représente un atome d'hydrogène, un équivalent de métal alcalin, alcalino-terreux, de magnésium, d'ammonium ou d'une base organique aminée ou A représente un groupement ester ou C0₂A représente un groupement C0₂-, n₂ représente un entier égal à 0, 1 ou 2.

Les produits répondant à la formule générale ci-dessus peuvent se présenter sous forme racémique ou optiquement active.

La société demanderesse a maintenant découvert qu'une classe particulière de produits présente des propriétés pharmacologiques particulièrement intéressantes .

La présente demande a ainsi pour objet les produits répondant à la formule générale (1) : 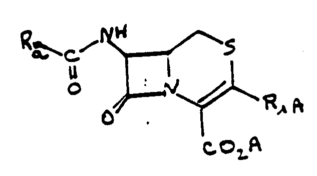 dans laquelle Rₐ représente un radical 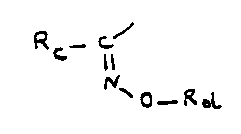 dans lequel R_{c} représente un radical 2-amino 4-thiazolyle. R_{d} représente : ou bien un atome d'hydrogène,

ou bien un radical alkyle, alkényle, alkynyle ou cycloalkyle ayant au plus 6 atomes de carbone, ou un radical phényle, lesdits radicaux alkyle, alkényle, alkynyle ou cycloalkyle étant éventuellement substitués par un radical choisi parmi les radicaux halogène, cyano, carbamoyle, nitro, amino, hydroxy, mercapto, oxo, carboxyle libre estérifié ou salifié,

ou bien un radical difluoro méthyle, trifluorométhyle, méthoxyméthyle, 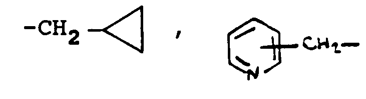 R_{lA} représente :
- soit un radical 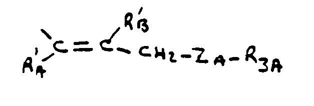
soit un radical 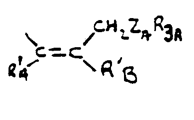 formules dans lesquelles R'_{A} et R'_{B} identiques ou différents représentent ou bien un atome d'hydrogène ou bien un radical alkyle ayant de 1 à 4 atomes de carbone, Z_{A} représente une simple liaison, un atome de soufre éventuellement oxydé ou un atome d'oxygène. R_{3A} représente ou bien un radical aryle carbocyclique ou hétérocyclique choisi parmi les radicaux phényle, diphényle, naphtyle, thiényle, furyle, pyrrolyle, imidazolyle, pyrazolyle, thiazolyle, isothiazolyle, oxazolyle, isoxazolyle, triazolyle, thiadiazolyle, oxadiazolyle, tétrazolyle, pyridinyle, pyrimidinyle, pyrazinyle, pyridazinyle, thiazinyle, oxazinyle, triazinyle, thiadiazinyle, oxadiazinyle, tétrazinyle, imidazolinyle, benzimidazolyle, benzothiazolyle, benzoxazole, ou bien à la condition que Z_{A} représente une simple liaison, un radical ammonium quaternaire choisi parmi les radicaux pyridinium, thienopyridinium, quinolinium, isoquinolinium, cyclopentylpyridinium, triméthylammonium, thiazolopyridinium, cyclohexenopyridinium, imidazopyridinium. Les radicaux R_{3A} pouvant être substitués par un ou plusieurs radicaux choisis parmi soit les radicaux alkyle ayant 1 à 4 atomes de carbone, ces radicaux alkyle étant eux-mêmes éventuellement substitués par un radical phényle ou thiényle, les radicaux phénoxy, méthoxy, éthoxycarbonyle, halogènes, hydroxy, carboxy libre estérifié ou salifié ou amino, soit les radicaux vinyle, allyle, butenyle, éthynyle, propargyle, phényle, tolyle, halogènes, amino, nitro, alkoxy ayant 1 à 4 atomes de carbone, méthylthio, hydroxy, mercapto, amino, carboxyle libre estérifié ou salifié ou carbamoyle, ou bien R_{3A} représente un radical acétyle, carbamoyle, alkoxycarbonyle, alkyle ou haloalkyle ayant 1 à 4 atomes de carbone, nitrile ou azido. R₄ représente un atome d'hydrogène ou un radical méthoxy. A représente un atome d'hydrogène, un équivalent de métal alcalin, alcalino-terreux, de magnésium, d'ammonium ou d'une base organique animée ou A représente un groupement ester ou CO₂A représente un groupement C0₂^{8.} n₂ représente un nombre entier égal à 0, 1 ou 2, ainsi que les sels des produits de formule (I) avec les acides minéraux ou organiques.

Les produits de formule (I) peuvent se présenter sous forme racémique ou optiquement active.

Parmi les valeurs de Rd, on peut citer les valeurs hydrogène, alkyle, alkényle éventuellement cyclique, alkynyle, aryle, hétéroaryle mono ou polycyclique et notamment les valeurs -CH₃, -CH₂-CH₃, -CHF₂, -CH₂-O-CH₃, -CH₂CN, -CH₂-CONH₂,

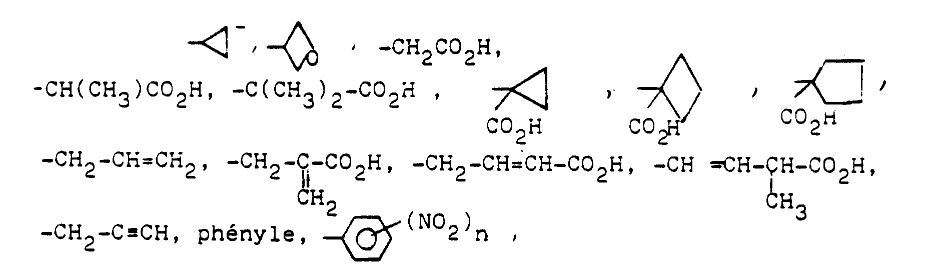

Parmi les valeurs de Rd, on préfère les valeurs méthyle, hydrogène, éthyle, allyle, 1-méthyl-1-carboxyéthyle, carboxyméthyle ou difluorométhyle.

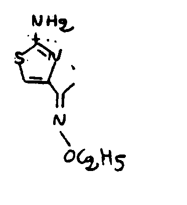

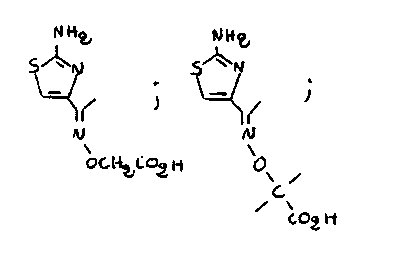

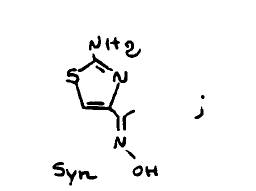

Parmi les valeurs de R_{3A}, on peut citer les radicaux phényle, diphényle, naphtyle, thiényle, furyle, pyrrolyle, imidazolyle, pyrazolyle, thiazolyle, isothiazolyle, oxazolyle, isoxazolyle, triazolyle, thiadiazolyle, oxadiazolyle, tétrazolyle, pyridinyle, pyrimidinyle, pyrazinyle, pyridazinyle, thiazinyle, oxazinyle, triazinyle, thiadiazinyle, oxadiazinyle, tétrazinyle, imidazolinyle, benzimidazolyle, benzothiazolyle, benzoxazole.

Parmi les différents radicaux aryles, on préfère les radicaux thiazol-2-yle, 1,3,4-thiadiazol-5-yle et 1,2,4-thiadiazol-5-yle, imidazol-2-yle, 1,3,4-triazol-5-yle, 1,3,4-thiadiazol-2-yle, 1,2,3-thiadiazol-5-yle, 1 H-tétrazol-5- yle, pyridin-2-yle, 1,3,4-triazin-2-yle, 1,3,5-triazin-4-yle, pyridinium, quinolinium, isoquinolinium, cyclopentylpyridinium, triméthylammonium.

Les radicaux R_{3A} peuvent éventuellement être substitués par un ou plusieurs radicaux choisis parmi les radicaux alkyle tels que méthyle, éthyle, propyle, isopropyle, butyle linéaire ou ramifié. Les radicaux alkyle peuvent eux-mêmes être substitués par un radical aryle tel que phényle ou thiényle, aryloxy tel que phénoxy alkyloxy inférieur tel que méthoxy, alcoxy carbonyle tel que éthoxy carbonyle, par un halogène tel que chloro ou bromo hydroxy libre ou protégé, carboxy libre estérifié ou salifié, amino, alkylamino ou dialkylamino, acylamido.

Les radicaux R_{3A} peuvent également être substitués par un ou plusieurs radicaux alkényle tel que vinyle, allyle, butényle, un radical alkynyle tel que éthynyle ou propargyle, un groupe aryle tel que phényle, tolyle, un halogène tel que chloro, bromo, iodo, fluoro, un groupe amino ou nitro, un radical alkoxy ayant de 1 à 4 atomes de carbone tel que méthoxy, alkylthio tel que méthylthio, un radical hydroxy, mercapto, amino, carboxyle libre estérifié ou salifié, carbamoyle, R_{3A} peut également être substitué par deux substituants formant ensemble un radical cyclique tel que cyclopentyle ou cyclohexyle. R_{3A} peut également représenter, de préférence, un radical ammonium quaternaire non cyclique, tel que triméthylammonium.

Parmi les valeurs de R_{1A}, on préfère les valeurs suivantes :

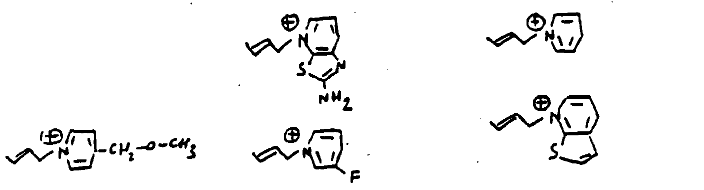

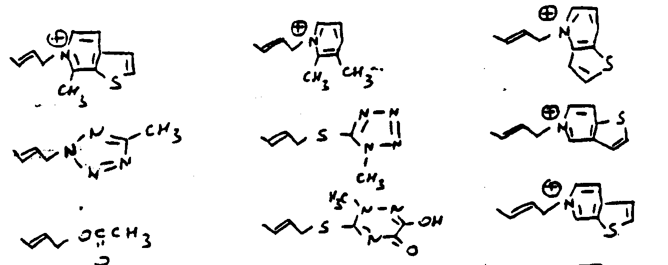

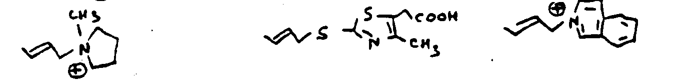

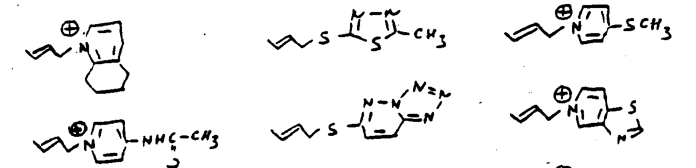

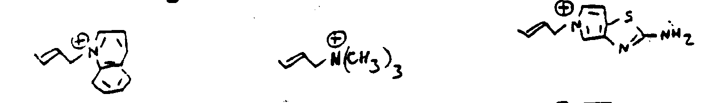

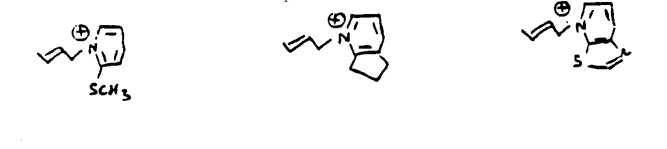

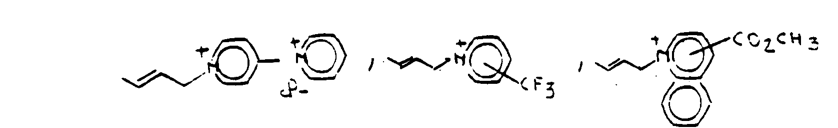

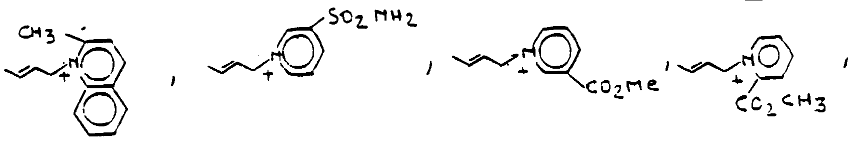

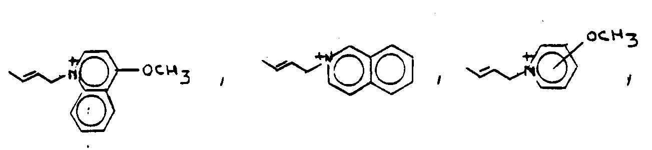

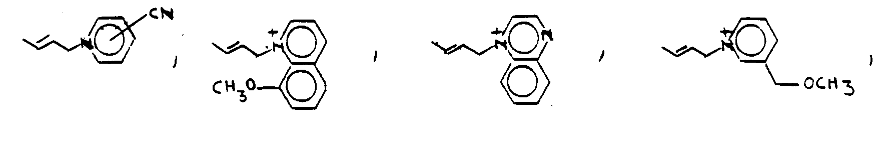

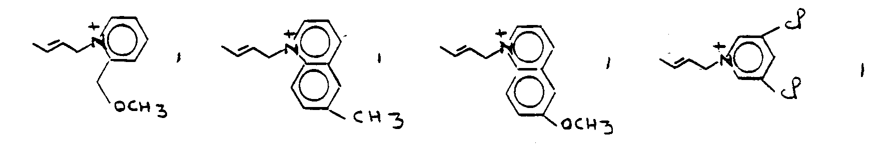

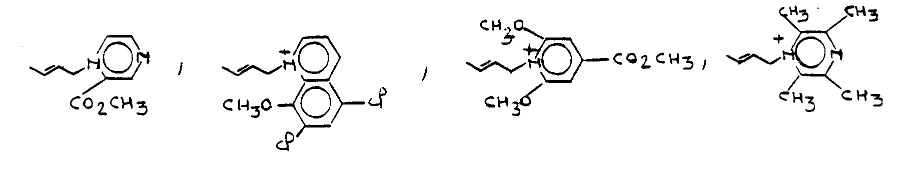

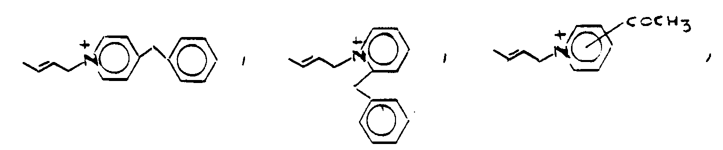

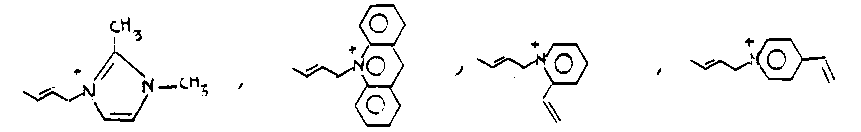

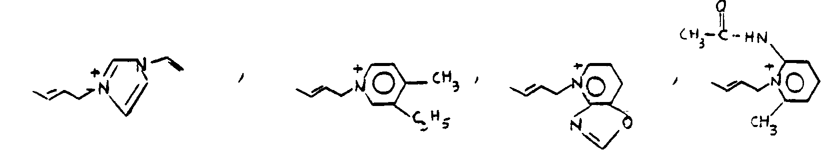

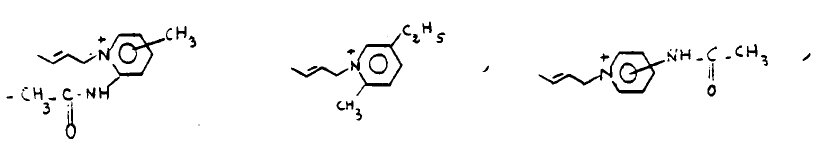

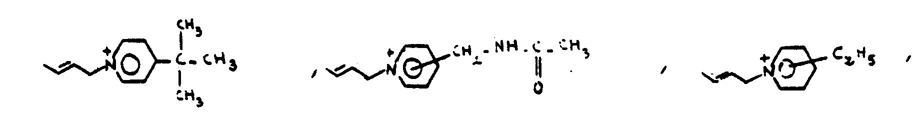

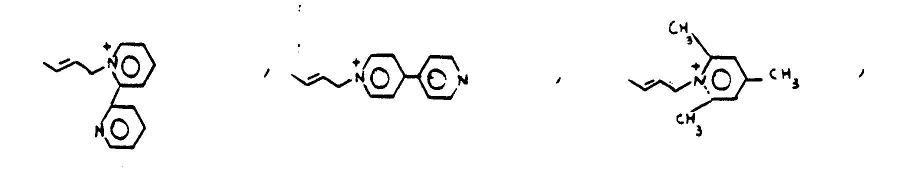

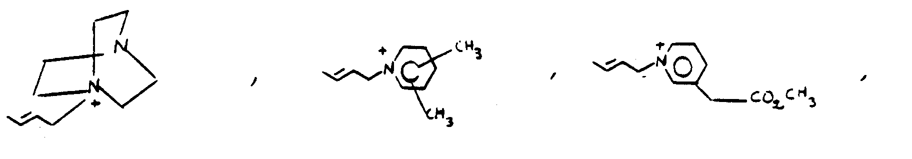

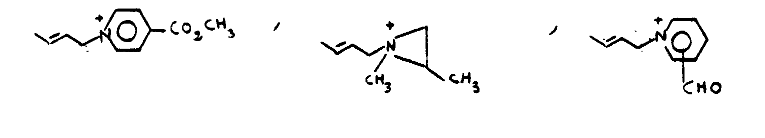

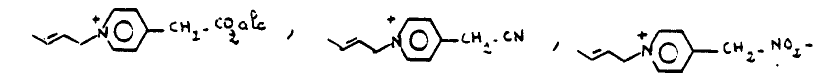

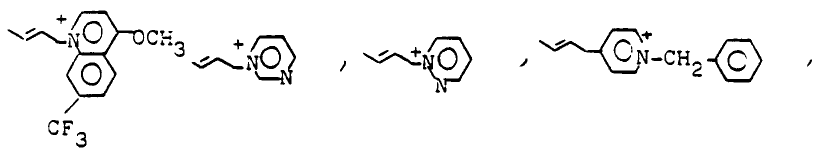

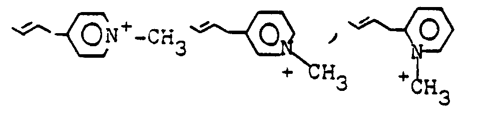

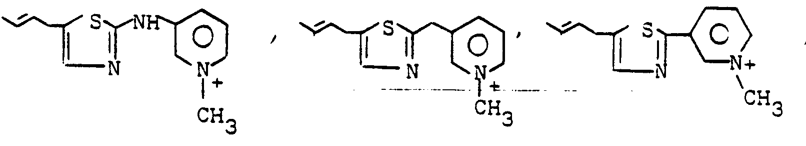

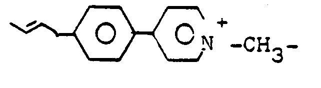

Parmi les valeurs de A, on peut citer un équivalent de sodium, de potassium, de lithium, de calcium, de magnésium, d'ammonium ou d'une base organique. On peut citer, parmi les bases organiques, la méthylamine, la propylamine, la triméthylamine, la diéthylamine, la triéthylamine, la N,N-diméthyléthanola- mine, le tris(hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements esters facilement clivables que peut représenter A, les groupements méthoxyméthyle, éthoxyméthyle, isopropyloxyméthyle, a-méthoxyéthyle, a-éthoxyéthyle, méthylthiométhyle, éthylthiométhyle, isopropylthiométhyle, pivaloylloxyméthyle, acétoxyméthyle, propionyloxyméthyle, butyryloxyméthyle, isobutyryloxyméthyle, valéryloxyméthyle, isovaléryloxyméthyle, tert-butyl- carbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1-hexadécanoy- loxyéthyle, 1-propionyloxypropyle, 1-méthoxycarbonyloxyéthyle, méthoxycarbonyloxyméthyle, 1-acétyloxy- butyle, 1-acétyloxyhexyle, 1-acétyloxyheptyle, phtalidyle, 5,6-diméthoxyphtalidyle, tert-butylcarbonylméthy- le, allyle, 2-chloroallyle, méthoxycarbonylméthyle, benzyle ou tert-butyle.

On peut encore citer entre autres restes de groupements esters que peut représenter A, les groupements méthoxyéthoxyméthyle, diméthylaminoéthyle, cyanométhyle, tert-butyloxycarbonyméthyle, 2,2-éthy- lènedioxyéthyle, cyanoéthyle, 2,2-diméthoxyéthyle, 2-chloroéthoxyméthyle, 2-hydroxyéthoxyéthyle, 2,3- époxypropyle, 3-diméthylamino, 2-hydroxypropyle, 2-hydroxyéthyle, 2-méthylaminoéthoxyméthyle, 2-ami- noéthoxyméthyle, 3-méthoxy 2,4-thiadiazol-5-yle, 2-tétrahydropyranyle, 2-méthoxyprop-2-yle, 1-hydroxyprop-2-yle, isopropyle, carbamoylméthyle, chlorométhyle, 2-chloroéthyle, acétylméthyle, 2-méthyl- thioéthyle ou thiocyanatométhyle.

On peut encore citer entre autres restes de groupements esters que peut représenter A, les groupements 2-chloro 1-acétyloxyéthyle, 2-bromo 1-acétyloxyéthyle, 2-fluoro 1-acétyloxyéthyle, 2-méthoxy 1-acétyloxyéthyle, 2-méthyl 1-acétyloxypropyle, 2-acétyloxyprop-2-yle, 1-méthoxyacétyloxyéthyle, 1-acétyl- carbonyloxyéthyle, 1-hydroxyacétyloxyéthyle, 1-formylcarbonyloxyéthyle, 1-(2-thiényl) carbonyloxyéthyle, 1-(2-furyl) carbonyloxyéthyle, 1-(5-nitro 2-furyl) carbonyloxyéthyle, 1-(2-pyrrolyl) carbonyloxyéthyle, 1-(propionyloxycarbonyloxy)éthyle, 1-(propyloxycarbonyloxy)éthyle, 1-(isopropyloxycarbonyloxy)éthyle, 1-(méthoxyéthoxycarbonyloxy) éthyle, 1-(allyloxycarbonyloxy)éthyle, 1-(2,3-époxy)propyloxycarbonyloxy éthyle, 1-(2-furyl)méthyloxycarbonyloxy éthyle, 1-(2-fluoro)éthyloxycarbonyloxy éthyle, 1-(méthoxycarbonyloxy)-propyle, (2-méthoxycarbonyloxy)prop-2-yle, (méthoxycarbonyloxy)chlorométhyle, 1-(méthoxycarbonyloxy)2-chloroéthyle, 1-(méthoxycarbonyloxy)2-méthoxyéthyle, 1-(méthoxycarbonyloxy)1-allyle. A peut également représenter le groupement :

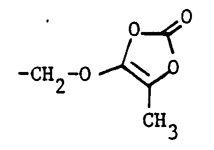

Les produits de formule (I) peuvent également se présenter sous forme de sels d'acides organiques ou minéraux.

Parmi les acides avec lesquels on peut salifier le ou les groupements amino ou le groupement ammonium quaternaire des produits (1), on peut citer entre autres, les acides acétique, trifluoroacétique, maléïque, tartrique, méthanesulfonique, benzène sulfonique, p-toluène sulfonique, trifluorométhanesulfonique, formique, phosphorique, sulfurique, chlorhydrique, bromhydrique, iodhydrique.

Parmi les valeurs de A, on peut notamment citer les groupements esters de formule: 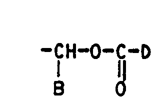 dans lesquels B représente un atome d'hydrogène ou un radical alkyl linéaire ou ramifié éventuellement substitué, renfermant de 1 à 5 atomes de carbone et D représente un radical alcoyle ou alcoxy linéaire ou ramifié, éventuellement substitué, renfermant de 1 à 15 atomes de carbone et notamment de 1 à 5 atomes de carbone et plus particulièrement les groupements esters dans lesquels B représente un atome d'hydrogène ou un radical méthyle ou éthyle et D représente un radical méthyle, éthyle, méthoxy ou éthoxy.

Parmi les valeurs de A, on peut encore citer le radical 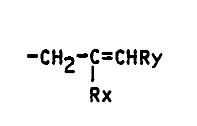 dans lequel Rx représente les valeurs hydrogène, alkyle, notamment méthyle, éthyle, halogène, notamment chlore, et Ry représente les valeurs hydrogène, halogène, aryle, notamment phényle, éventuellement substitué par méthyle, méthoxy ou halogène, ou Ry représente la valeur alkyle éventuellement substitué par acyloxy, par alcoxycarbonyl, par halogène.

Parmi les valeurs de A, on peut encore citer le radical 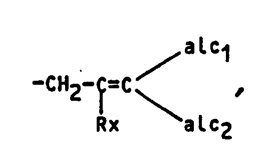 dans lequel Rx est défini comme ci-dessus et alc₁ et alc₂, identiques ou différents, représentent un radical alkyle renfermant de 1 à 4 atomes de carbone.

Parmi les produits de formule (1), la présente demande a plus spécialement pour objet les produits répondant à la formule (I_{A}): 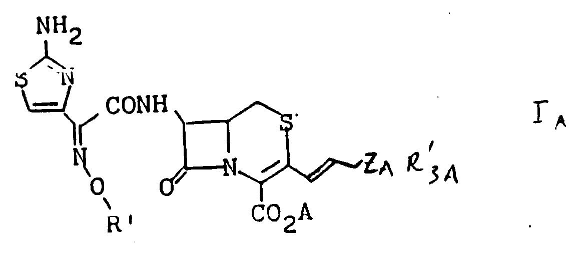 isomère syn dans laquelle R' représente un atome d'hydrogène ou un radical alkyle alkényle, alkynyle ou cycloalkyle ayant au plus 6 atomes de carbone, éventuellement substitué par un radical carboxy libre, estérifié ou salifié ou un radical difluorométhyle ou méthoxyméthyle, Zₐ représente une simple liaison et R'_{3A} représente un radical pyridinium, thiénopyridinium, thiazolopyridinium, cyclopentylpyridinium, cyclohexénopyridinium, triméthylammonium, ces radicaux pouvant être substitués par un radical choisi parmi les radicaux alkyle et amino.

Parmi les produits préférés, on peut citer ceux décrits ci-après dans les exemples, et notamment les produits suivants :
- le sel interne de 7-/3-/7-//(2-amino 4-thiazolyl) 2-/Z-(difluorométhoxy) imino/ acétyl/ amino/ 2-carboxy 8-oxo 4-thia 1-azabicyclo /4.2.0./ oct-2-èn-3-yl/ 2-(E)-propényl/ thiéno /2,3-b/ pyridinium (6S)(7S) ;
- le sel interne de 5-/3-/7-//(2-amino 4-thiazolyl) (méthoxyimino) acétyl/ amino/ 2-carboxy 8-oxo 4-thia 1-azabicyclo /4.2.0./ oct-2-èn-3-yl/ 2(E)-propényl/ 2-méthyl thiazolo /4,5-c/pyridinium syn (6S)(7S) ;
- le sel interne de 7-/3-/7-//(2-amino 4-thiazolyl) 2-(Z)-(méthoxyimino) acétyl/ amino/ 2-carboxy 8-oxo 4-thia 1-azabicyclo /4.2.0./ oct-2-èn-3-yl/ propèn-2-(E)-yl/ thiéno /2,3-b/ pyridinium (6S)(7S) ;
- l'acide 7-//(2-amino 4-thiazolyl) (méthoxyimino) acétyl/ amino/ 3-//3-(1,3,4-thiadiazol-2-yl) thio/ 1-(E)-propényl/ 8-oxo 4-thia 1-azabicyclo /4.2.0./ oct-2-èn-2-carboxylique syn (6S)(7S);
- le sel interne de 5-/3-/7-//(2-amino 4-thiazolyl) (difluorométhoxy) imino/ acétyl/ amino/ 2-carboxy 8-oxo 4-thia 1-azabicyclo /4.2.0./ oct-2-èn-3-yl/ 2-(E)-propényl/ 2-méthyl thiazolo /4,5-c/ pyridinium (6S)(7S) Z.

L'invention a également pour objet un procédé de préparation des produits de formule générale I telle que définie ci-dessus, caractérisé en ce que l'on traite un produit de formule VII: 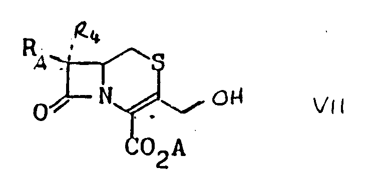 dans laquelle R₄ et A ont la signification indiquée ci-dessus et R_{A} représente soit un radical amino libre ou protégé par un groupement protecteur mono ou divalent, soit R_{A} représente le radical 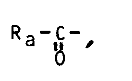 Rₐ ayant la signification indiquée ci-dessus, soit par un agent d'halogénation soit par un agent de sulfonation puis par la triphénylphosphine, ou par un réactif de formule P(OAlk)₃ dans laquelle Alk représente un radical alkyle ayant de 1 à 4 atomes de carbone, pour obtenir ou bien un produit de formule VIII_{A}: 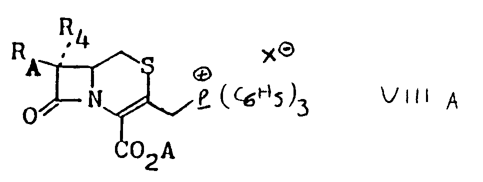 dans lequel R_{A}, R₄, et A ont la signification précédente et X^{⊖} représente le reste d'un anion, ou bien un produit de formule VIII'A: 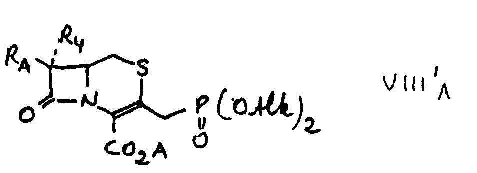 dans laquelle R_{A}, R4, A et Alk ont la signification précédente, produits de formules VIII_{A} ou VIII'_{A} que, si désiré l'on traite d'abord par une base forte puis par un halogène d'alkyle de formule hal-R"_{A} dans laquelle hal représente un atome d'halogène et R"A a les valeurs de R'A différentes d'un atome d'hydrogène, pour obtenir les produits de formules VIII_{B} ou VIII'_{B}: 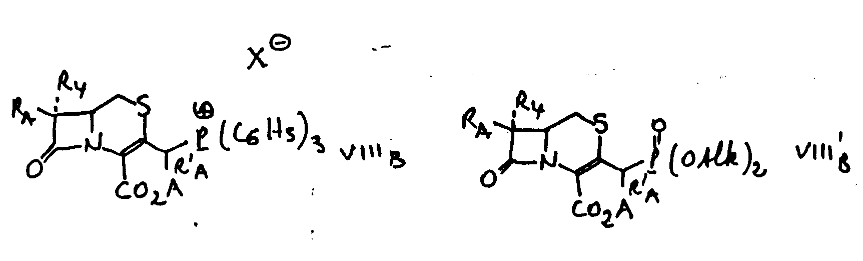 formules dans lesquelles R'_{A} a la signification indiquée ci-dessus, les produits de formules VIII_{B} et VIII'_{B} représentant l'ensemble des produits de départ VIII_{A} et VIII'_{A} et des produits obtenus après action sur ces produits du dérivé hal-R"_{A}, produits de formules VIII_{B} ou VIII'_{B} que l'on traite par un produit de formule 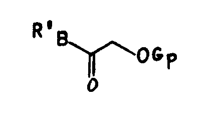 dans laquelle R'_{B} a la valeur indiquée ci-dessus et Gp représente un groupement protecteur du radical hydroxyle pour obtenir un produit de formule IX: 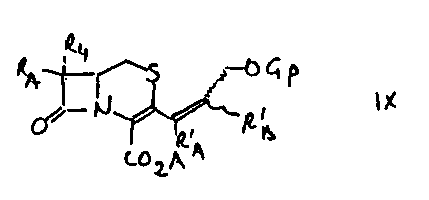 sous forme d'un mélange des isomères E et Z ou sous forme d'isomère E ou Z produit de formule IX sur lequel on effectue les réactions suivantes dans un ordre quelconque:
a) coupure du groupement protecteur Gp;
b) lorsque R_{A} représente un radical amino protégé par un groupement protecteur mono ou divalent, élimination de ce groupement protecteur et traitement du produit dans lequel R_{A} représente un radical amino par un acide de formule IVa : 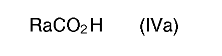 formule dans laquelle Ra a la signification indiquée ci-dessus, ou par un dérivé fonctionnel de cet acide,
c) Séparations des isomères E et Z; pour obtenir un produit de formule X: 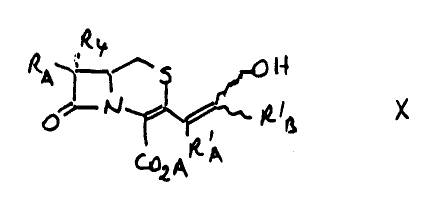 dans laquelle R_{A} a la signification indiquée ci-dessus, produit de formule X sur lequel on fait agir soit un dérivé réactif du groupement R_{3A} pour obtenir un produit de formule XI_{A}: 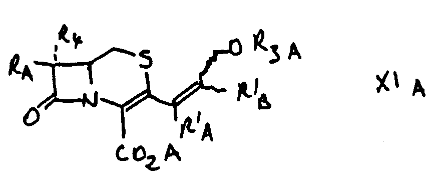 soit un dérivé d'un groupement d'activation du radical hydroxyle puis un dérivé ou un dérivé réactif du groupement R_{3A} pour obtenir un produit de formule XI_{B}: 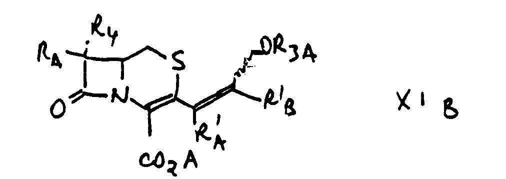 soit un dérivé d'un groupement d'activation du radical hydroxyle puis le produit de formule HS-R_{3A} ou un dérivé réactif de ce produit, pour obtenir un produit de formule Xl_{C}: 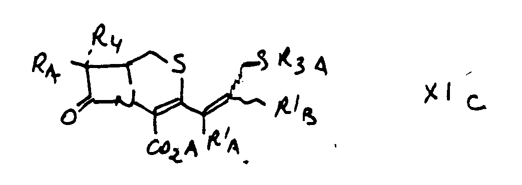 produits de formule XI_{A}, XI_{B} ou Xl_{c} que l'on soumet si nécessaire ou si désiré, à l'une quelconque ou à plusieurs des réactions suivantes, dans un ordre quelconque:
   a) coupure par hydrolyse, hydrogénolyse ou par action de la thiourée de tout ou partie du ou des groupements protecteurs;
   b) estérification ou salification par une base du ou des groupements carboxy ou sulfo;
   c) salification par un acide du ou des groupements amino;
   d) dédoublement de la molécule pour obtenir un produit optiquement actif;

L'agent d'halogénation que l'on utilise de préférence est le chlorure de thionyle dans un solvant chloré tel que le chlorure de méthylène ou dans le diméthyl formamide. On obtient alors intermédiairement un produit de formule Vlla:

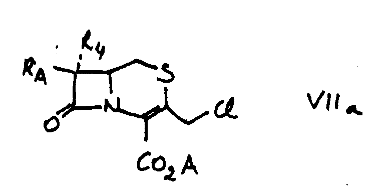

On peut également utiliser d'autres réactifs d'halogénation tels que le pentachlorure de phosphore, l'oxychlorure de phosphore ou le chlorure de tosyle.

L'agent de sulfonation peut être par exemple l'anhydride trifluorométhylsulfonique. On opére alors éventuellement en présence d'une base de préférence la 2,6-diméthylpyridine ou lutidine. De manière générale, les produits de sulfonation sont instables et ne sont pas isolés.

L'action de la triphénylphosphine ou du réactif de formule P(OAlk)3 dans laquelle Alk représente de préférence un radicl éthyle est réalisée dans les conditions usuelles. L'action de la triphénylphosphine ou du réactif de formule P(OAlk)3 est de préférence réalisée en présence de silice sur le produit halogéné de formule Vlla; on opère en général en chauffant au reflux d'un solvant tel que le toluène ou le chloroforme. Dans le cas où l'on fait agir un agent de sulfonation sur un produit de formule VII, l'action de la triphénylphosphine ou du produit de formule P(OAlk)3 est effectuée de préférence en même temps, en raison de l'instabilité des produits de sulfonation.

La base forte avec laquelle on traite éventuellement les produits de formule VillA et VIII'_{A} peut être choisie parmi le tert butylate de potassium ou de lithium, le disopropylamidure de lithium, le bis triméthyl silyl amidure de lithium.

L'halogénure d'alkyle de formule hal-R"A est de préférence le chlorure ou le bromure.

L'action du produit de formule 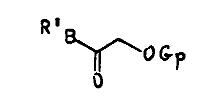 sur les produits de formules VIII_{B} ou VIII'_{B} est effectuée de préférence dans un solvant chloré tel que le chlorure de méthylène. On peut opérer en présence d'une base relativement faible telle que la triéthylamine ou le DBU (1,8-diazabicyclo /5,4.0/ undec-7-ène) ou le DBN (1,5-diazabicyclo /4,3,0/ non-5-ène). On opère alors à température ambiante ou en chauffant.

On peut également opérer en présence d'une base forte telle que le tert-butyl lithium ou un alcoolate tel que le tert-butylate de lithium, le bistriméthylsilyl amidure de lithium ou le disopropylamidure de lithium. On opère alors à faible température. La réaction peut par exemple être conduite de -70αC à la température ambiante.

Le groupement protecteur que représente Gp peut être un groupement silyle tel que le triméthylbusilyle ou le tert-butyl diméthylsilyle. On peut cependant utiliser un groupement tel que le tétrahydropyranyle.

La coupure du groupement de protection Gp est effectuée dans les conditions classiques. On opère par exemple en présence d'un acide ou d'un ion fluorure apporté par l'acide fluorhydrique ou le fluorure de tétrabutylammonium.

Lorsqu'on obtient un produit de formule IX dans lequel R_{A} représente un radical amino, dans un mode préférentiel d'exécution du procédé, on traite le produit de formule (IX) par un dérivé fonctionnel d'un produit de formule (IVₐ). Ce dérivé fonctionnel peut être par exemple un halogénure, un anhydride symétrique ou mixte, un amide ou un ester activé.

Comme exemple d'anhydride mixte, on peut citer par exemple celui formé avec le chloroformiate d'isobutyle et celui formé avec le chlorure de pivaloyle et les anhydrides mixtes carboxylique sulfonique formés par exemple avec le chlorure de paratoluènesulfonyle. Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

On peut citer également l'azide d'acide ou l'amide d'acide.

L'anhydride peut être formé in situ par action de carbodiimide NN'-disubstitué, par exemple la N,N-dicyclohexylcarbodiimide.

La réaction d'acylation est conduite de préférence dans un solvant organique tel que le chlorure de méthylène. On peut cependant utiliser d'autres solvants tels que le tétrahydrofuranne, le choroforme ou le diméthylformamide.

Lorsqu'on utilise un halogénure d'acide et de manière générale lorsqu'une molécule d'acide halohydri- que est libérée au cours de la réaction, on réalise la réaction de préférence en présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en général inférieure ou égale à la température ambiante.

La réaction subséquente de séparation éventuelle des isomères E et Z est effectuée selon les technique usuelles. On opère de préférence par chromatographie sur silice.

La transformation des produits de formule X en produits de formule XI_{A} est effectuée également selon les méthodes classiques.

Le dérivé du groupement R_{3A} que l'on utilise, peut par exemple être un halogénure dérivé d'un radical aryle carboxyclique ou hétérocyclique éventuellement substitué. On utilise alors de préférence le fluorure lorsque R_{3A} représente par exemple un radical acétyle ou carbomoyle. On opère de préférence en présence d'un dérivé réactif tel qu'un anhydride ou un halogénure.

La transformation des produits de formule X en produits de formule XI_{B} est effectuée de préférence selon les conditions suivantes : on prépare un dérivé activé du radical hydroxyle par action, par exemple, d'un dérivé d'un acide sulfonique tel que l'anhydride de l'acide trifluorométhyl sulfonique. On opère à basse température ou à température ambiante.

Lorsque R_{3A} ne représente pas un ammonium quaternaire, on opère de préférence en présence d'une base.

La transformation des produits de formule X en produits de formule XIα est effectuée dans des conditions similaires.

On prépare d'abord un dérivé activé du groupement hydroxyle dans les conditions énoncées précédemment, notamment à partir de l'anhydride de l'acide trifluoro méthyl sulfonique. L'action du produit de formule HSR_{3A} est effectuée de préférence en présence d'une base organique telle que la 2,6-diméthylpyridine et d'une source soluble d'iodure. On opère alors de préférence en présence d'iodure de tétraalkylam- monium.

Les produits de formules XI_{A}, XI_{B} et Xl_{C} constituent des produits de formule I_{A} lorsqu'ils ne comportent aucun groupement protecteur et lorsque A ne représente pas, parmi les groupements esters facilement clivables, l'un de ceux que l'on désire éliminer.

Dans les autres cas, l'action sur le produit de formule XI_{A},XI_{B} ou Xl_{C} d'un ou plusieurs agents d'hydrolyse, d'hydrogénolyse ou de la thiourée a pour but d'éliminer le ou les groupements protecteurs que peuvent comporter les différents produits.

La nature des réactifs à mettre en jeu dans tous ces cas est bien connue de l'homme de métier. Des exemples de telles réactions sont donnés plus loin dans la partie expérimentale.

On donne ci-après une énumération non exhaustive des moyens pouvant être mis en oeuvre pour éliminer les différents groupements.

L'élimination du ou des groupements protecteurs peut être effectuée par hydrolyse, celle-ci étant acide, basique ou utilisant l'hydrazine.

On utilise préférentiellement l'hydrolyse acide pour éliminer les groupements alkoxy et cycloalkoxycar- bonyle éventuellement substitués, tels que tert-pentyloxycarbonyle ou tert-butyloxycarbonyle, les groupements aralcoxycarbonyle éventuellement substitués tels que benzyloxycarbonyle, les groupements trityle, benzhydryle, tert-butyle ou 4-méthoxybenzyle.

L'acide que l'on utilise de préférence peut être choisi dans le groupe constitué par les acides chlorhydrique, benzène sulfonique ou para-toluène sulfonique, formique ou trifluoroacétique. On peut cependant utiliser d'autres acides minéraux ou organiques.

L'hydrolyse basique est utilisé préférentiellement pour éliminer les groupements acyle tels que trifluoroacétyle.

La base que l'on utilise de préférence est une base minérale telle que l'hydroxyde de sodium ou de potassium. On peut également utiliser la magnésie, la baryte ou un carbonate ou carbonate acide de métal alcalin tel que les carbonates et carbonates acides de sodium ou de potassium ou d'autres bases.

On peut également utiliser l'acétate de sodium ou de potassium.

L'hydrolyse utilisant l'hydrazine est utilisée de préférence pour éliminer des groupes tels que phtaloyle.

Certains groupements peuvent également être éliminés par le système zinc-acide acétique (pour le groupement trichloroéthyle), les groupements benzhydryle, benzyloxycarbonyle sont éliminés de préférence par l'hydrogène, en présence d'un catalyseur.

Le groupement chloroacétyle est éliminé par action de la thiourée en milieu neutre ou acide selon le type de réaction décrit par MASAKI J.A.C.S., 90, 4508, (1968).

On peut également utiliser d'autres méthodes de déprotection connues dans la littérature.

Parmi les groupes préférés, on peut citer les groupements formyle, acétyle, éthoxycarbonyle, mésyle, trifluoroacétyle, chloroacétyle, trityle. On préfère particulièrement les radicaux trityle et chloroacétyle.

L'acide que l'on utilise de préférence est l'acide trifluoroacétique.

La salification des produits peut être effectuée selon les méthodes usuelles.

La salification peut, par exemple, être obtenue par action sur un produit sous forme acide ou sur un solvat, par exemple le solvat éthanolique ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate tri-sodique. On peut également faire appel à des sels d'acides organiques.

Comme sels d'acides organiques, on peut mentionner, par exemple, les sels de sodium d'acides carboxyliques aliphatiques, linéaires ou ramifiés, saturés ou insaturés de 1 à 18 et de préférence de 2 à 10 atomes de carbone. Ces radicaux aliphatiques peuvent être interrompus par un ou plusieurs hétéroatomes tels que l'oxygène ou le soufre ou substitués par des radicaux aryle, comme par exemple : phényle, thiényle, furyle, ou par ou plusieurs radicaux hydroxyle ou par un ou plusieurs atomes d'halogène tels que fluor, chlore ou brome, préférentiellement chlore, par un ou plusieurs radicaux carboxyliques ou alkoxycar- bonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques des acides aromatiques suffisamment solubles comme par exemple des acides benzoïques substitués, de préférence, par des radicaux alkyles inférieurs.

Comme exemples de tels acides organiques, on peut mentionner : les acides formique, acétique, acrylique, butyrique, adipique, isobutyrique, n-caproïque, isocaproïque, chloropropioniques, crotonique, phénylacétique, 2-thiénylacétique, 3-thiénylacétique, 4-éthylphénylacétique, glutarique, l'ester monoéthyli- que de l'acide adipique, les acides hexanoïque, heptanoïque, décanoïque, oléïque, stéarique, palmitique, 3-hydroxypropionique, 3-méthoxypropionique, 3-méthylthiobutyrique, 4-chlorobutyrique, 4-phénylbutyrique, 3- phénoxybutyrique, 4-éthylbenzoïque, 1-propylbenzoïque.

On utilise cependant de préférence comme sels de sodium l'acétate de sodium, le 2-éthyl hexanoate de sodium ou le diéthyl acétate de sodium.

La salification peut également être obtenue par action d'une base organique comme la triéthylamine, la diéthylamine, la triméthylamine, la propylamine, la N,N-diméthyl-éthanolamine, le tris(hydroxyméthyl) amino méthane, la méthylamine, l'éthanolamine, la pyridine, la picoline, la dicyclohexylamine, la morpholine et la benzylamine.

Elle peut également être obtenue par action de l'arginine, de la lysine, de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opère en général en faisant réagir l'acide de formule (I) avec un dérivé de formule: Z-^{R}s dans laquelle Z représente un radical hydroxyle ou un atome d'halogène tel que le chlore, le brome, l'iode et Rₛ désigne le groupement ester à introduire, groupement dont une liste non exhaustive figure ci-dessus. Dans certains cas, il peut être avantageux d'effectuer une estérification sur un produit dont l'amine est bloquée avant d'enlever le groupement protecteur de l'amine.

L'éventuel dédoublement des composés de formules générales XI_{A}, XI_{B}, XIα peut être effectué au moyen d'un acide organique carboxylique ou sulfonique optiquement actif comme l'acide tartrique, dibenzoyltartrique, camphosulfonique ou glutamique, la décomposition du sel ainsi obtenue étant effectuée au moyen d'une base minérale telle que le bicarbonate de sodium ou d'une base organique telle qu'une amine tertiaire comme la triéthylamine. De plus, on peut éventuellement utiliser une base optiquement active.

L'oxydation éventuelle des produits de formule XI_{A}, XI_{B} ou XIα peut être effectuée à l'aide d'oxygène, de péroxydes, d'hydropéroxydes, de péracides ou d'eau oxygénée. La réaction est avantageusement sensibilisée à la lumière. Ces réactifs peuvent être en mélange avec des acides organiques ou minéraux. On utilise de préférence l'acide métachloroperbenzoOlque. Les conditions réactionnelles sont connues de l'homme de métier. On trouve de telles conditions exposées par exemple dans le brevet français 2.387.234.

Les produits de formule générale (I) ainsi que leurs sels pharmaceutiquement acceptables possèdent une très bonne activité antibiotique sur les bactéries gram (+) telles que les staphylocoques, les streptocoques et, notamment, sur les staphylocoques pénicillino-résistants. Leur efficacité sur les bactéries gram (-), notamment, sur les bactéries coliformes, les klebsiella, les salmonella et les proteus est particulièrement ramarquable.

Ces propriétés rendent aptes lesdits produits à être utilisés comme médicaments dans le traitement des affections à germes sensibles et, notamment, dans celui des staphylococcies, telles que septicémies à staphycocoques, staphylococcies malignes de la face ou cutanée, pyodermites, plaies septiques ou suppurantes, anthrax, phlegmons, érésipèles, staphylococcies aigües primitives ou post grippales, bron- chopneumonies, suppuration pulmonaire.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

La présente invention a donc également pour objet, à titre de médicaments et notamment de médicaments antibiotiques, les produits de formule (1), tels que définis ci-dessus, ainsi que leurs sels d'acides, pharmaceutiquement acceptables.

L'invention a particulièrement pour objet, à titre de médicaments et notamment de médicaments antibiotiques, les produits de formule (I_{A}): 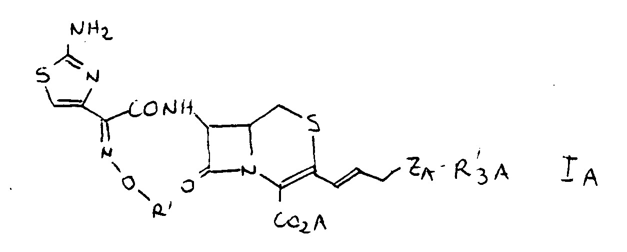 isomère syn dans laquelle R' représente un atome d'hydrogène ou un radical alkyle, alkényle, alkynyle ou cycloalkyle ayant au plus 6 atomes de carbone, éventuellement substitué par un radical carboxy libre, estérifié ou salifié ou un radical difluorométhyle ou méthoxyméthyle, Zₐ représente une simple liaison et R'_{3A} représente un radical pyridinium, thiénopyridinium, thiazolopyridinium, cyclopentylpyridinium, cyclohexénopyridinium, triméthylammonium, ces radicaux pouvant être substitués par un radical choisi parmi les radicaux alkyle et amino.

L'invention a tout particulièrement pour objet, à titre de médicament et notamment à titre de médicaments antibiotiques, les produits décrits ci-après dans les exemples.

L'invention s'étend aux compositions pharmaceutiques renfermant comme principe actif au moins un des médicaments définis ci-dessus.

Ces compositions peuvent être administrées par voie buccale, rectale, parentérale, intramusculaire ou par voie locale en application topique sur la peau et les muqueuses.

En particulier, les produits de formule (I) dans laquelle A représente un groupement ester clivable tel que l'ester de propionyloxyméthyle peuvent être administrés par voie orale.

Elles peuvent être solides ou liquides et se présenter sous les formes pharmaceutiques couramment utilisées en médecine humaine comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent, notamment, se présenter sous forme d'une poudre, destinées à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 0,500 g et 1 g trois fois par jour, par voie intramusculaire pour les produits des exemples 1 ou 2.

Les produits de formule (I) peuvent également être utilisés comme désinfectants des instruments chirurgicaux.

Les produits de formule VII utilisés au départ du procédé de préparation des produits de formule 1 peuvent être préparés selon le procédé décrit dans le brevet français 2 559 486.

Un exemple d'une telle préparation est donné ci-après dans les exemples de référence.

En plus des produits décrits ci-après dans les exemples qui illustrent l'invention sans toutefois la limiter, les produits suivants constituent les produits pouvant être obtenus dans le cadre de la présente invention.

Les produits répondant à la formule : 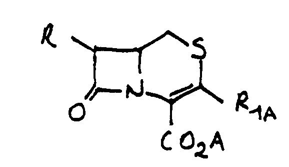 dans laquelle R, R_{lA} et A ont la signification indiquée dans les tableaux ci-dessous.

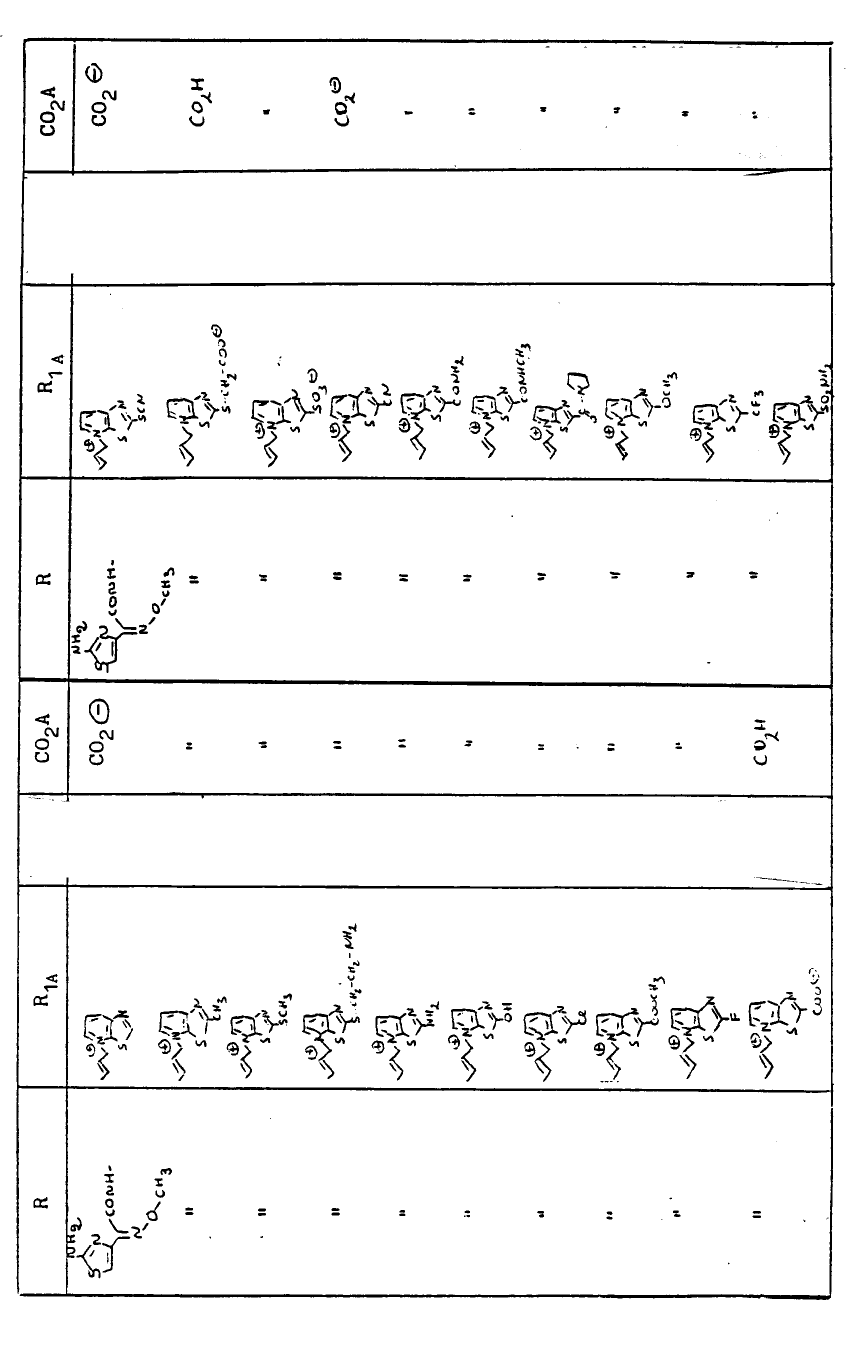

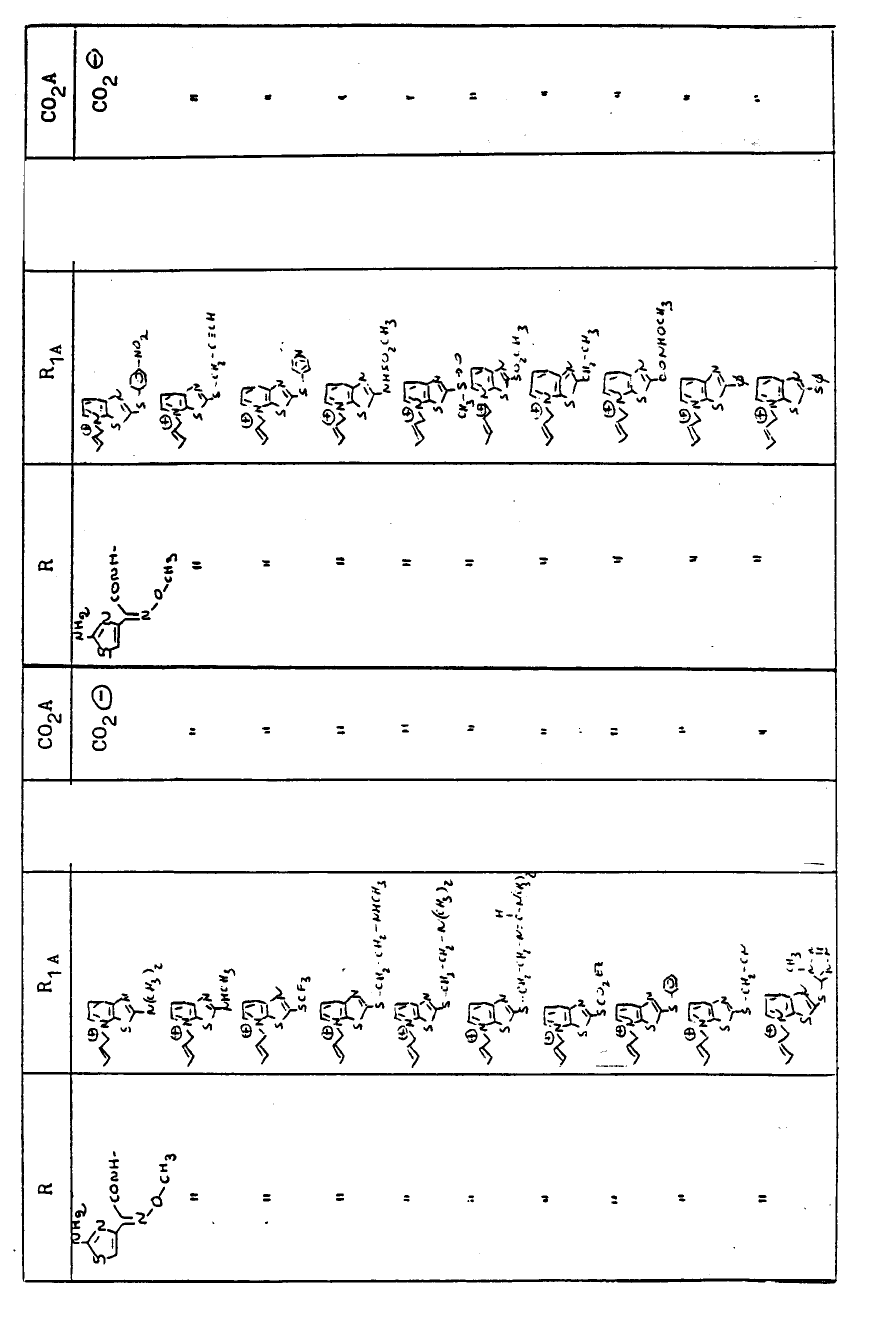

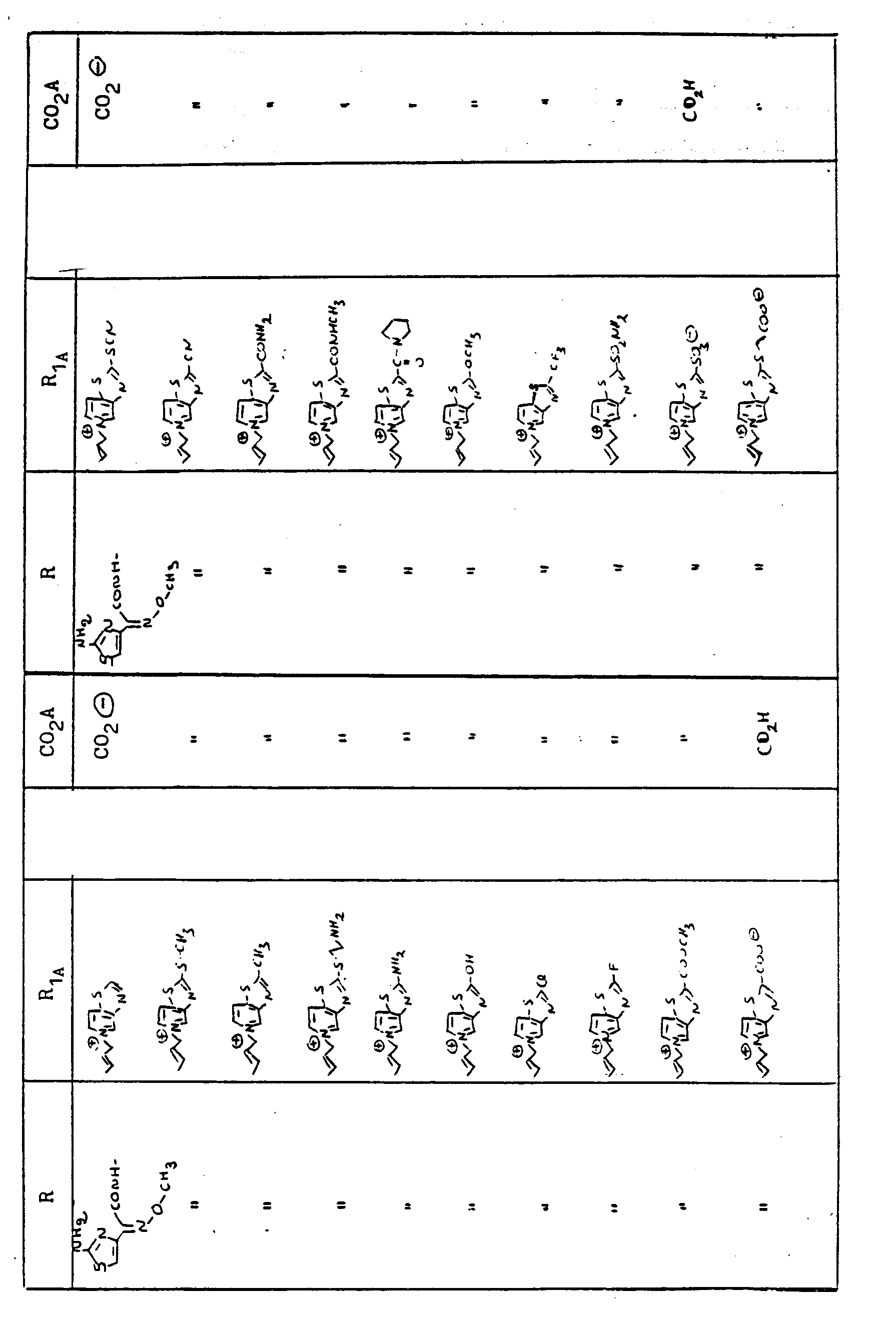

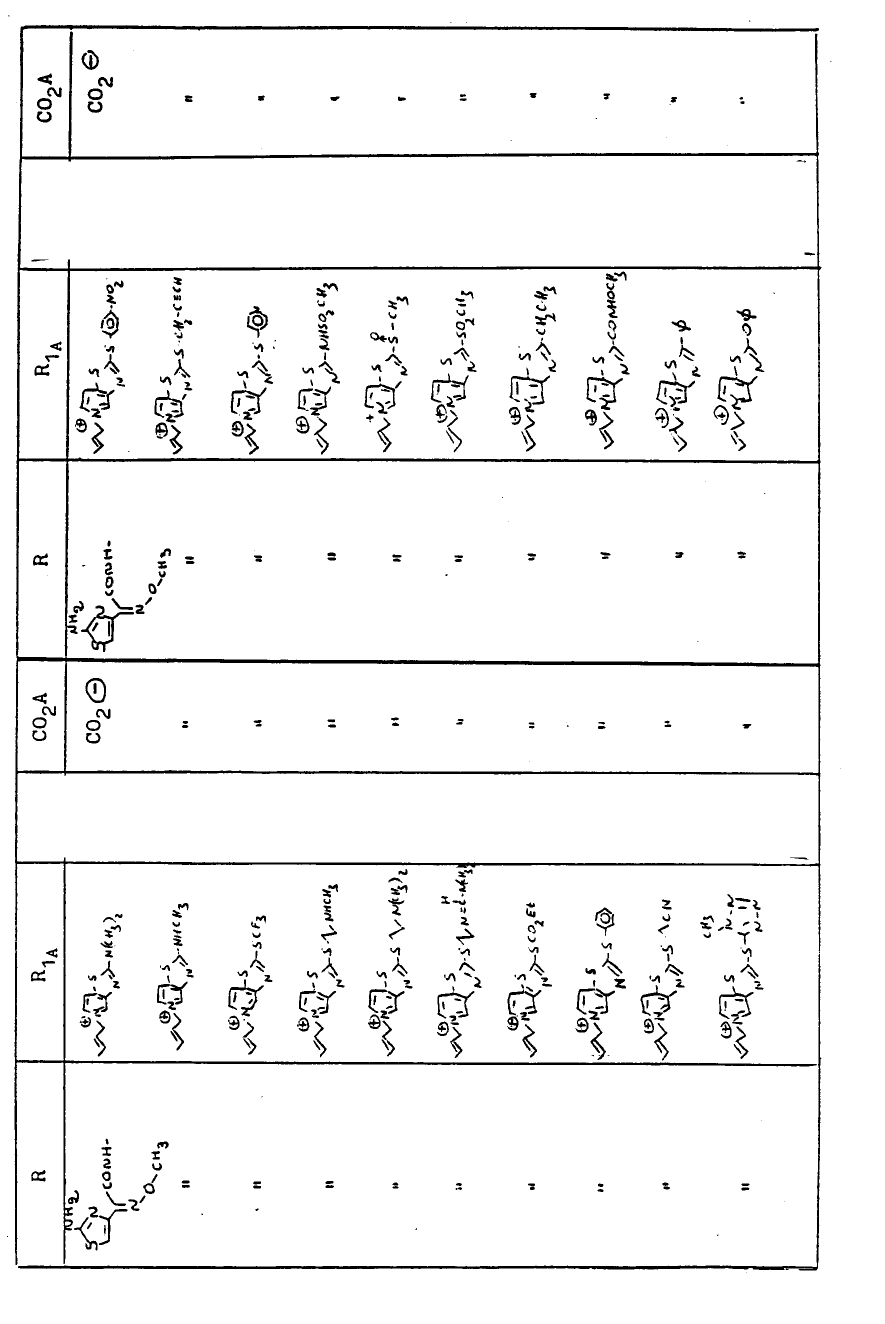

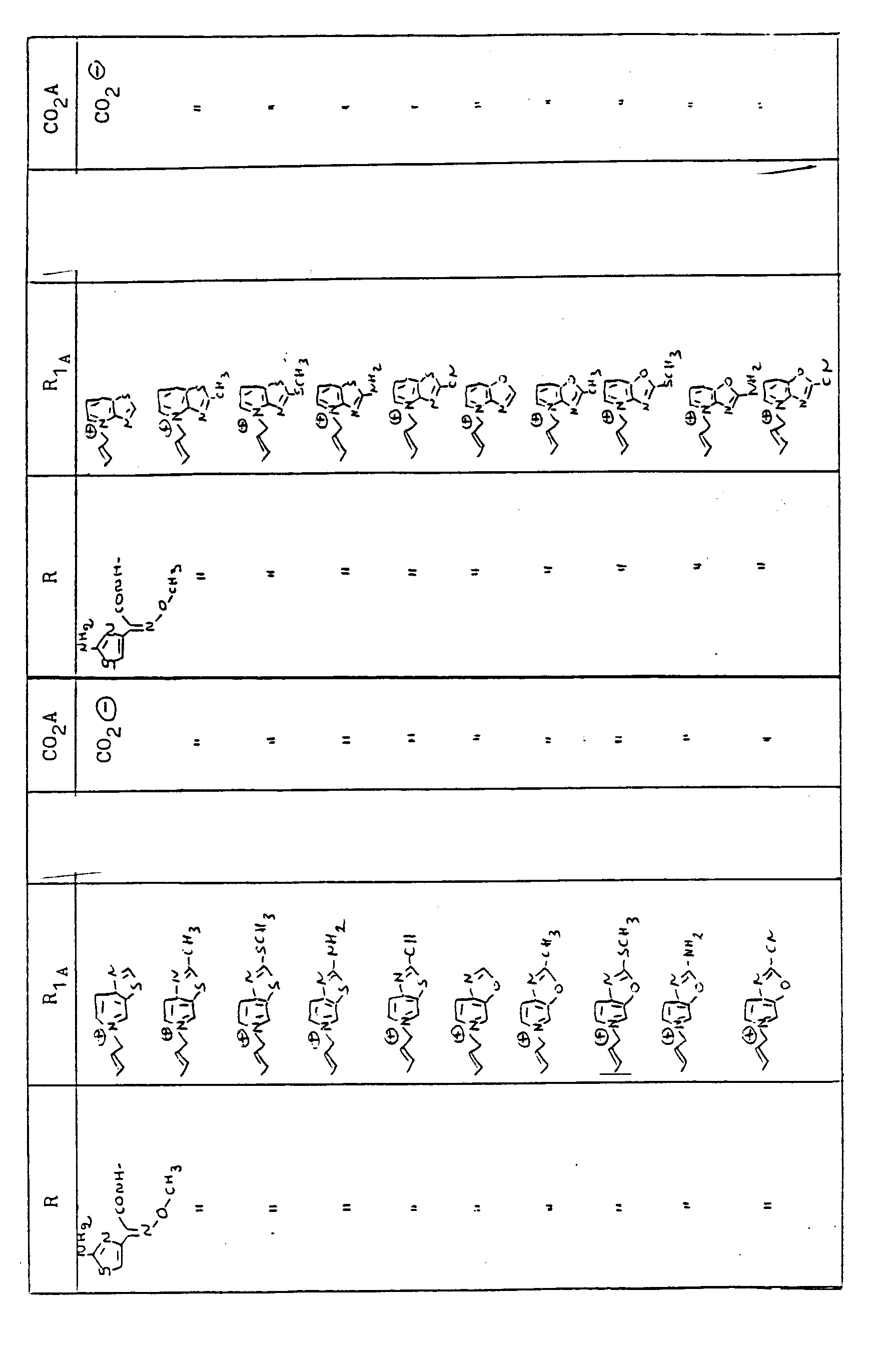

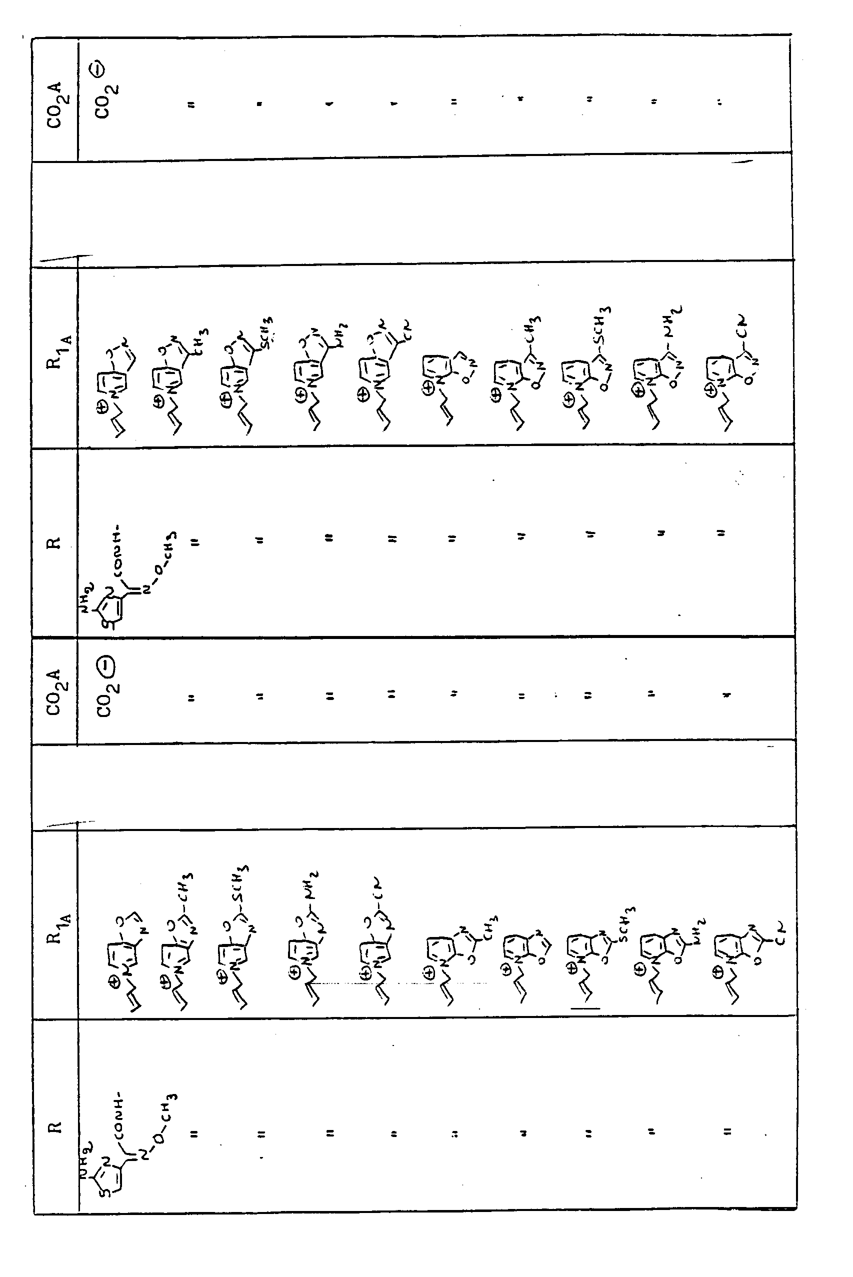

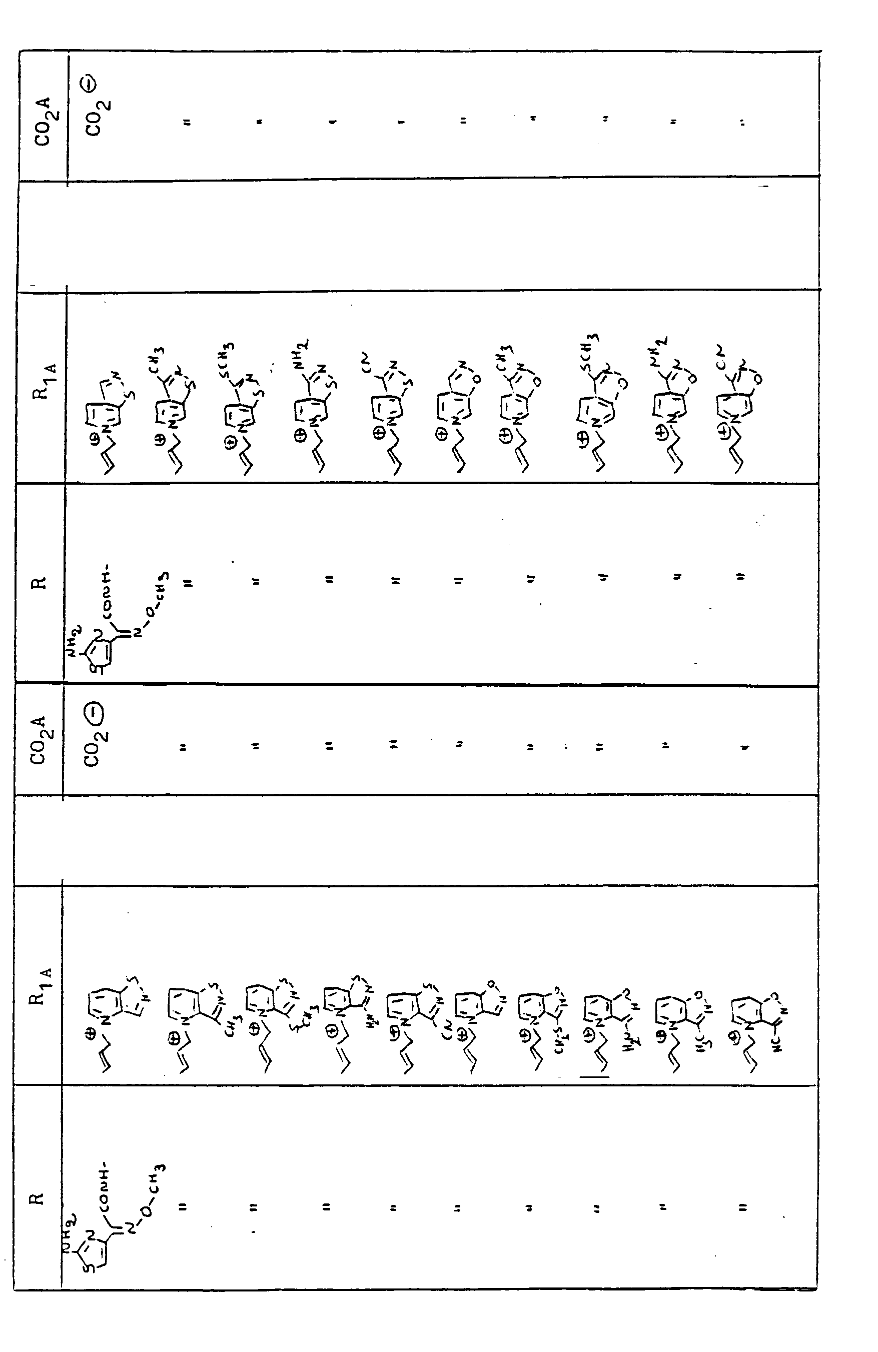

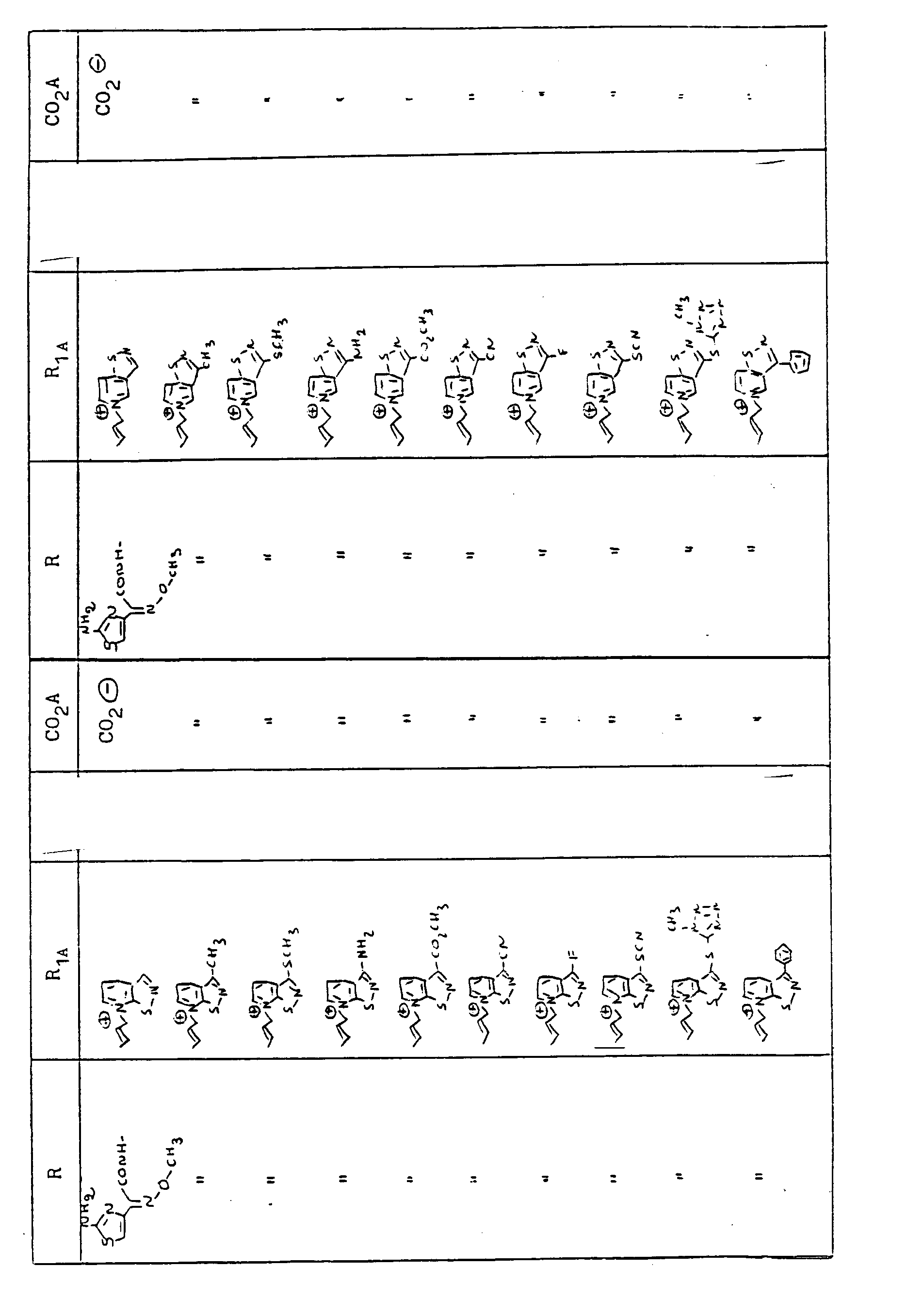

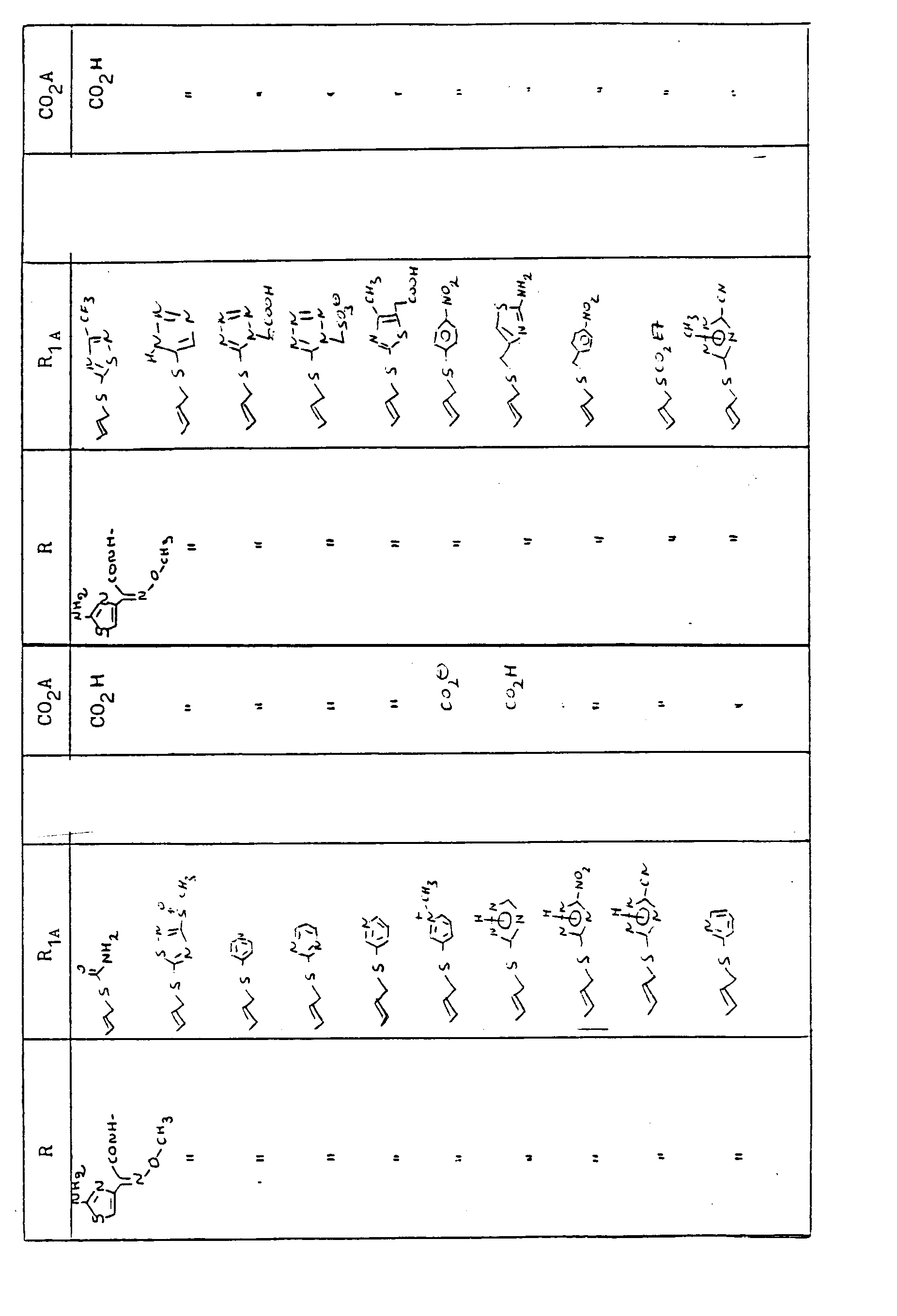

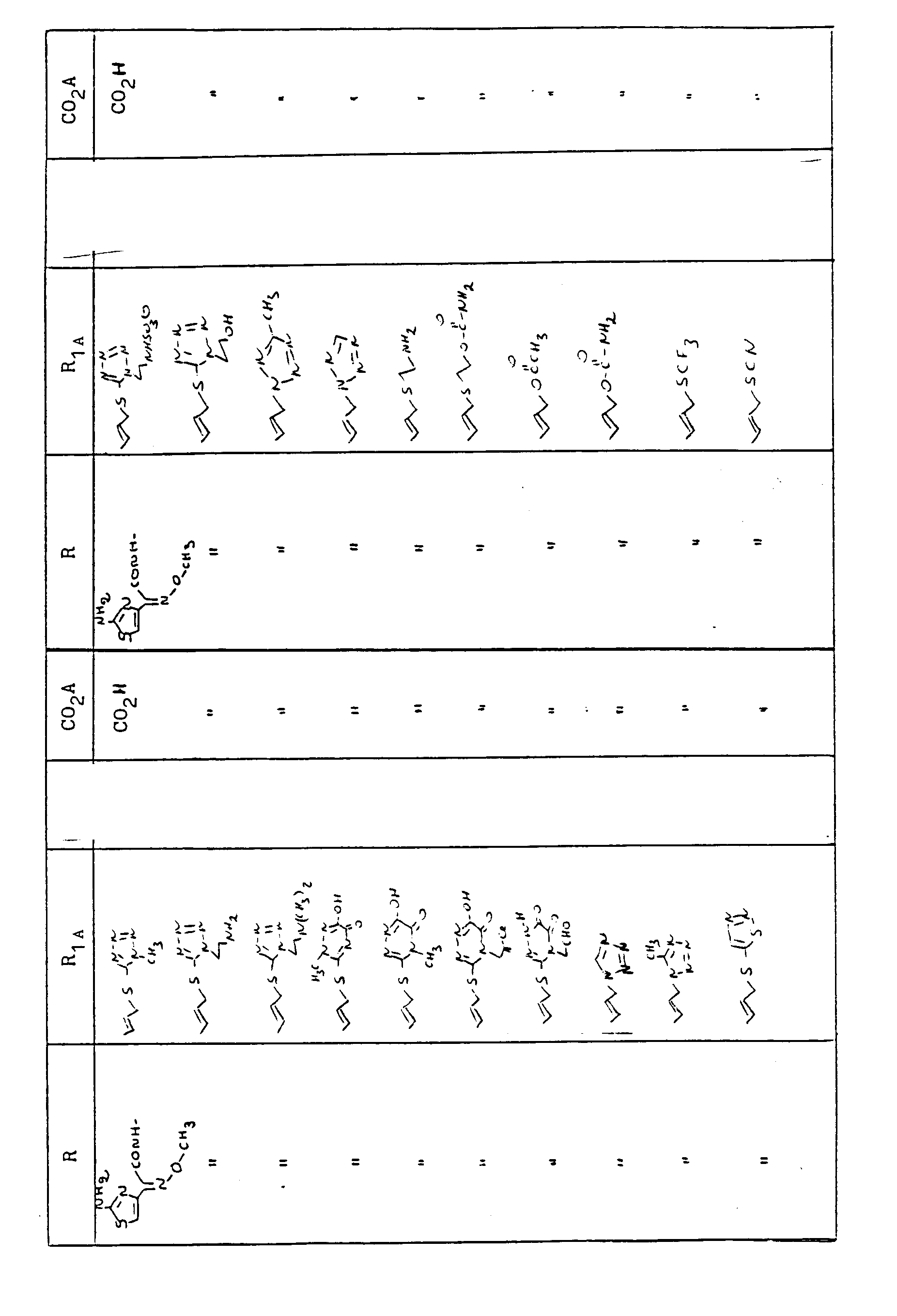

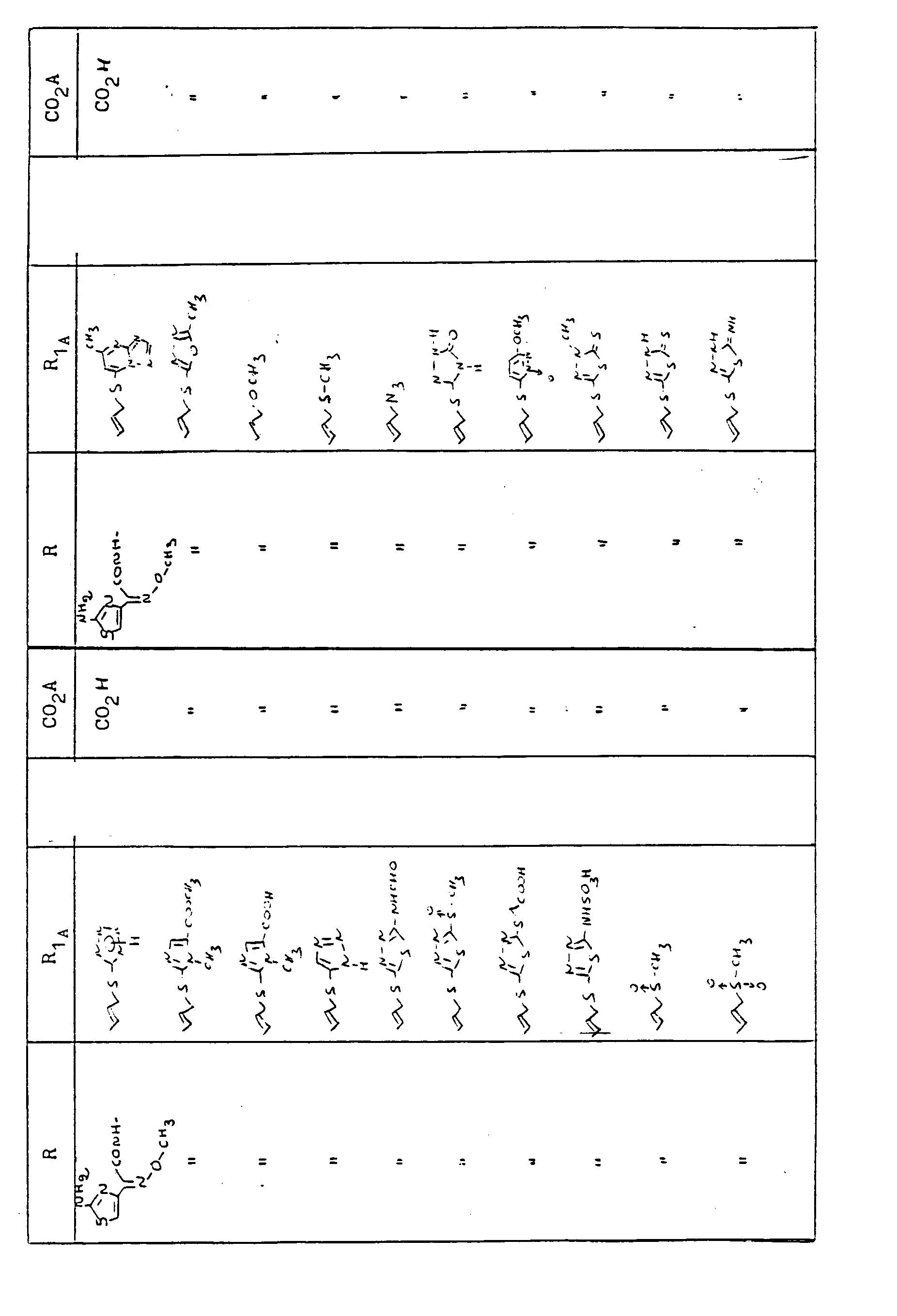

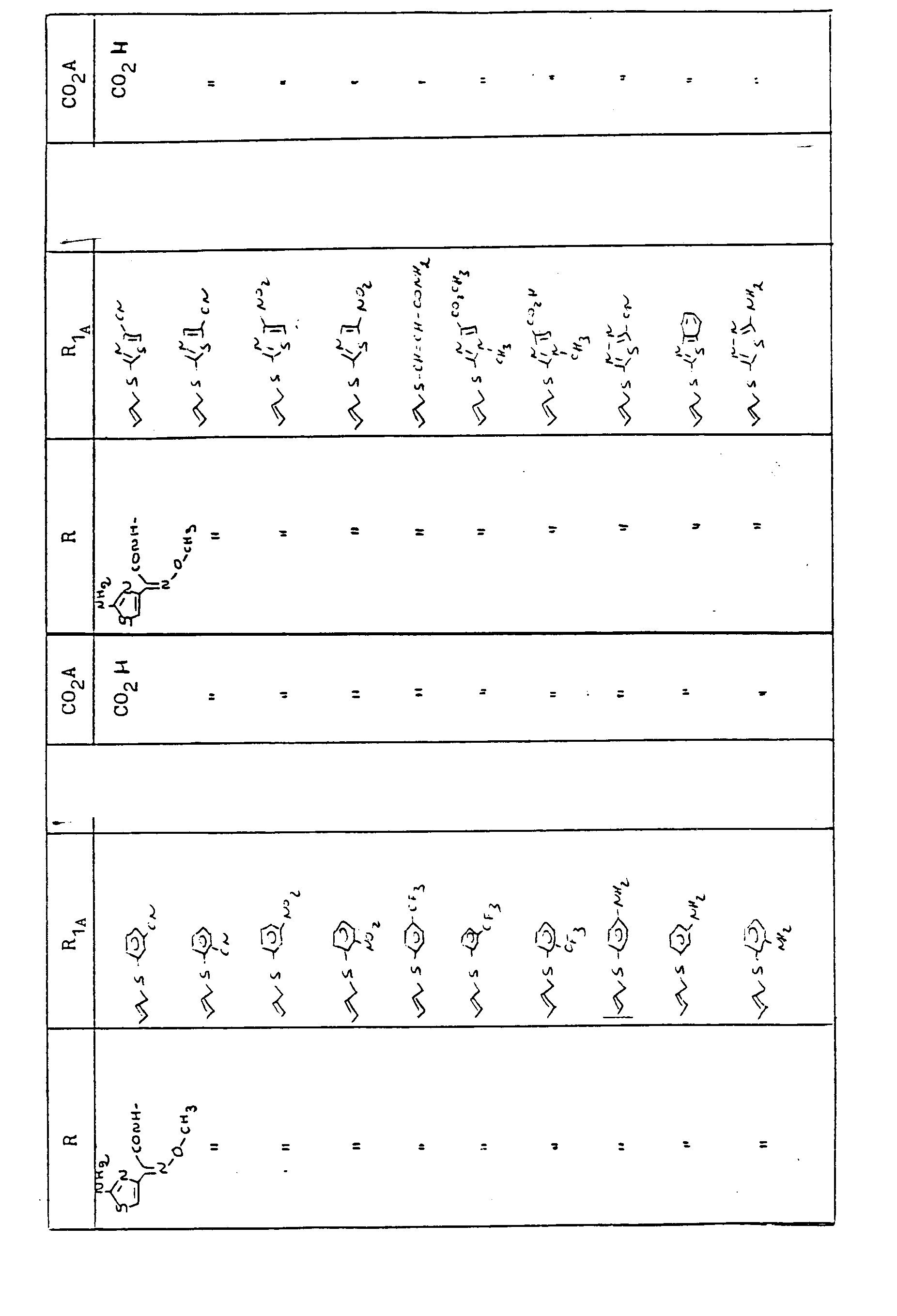

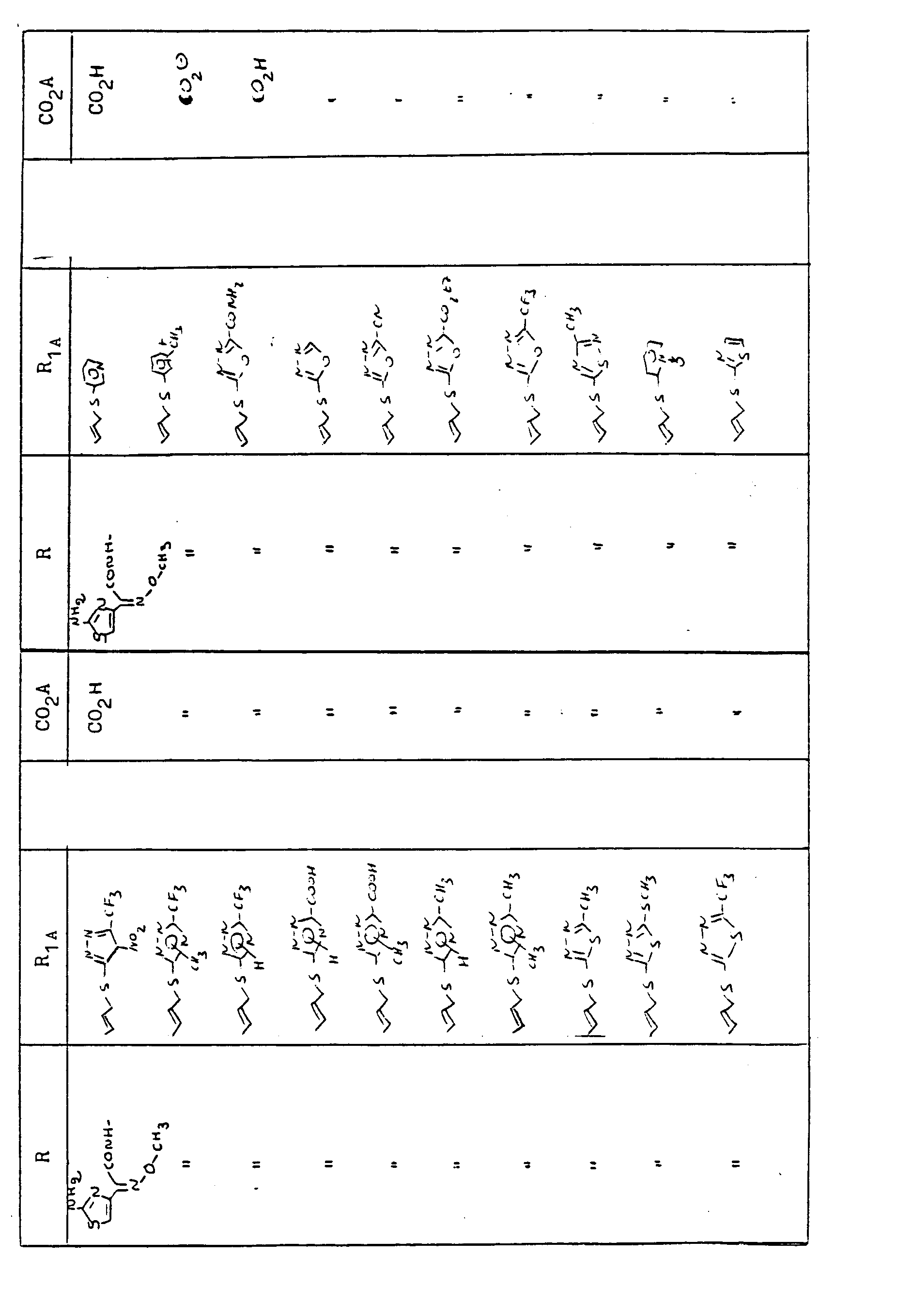

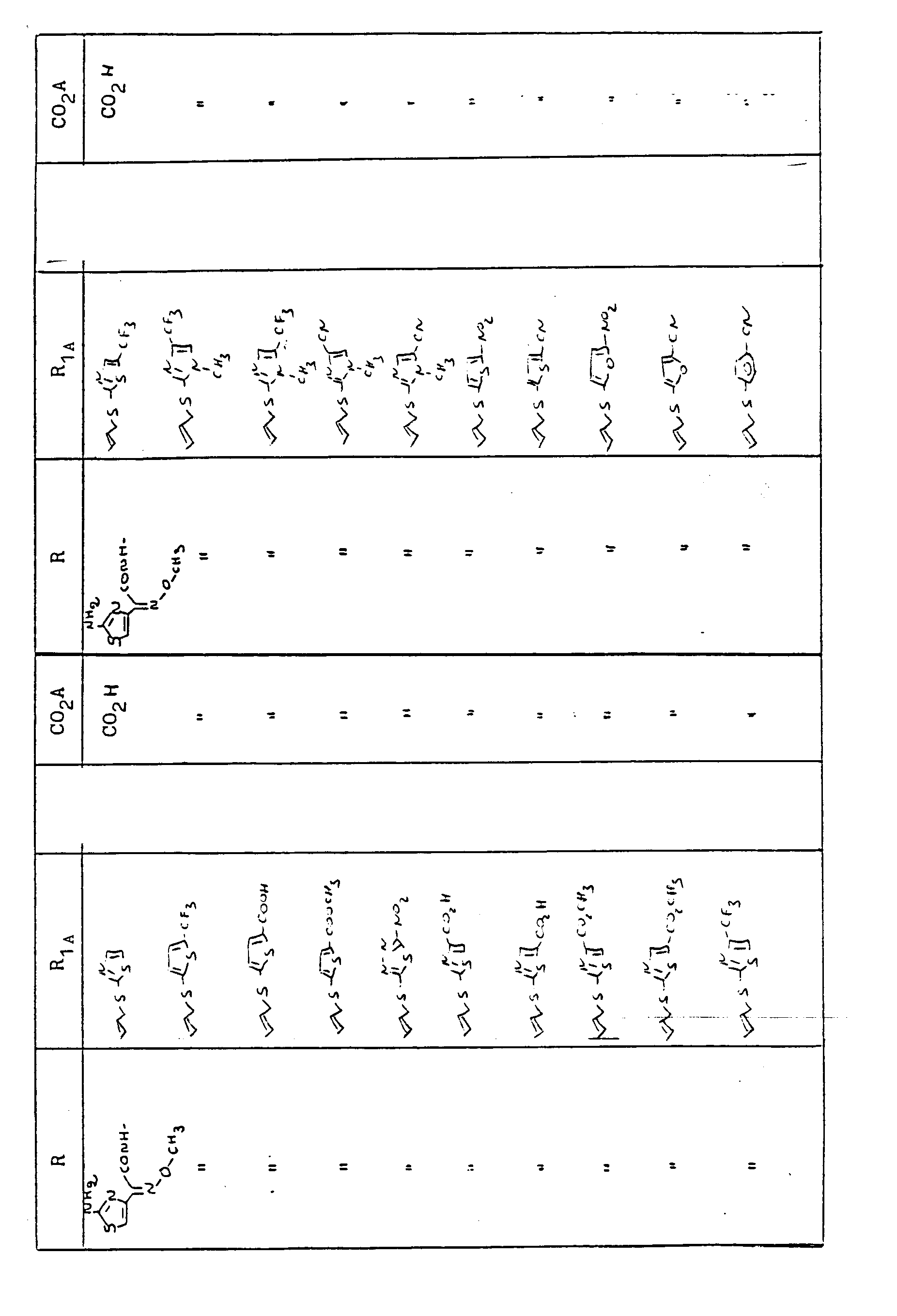

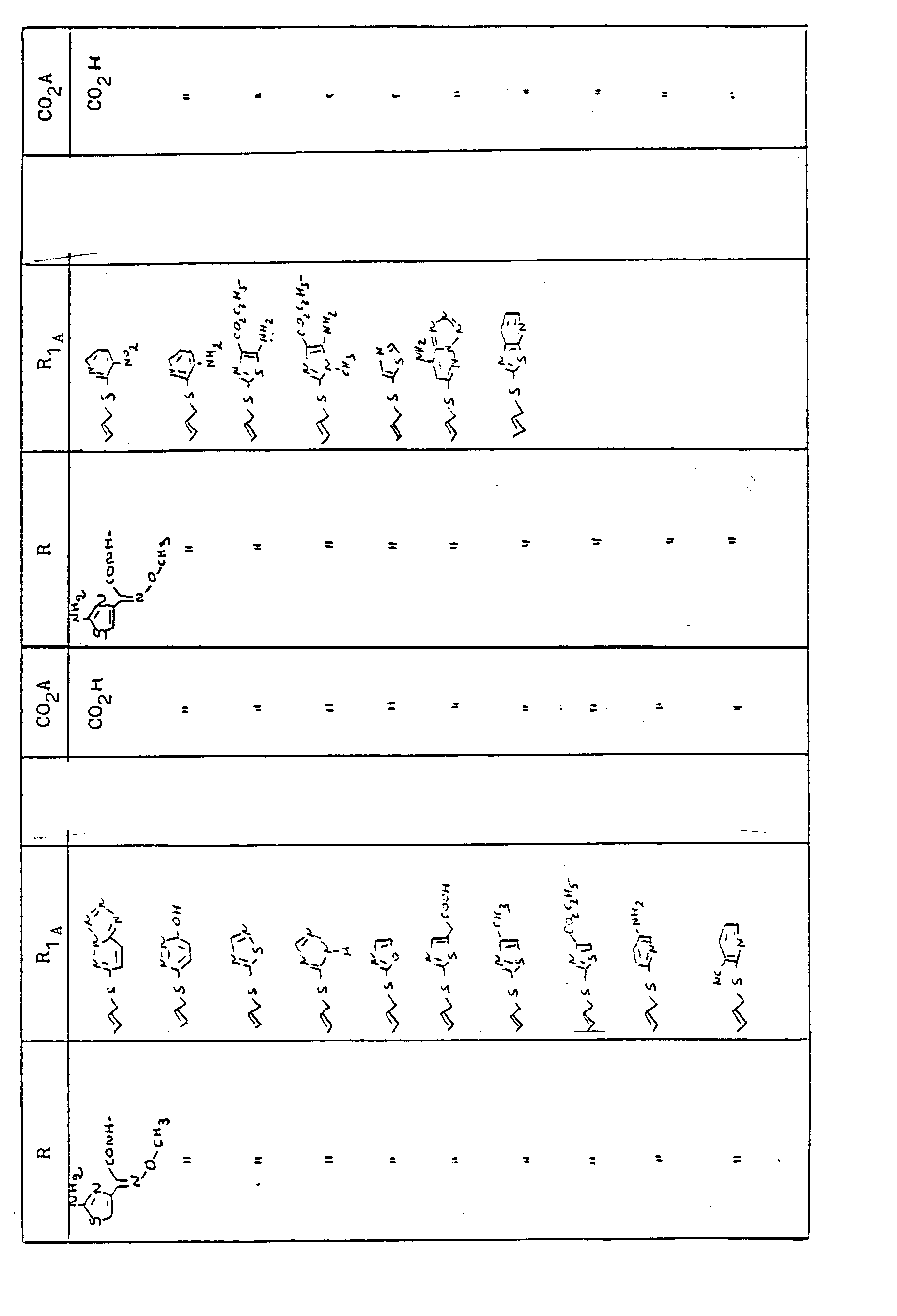

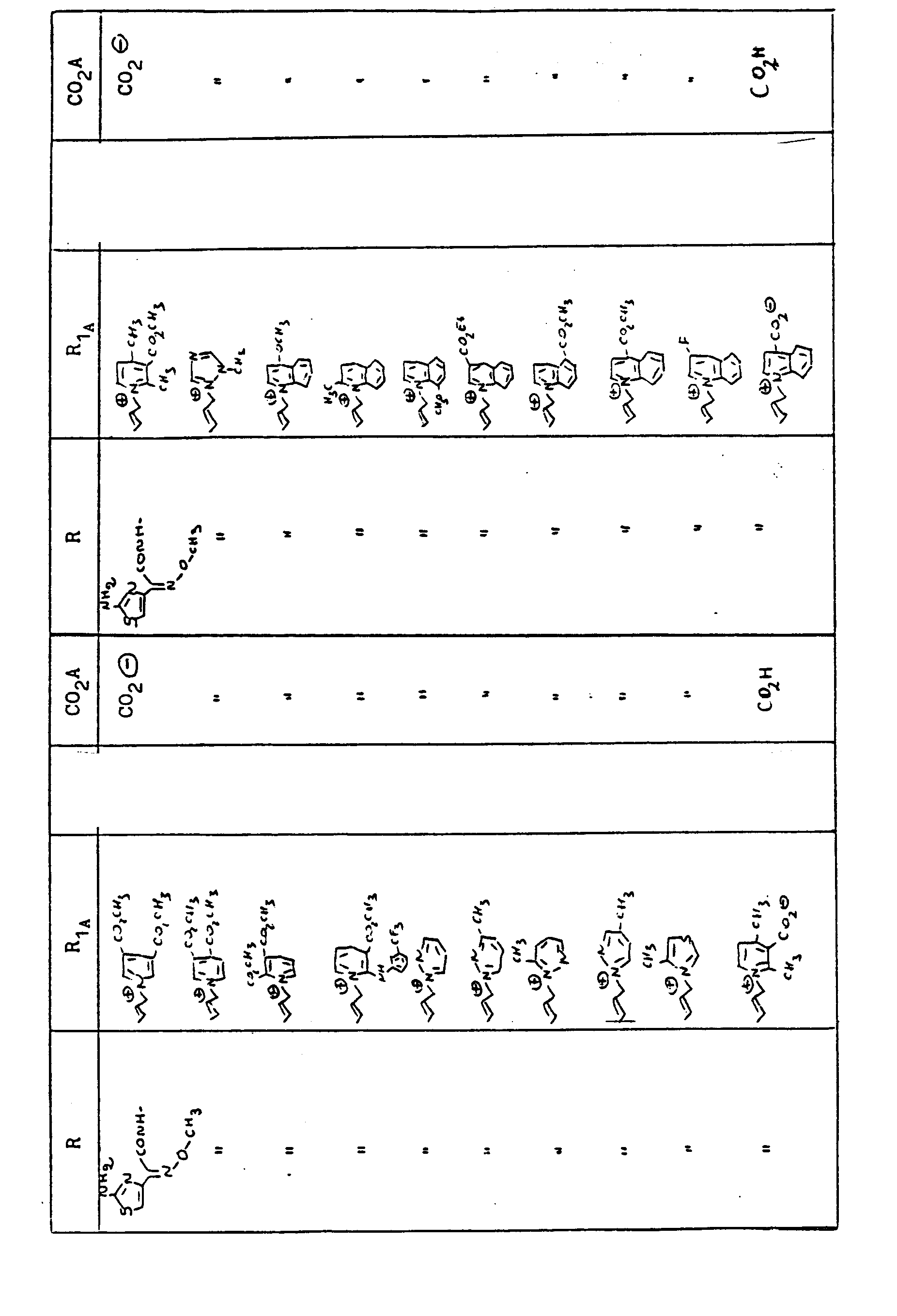

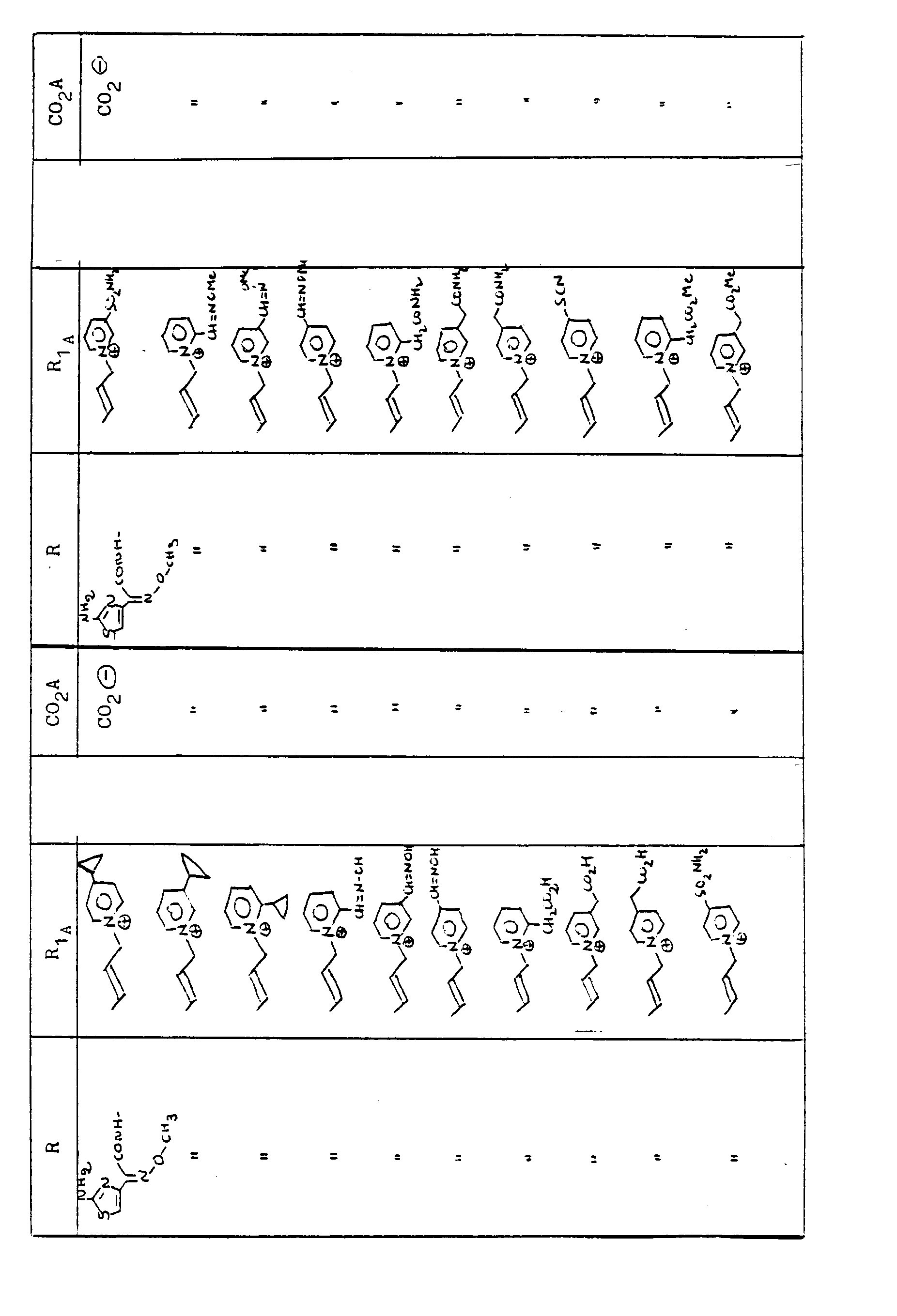

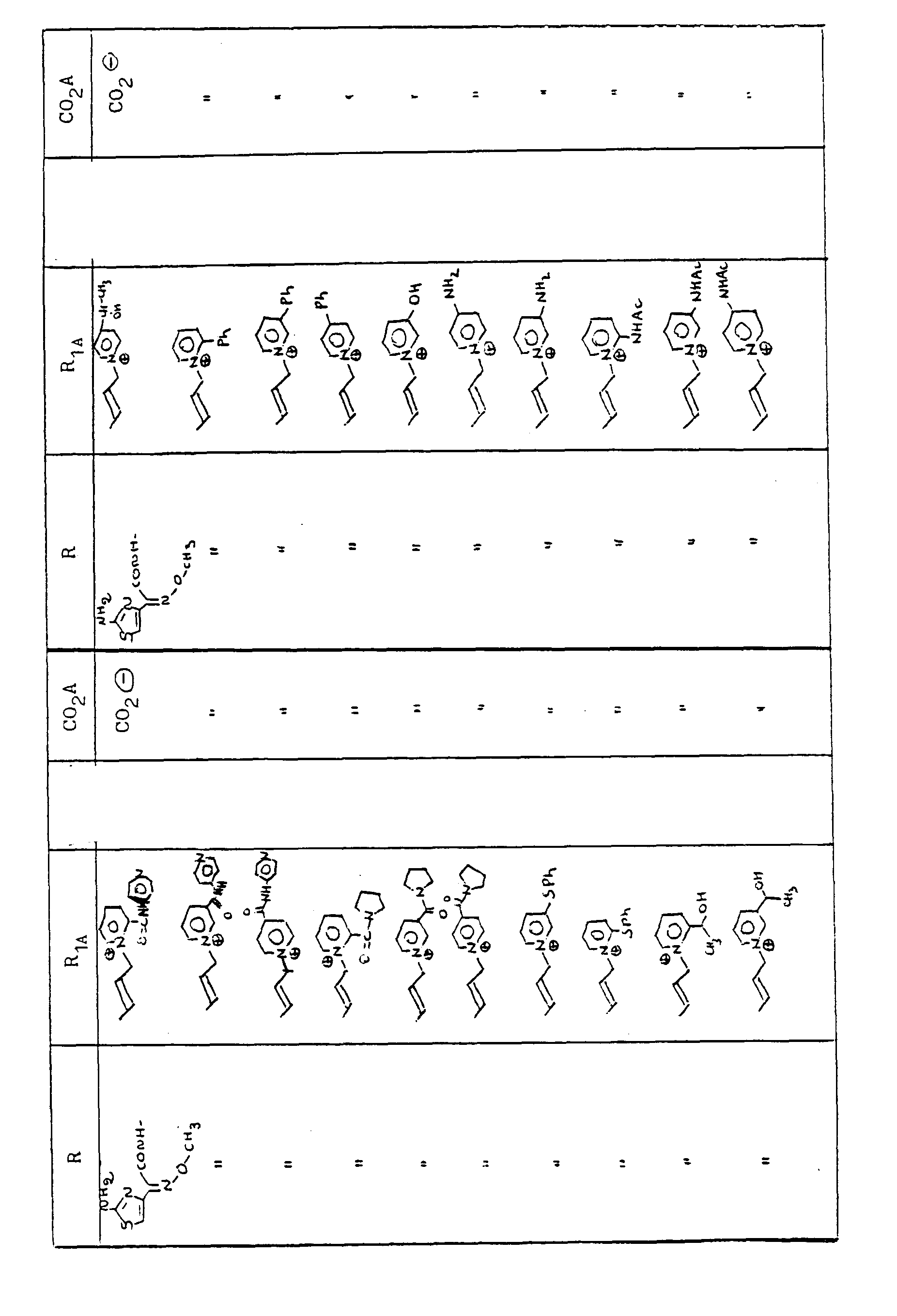

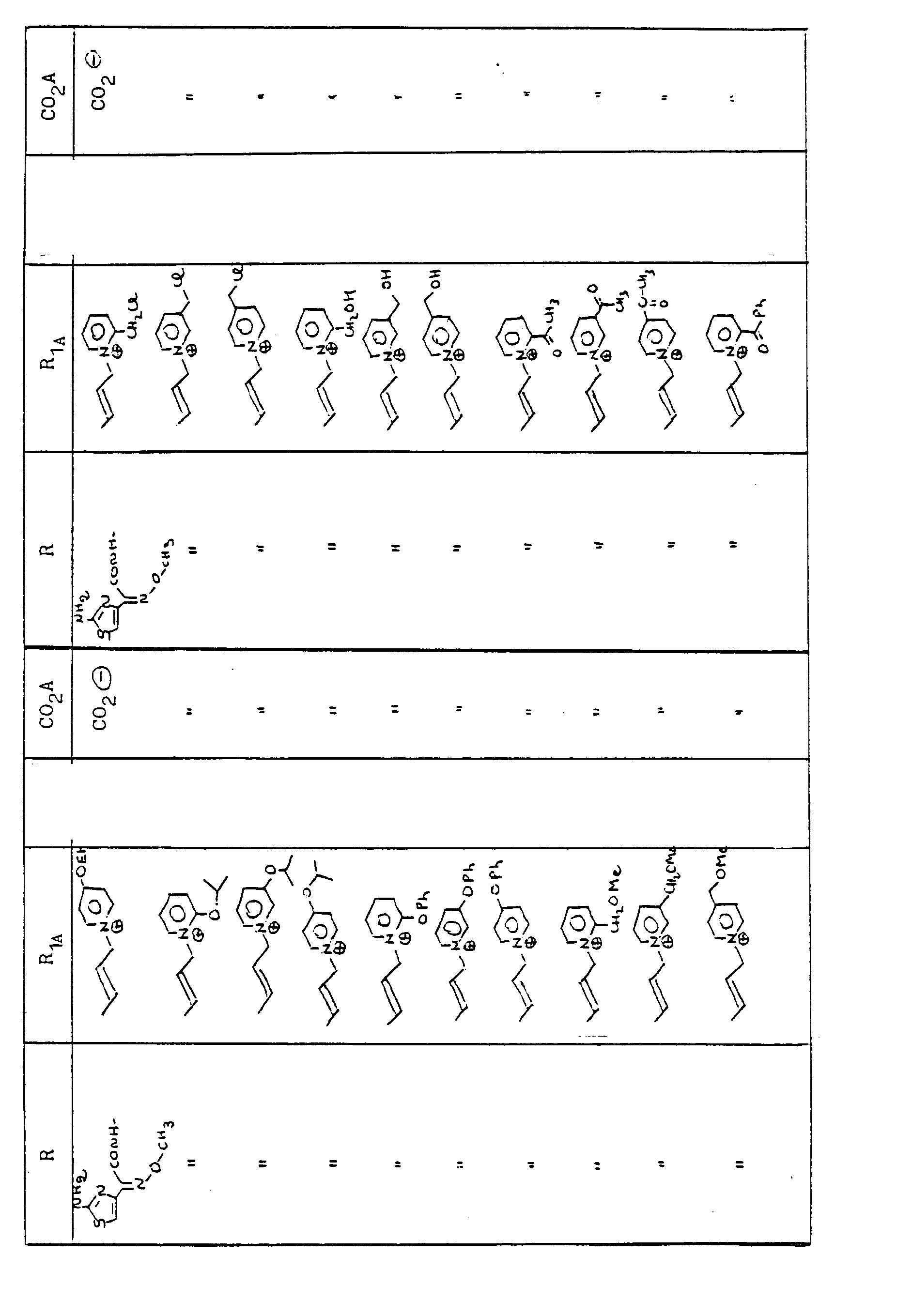

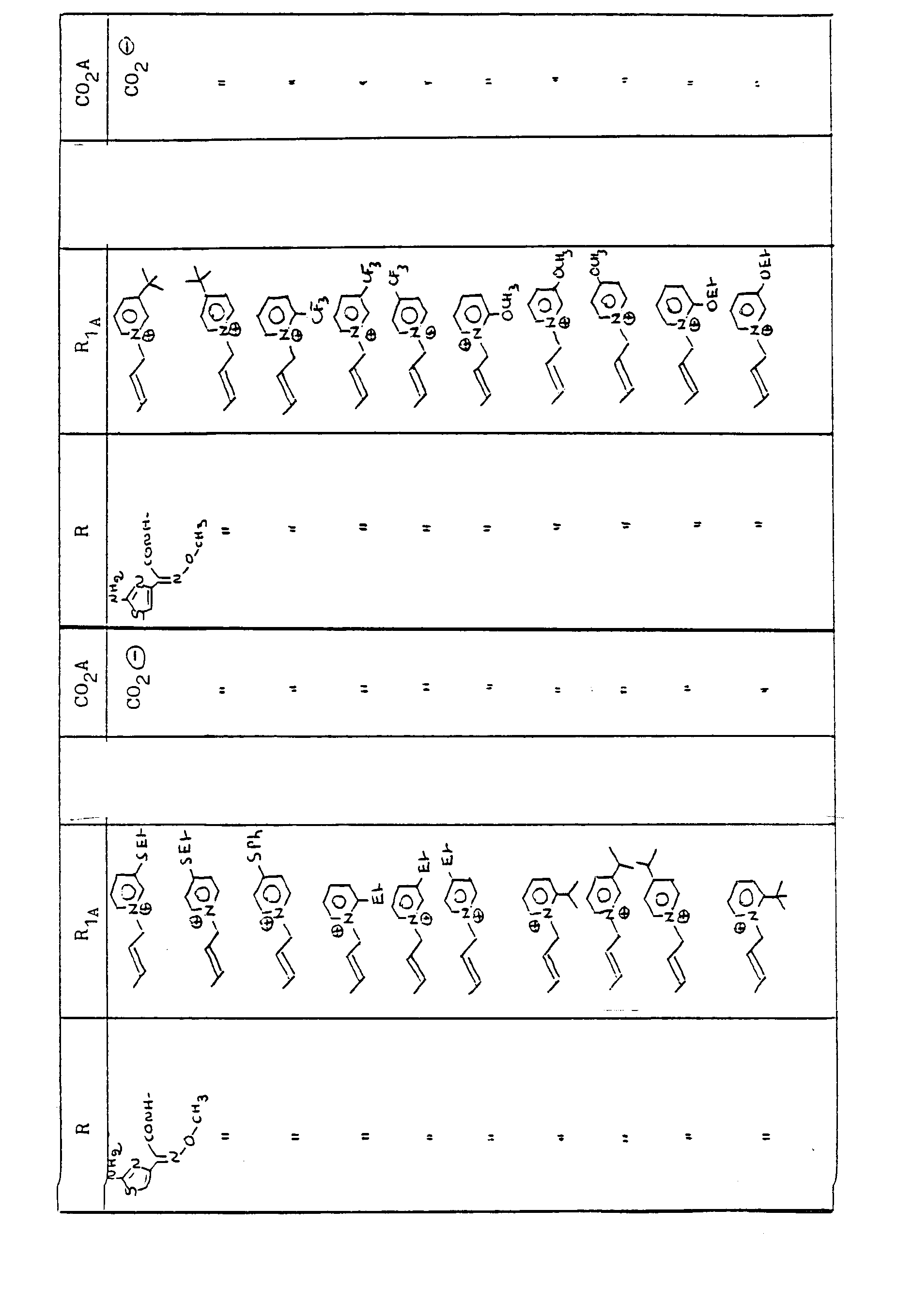

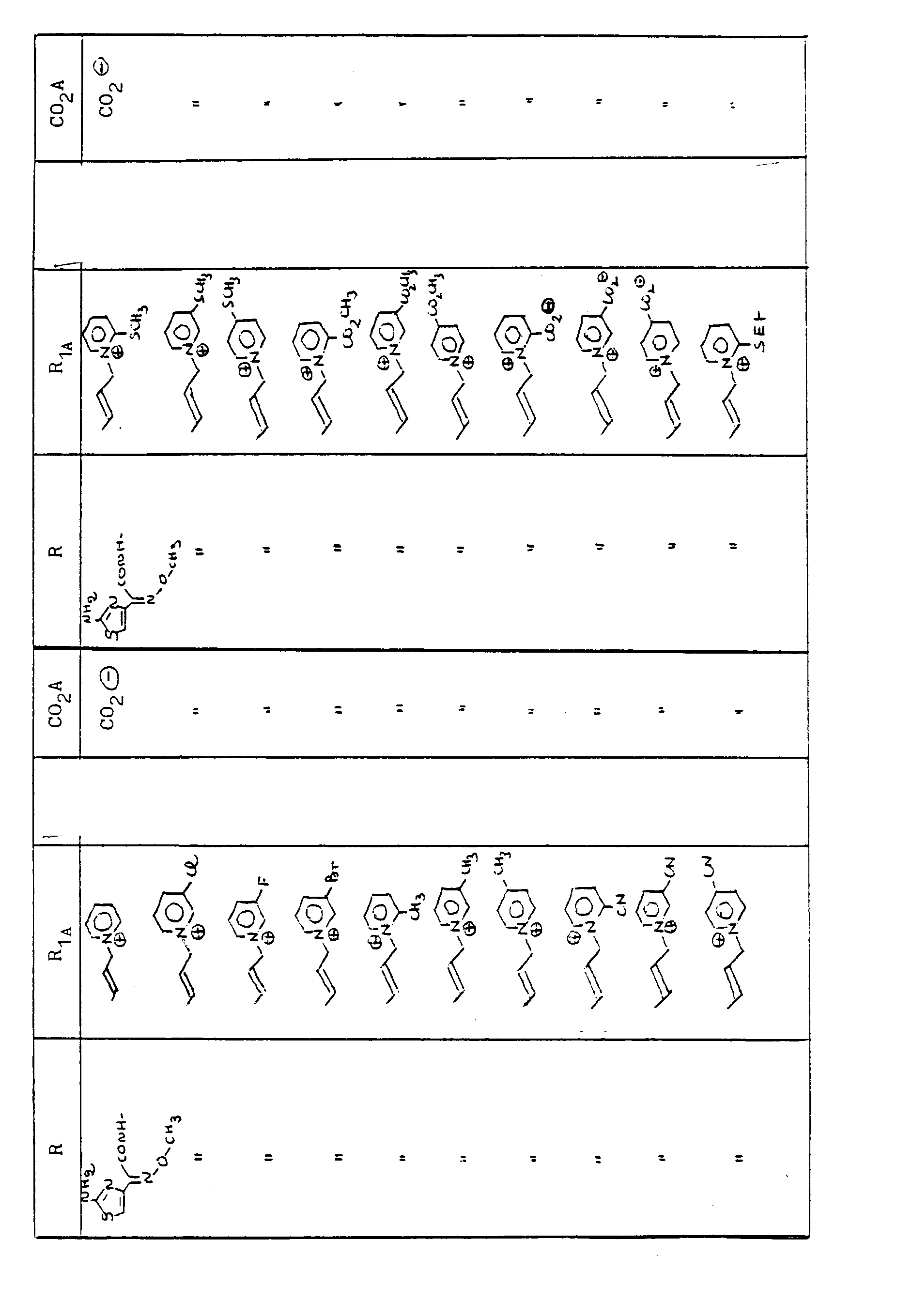

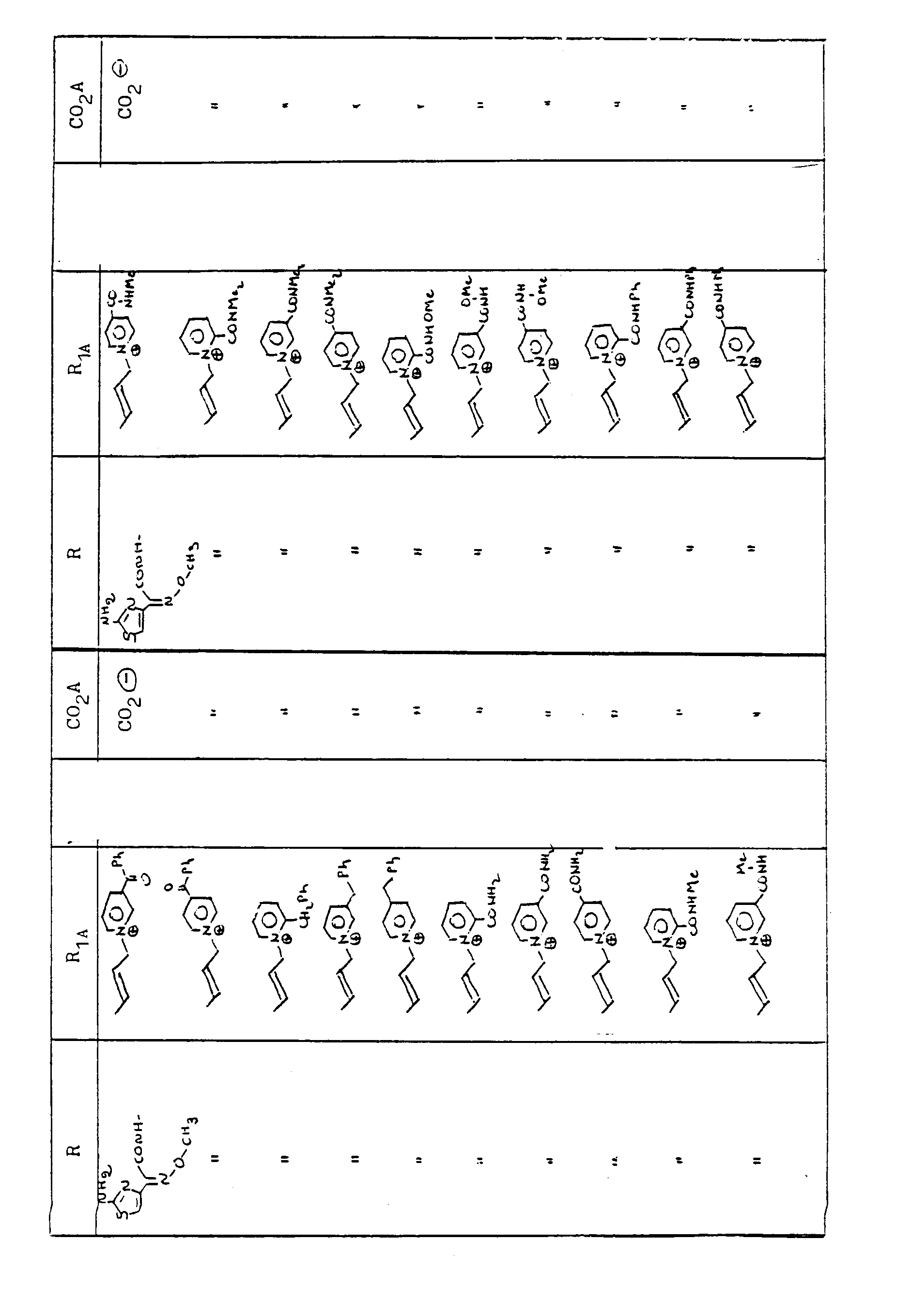

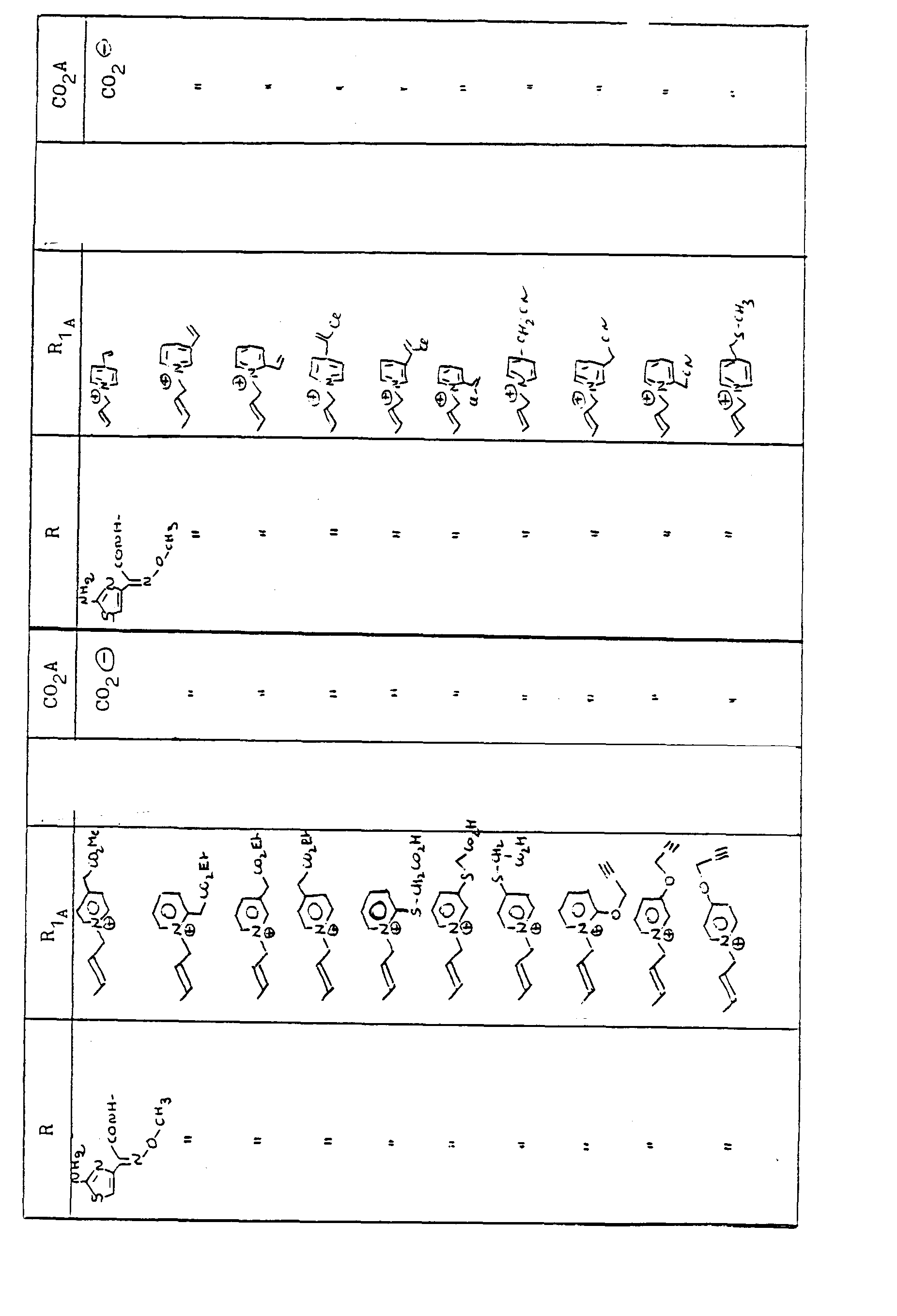

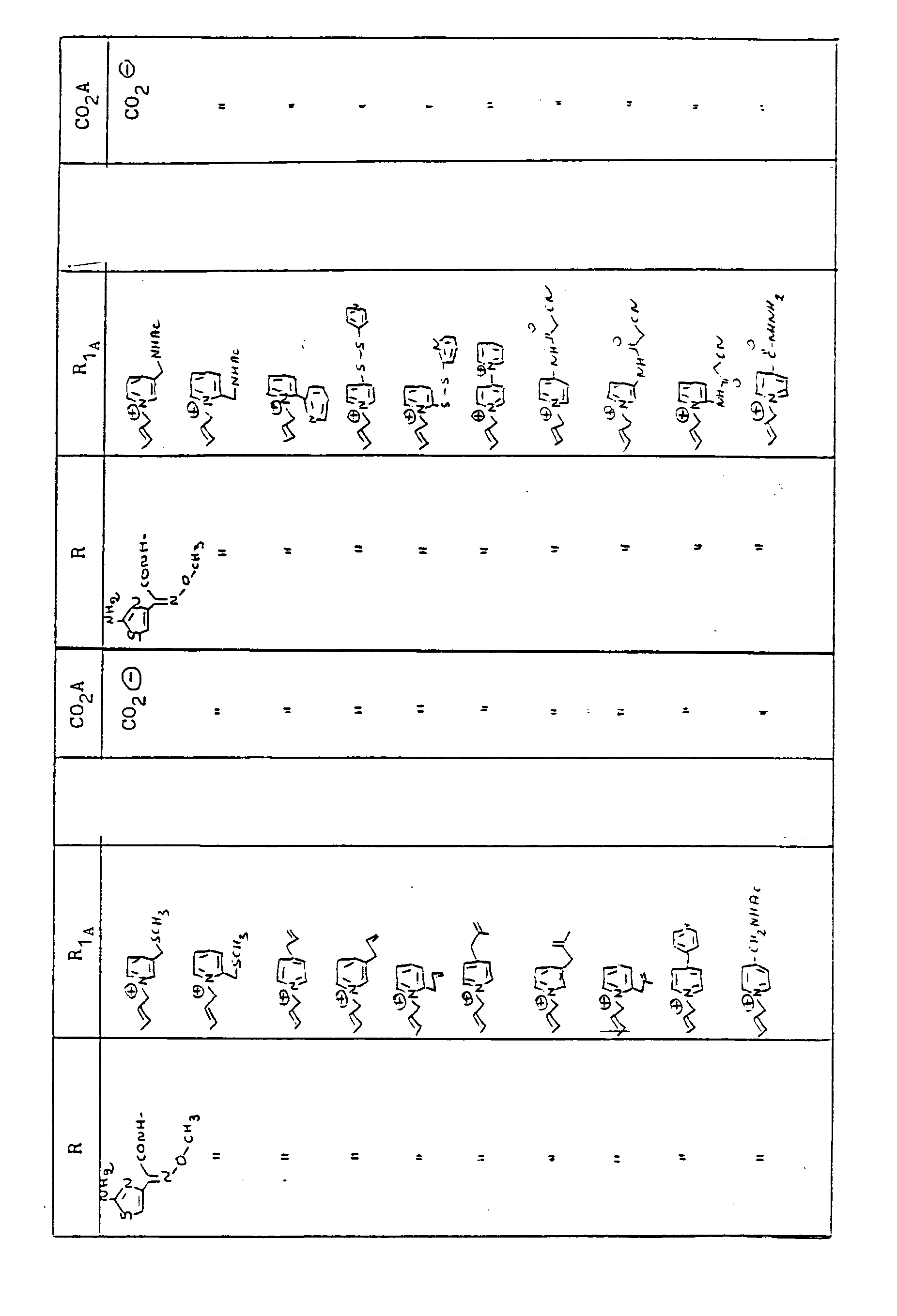

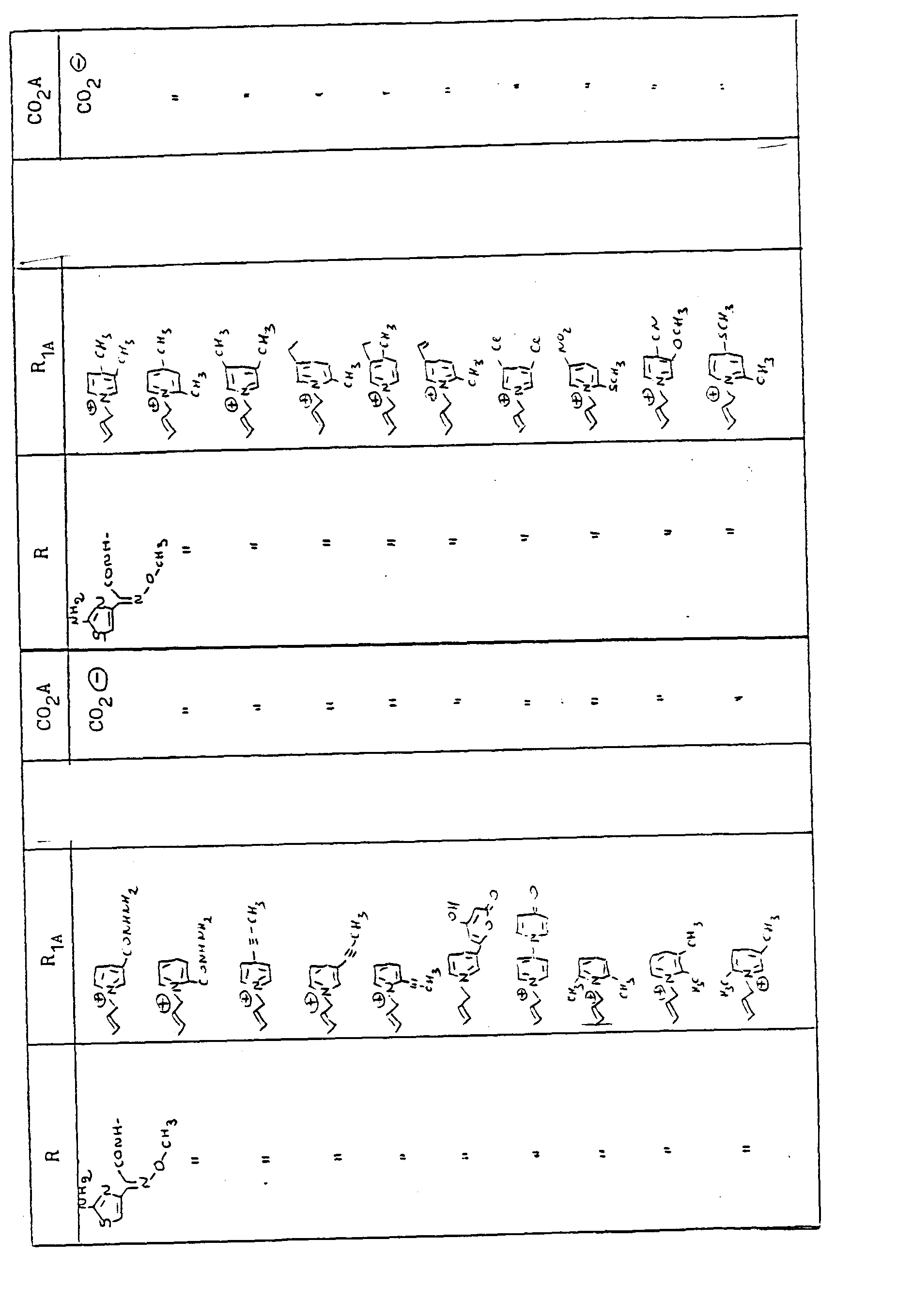

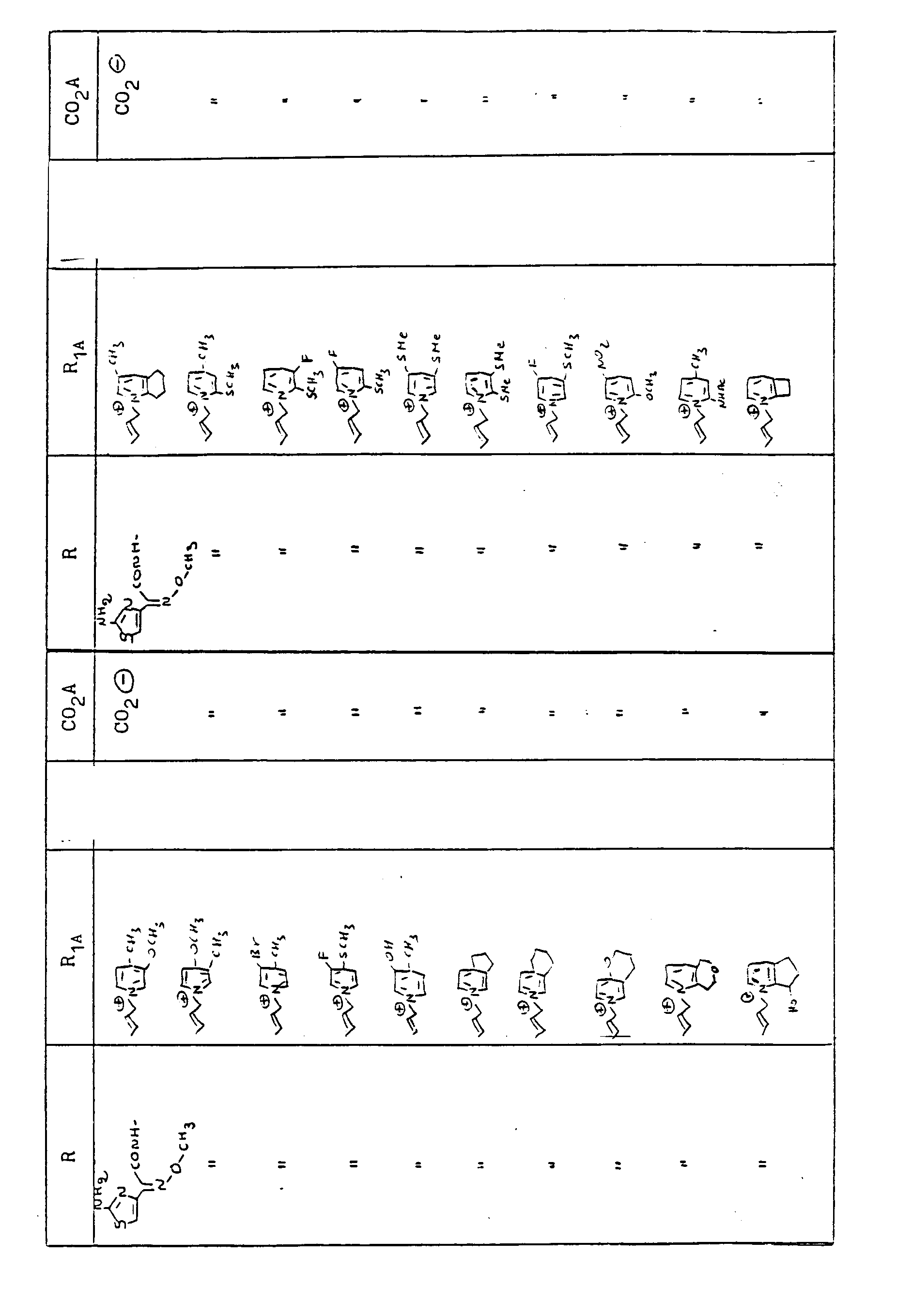

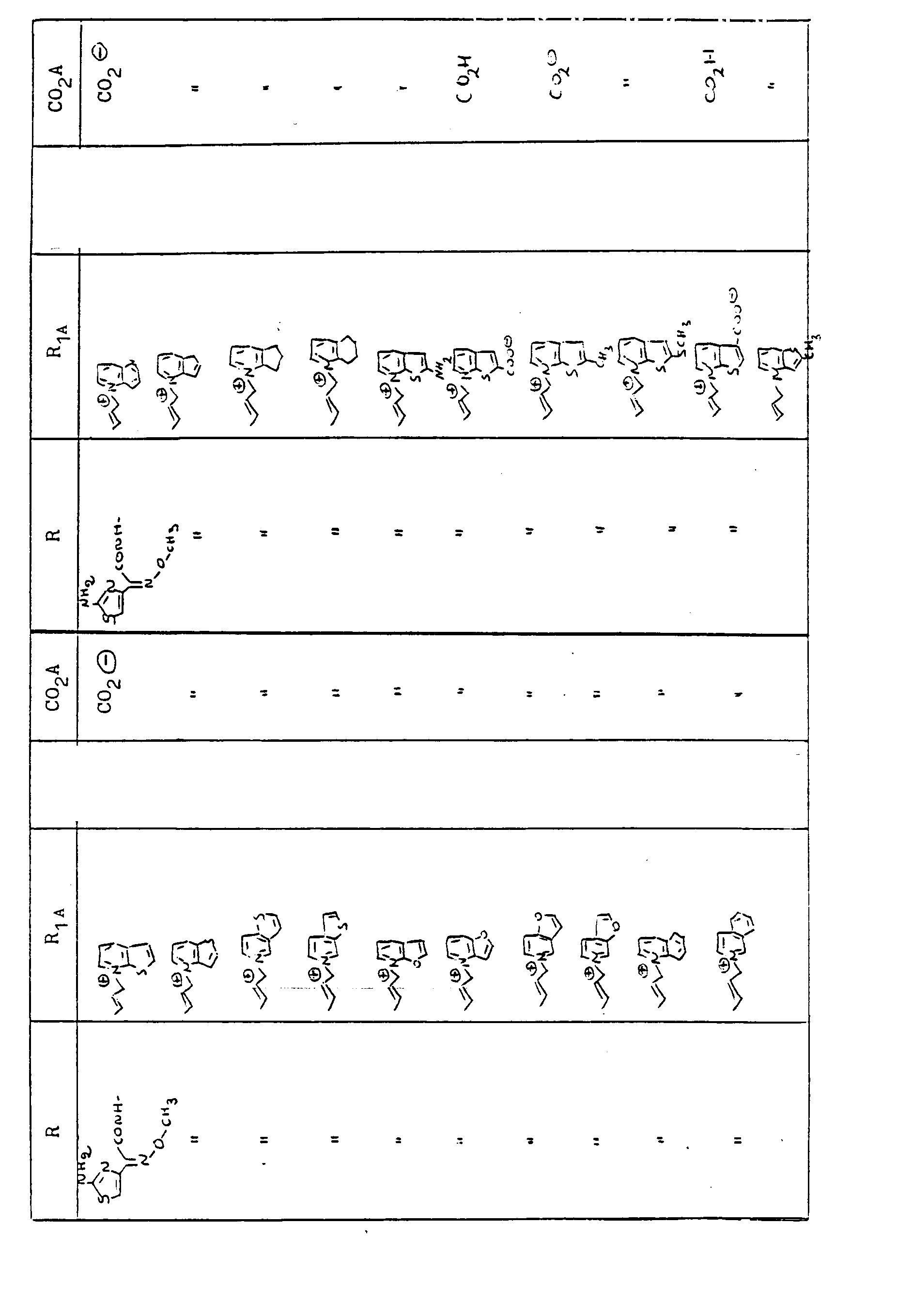

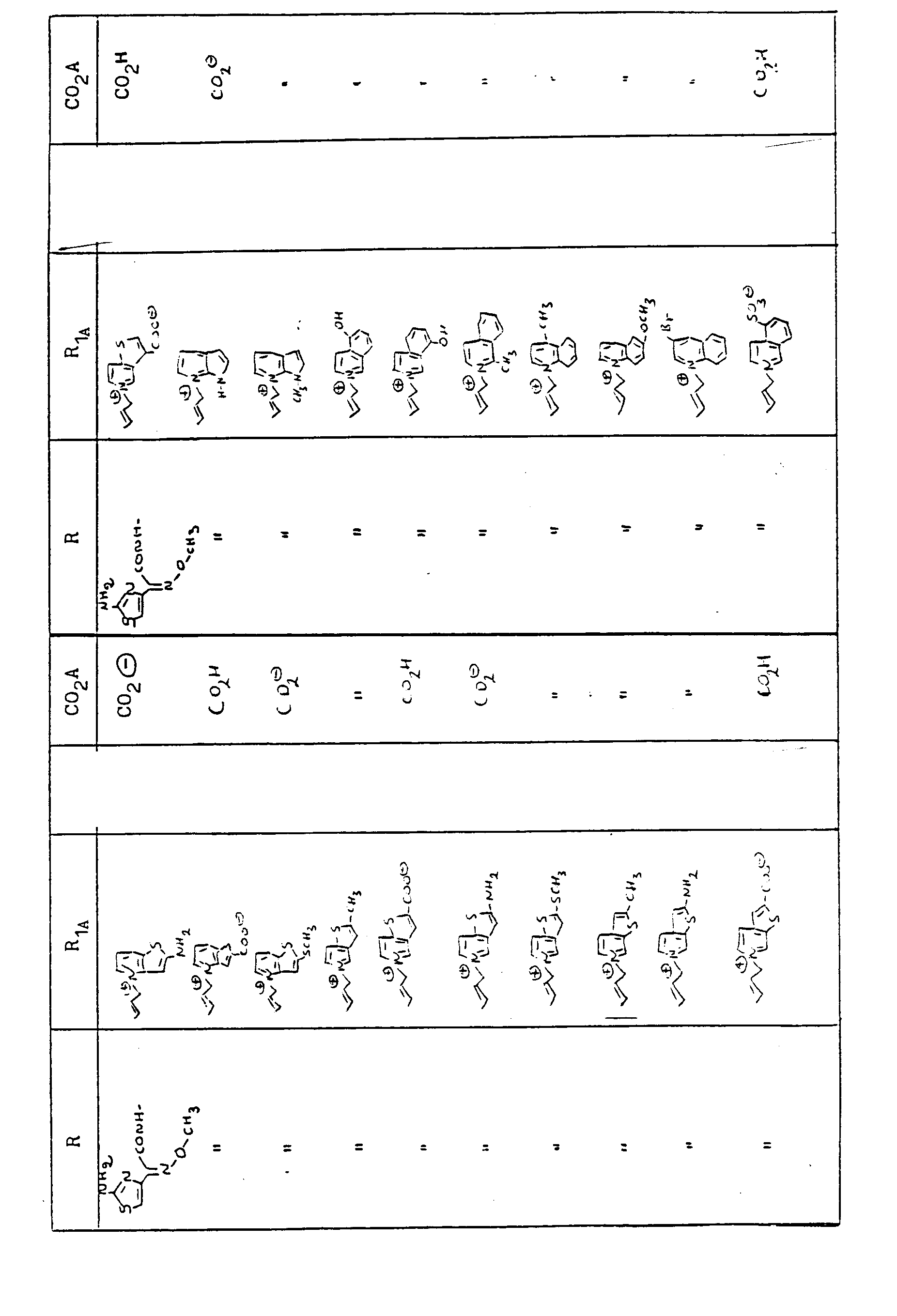

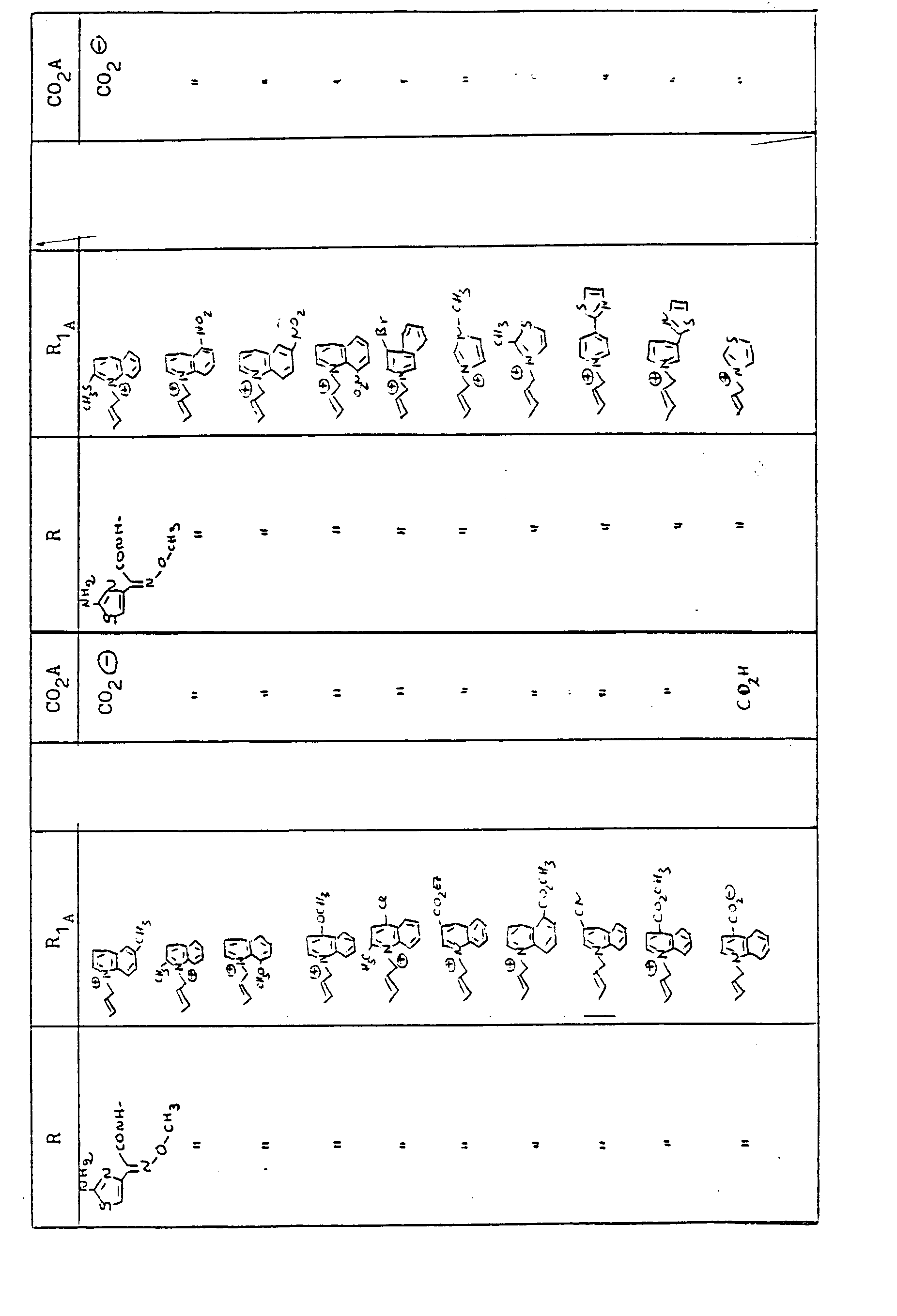

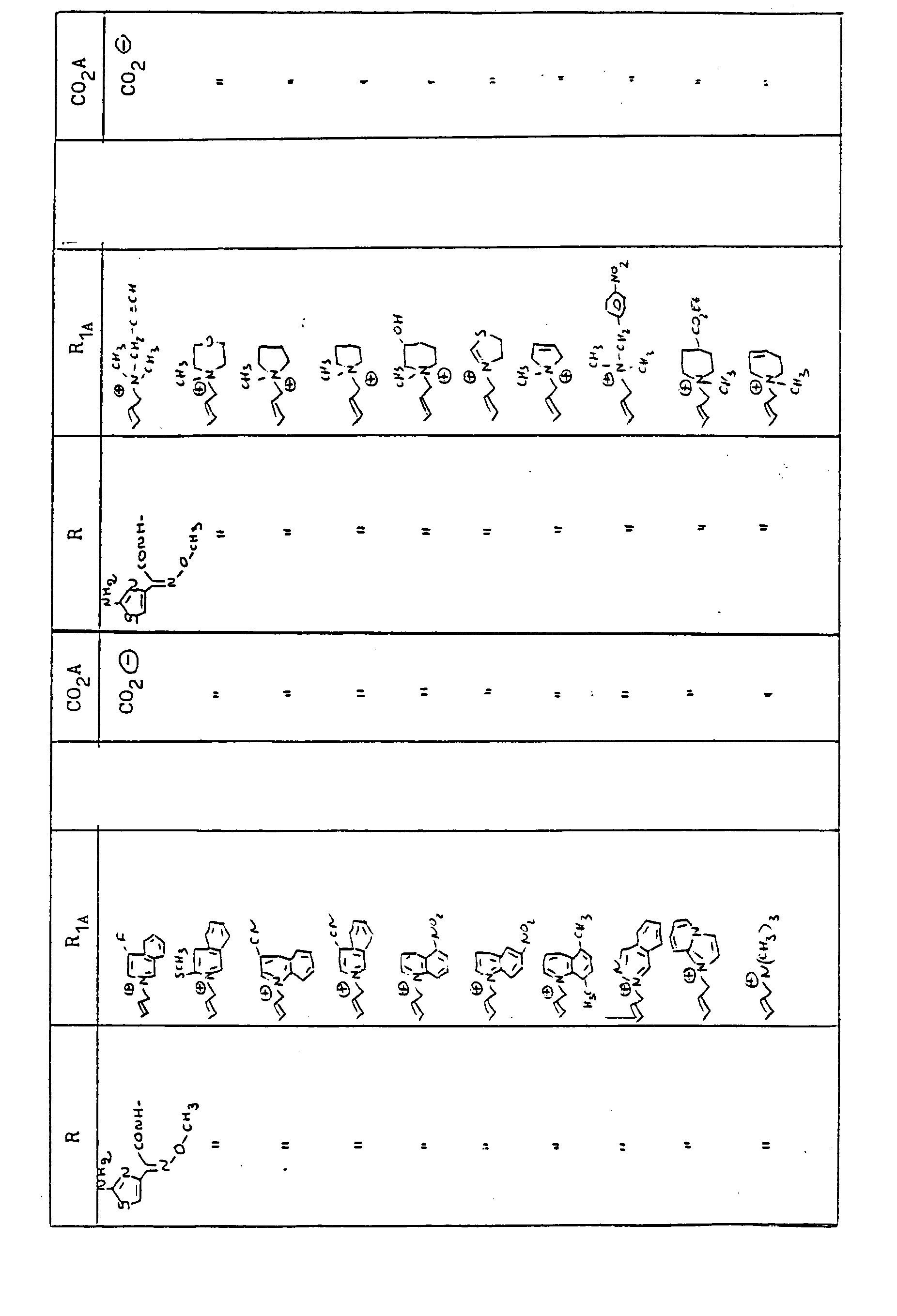

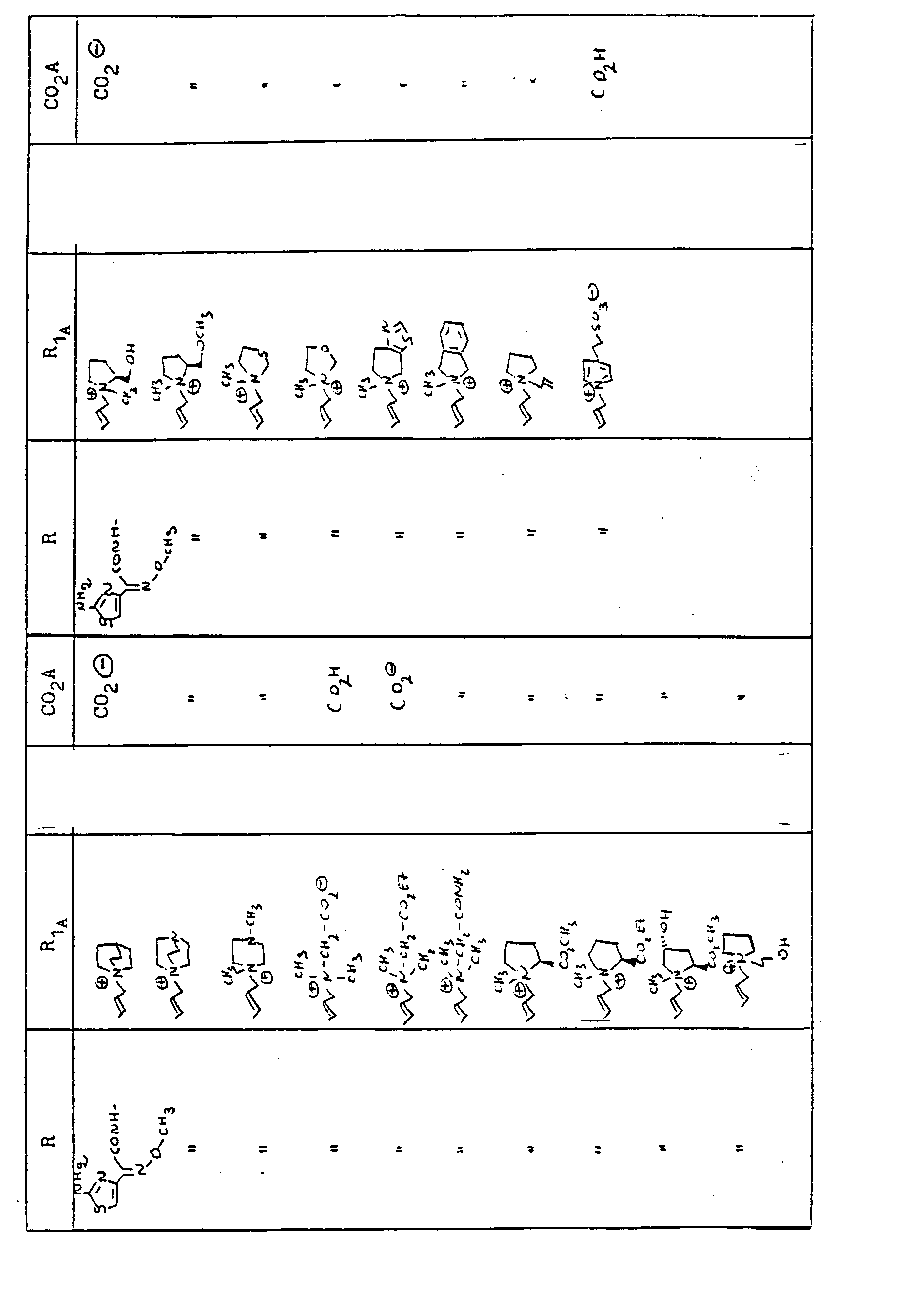

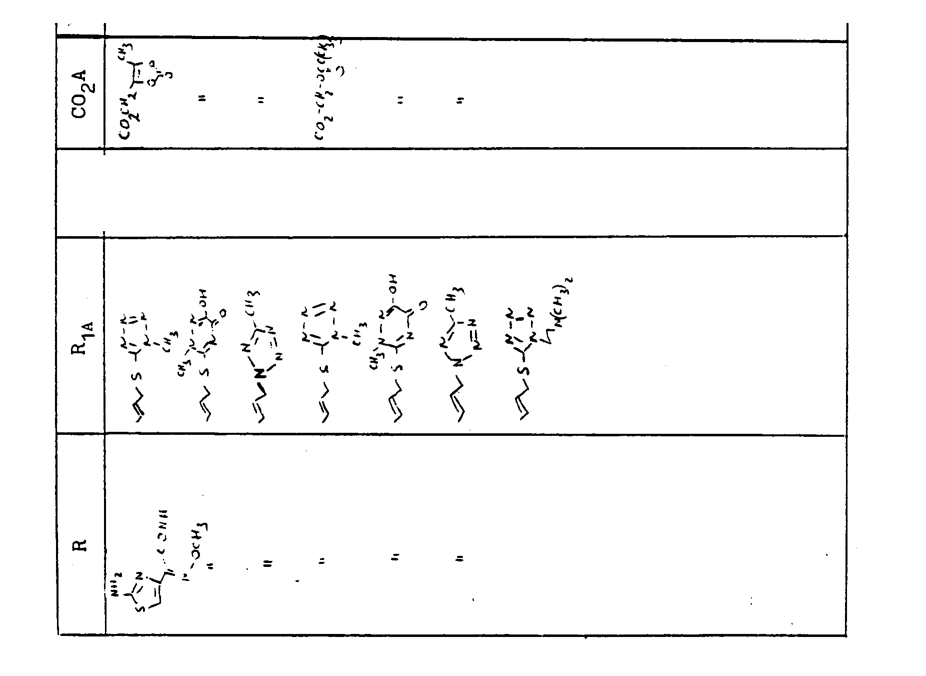

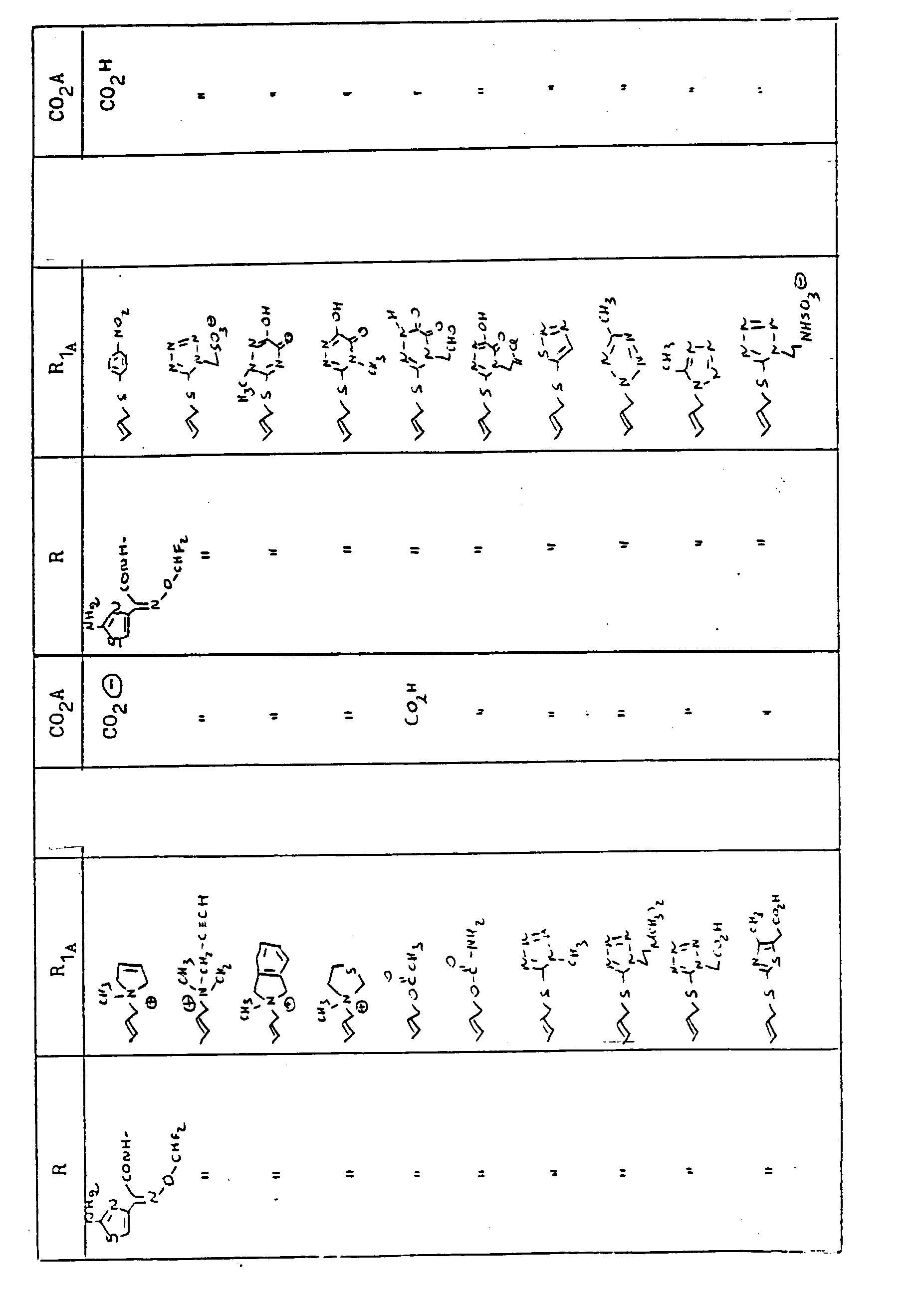

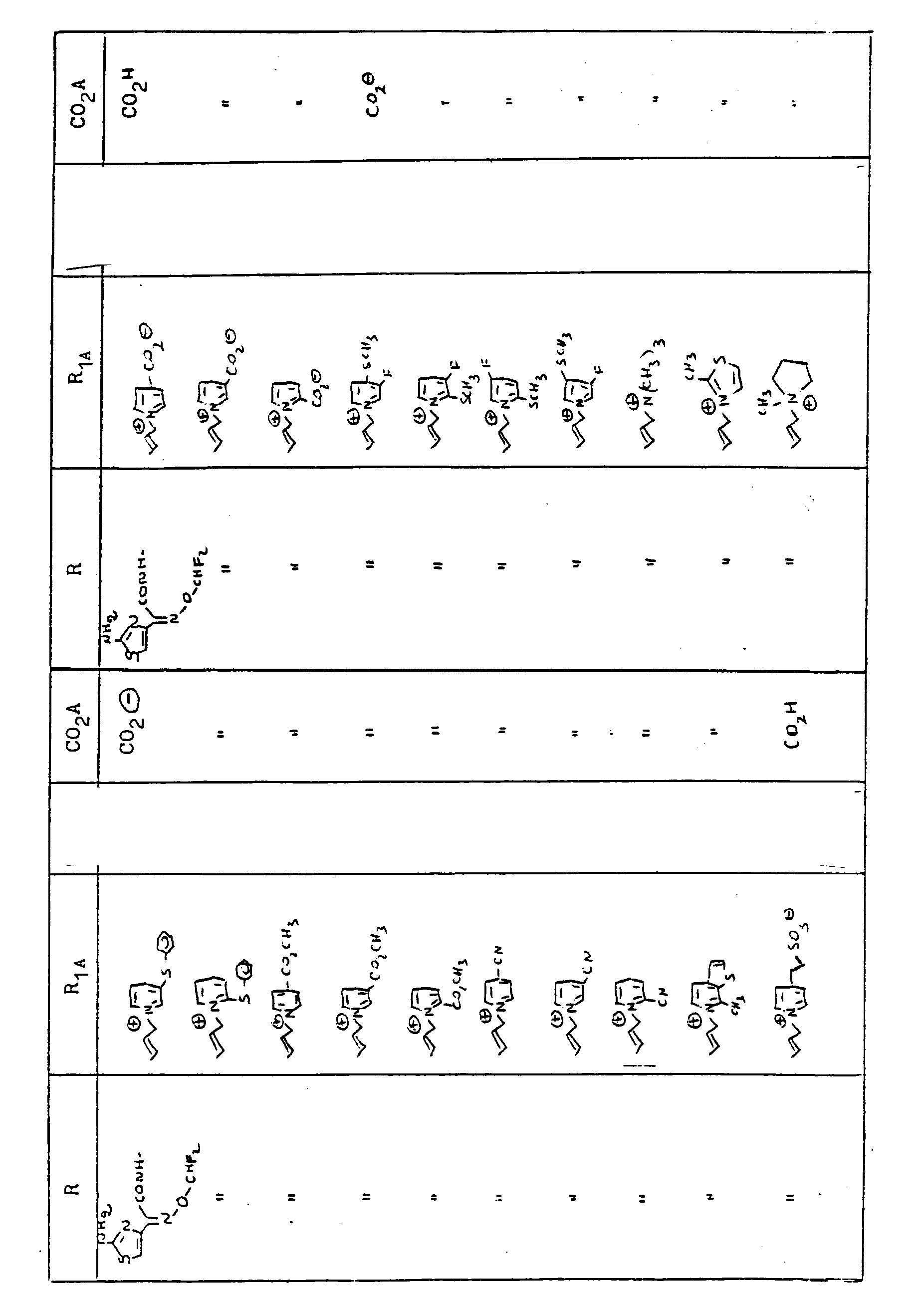

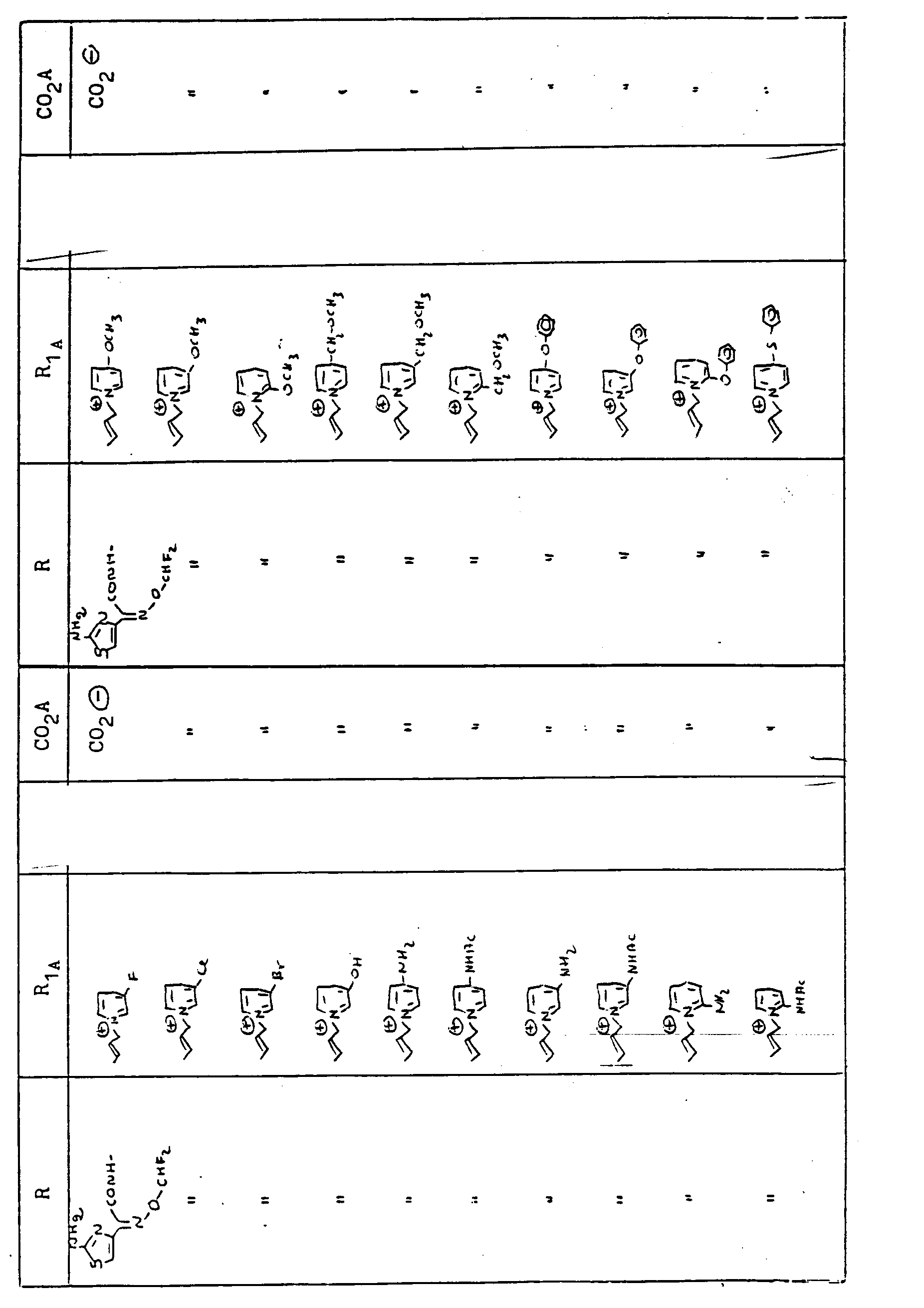

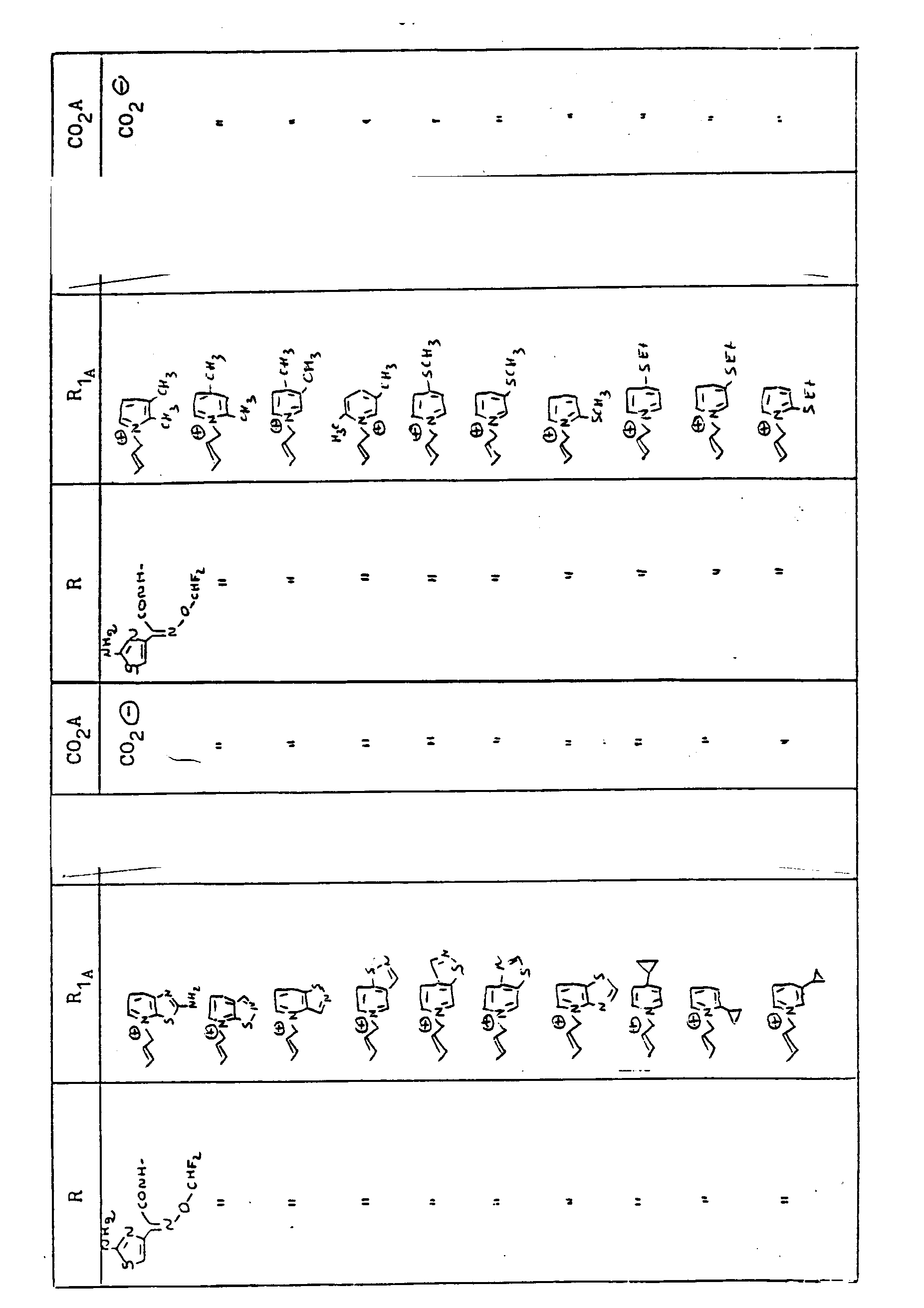

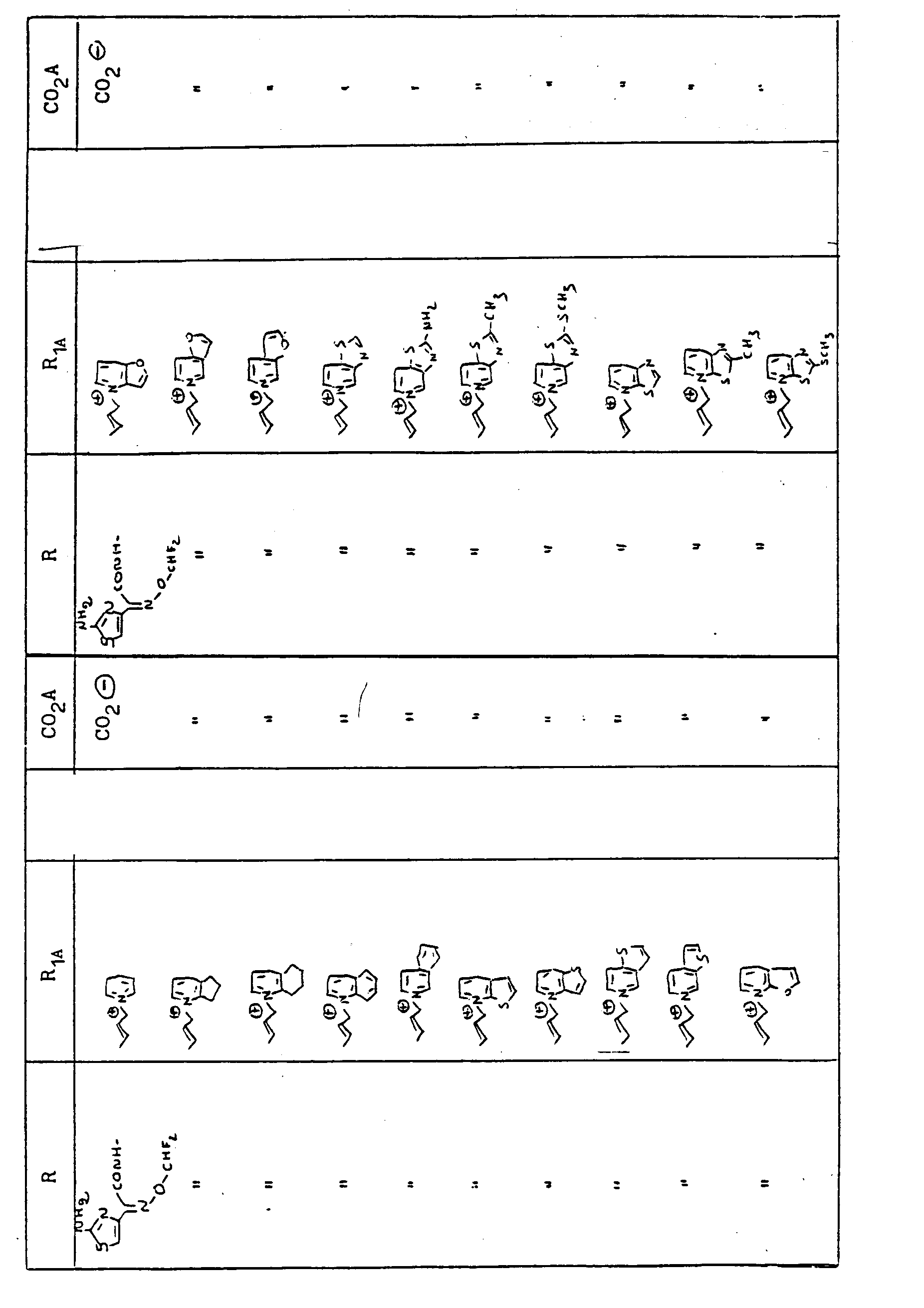

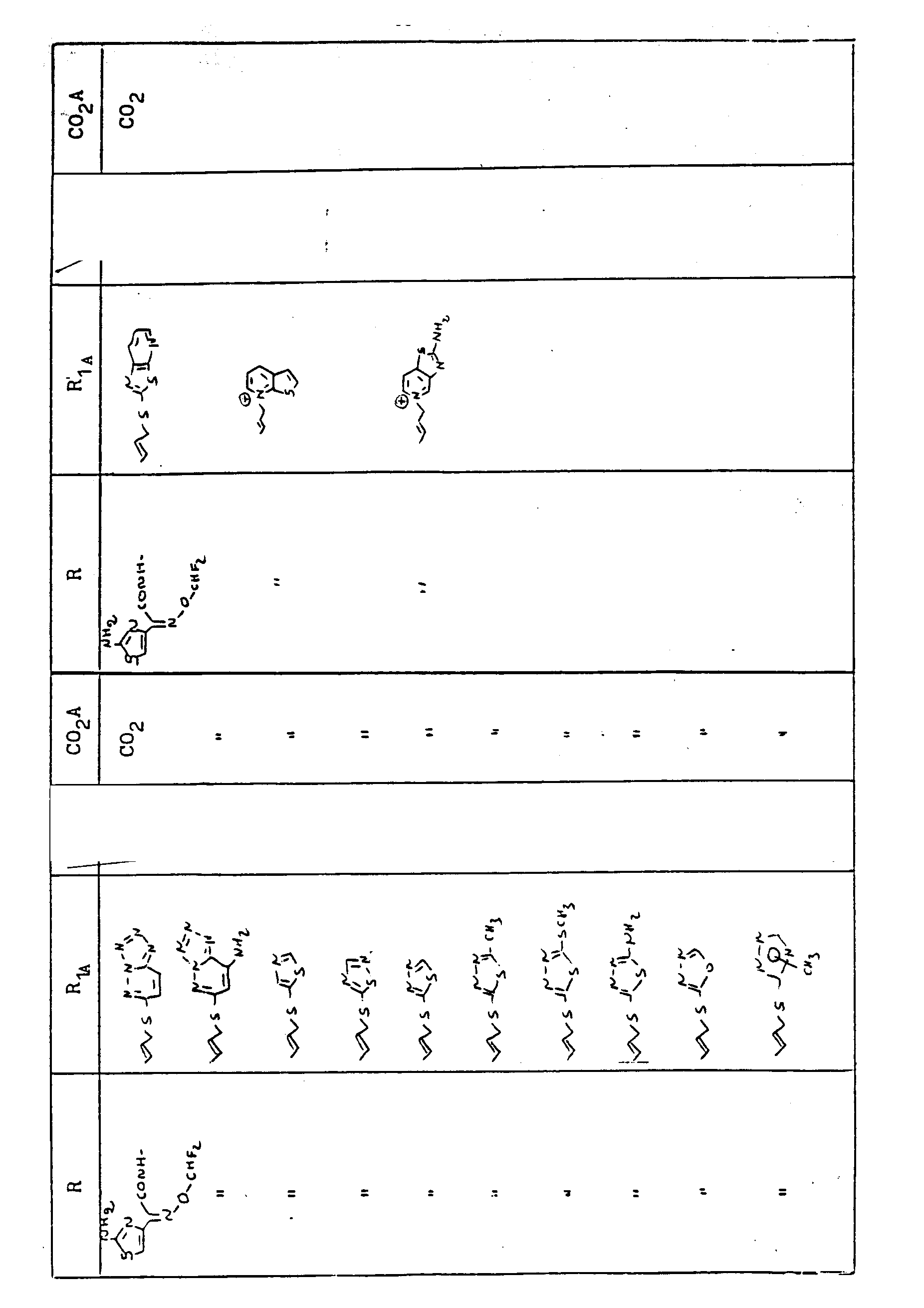

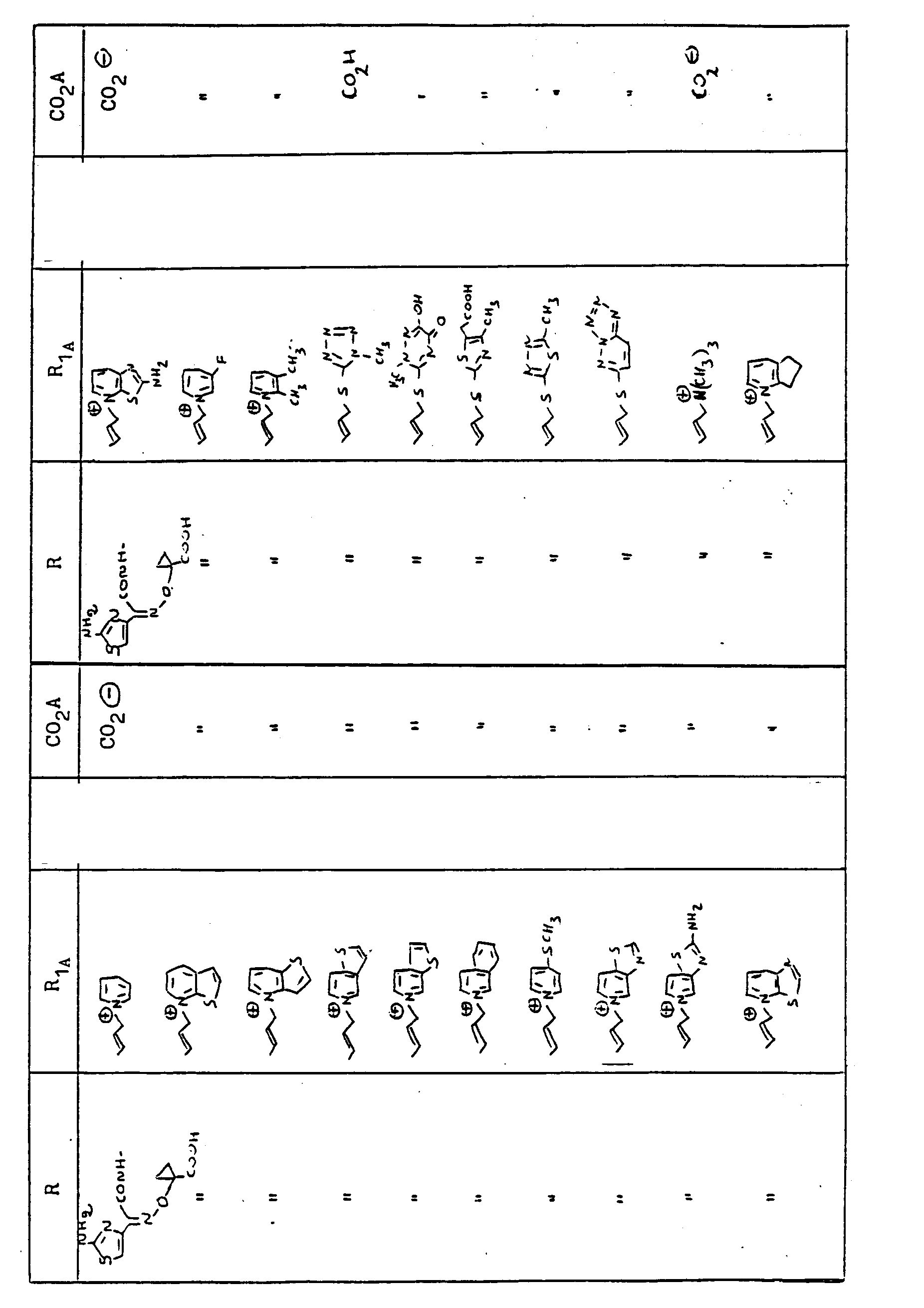

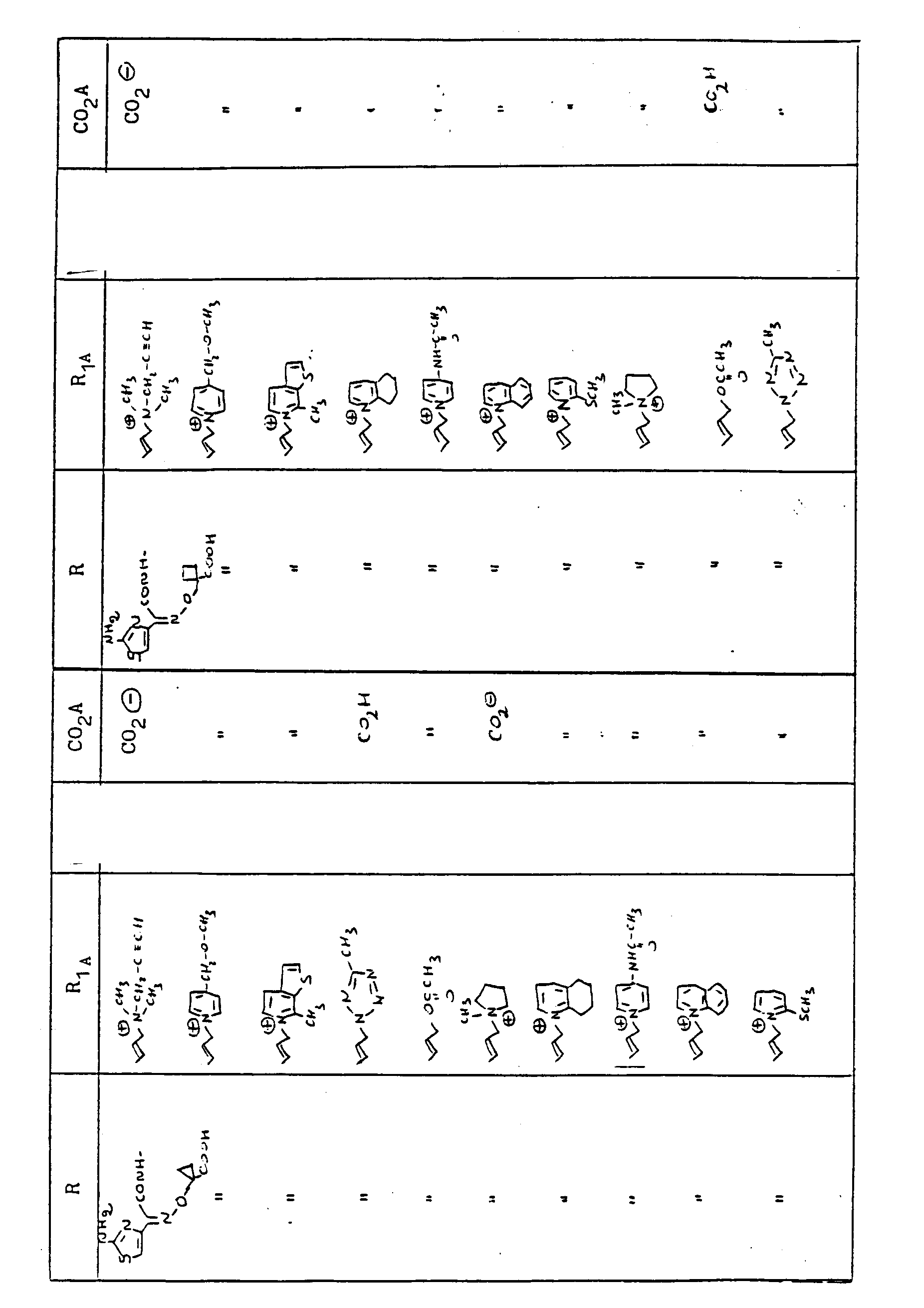

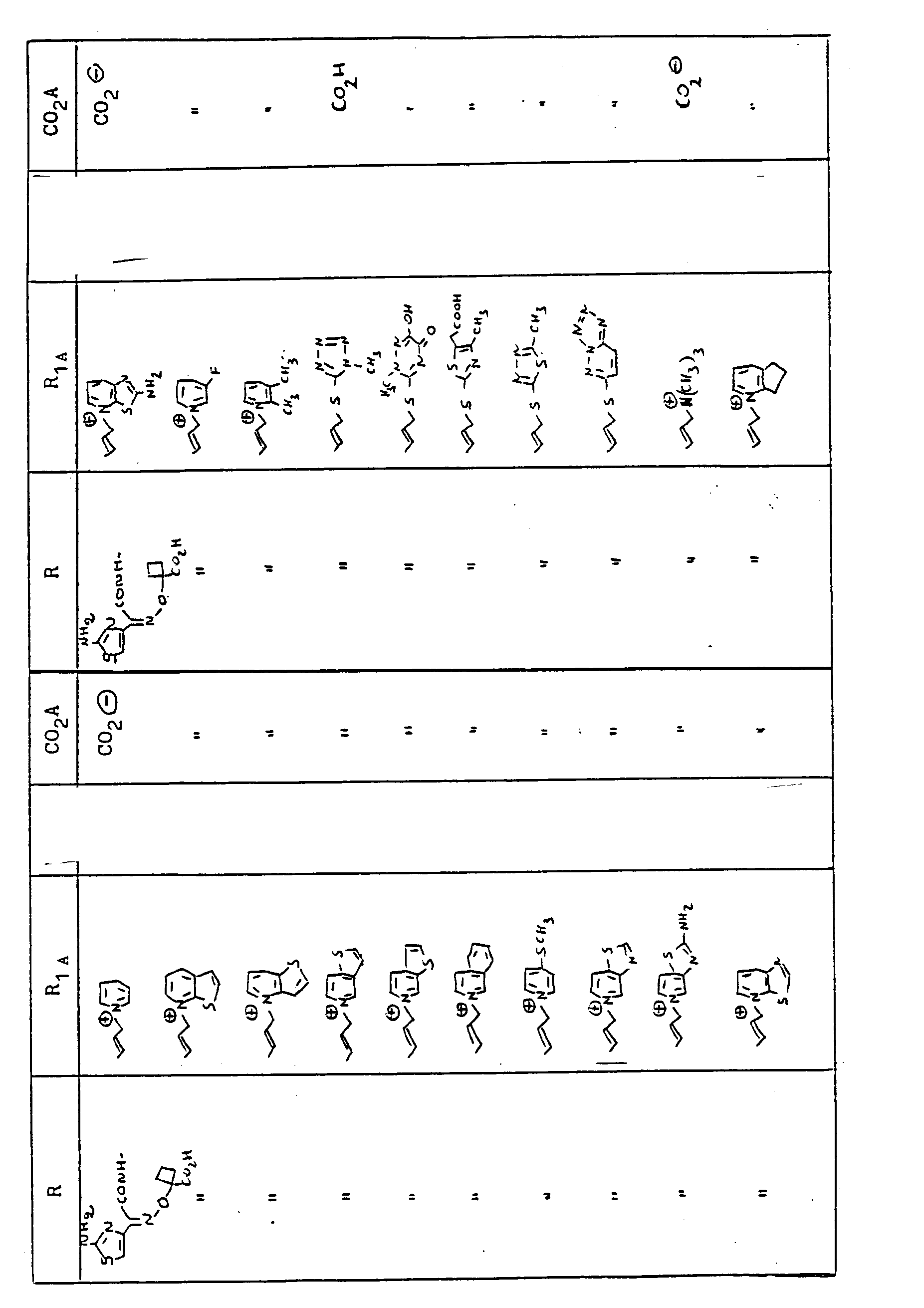

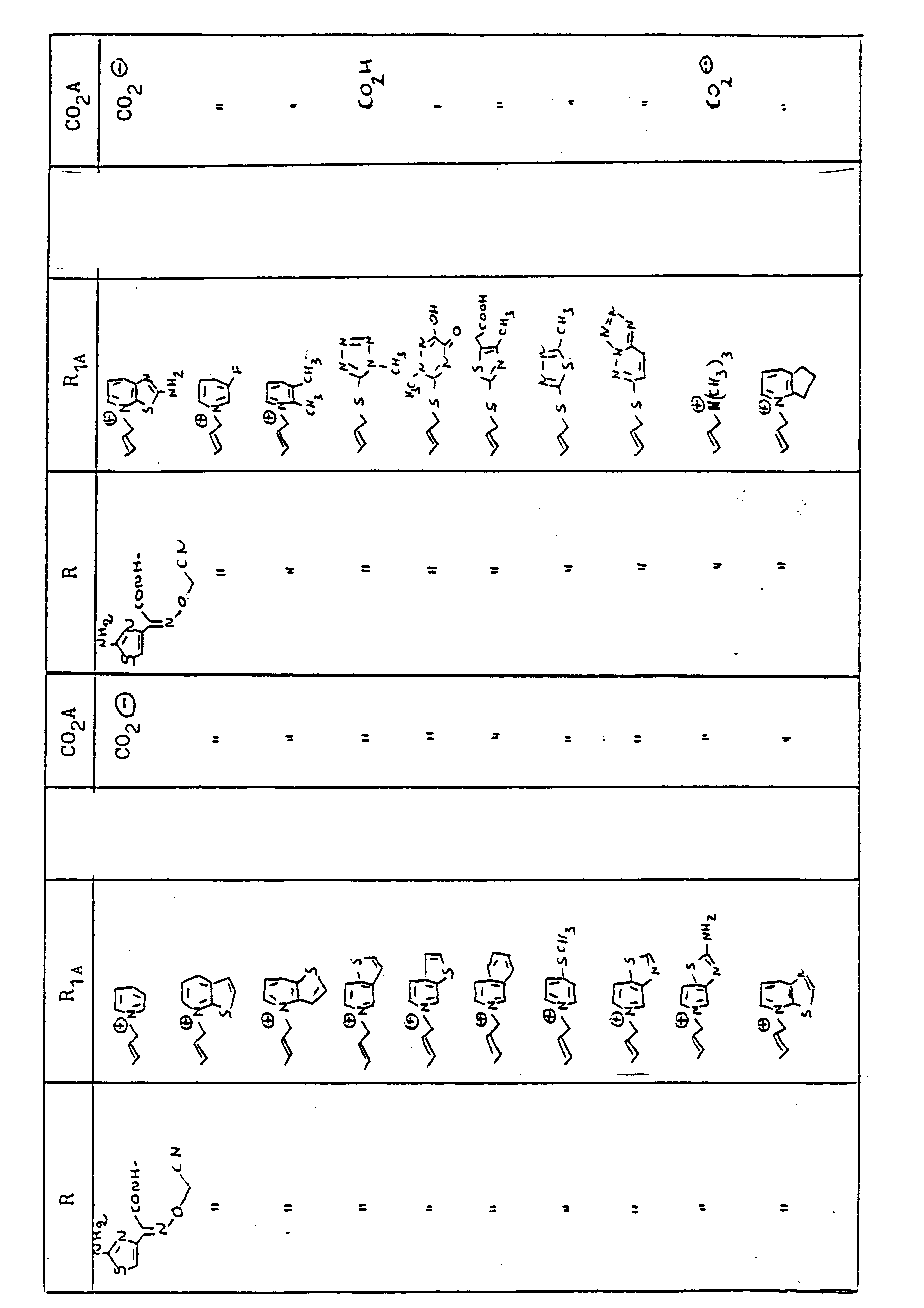

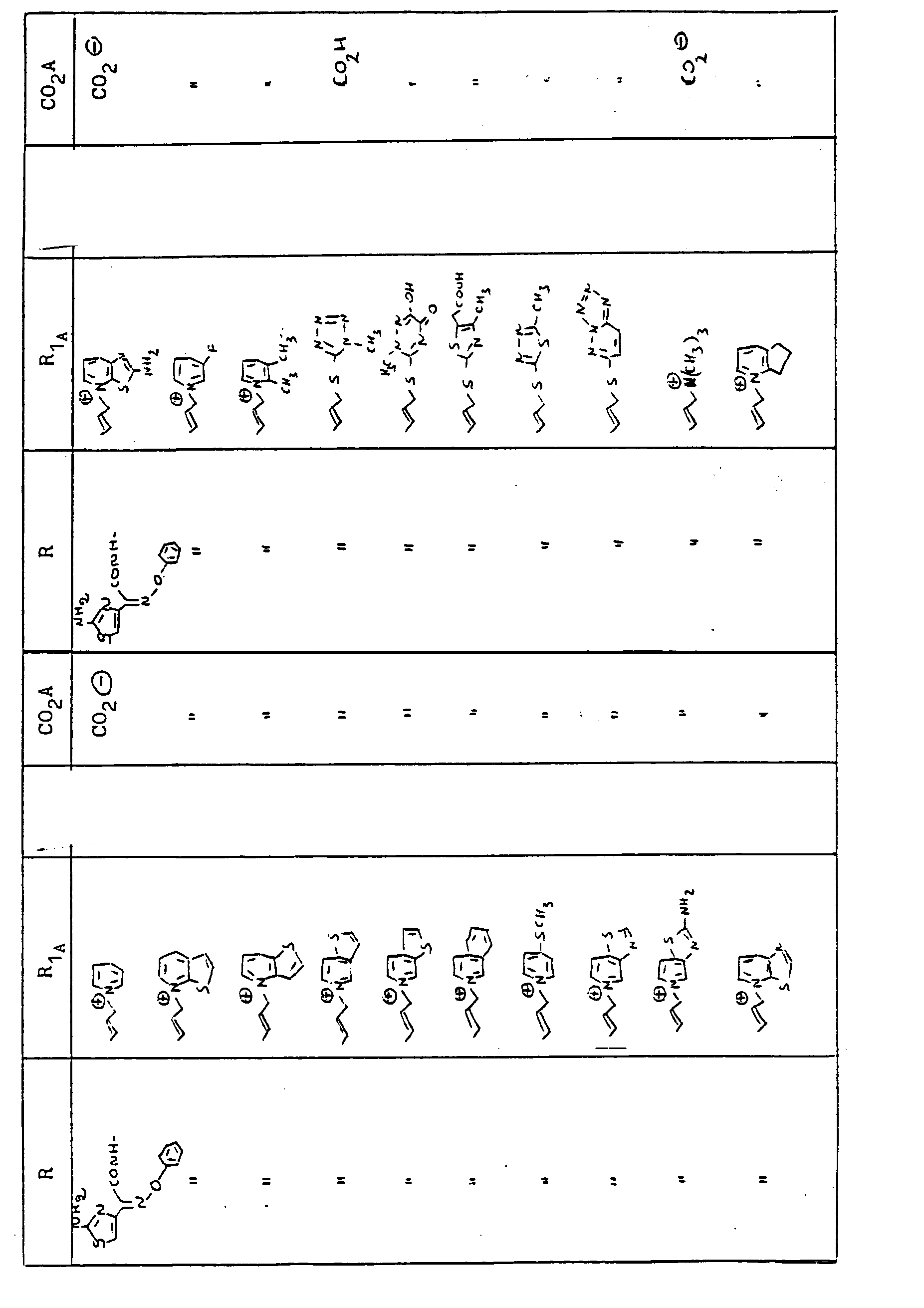

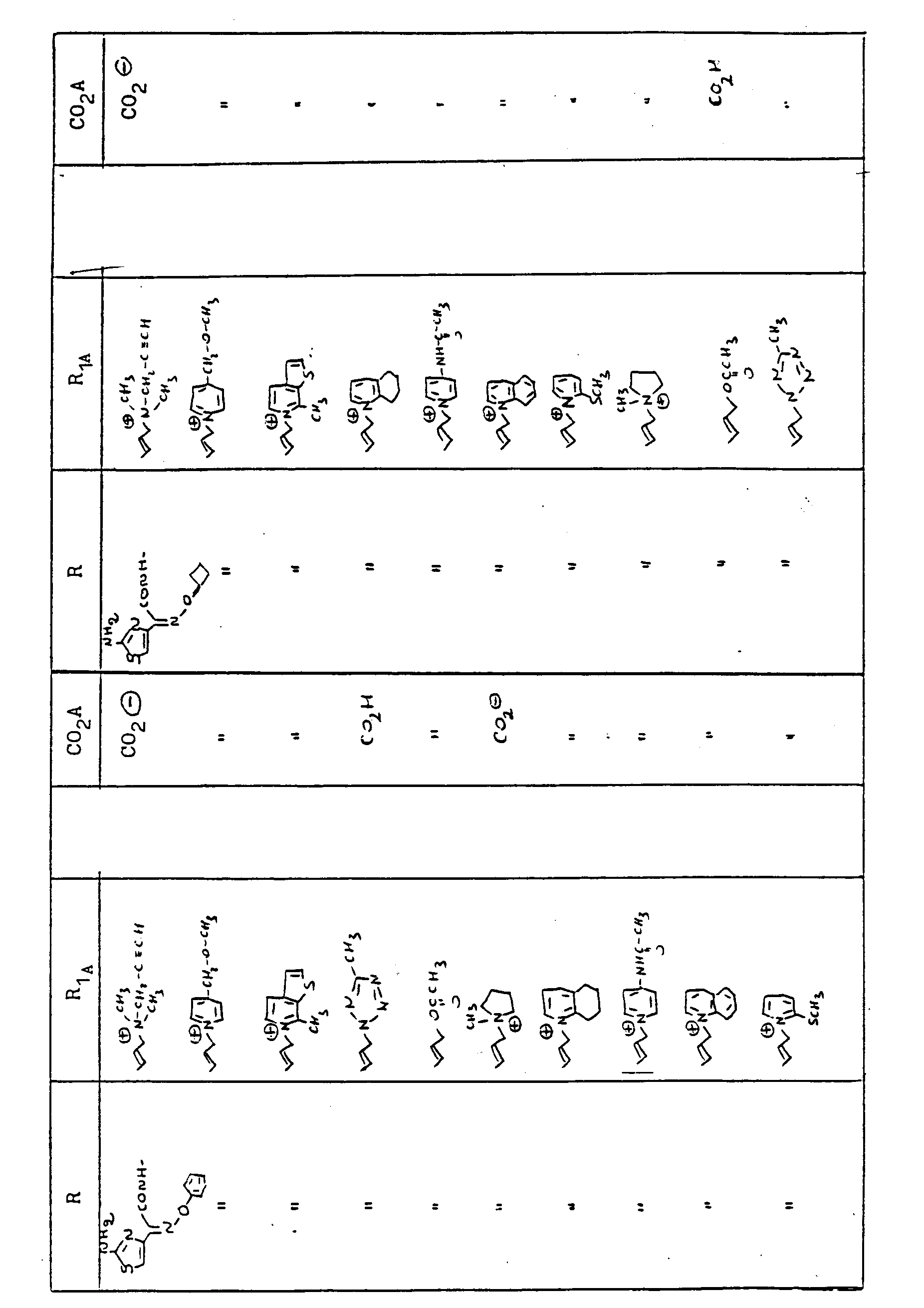

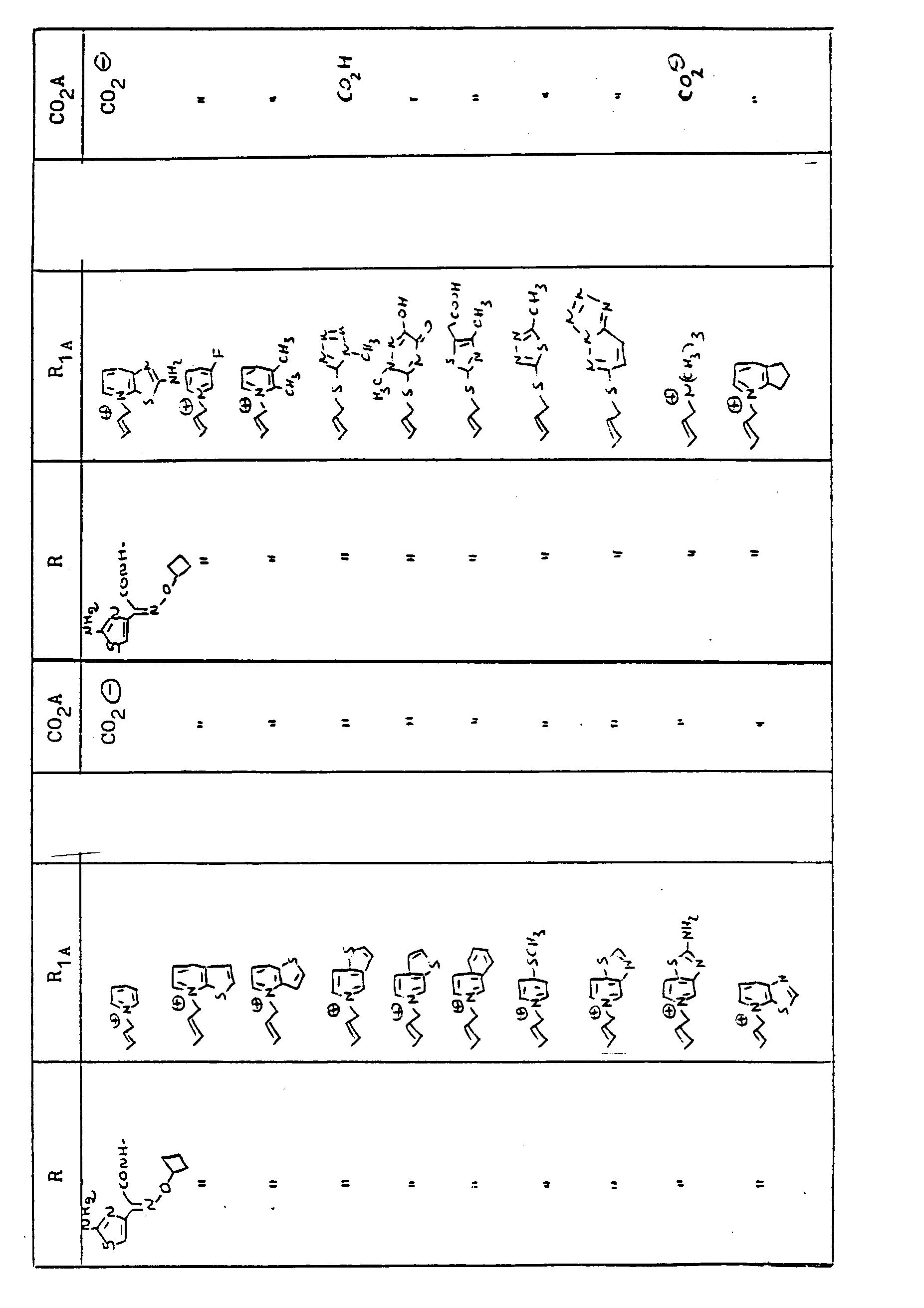

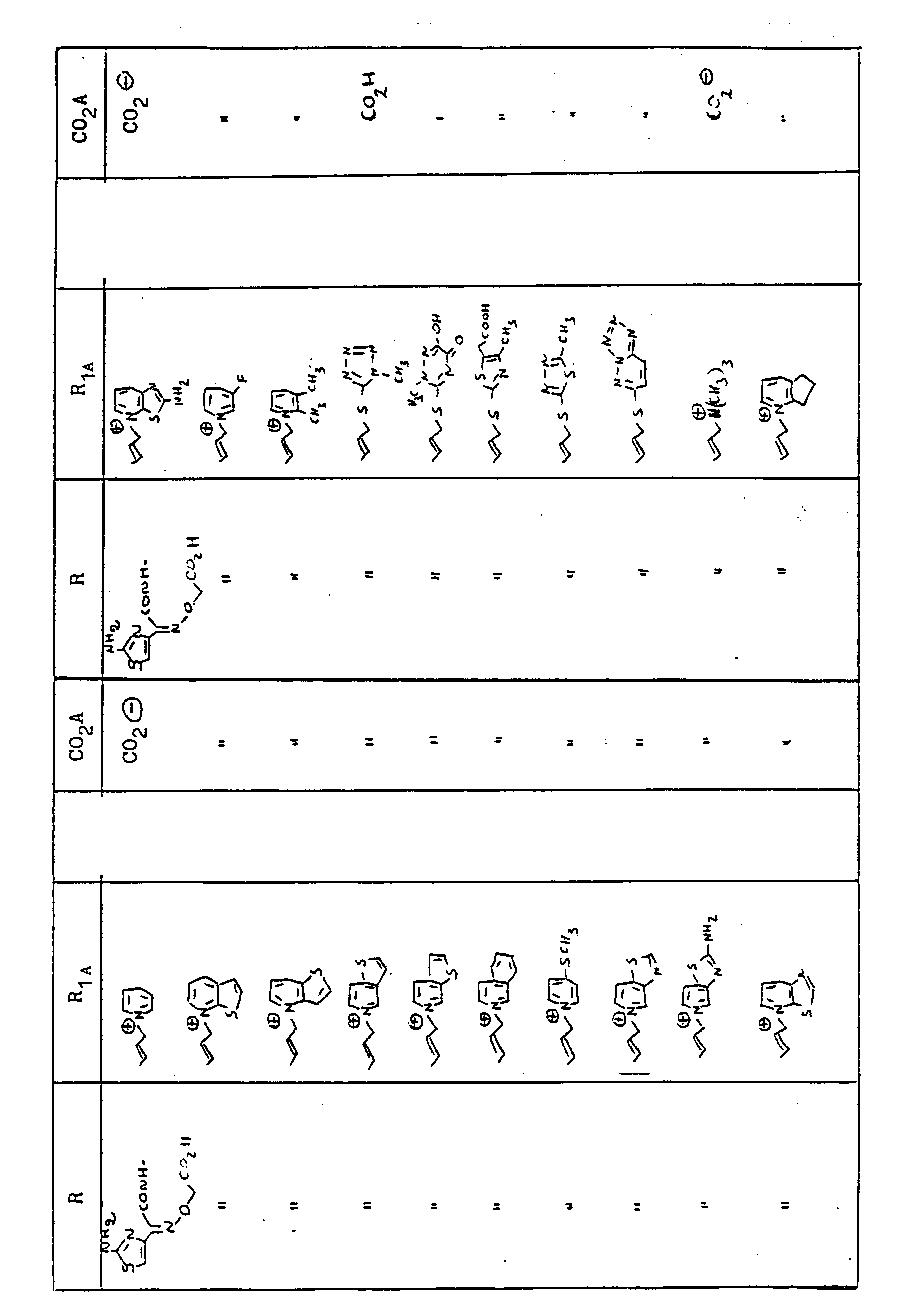

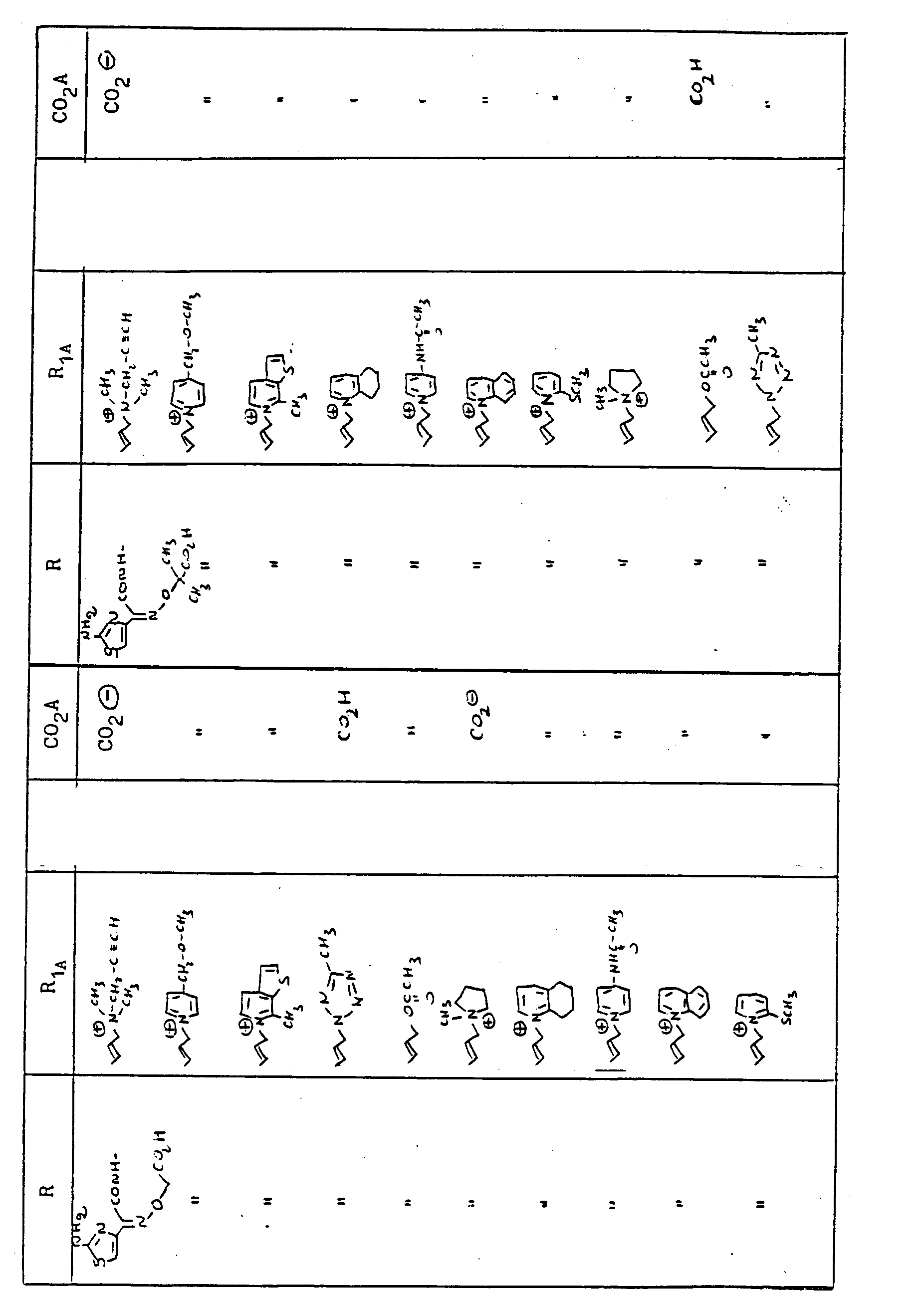

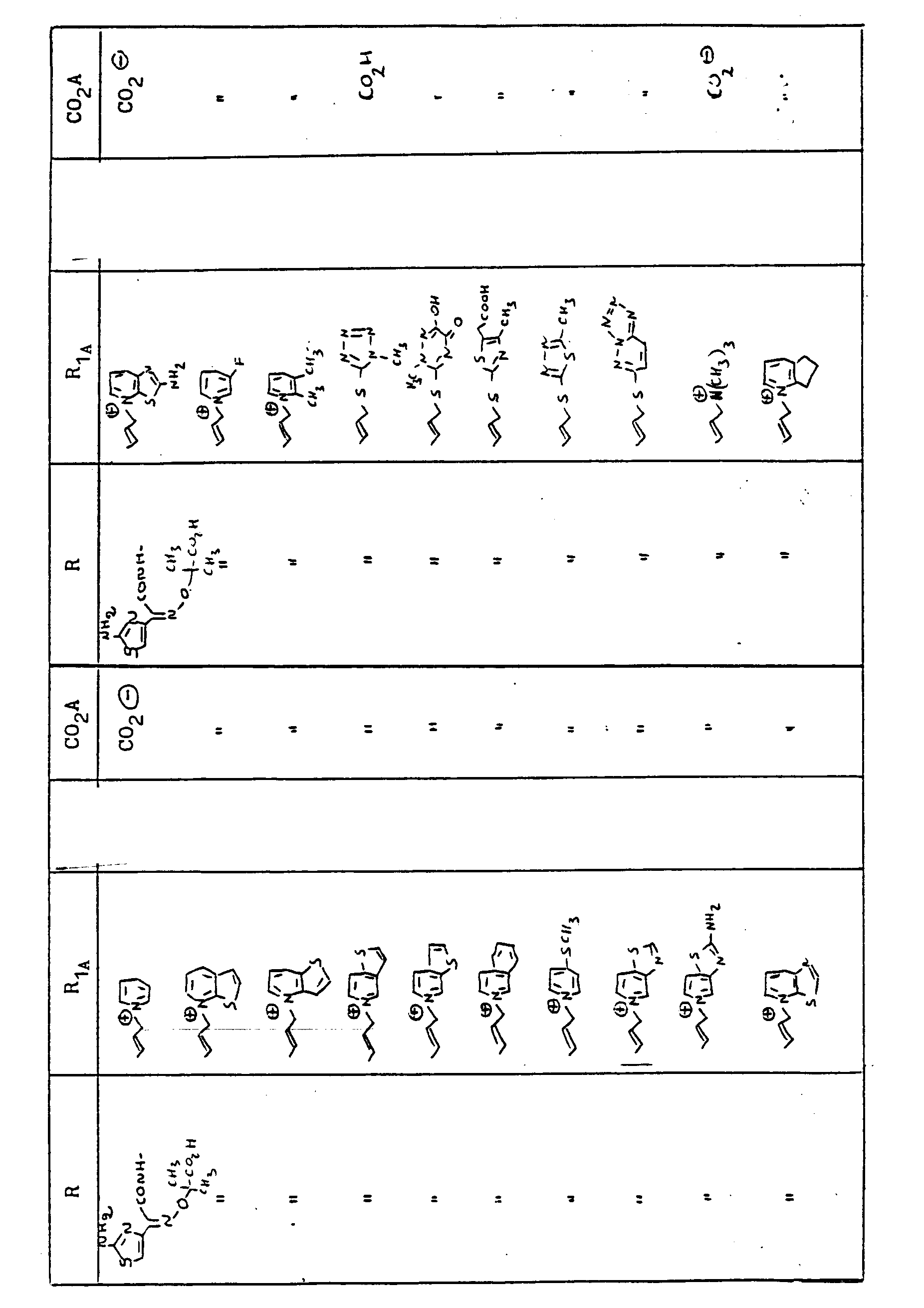

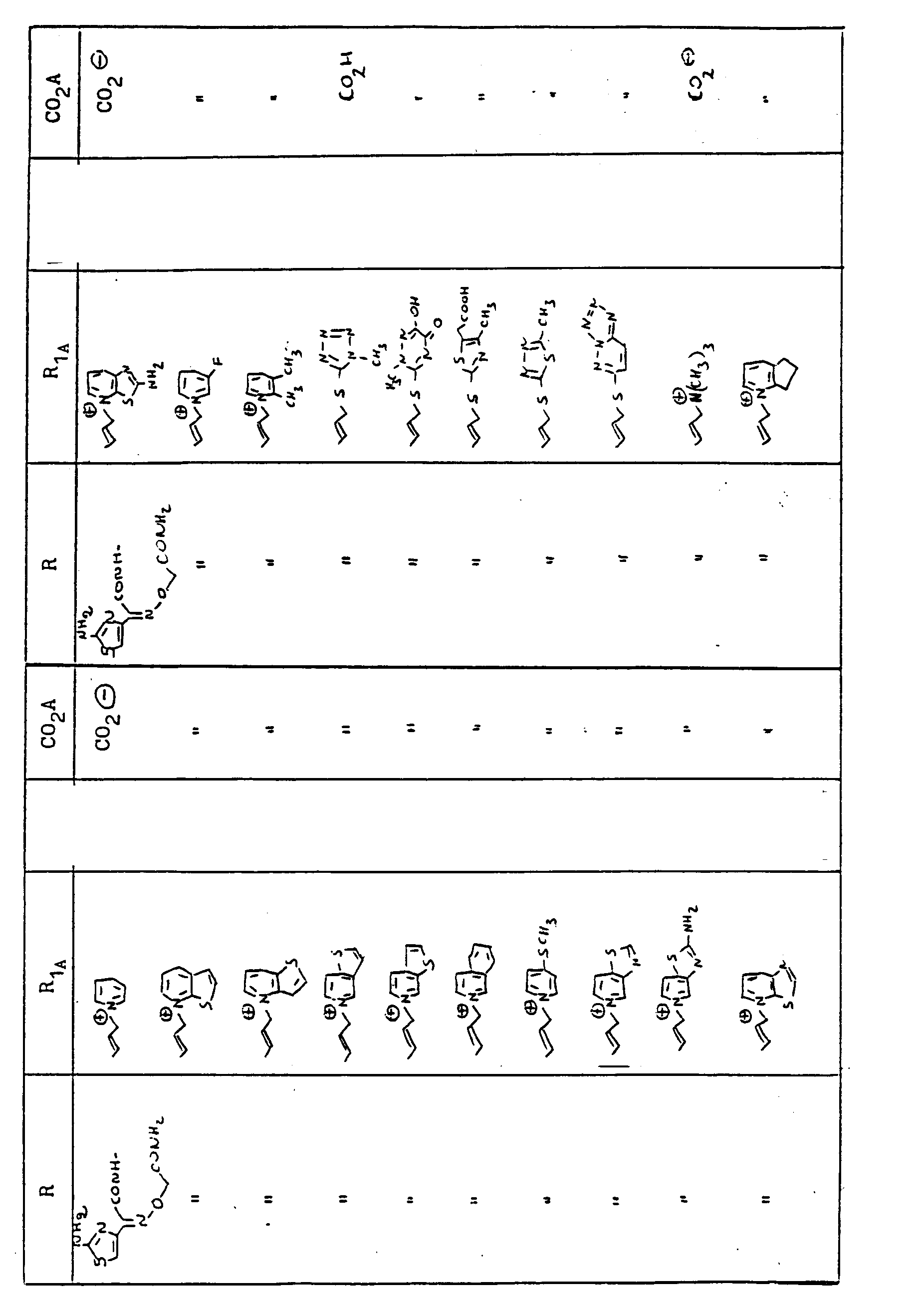

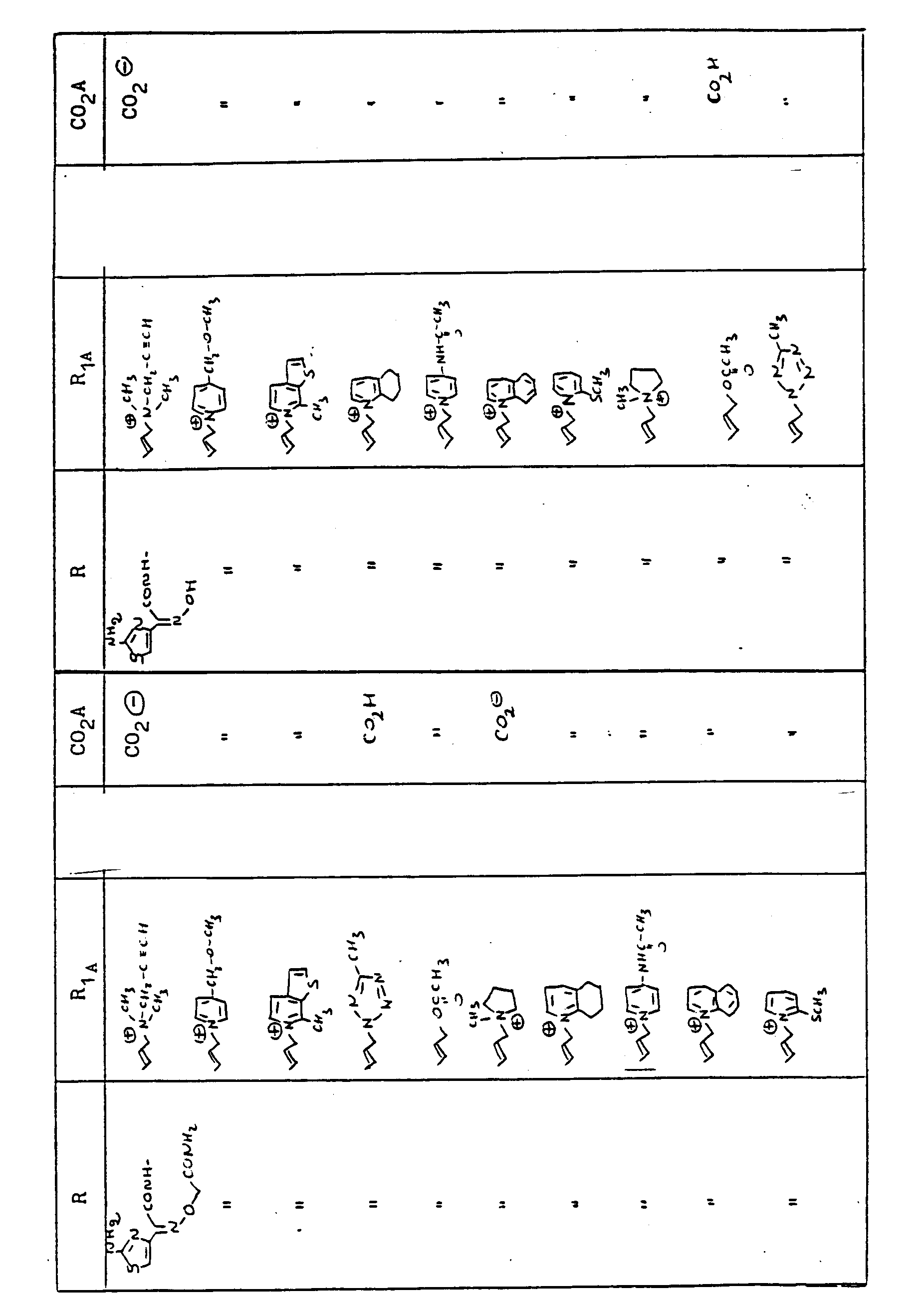

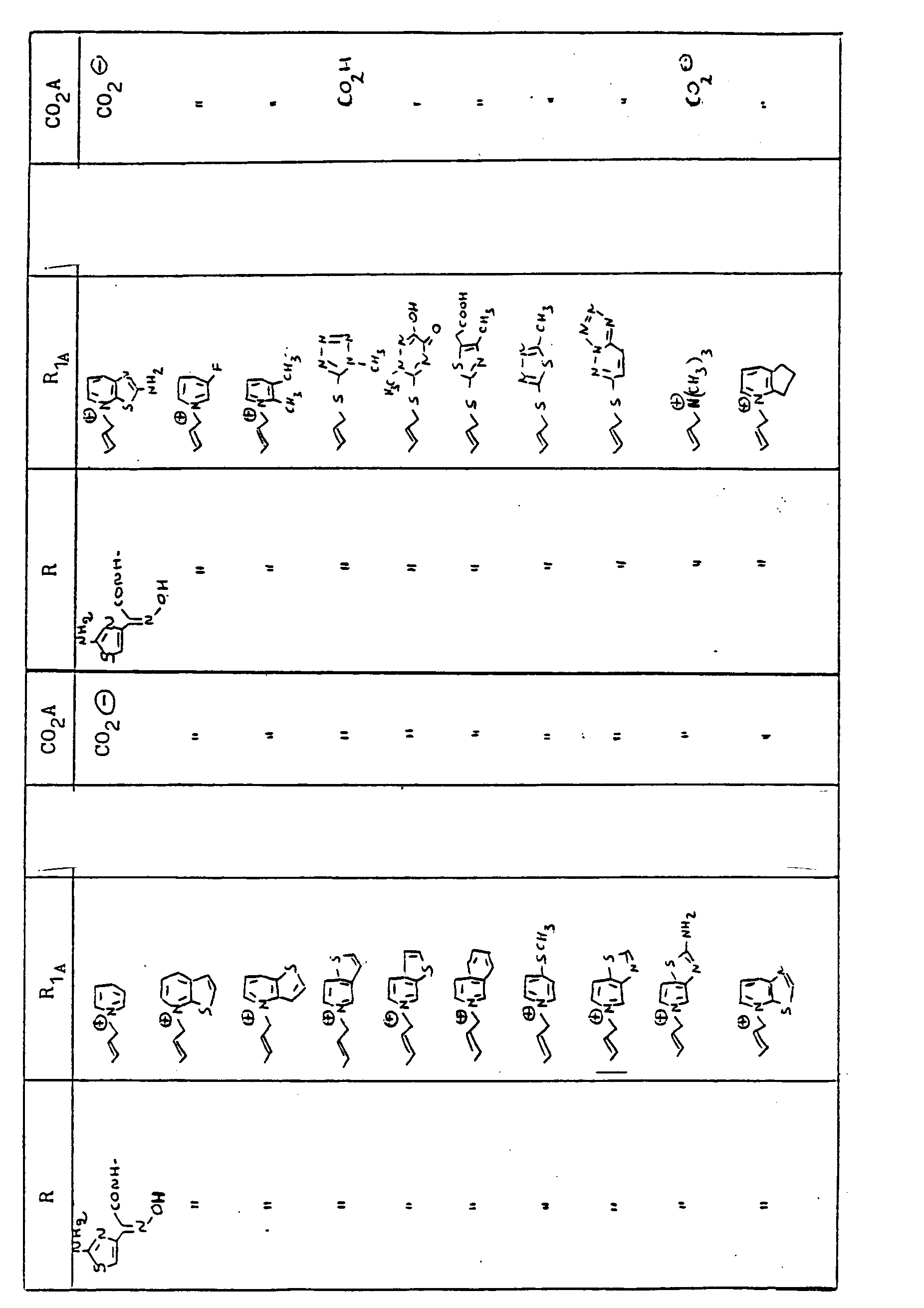

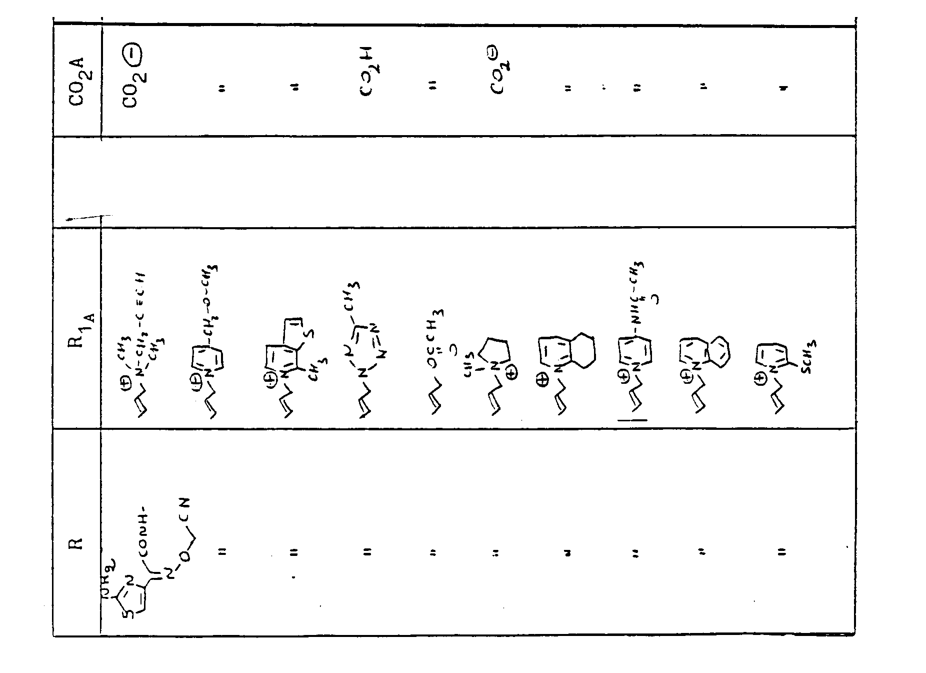

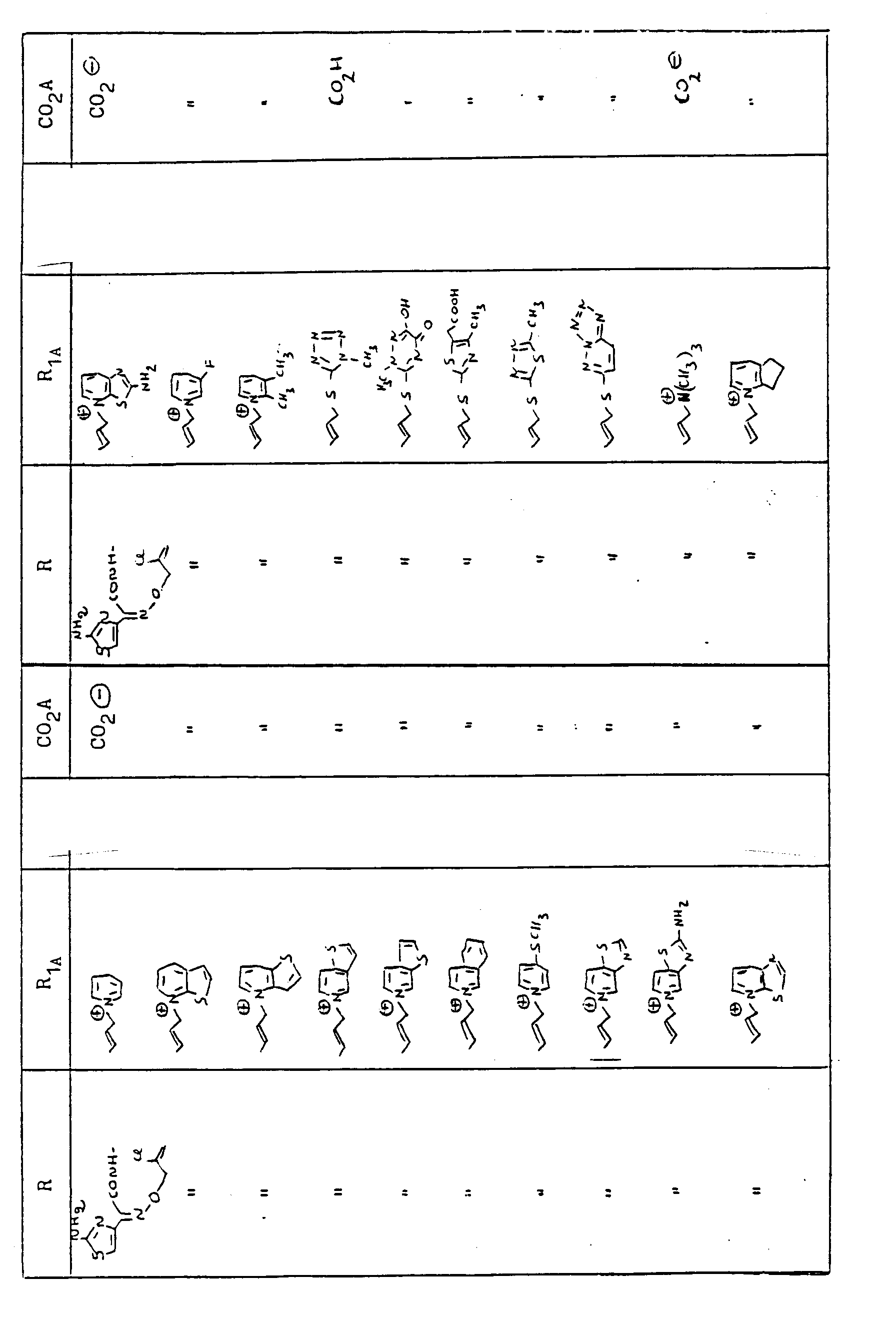

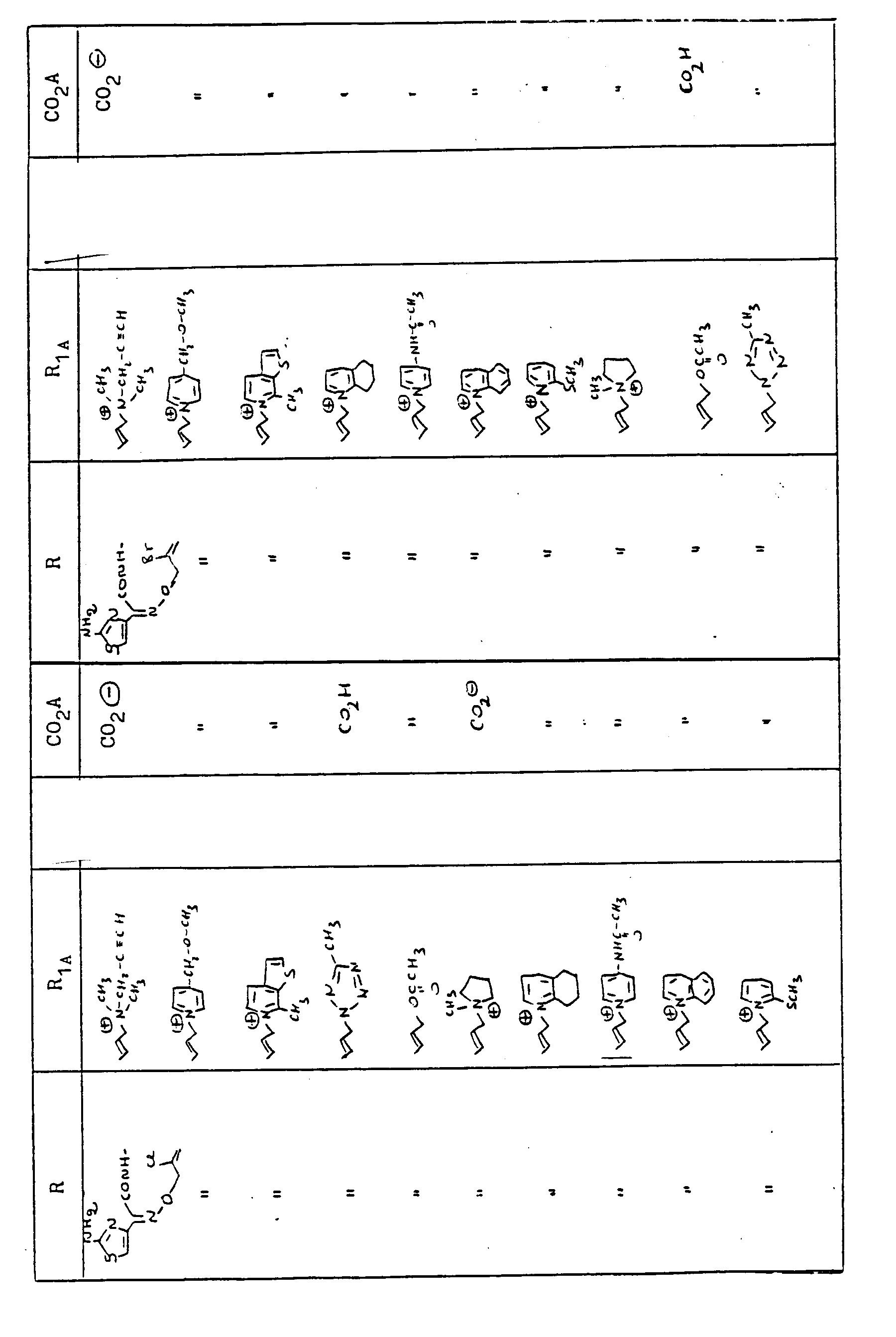

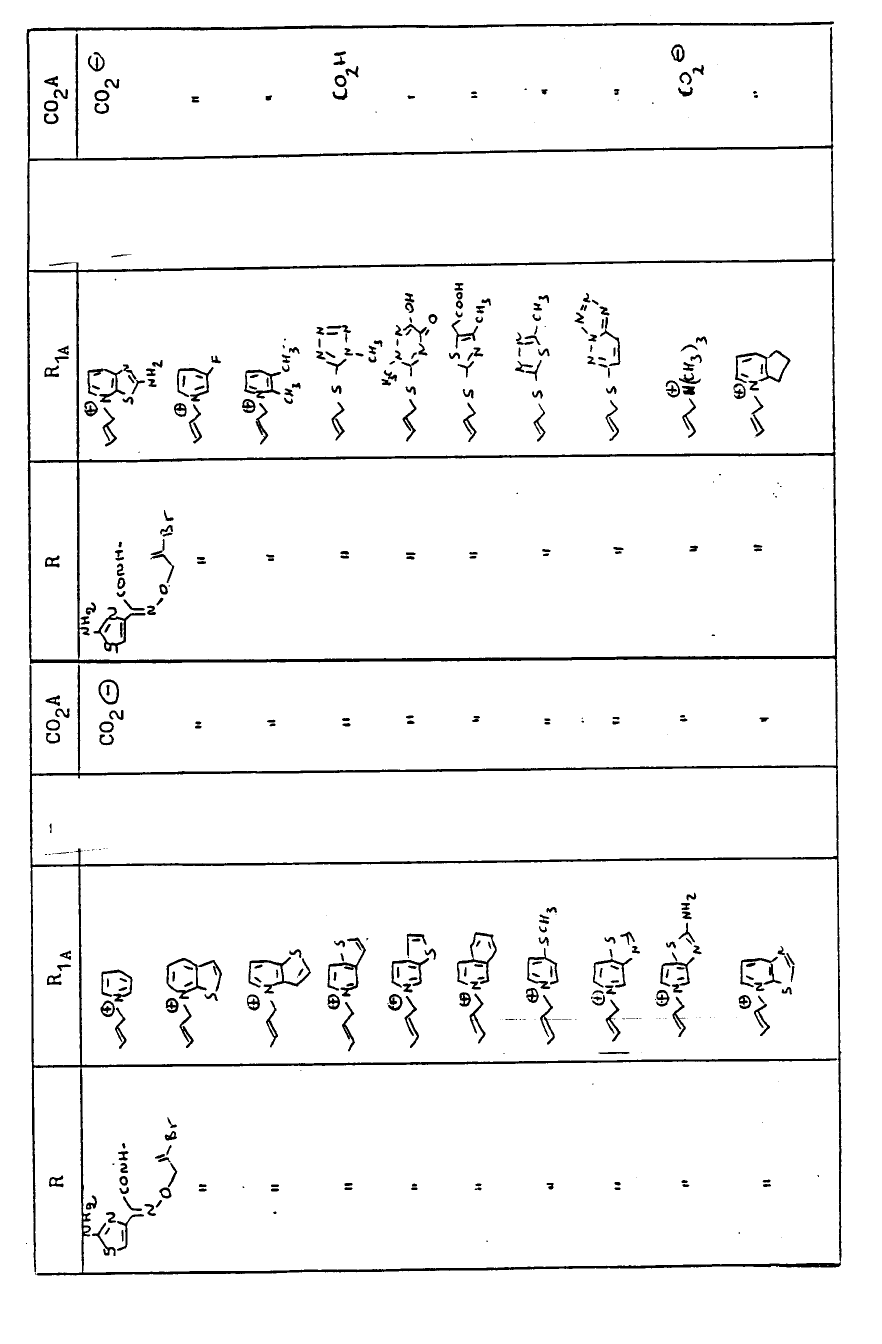

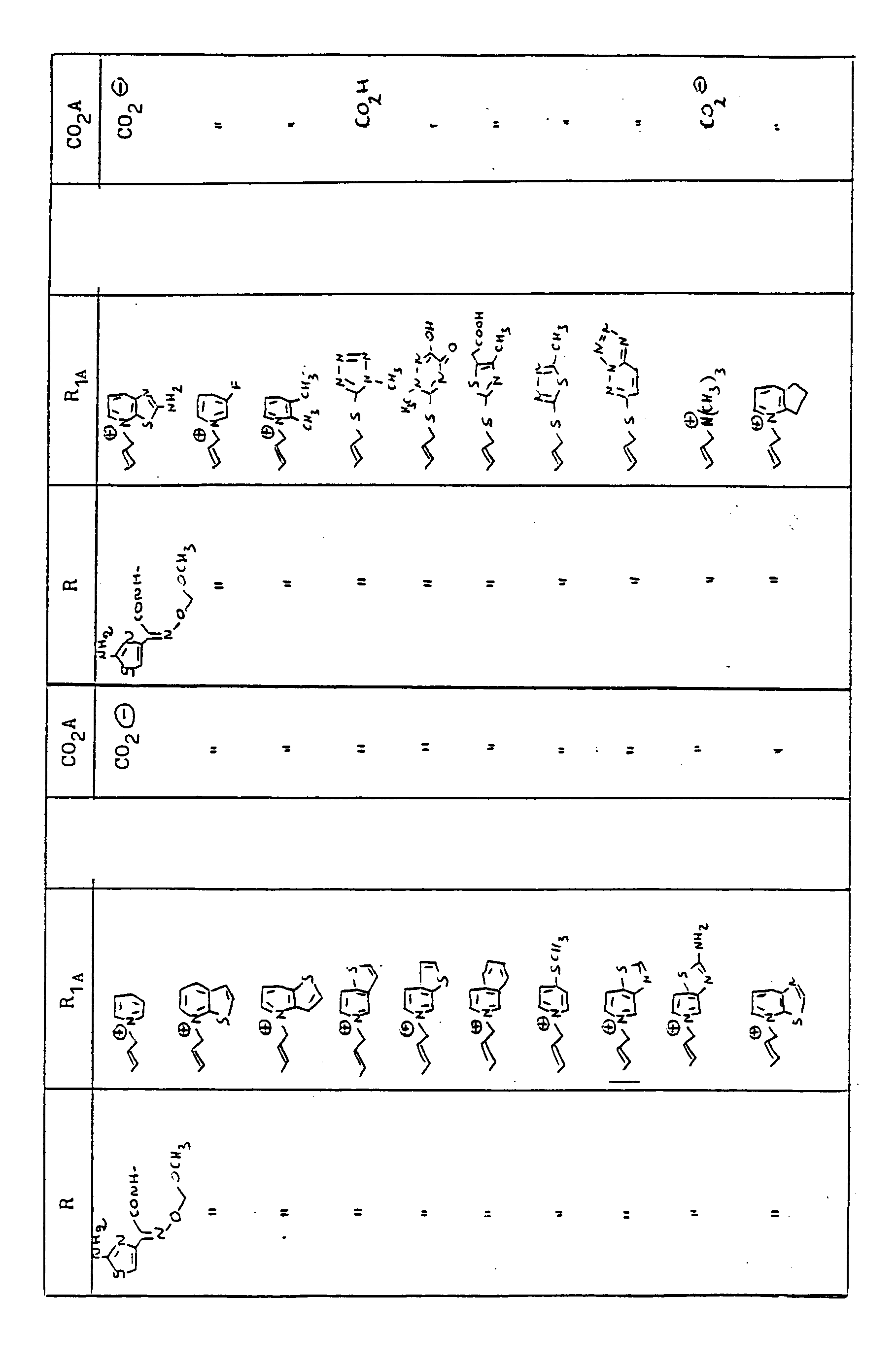

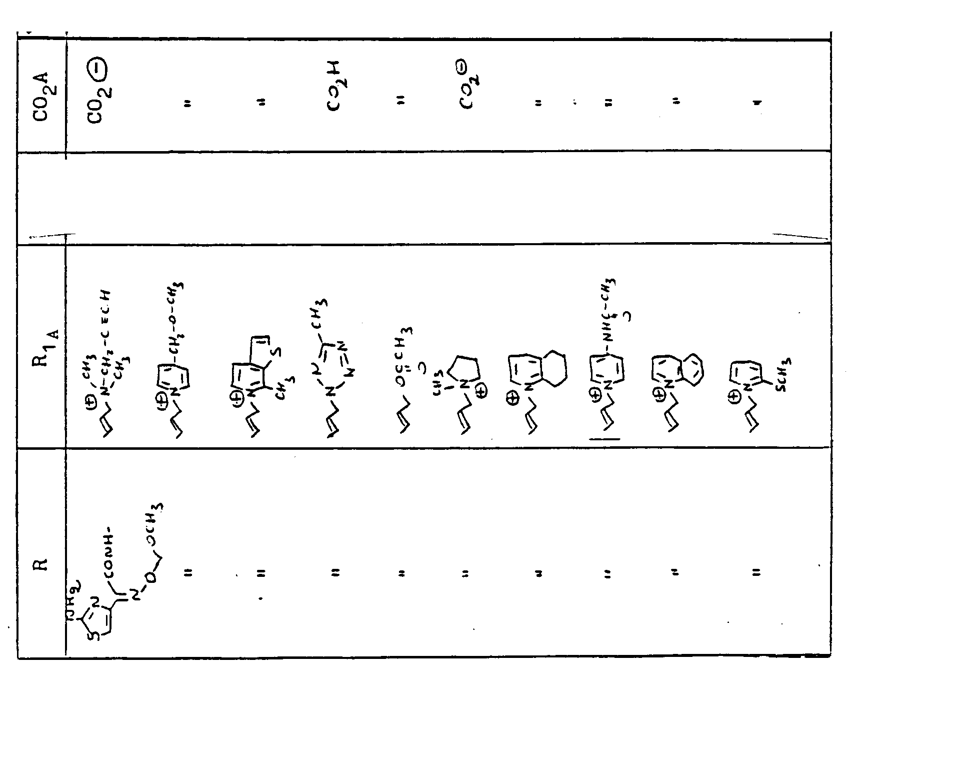

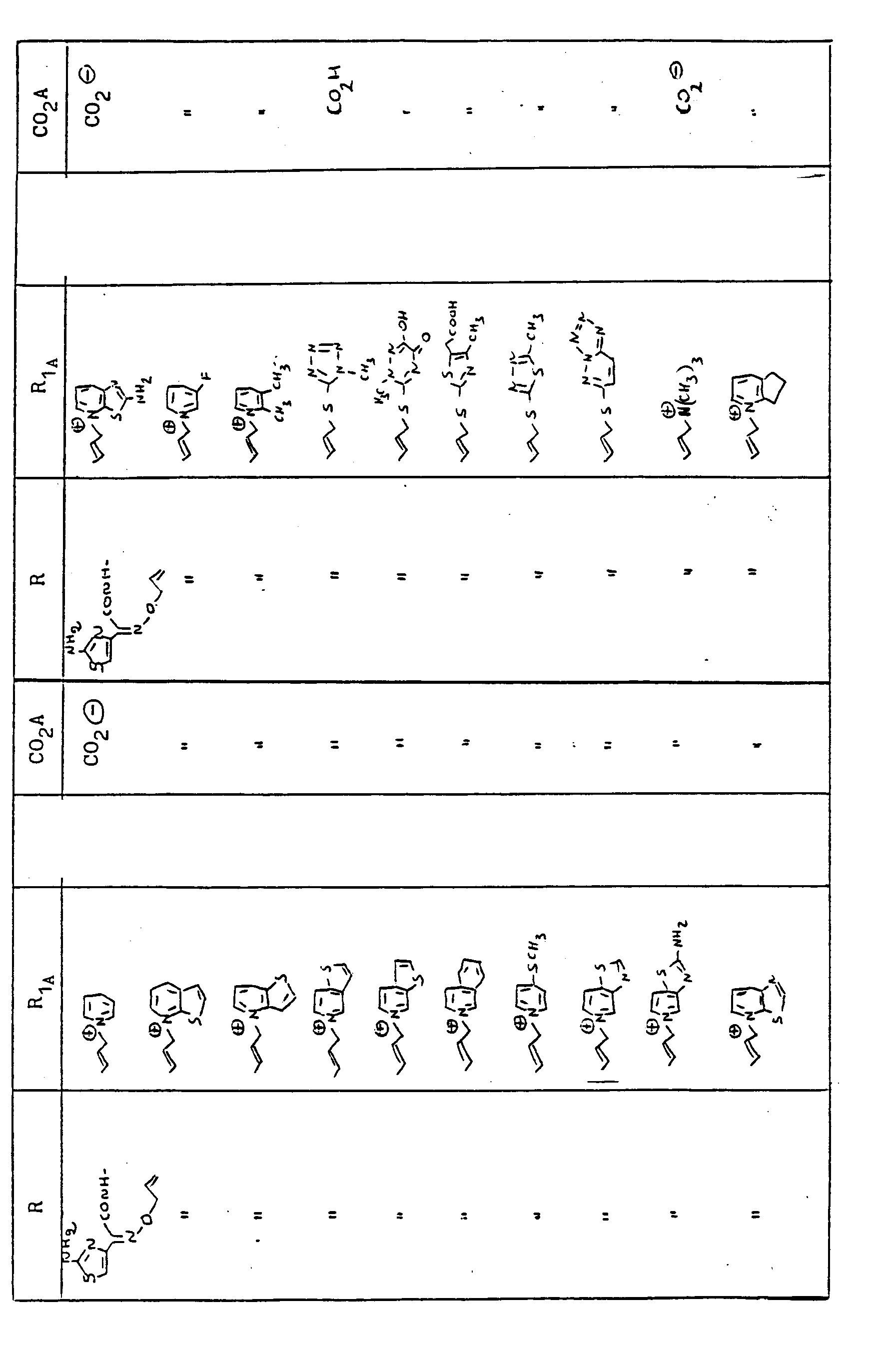

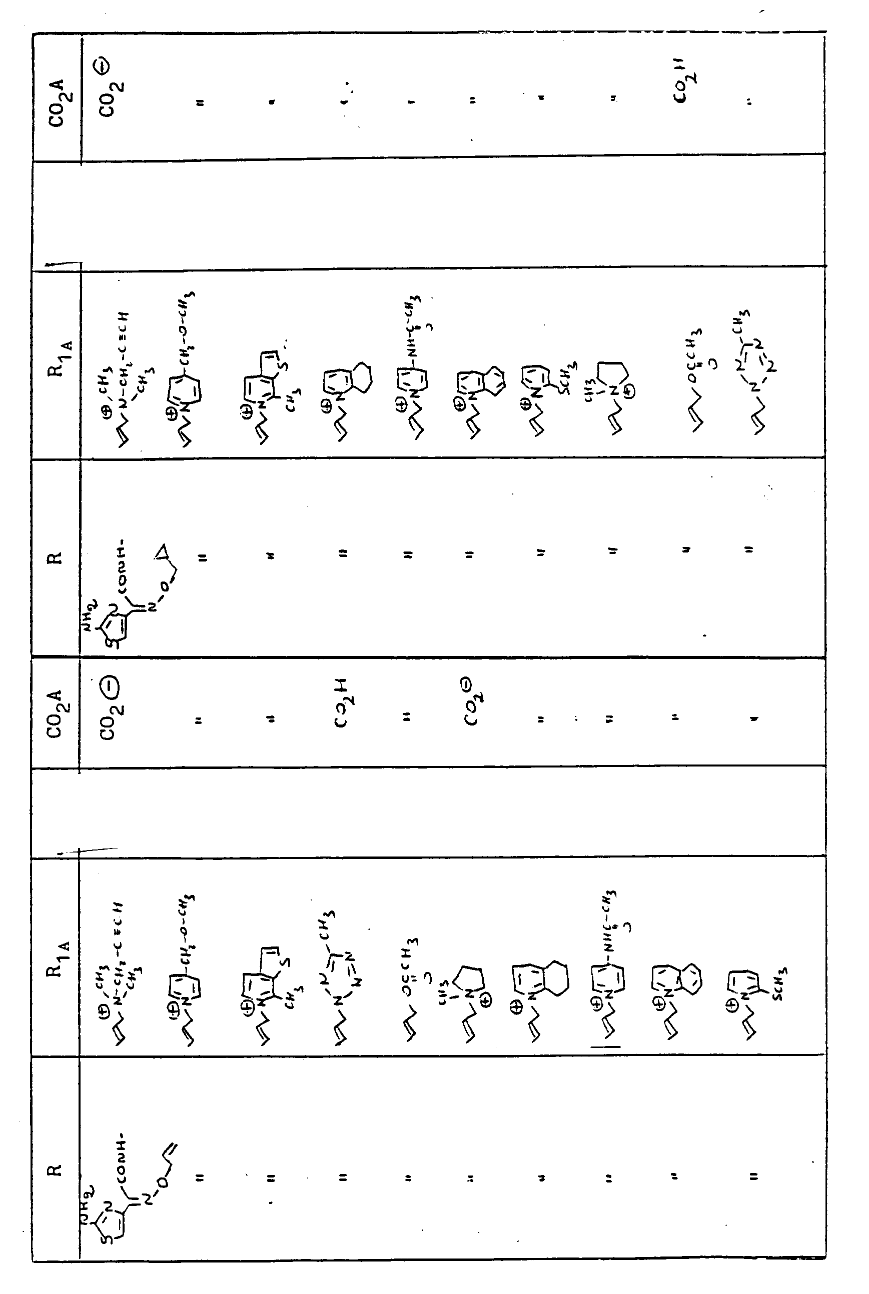

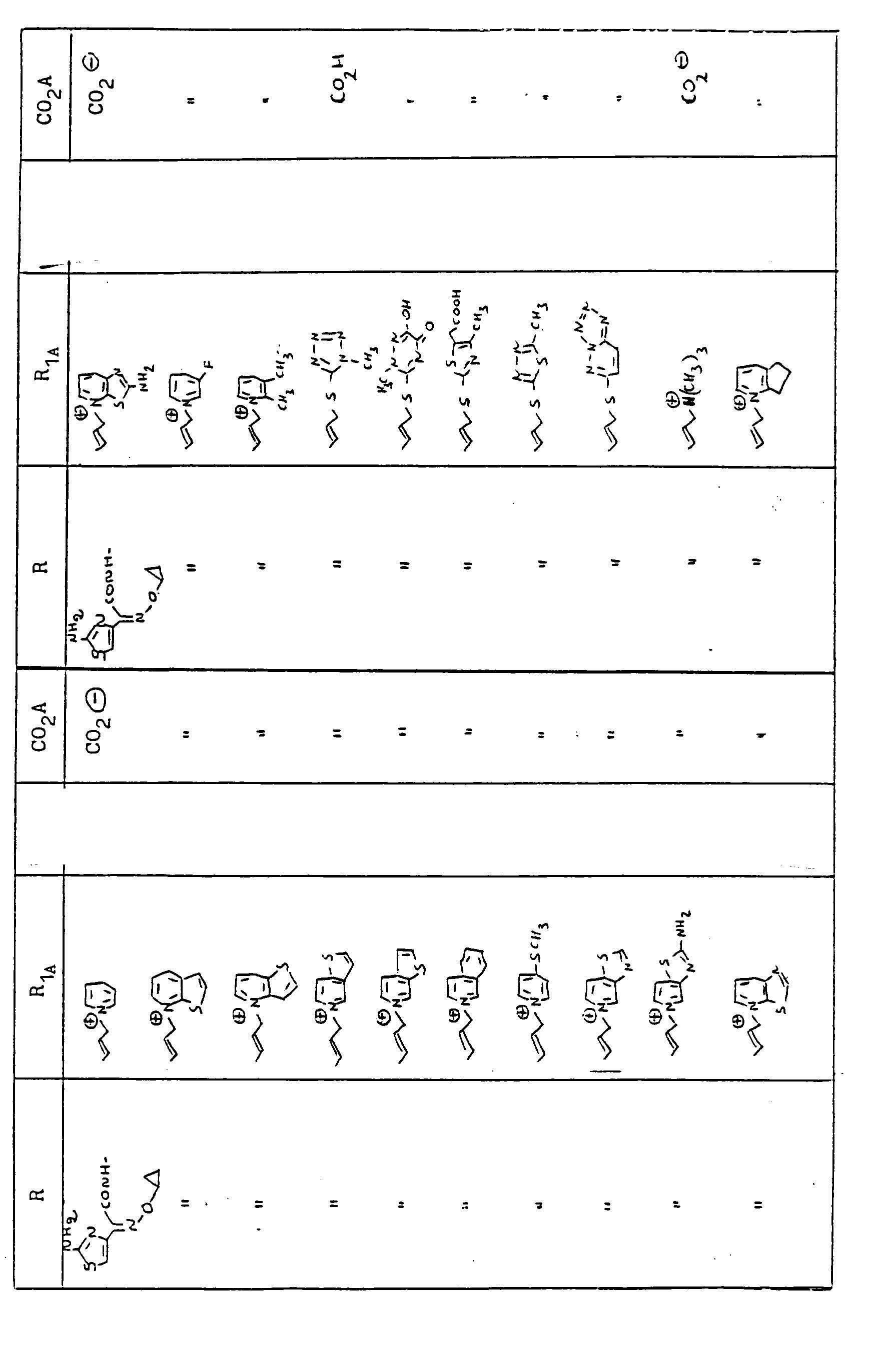

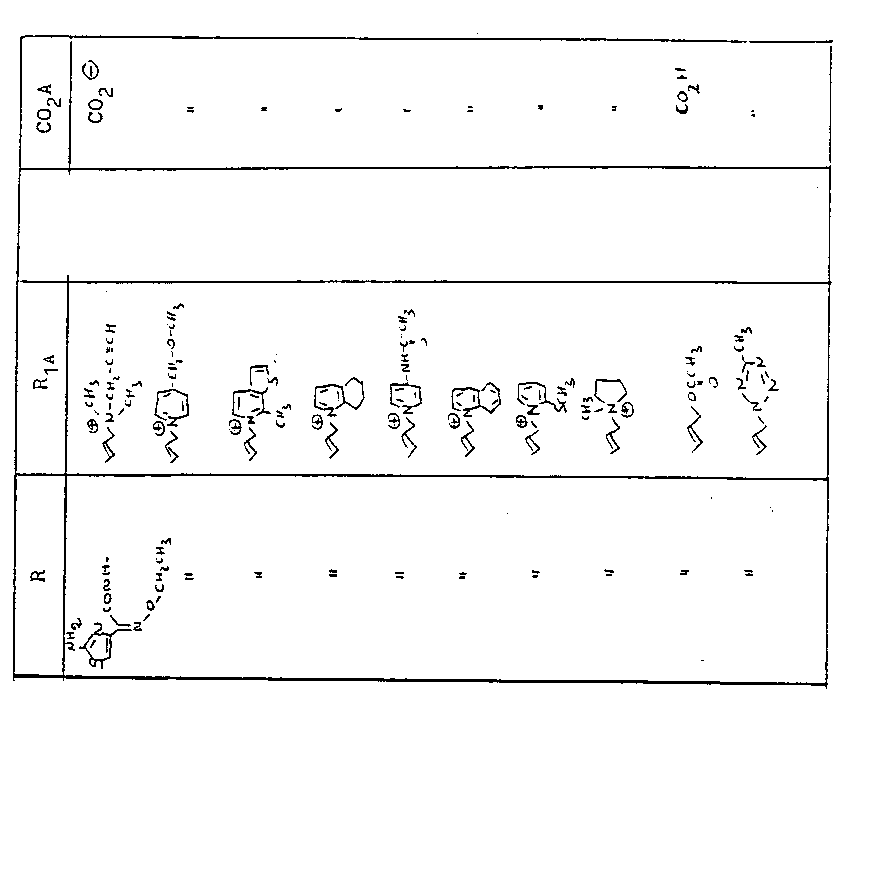

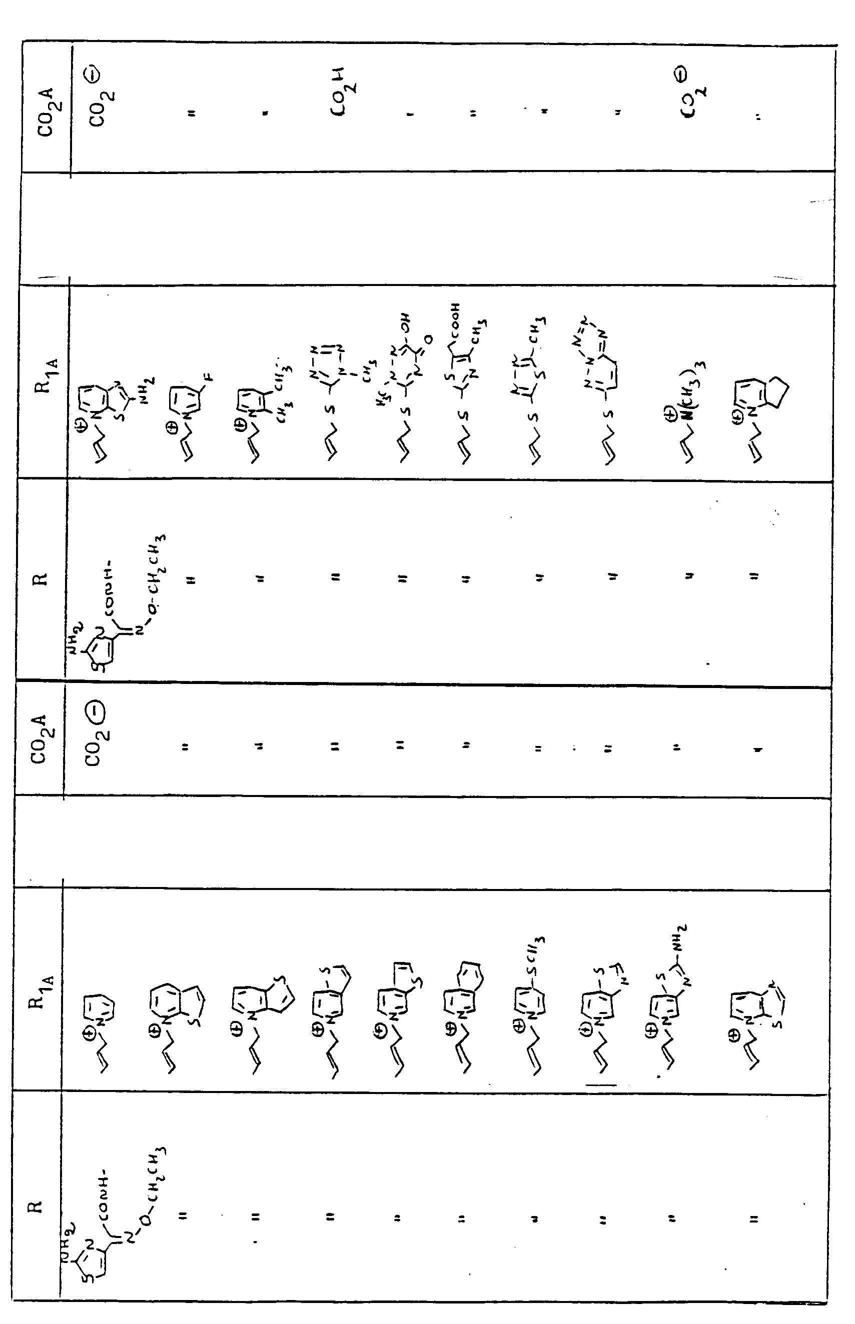

### EXEMPLE de REFERENCE A : Acide 7-/2-(2-aminothiazol-4-yl) 2-méthoxyimino acétamido/8-oxo 4-thia 3-méthoxyméthyl 1-azabicyclo /4-2-0/ oct-2-ène 2-carboxylique syn cis racémique.

### STADE A : méthoxyacétaldéhyde.

On porte au reflux 100 cm3 de diméthycétal du méthoxyacétaldéhyde, 100 cm3 d'eau et 3,2 cm3 d'acide chlorhydrique concentré puis effectue plusieurs distillations fractionnées et obtient 8,7 g de produit attendu.

### STADE B :2-chloro 3-méthoxyméthyl oxirane carboxylate de 1,1-diméthyl éthyle.

On refroidit à -20αC un mélange constitué de 2,106 g de méthoxyacétaldéhyde, 3,8 cm3 de dichloroacétate de terbutyle et 25 cm3 de tétrahydrodrofuranne. On introduit en 15 minutes 29 cm3 de terbutylate de potassium dans du tétrahydrofuranne (0,9 M/L) et laisse en contact 1 heure 20 mn. On ajoute 25 cm3 d'éther et 25 cm3 d'eau et extrait à l'éther. On lave la phase organique à l'eau saturée en chlorure de sodium, sèche et concentre à sec sous pression réduite. On chromatographie sur silice le résidu obtenu, élue au chlorure de méthylène et obtient 1,44 g de produit attendu.

STADE C :7-/2-(2-tritylamino thiazol-4-yl) 2-méthoxyimino acétamido/ 8-oxo 2-hydroxy 3-méthoxyméthyl 4-thia 1-azabicyclo /4-2-0/ octane 2-carboxylate de 1,1-diméthyléthyle.

On mélange 1,38 g de 2-chloro 3-méthoxyméthyl oxirane carboxylate de 1,1-diméthyléthyle, 2,736 g de 4-mercaptométhyl 3-/2-(2-tritylaminothiazol-4-yl) 2-méthoxyimino acétamido/ 2-oxo 1-azétidine cis syn racémique préparé selon le procédé décrit dans le brevet belge 894.795 et 12 cm3 de diméthylformamide. Après 10 minutes de contact, on ajoute 458 mg de carbonate de lithium et agite pendant 2 heures 50 minutes. On verse le mélange réactionnel dans 100 cm3 d'eau et 60 cm3 d'acétate d'éthyle. On extrait à l'acétate d'éthyle, lave la phase organique à l'eau, sèche et concentre à sec sous pression réduite. On reprend le résidu à l'éther et obtient 3,058 g de produit attendu.

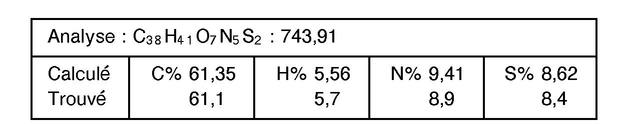

### STADE D : 7-/2-(2-tritylamino thiazol-4-yl) 2-méthoxy imino acétamido/ 8-oxo-3-méthoxy méthyl 4-thia 1-azabicyclo /4-2-0/ oct-2-ène 2-carboxylate de 1,1-diméthyléthyle.

On met en suspension 4,44 g de tétraiodure de diphosphore dans 35 cm3 de pyridine, agite 5 minutes et ajoute, en une seule fois, 3,058 g de 7-/2-(2-tritylamino thiazol-4-yl) 2-méthoxyimino acétamido/ 8-oxo 2-hydroxy 3-méthoxyméthyl 4-thia 1-azabicyclo /4-2-0/ octane 2-carboxylate de 1,1-diméthyléthyle et agite pendant 2 heures 40 minutes. On distille la pyridine, reprend le résidu avec 50 cm3 d'acétate d'éthyle, filtre, ajoute 50 cm3 d'acide chlorhydrique N au filtrat, agite vigoureusement et décante la phase organique, lave à l'eau, sèche, concentre à sec sous pression réduite. On chromatographie le résidu sur silice par un mélange chlorure de méthylène-acétate d'éthyle (85-15). On concentre à sec les fractions intéressantes, recristallise dans le méthanol et obtient 703 mg de produit attendu.

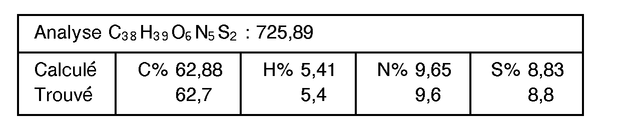

### STADE E : Acide 7-/2-(2-aminothiazol-4-yl) 2-méthoxyimino acétamido/ 8-oxo 4-thia 3-méthoxyméthyl 1-azabicyclo /4-2-0/ oct-2-ène 2-carboxylique syn cis racémique.

On dissout 246 mg de 7-/2-(2-tritylaminothiazol-4-yl) 2-méthoxyimino acétamido 8-oxo 3-méthoxyméthyl 4-thia 1-azabicyclo /4-2-0/ oct-2-ène 2-carboxylate de 1,1-diméthyléthyle dans 1 cm3 d'acide trifluoroacétique et laisse en contact pendant 50 minutes à température ambiante. On ajoute 12 cm3 d'éther isopropylique, filtre le précipité formé et obtient 176 mg de trifluoroacétate brut que l'on dissout dans l'éthanol. On ajoute 2 gouttes de pyridine, laisse cristalliser pendant 1 heure et obtient 88 mg de produit attendu.

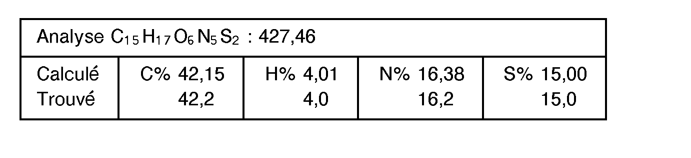

### EXEMPLE DE REFERENCE B : Acide 7-/2-(2-aminothiazol-4-yl) 2-méthoxyimino acétamido/ 8-oxo 3-/(1-méthyl 1 H tétrazol-5-yl) thio méthyl/ 4-thia 1-azabicyclo /4-2-0/ oct-2-ène 2-carboxylique syn cis racémique.

STADE A : 2-(1,1-diméthyl éthyle) diméthyl silyloxy/ éthanol.

A 20αC, on mélange sous azote :
- 18 g de chlorure de tertbutyl-méthyl silyle,
- 150 cm3 de dichloro méthane,
- 17,5 cm3 de diméthyl formamide,
- 33,6 cm3 d'éthylène glycol.

Après dissolution complète, on ajoute en 5 minutes, 20,1 cm3 de triéthylamine puis ajoute 1,8 g de 4-diméthyl amino pyridine. Après 2 h 45 d'agitation, on verse la solution dans 120 cm3 d'eau et neutralise avec de l'acide chlorhydrique N (environ 40 cm3) de manière à obtenir un pH de 3. On décante, réextrait la phase aqueuse avec 20 cm3 de pentane, puis on lave la phase organique avec 60 cm3 d'eau qu'on réextrait avec 20 cm3 de pentane. La phase oranique est séchée et distillée sous pressionréduite. Après rectification, on isole 13,9 g de produit attendu. Eb 16 mm/Hg = 82 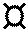 -86 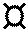 C

### STADE B : 2-/(1,1-diméthyl éthyl) diméthyl silyloxy/ acétaldéhyde.

Sous agitation et azote, on dissout 4,71 cm3 de chlorure d'oxalyle dans120 cm3 de dichlorométhane. On refroidit à -70 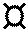 C et introduit en 12 minutes en maintenant la température à -65αC, une solution comprenant 8, cm3 de diméthylsulfoxyde et 26 cm3 de dichlorométhane. Puis après 10 minutes de contact à cette température, on introduit en 12 minutes la solution suivante à -65αC qui comprend 8,81 g de 2/(1,1-diméthyl éthyl) diméthyl silyloxy/ éthanol obtenu au stade A, 50 cm3 de dichlorométhane et 8,86 cm3 de pyridine. Après 15 minutes de contact à cette température, on ajoute en 8 minutes à 65αC, 35 cm3 de triéthylamine. A + 13αC, on neutralise par de l'acide chlorhydrique N, de manière à obenir un pH de 4. On décante, réextrait avec 50 cm3 de dichlorométhane, sèche la phase organique et distille sous pression réduite. Le produit brut est chromatographié sur silice en éluant au dichlorométhane. On isole finalement 7,95 g de produit attendu.

### STADE C : 2-chloro-3-(tertbutyl diméthyl silyloxy méthyl) oxirane carboxylate de 1,1-diméthyl éthyle.

On opère comme au stade B de l'exemple de référence A en prenant la précaution d'introduire en même temps les solutions de terbutylate de potassium et 7,95 g d'aldéhyde obtenu au stade B à -20 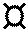 C, dans une solution de dichloroacétate de 1,1-diméthyl éthyle et obtient, après chromatographie sur silice (éluant : hexane-dichlorométhane 6-4), 9,4 g de produit attendu.

STADE D : 7-/2-(2-tritylaminothiazol-4-yl) 2-méthoxyimino acétamido/8-oxo 2-hydroxy 3-/(1,1-diméthyl éthyl) diméthyl silyloxy méthyl/4-thia 1-azabicyclo /4-2-O/ octane-2-carboxylate de 1,1-diméthyl éthyle.

On opère comme au stade C de l'exemple de référence A, avec un temps de réaction de 16 heures. Après chromatographie sur silice et élution au dichloromé-thane-acétate d'éthyle(75-25), on isole à partir de 8,31 g de 4-mercaptométhyl 3 /2-(2-tritylamino thiazol-4-yl) 2-méthoxyimino acétamido/2-oxol-azétidine cis syn racémique et du produit obtenu ci-dessus, 9,09 g de produit cyclisé.

STADE E : 7-/2-(2-tritylaminothiazol-4-yl) 2-méthoxyimino acétamido/8-oxo 3-/(1,1-diméthyl éthyl) diméthyl silyloxy méthyl/ 4-thia 1-azabicyclo /4-2-O/ oct-2-ène 2-carboxylate de 1,1-diméthyl éthyle.

On opère comme précédemment dans l'exemple de référence A, stade D. Le contact est réduit à 55 mn. Le melange réactionnel est versé dans l'eau, acidifié avec de l'acide chlorhydrique 2N jusqu'à pH 1,4 et extrait à l'acétate d'éthyle. En partant de 9,09 g de produit obtenu ci-dessus, on isole, après chromatographie, 4 g de produit attendu.

### Spectre UV

- dans l'éthanol infl : 233 nm E₁¹ : 364 infl : 265 nm E₁¹ : 173 max : 302 nm E₁¹ : 229 ∈ = 18900

### STADE F : 7-/2-(2-tritylamino thiazol-4-yl) 2-méthoxyimino acétamido/ 8-oxo3-hydroxy méthyl 4-thia 1-azabicyclo /4-2-O/ oct-2-ène 2-carboxylate de 1,1-diméthyl éthyle.

On met en suspension 2,595 g de dérivé silylé obtenu au stade E dans 30 cm3 d'acétone et 4,7 cm3 d'acide chlorhydrique N. La solution devient limpide peu à peu et après 3 heures d'agitation, on ajoute 7,7 cm3 d'eau bicarbonatée saturée et distille l'acétone sous pression réduite. On ajoute20 cm3 de dichloromé- thane, agite, décante, réextrait et sèche la phase organique et distille sous pression réduite. La gomme résiduelle est dissouteavec 5,5 cm3 d'acétate d'éthyle, auquel on ajoute 43 cm3 d'éther. Après 3 h 15 d'agitation, on filtre les cristaux formés, rince et sèche. On isole ainsi 2,232 g de produit attendu.

### STADE G : 7-/2-(2-tritylaminothiazol-4-yl) 2-méthoxyimino acétamido/8-oxo 3-chlorométhyl 4-thia 1-azabicyclo /4-2-O/ oct-2-ène 2-carboxylate de 1,1-diméthyl éthyle.

On dissout 623 mg d'alcohol obtenu au stade F dans 8 cm3 de dichlorométhane avec 834 mg de chlorure de tosyle et introduit en 20 minutes, la solution suivante comprenant 534 mg de 4-diméthyl amino pyridine et 5 cm3 de dichlorométhane. Après 1 heure d'agitation, on ajoute 2,2 cm3 d'acide chlorohydrique N, agite, décante, sèche la phase organique. Après distillation sous pression réduite, le résidu est chromatographiésur silice én éluant avec du dichlorométhane-acétate d'éthyle (9-1). Le produit attendu cristallise dans l'éther ; on en obtient 245 mg.

### Spectre UV

- dans EtOH infl : 224 nm E₁¹ : 441 ∈ = ₃₈₂₀₀ infl : 264 nm E₁¹ : 179 infl : 271 nm E₁¹ : 164 max : 306 nm E₁¹ : 222 ∈ = 19200

### STADE H : 7-/2-(2-tritylamino thiazol-4-yl) 2-méthoxyimino acétamido/8-oxo 3-/(1-méthyl 1 H tétrazol-5-yl) thiométhyl/ 4-thia 1-azabicyclo /4-2-0/ oct-2-ène 2-carboxylate de 1,1-diméthyl éthyle.

On dissout 301 mg du produit obtenu au stade G, 114 mg du sel de sodiumdu 1-méthyl 5-mercapto 1,2,3,4-tétrazole dans 3 cm3 de diméthylformamide.Aprèsl h 40 d'agitation, on verse la solution dans 30 cm3 d'eau etextrait à l'acétate d'éthyle. La phase organique est séchée et distillée sous pression réduite. La gomme obtenue est dissoute dans de l'acétate d'éthyle (2 cm3) et on ajoute de l'éther jusqu'à la limite de la solubilité. On filtre les cristaux obtenus après 45 minutes, rince à l'éther, sèche et obtient en 2 jets, 283 mg du dérivé cherché.

### STADE 1 : Acide 7-/2-(2-aminothiazol-4-yl) 2-méthoxyimino acétamido/ 8-oxo 3-/(1-méthyl 1 H tétrazol 5-yl) thiométhyl/ 4-thia 1-azabicyclo /4-2-0/ oct-2-ène 2-carboxylique.

On opère comme dans l'exemple A, stade E, mais le produit brut est dissout dans l'eau bicarbonatée et acidifiée à pH 4, puis filtré après avoirdistillé une grande partie de l'eau. A partir de 371 mg de produit obtenu au stade H, on isole 61 mg de produit attendu.

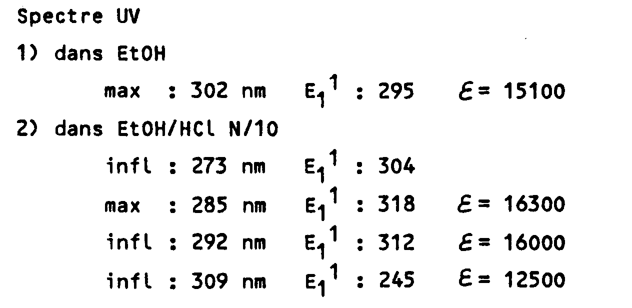

On prépare les produits optiquements actif correspondants et en particulier le 7-(2-(2-tritylamino thiazol-4-yl) 2 méthoxy imino acétamidio/8-oxo 3 hydroxyméthyl 4-thia 1-azabicyclo/4,2,0/ oct2 ène 2-carboxylate de 1,1-diméthyl éthyle syn 6S,7S en opérant comme à l'exemple de référence A en partant du 4- mércaptométhyl 3-/2-(2-tritylamino thiazol 4-yl) 2-méthoximino acétamido/2-oxo 1 azetidine cis syn 3,S,4 S décrite dans la demande française RF 2.538.389.

Le produit obtenu possède les caractéristiques physico-chimiques suivantes : F = 180αC. Ultra violet : 1) Dans l'éthanol : inf. : 222 nm E₁¹ = 444 inf. : 237 nm E₁¹ = 352 max. : 302 nm E₁¹ = 223 ; _{E} = 15900
2) Dans l'éthanol et Hcl 0,1 N
   inf. : 221 nm E₁¹ = 464
   inf. : 263 nm E₁¹ = 204
   inf. : 285 nm E₁¹ = 294
   max. : 293 nm E₁¹ = 340 _{E} = 22000
   inf. : 300 nm E₁¹ = 299
   inf. : 310 nm E₁¹ = 230

### EXEMPLE DE REFERENCE C : chlorure de/7//(2-triphénylméthylamino thiazol-4-yl) 2-(Z) méthoxyimino) acétyl/amino/2-/(1,1-diméthyl éthyl) oxy carbonyl 8-oxo 4-thia 1-azabicydo /4,2,0/ oct-ène 3yl/méthyl/triphényl phosphonium 6S,7S.

On dissout 2,182 g de 7/2(2-trilylamino thiazol-4-yl) 2-méthoxiimino acétamido/8-oxo 3-chlorométhyl 4-thia 1-aza bicyclo/4,2,0/oct-2ène 2-carboxylate de 1,1-diméthyl éthyle 6S,7S préparé comme au stade G de l'exemple de référence B et 1, 679 g de triphényl phosphine dans 24 cm3 de tétrahydrofuranne, ajoute 14,1 g de silice, distille le tétrahydrofuranne sous-pression réduite pendant 2 heures, refroidit, agite pendant 26 heures à 20 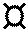 C, chromatographie sur silice (éluant dichlorométhane-méthanol 9.1) et obtient 1,89 de produit cherché.

Exemple 1 : Trifluorométhane sulfonate de 7-/3/7-//2-amino 4-thiazolyl) (méthoxy imino) acétyl/amino/ 2-carboxy 8-oxo 4-thia 1-azabicyclo/4.2.0/ oct-2-èn-3-yl/ propèn 2(E)-yl/thiéno /2,3-b/pyridinium 6S,7S syn.

### Stade A : 7-//2-(2-triphénylméthylamino thiazol-4-yl/2(Z) méthoxy imino acétyl/amino 3-/3-/(1,1-diméthyl éthyl)diméthyl silyl/oxy/ propèn-1-yl/8-oxo 4-thia 1-azabicyclo /4.2.0/ oct-2-èn 2-carboxylate de (1,1-diméthyl) éthyl 6S,7S.

On mélange 1,89 g de chlorhydrate de 7-//2-(2-triphénylméthylamino thiazol-4-yl/ 2(Z) méthoxy iminoacétyl/amino 3-méthyl triphényl phosphonium 8-oxo 4-thia 1-azabicyclo /4.2.0/ oct-2-èn 2-carboxylate de (1,1-diméthyl) éthyle, 30 cm3 de dichlorométhane, 0,68 cm3 de 2/1,1-diméthyléthyl diméthylsilyl oxy/ acétaldéhyde, 0,53 cm3 de triéthylamine, agite à + 20 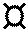 C pendant 14 heures, chromatographie la solution sur silice en éluant par un mélange de dichlorométhane et d'acétate d'éthyle (9/1) et isole 1,305 g de produit attendu contenant un mélange d'isomère E (2/3) et Z (1/3).

Spectre de RMN (CDCl₃) 0,90 - 0,92 ppm SitBu ; 1,50 - 1,53 ppm C0₂tBu ; 2,95 - 3,11 ppm CH₂S ; 4,05 ppm OCH₃ ; 4,31 - 4,37 ppm CH₂0 4,11 ppm H₆ ; 5,4 à 5.5 ppm H₇ 5,75 - 5,87 ppm et 6,11 - 6,24 ppm ΔZ(1/3) ; 6,17 - 6,34 ppm et 6,97 - 7,16 ppm E(2/3) ; 6,50 - 6,56 ppm H₅ thiazole syn ; 7,34 ppm 0₃.

### Stade B : 7-//2-(2-triphénylméthylamino thiazol-4-yl) 2(Z)méthoxy imino acétyl/ amino/ 3-/(3-hydroxy propèn-1 (E) yl) 8-oxo 4-thia 1 azabicyclo /4.2.0/ oct-2-èn 2-carboxylate de (1,1-diméthyl éthyle) et,

### 7-//2-(2-triphénylméthylamino thiazol-4-yl) 2(Z) méthoxy imino acétyl/ amino/ 3-/3-hydroxy propèn-1 (Z) yl/ 8-oxo 4-thia 1-azabicyclo /4.2.0/ oct-2-èn 2-carboxylate de (1,1-diméthyl éthyle).

On dissout 1,305 g de produit obtenu au stade A de l'exemple 1 dans 15 cm3 d'acétone, ajoute 2 cm3 de solution aqueuse normale d'acide chlorhydrique, agite pendant 2 heures et 30 minutes, concentre à sec par distillation sous pression réduite, ajoute de l'eau bicarbonatée, extrait au dichlorométhane, concentre à sec par distillation sous pression réduite, chromatographie le résidu sur silice en éluant par un mélange de dichlorométhane et d'acétate d'éthyle (1/1) et isole :
536 mg d'isomère E et,
369 mg d'isomère Z du produit attendu.
Contrôles de l'isomérie (E).
Spectre I.R. (chloroforme)
3607^{cm-1} OH ; 3405^{cm-1} amide NH ; 1684 ^{cm-1} C=0 ;
1515 ^{cm-1} amide II ; 1772 ^{cm-1} lactame C = ₀ ; 1595 ^{cm-1} ;
1544 ^{cm-1} ; 1527 ^{cm-1} : aromatique, thiazole, système conjugué ;
1700 ^{cm-1} ester terbutylique ; 1370 ^{cm-1} ; CH₃ ;
1154 ^{cm-1} C-0-C ; 2820 ^{cm-1} OMe ; 1049 ^{cm-1} C = N-OR.

### Spectre U.V. : éthanol.

Max. 231 nm E 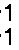 = 497 ∈ = 32 200.
Inflexion 258 nm E 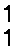 = 290 ;
Inflexion 264 nm E 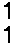 = 264 ;
Inflexion 270 nm E 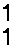 = 240 ;
Max. 320 nm E 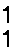 = 245 ; ∈ = 18 100.

### Spectre de RMN (CDCl₃)

1,51 ppm tBu ; 3,05 ppm CH₂S ; 4,02 ppm OCH₃ ; 4,28 ppm CH₂0H ; 5,48 ppm : H₇ ; 6,24 - 6,41 et 6,97 - 7,13 ppm H éthyléniques AE J = 15 Hz ; 7,29 ppm Ø₃ ; 6,53 ppm H₅ thiazole "syn".

Contrôles de l'isomère Z.

### Spectre I.R. (chloroforme)

3605 ^{cm-1} OH ; 3405 ^{cm-1} amide NH ; 1685 ^{cm-1} C=0 ;
1505 ^{cm-1} amide II ; 1773 ^{cm-1} βlactame C = 0
1704 ^{cm-1} ester terbutylique ; 1368 ^{cm-1} Me ; 11₅4 ^{cm-1} C-O-C ;
₁₅₈₅ ^{cm-1}, 1573 ^{cm-1}, 1527 ^{cm-1}, 1493 ^{cm-1}, trityle,
aromatiques, thiazole, système conjugué ; 2820 ^{cm-1} C = N-OMe ;
1050 ^{cm-1} C ₌ N-OR.

### Spectre U.V. (éthanol)

Inflexion 230 nm E 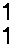 = 411 ; Inflexion 260 nm E 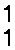 = 220 ;
Inflexion 265 nm E 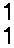 = 198 ; Inflexion 271 nm E 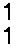 = 176 ;
Max. 308 nm E 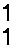 = 194 = 14 300.

### Spectre de RMN (CDCl₃)

1,49 ppm tBu ; 3,06 ppm S-CH₂ ; 4,04 ppm OMe ; 4,27 ppm CH₂0H ;
5,48 ppm H₇ ; 5,85 - 5,98 ppm et 6,21 - 6,34 ppm H éthyléniques
AZ J = 11 Hz ; 6,56 ppm H₅ thiazole syn ; 7,29 ppm Ø₃.

### Stade C : Trifluorométhane sulfonate de 7-/3-/7-//2-(2-triphénylméthyl amino thiazol-4-yl) 2(Z) méthoxy imino)acétyl/ amino/ 2-//1,1-diméthyl éthyl/ oxy/ carbonyl/ 8-oxo 4-thia 1-azabicyclo /4.2.0/ oct-2-èn 3-yl/ propèn 2(E) yl/ thiéno /2,3-b/ pyridinium 6S,7S.

On dissout 222 mg d'igomère E obtenu au stade B de l'exemple 1 dans 4,8 cm3 de dichlorométhane, ajoute 3 cm3 de solution comprenant 536 mg de thiéno pyridine pour 10 cm3 de chlorure de méthylène, refroidit à -70αC, ajoute goutte à goutte, 2,7 cm3 de solution d'anhydride trifluorométhane sulfonique comprenant 0,42 cm3 pour 10 cm3 de chlorure de méthylène, amène lentement à + 20 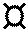 C, concentre à sec par distillation sous pression réduite, ajoute de l'acétate d'éthyle, lave à l'eau contenant 0,7 cm3 de solution aqueuse N d'acide chlorhydrique, décante, lave à l'eau, extrait à l'acétate d'éthyle, concentre à sec par distillation sous pression réduite, chromatographie le résidu sur silice en éluant par un mélange de dichlorométhane et de méthanol (92/8) et isole 236 mg de composé attendu (isomère E).

### Stade C' : Trifluorométhane sulfonate de 7-/3/7//(2-triphénylméthylamino thiazol-4-yl) 2(Z) méthoxy imino) acétyl/amino/ 2-//1,1-diméthyl éthyl/ oxy/ carbonyl/ 8-oxo 4-thia 1-azabicyclo /4.2.0/ oct-2-èn 3-yl/ propèn 2-(Z)-yl/ thiéno /2,3-b/ pyridinium 6S,7S.

On dissout 145 mg d'isomère Z obtenu au stade B de l'exemple 1 dans 5 cm3 de dichlorométhane, ajoute 120 I de thiéno /2,3-b/ pyridine, refroidit à -70 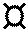 C, ajoute goutte à goutte 1,76 cm3 de solution d'anhydride trifluorométhane sulfonique dans le chlorure de méthylène comportant 0,42 cm3 d'anhydride pour 10 cm3 de chlorure de méthylène, amène lentement la température à + 20 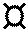 C, chromatographie la solution sur silice en éluant par un mélange de dichlorométhane et de méthanol (92/8) et isole 157 mg de composé attendu.

Stade D : Trifluorométhane sulfonate de 6-/3/7-//2-amino 4-thiazolyl) (méthoxy imino) acétyl/ amino/ 2-carboxy 8-oxo 4-thia 1-azabicyclo /4.2.0/ oct-2-èn 3-yl/ propèn 2(E)-yl/ thiéno /2,3-b/ pyridinium 6S,7S syn.

On mélange 221 mg d'isomère (E) obtenu au stade C de l'exemple 1, 2,5 cm3 d'acide formique à 33 % (solution aqueuse), chauffe à 65 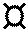 C pendant 55 minutes, refroidit, dilue à l'eau, filtre, lave à l'éther, concentre la phase aqueuse à sec par distillation sous pression réduite, ajoute de l'eau, essore, sèche et obtient 93 mg de produit attendu.

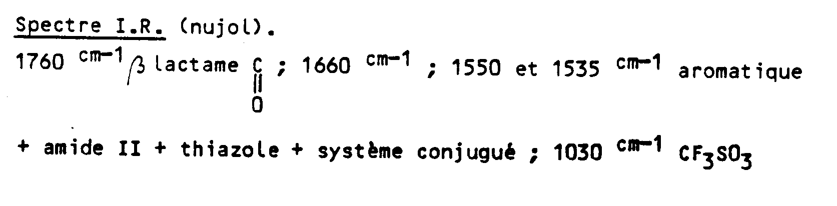

### Spectre U.V. (éthanol).

Max. 238 nm E 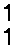 = 625 ∈ = 44.200
Max. 307 nm E 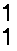 = 251 ∈ = 17.700
Inflexion 318 nm E 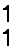 = 232
Inflexion 338 nm E 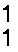 = 166

### Spectre de RMN (DMSO)

3,83 ppm : OCH₃; 5,55 à 6,44 ppm CH₂N^{⊕}; H₇; 1 H éthylénique;

6,81 ppm : H₅ thiazole; 7,23 ppm NH₂; 7,84 à 9,24 ppm aromatiques.

L'isomère Z par hydrolyse formique d'hydrolyse totalement en isomère E (isomérisation).

### Exemple 2 : Acide 7-//2-(2-amino thiazol 4-yl) 2(Z) méthoxy imino acétyl/ amino/ 3-//3-/1-méthyl 1 H. 1,2,3,4-tétrazol-5-yl) thio/ propèn (E) yl/ 8-oxo 4-thia 1-azabicyclo /4.2.0/ oct-2-èn 2-carboxylique 6S,7S syn.

### Stade A : /7-//2-(2-triphényl méthylamino thiazol 4-yl) 2(Z) méthoxy imino acétyl/ amino/ 3-//3-(1-méthyl 1 H, 1,2,3,4-tétrazol-5-yl) thio/ propèn (E) yl/ 8-oxo 4-thia 1-azabicyclo /4.2.0/ oct-2-èn 2-carboxylate de 1,1-diméthyl éthyle.

On mélange 37 mg de 7-//2-(2-triphényl méthyl amino thiazol-4-yl/ 2(Z) méthoxy imino acétyl/ amino/ 3- //(3-hydroxy) propèn 1(E) yl/8-oxo 4-thia 1-azabicyclo /4.2.0/ oct-2-èn 2-carboxylate de 1,1-diméthyl éthyle, 24 mg d'iodure de tétrabutyl ammonium, 0,8 cm3 de chlorure de méthylène, 23 µl de 2,6 lutidine, refroidit à -70αC, ajoute goutte à goutte 0,25 cm3 de solution d'anhydride trichlorométhane sulfonique titrant 0,42 cm3 d'anhydride pour 10 cm3 de chlorure de méthylène, ajoute 14 mg de sel de sodium de 1-méthyl 5-mercapto 1,2,3,4-tétrazole puis 0,5 cm3 de diméthyl formamide, agite à 20αC, concentre à sec, chromatographie le résidu sur silice en éluant par un mélange de chlorure de méthylène et d'acétate d'éthyle (9/1) et isole le produit attendu.

Stade B : Acide 7-//2-(2-amino thiazol 4-yl) 2(Z) méthoxy imino acétyl/ amino/ 3-//3-/1-méthyl 1 H, 1,2,3,4-tétrazol 5-yl) thio/ propèn (E) yl/ 8-oxo 4-thia 1-azabicyclo /4.2.0/ oct-2èn 2-carboxylique.

On mélange 329 mg de composé obtenu au stade A de l'exemple 2, 9,6 cm3 de solution aqueuse d'acide formique à 33 %, chauffe pendant une heure et 15 minutes à +65 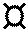 C, refroidit, dilue à l'eau, filtre, concentre à sec par distillation sous pression réduite, empâte le résidu dans l'eau, élimine par filtration l'insoluble formé, concentre à sec et isole 142 mg de composé attendu. ^{/α/}D = -99 (c = 0,8 % DMSO).

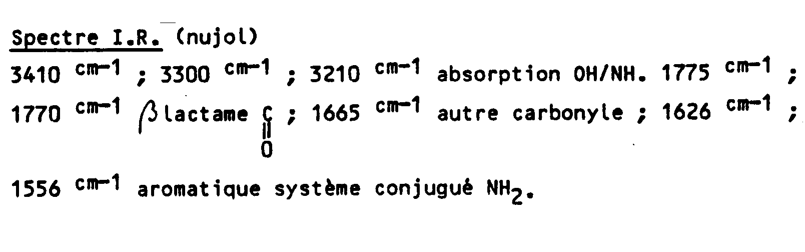

Spectre U.V. (éthanol HCI, 0,1 N).
Max. 239 nm E 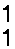 = 390 ∈ = 21 000 ;
Max.266 nm E 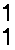 = 414 ∈ = 22 300 ;
Infl. 280 nm E 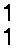 = 390 ;
Max. 329 nm E 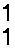 = 304 ∈ = 16 300.

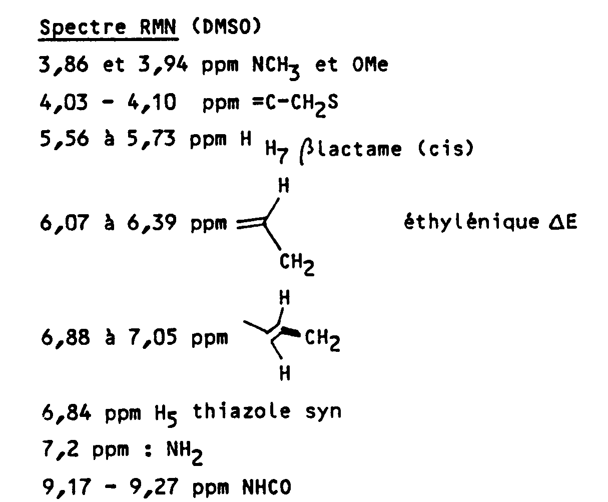

### Exemple 3 : Sel interne de 7-/3-/7-/2-(2-aminothiazol-4-yl) 2-(méthoxyimino) acétamido/ 2-carboxy 8-oxo 4-thia 1-azabicyclo /4.2.0./ oct-2-èn-3-yl/ propèn-2-(E)-yl/ thiéno /2,3-b/ pyridinium syn (6S) (7S).

On dissout 281 mg de produit obtenu au stade C de l'exemple 1 dans 3,17 cm3 d'acide formique à 66%, chauffe à 65 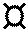 C pendant 2 heures. On dilue avec 3,2 cm3 d'eau, filtre et concentre à sec le filtrat sous pression réduite. On dissout le résidu dans 1,3 cm3 d'une solution aqueuse 1 M de carbonate de triéthylamine et 1,3 cm3 d'acétonitrile, chromatographie sur silice (éluant : eau-acétonitrile 9-1 puis 85-15). On obtient 77 mg de produit attendu.

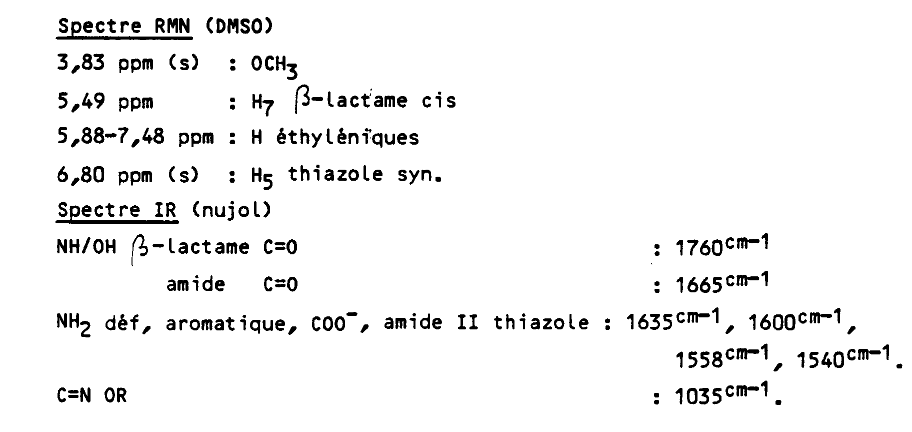

Exemple 4 : Trifluoroacétate de l'acide 7-/2-(2-aminothiazol-4-yl) 2-(méthoxyimino) acétamido/ 3-/(3-acétoxy)

### 1-(E)-propényl/8-oxo 4-thia 1-azabicyclo /4.2.0./ oct-2-èn-2-carboxylique syn (6S) (7S).

Stade A : 7-/2-(2-tritylaminothiazol-4-yl) 2-(Z)-méthoxyimino acétamido/ 3-/(3-acétoxy) propèn-1 (E)-yl/ 8-oxo 4-thia 1-azabicyclo /4.2.0./ oct-2-èn-2-carboxylate de 1,1-diméthyléthyle.

On agite 2 heures à température ambiante 300 mg de l'isomère E obtenu à l'exemple 1 stade B, 1,5 cm3 de pyridine et 0,75 cm3 d'anhydride acétique. On ajoute 20 cm3 d'eau et 10 cm3 d'acide chlorhydrique N et extrait au chlorure de méthylène. On obtient 395 mg de produit brut que l'on chromatographie sur silice en éluant par un mélange chlorure de méthylène-acétate d'éthyle (6-4). On recueille 255 mg de produit attendu. F = 170αC.

Stade B : Trifluoroacétate de l'acide 7-/2-(2-aminothiazol-4-yl) 2-(méthoxyimino) acétamido/ 3-/(3-acétoxy) 1-(E)-propényl/ 8-oxo 4-thia 1-azabicyclo /4.2.0./ oct-2-èn-2-carboxylique syn (6S) (7S).

On agite 55 minutes à température ambiante 210 mg de produit obtenu ci-dessus et 1,05 cm3 d'acide trifluoroacétique à 10% d'eau. On ajoute 5 cm3 d'éther et agite encore 1 heure, essore et obtient 133 mg de produit attendu.

### Spectre RMN (DMSO)

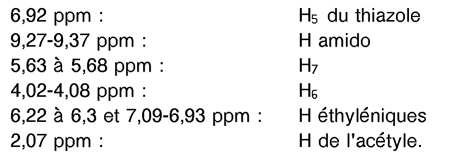

### Spectre IR (nujol)

Absorption région OH/NH β-lactame C = O : 1767 ^{cm-1}, 1740^{cm-1}, 1698^{cm-1}, 1667^{cm-1}, 1640^{cm-1}, Absorptions fortes : 1140^{cm-1}, 1250^{cm-1}.

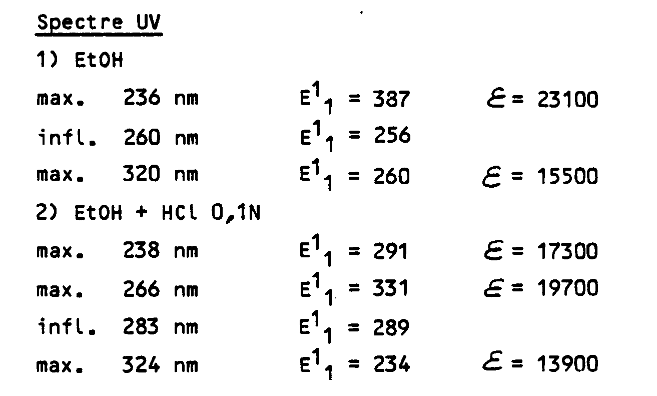

### Exemple 5 : Trifluorométhane sulfonate de 3-/7-/2-(2-aminothiazol-4-yl) 2-méthoxyimino acétamido/ 2-carboxy 8-oxo 4-thia 1-azabicyclo /4.2.0./ oct-2-èn-3-yl 2-(Z)-propényl triméthylamonnium syn (6S) (7S) et

### l'isomère (E) propényl.

### Stade A : Trifluorométhane sulfonate de 3-/7-/2-(2-tritylaminothiazol-4-yl) 2-méthoxyimino acétamido/ 2-/(1,1-diméthyléthoxy) carbonyl/ 8-oxo 4-thia 1-azabicyclo /4.2.0./ oct-2-èn-3-yl/ propèn-2-(E)-yl-trimétylammonium syn (6S) (7S) et l'isomère (propèn-2(Z)-yl).

On dissout 282 mg de 7-/2-(2-tritylaminothiazol-4-yl) 2-méthoxyimino) acétamido 3-/3-hydroxy 1 (E + Z) propényl/8-oxo 4-thia 1-azabicyclo /4.2.0./ oct-2-èn carboxylate de terbutyle dans 3 cm3 de chlorure de méthylène. On refroidit à -70αC et ajoute 1,6 cm3 d'une solution 1,53 mmole de triméthylamine. On ajoute goutte à goutte 0,094 cm3 d'anhydride trifluorométhane sulfonique et agite à -70αC pendant 30 minutes. On ajoute 7 cm3 de chlorure de méthylène et 5 cm3 d'acide chlorhydrique 0,1 N. On laisse le mélange se réchauffer, sépare la phae organique, la sèche et la chromatographie sur silice. Après élution par un mélange chlorure de méthylène-méthanol (95-5). On obtient les produits attendus 108 mg d'isomère Z (30 %), 117 mg d'isomère E (33%) et 53 mg de mélange E et Z.

### Stade B : Trifluorométhane sulfonate de 3-/7-/2-(2-aminothiazol-4-yl) 2-méthoxyimino acétamido/ 2-carboxy 8-oxo 4-thia 1-azabicyclo /4.2.0/ oct-2-èn-3-yl 2-(Z)-propényl triméthylammonium syn (6S) (7S) et l'isomère 2(E) propényl.

On dissout 108 mg de produit obtenu(isomère Z)dans 2 cm3 d'acide formique à 66% et agite pendant 2 heures à 65αC. On refroidit et dilue avec 2 cm3 d'eau; On lave le mélange réactionnel avec de l'éther puis du chlorure de méthylène. On évapore la phase aqueuse. On reprend le résidu 3 fois, avec 5 cm3 d'acétonitrile et 1 cm3 de méthanol On ajoute au résidu 5 cm3 de chlorure de méthylène, agite 1 heure, essore et obtient 55 mg de produit attendu.

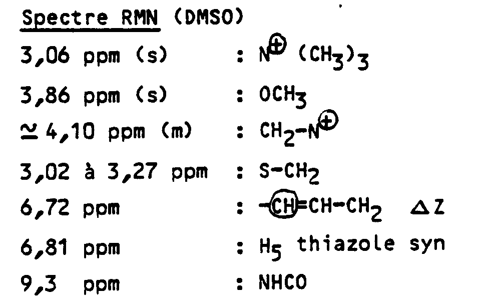

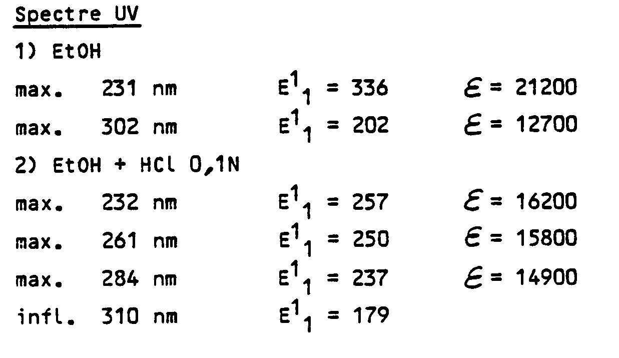

En partant de l'isomère (E) obtenu au stade A, on opère comme ci-dessus et obtient 56 mg de produit attendu.

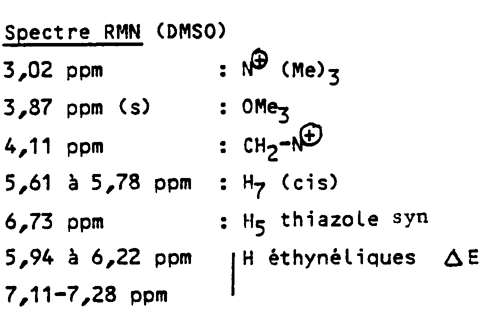

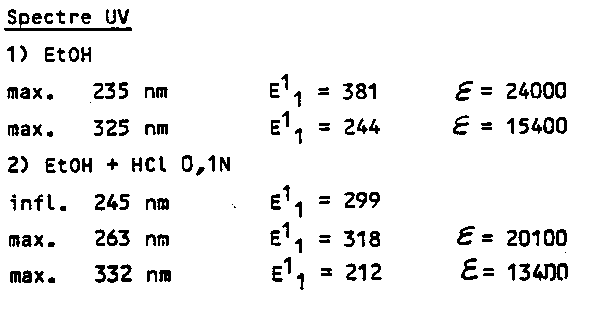

Exemple 6 : le produit suivant a également été obtenu par le procédé de l'invention

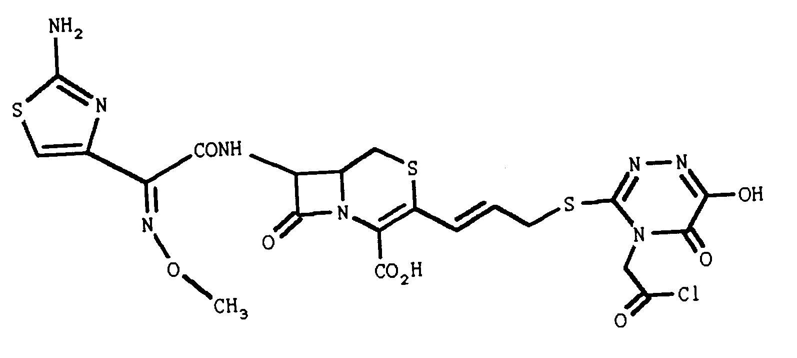

### Exemple 7 : Trifluorométhane sulfonate de 1-/3-/7-//2-(2aminothiazol-4-yl) 2-(méthoxyimino) acétamido/ 2-carboxy 8-oxo 4-thia 1-azabicyclo /4.2.0./ oct-2-èn-3-yl/ 2-(Z)-propényl/ 1,4-diazabicyclo octane (6S,7S) trifluoroacétate.

### Stade A : Trifluorométhane sulfonate de 7-/2-(2-tritylaminothiazol-4-yl) 2-(méthoxyimino) acétamido/ 3-/3-(1,4-diazabicyclo octane) 1(Z)-propényl/ 8-oxo 4-thia 1-azabicyclo /4.2.0./ oct-2-èn-carboxylate de 1,1-diméthyléthyle (6S) (7S).

On mélange 300 mg de 7-//2-(2-tritylaminothiazol-4-yl) 2-(méthoxyimino) acétamido/ 3-/3-hydroxy 1-(Z)-propényl/ 8-oxo 4-thia 1-azabicyclo /4.2.0./ oct-2-èn-carboxylate de 1,1-diméthyléthyle (6S) (7S), 6 cm3 de chlorure de méthylène et 179 mg de 1,4-diazabicyclo octane. On refroidit à -70αC et ajoute goutte à goutte 0,114 cm3 d'anhydride trifluorométhane sulfonate en agitant pendant 25 minutes à cette température. On laisse à -15αC pendant 15 minutes, agite puis verse 20 cm3 d'eau et 6 cm3 d'acide chlorhydrique 0,1N. On extrait au chlorure de méthylène, sèche et concentre à sec et obtient 350 mg de produit brut que l'on chromatographie sur silice, élue avec un mélange chlorure de méthylène-méthanol (9-1). On recueille 240 mg de produit attendu.

### Stade B : Trifluorométhane sulfonate de 1-/3-/7-//2-(2aminothiazol-4-yl) 2-(méthoxyimino) acétamido/ 2-carboxy 8-oxo 4-thia 1-azabicyclo /4.2.0./ oct-2-èn-3-yl/ 2-(Z)-propényl/ 1,4-diazabicyclo octane, (6S,7S) trifluoroacétate.

On agite 60 minutes 190 mg du produit obtenu ci-dessus et 1 cm3 d'acide trifluoroacétique à 10% d'eau. On précipite par 6 cm3 d'éther, filtre, sèche et obtient 146 mg de produit attendu. F = 190αC.

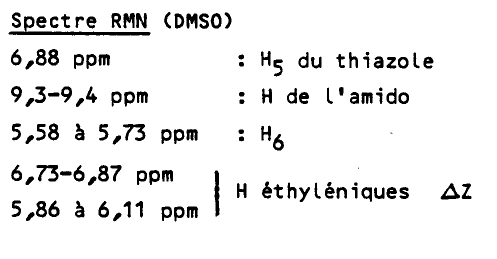

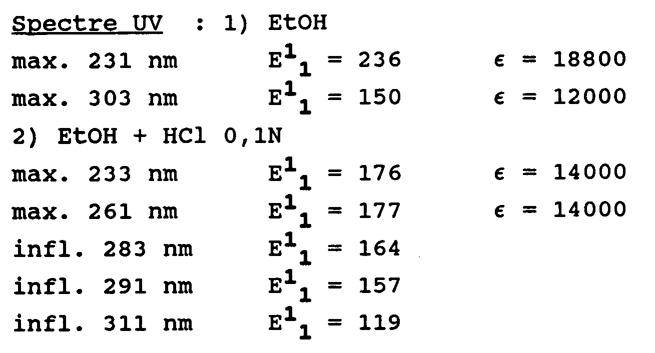

### Exemple 8 : Sel interne de 4-/3-/7-/2-(2-aminothiazol-4-yl) 2-(méthoxyimino) acétamido/ 2-carboxy 8-oxo 4-thia 1-azabicyclo /4.2.0./ oct-2-èn-3-yl/ 2-(E)-propényl/ 2-méthyl thiazolo /4.5-b/ pyridinium syn (6S)(7S).

### Stade A : Trifluorométhanesulfonate de 4-//7-(2-(2-tritylaminothiazol-4-yl) 2-(Z)-méthoxyimino) acétamido/ 2-(1,1-diméthyléthyloxycarbonyl/ 8-oxo 4-thia 1-azabicyclo /4.2.0./ oct-2-èn-3-yl/ 2-(E)-propényl/ 2-méthyl thiazolo /5,4-b/ pyridinium syn (6S)(7S).

On refroidit à -70 °C 350 mg d'isomère Z obtenu au stade B de l'exemple 1 dans 10 cm³ de chlorure de méthylène et 356 mg de méthylthiazolopyridine. On introduit 2,34 cm³ d'une solution constituée par 0,5 cm³ d'anhydride trifluorométhanesulfonique dans 10 cm³ de chlorure de méthylène. On agite 5 minutes à -70 °C sous azote puis laisse remonter la température à 20°C, dilue au chlorure de méthylène, lave à l'eau, sèche et amène à sec sous pression réduite. On chromatographie le résidu sur silice (éluant : chlorure de méthylène-acétone 9-1 puis chlorure de méthylène-méthanol 92-8). On obtient 347 mg de produit attendu.

### Stade B : Sel interne de 4-/3-/7-/2-(2-aminothiazol-4-yl) 2-(méthoxy imino) acétamido/ 2-carboxy 8-oxo 4-thia 1-azabicyclo /4.2.0./ oct-2-èn-3-yl/ 2-(E)-propényl/ 2-méthyl thiazolo /5,4-b/ pyridinium syn (6S)(7S).

On opère comme à l'exemple 3 à partir de 330 mg de produit obtenu ci-dessus. On élue par de l'eau distillée contenant 5 % d'acétonitrile puis 10 % enfin 20 %. On évapore l'acétonitrile, reprend le résidu dans 1 cm³ d'acétone, isole et rince avec un minimum d'acétone par centrifugation et obtient 98 mg de produit attendu.

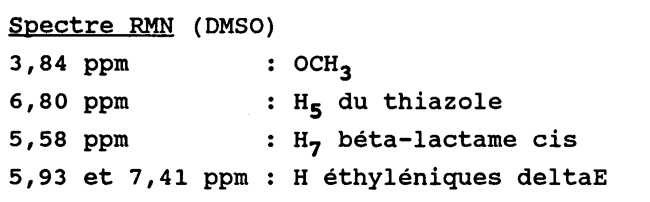

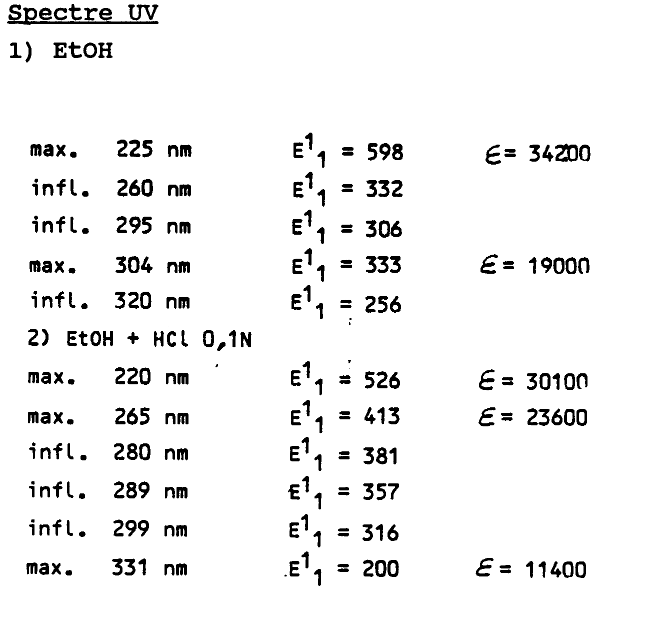

### Exemple 9 : Sel interne de 1-/3-/7-/2-(2-aminothiazol-4-yl) 2-méthoxyimino acétamido/ 2-carboxy 8-oxo 4-thia 1-azabicyclo /4.2.0./ oct-2-èn-3-yl/ 2-(E)-propényl/ 5,6,7,8-tétrahydroquinoléinium syn (6S)(7S).

### Stade A : Trifluorométhane sulfonate de 1-/3-/7-/2-(2-tritylaminothiazol-4-yl) 2-méthoxyimino acétamido/ 2-(1,1-diméthyléthyloxycarbonyl) 8-oxo 4-thia 1-azabicyclo /4.2.0./ oct-2-èn-3-yl/ 2-(E)-propényl/ 5,6,7,8-tétrahydroquinoléinium syn (6S)(7S).

On opère comme au stade A de l'exemple 8 en utilisant 162 µl de 2,3-cyclohexénopyridine et obtient 73 mg de produit attendu.

### Stade B : Sel interne de 1-/3-/7-/2-(2-aminothiazol-4-yl) 2-méthoxyimino acétamido/ 2-carboxy 8-oxo 4-thia 1-azabicyclo /4.2.0./ oct-2-èn-3-yl/ 2-(E)-propényl/ 5,6,7,8-tétrahydroquinoléinium syn (6S)(7S).

On dissout 136 mg de produit obtenu comme ci-dessus dans 1,5 cm3 d'acide formique à 66% et opère comme à l'exemple 3 pour obtenir 25 mg de produit attendu.

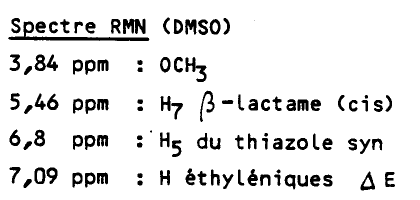

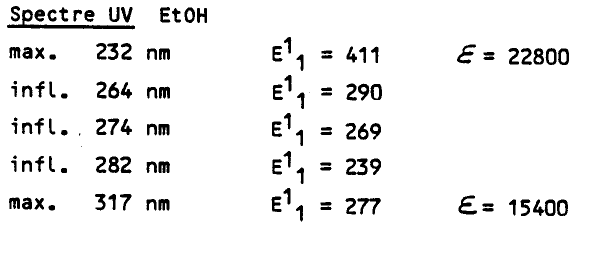

### Exemple 10 : Sel interne de 7-/3-/7-/2-(2-aminothiazol-4-yl) 2-difluorométhoxyimino acétamido/ 2-carboxy 8-oxo 4-thia 1-azabicyclo /4.2.0./ oct-2-èn-3-yl/2-(E)-propényl/ thiéno /2,3-b/ pyridinium syn (6S)(7S).

On opère comme à l'exemple 5 à partir de 2,527 g de 7-/2-(2-trityl aminothiazol-4-yl) 2-(Z)-difluorométhoxyimino acétamido/ 3-(3-hydroxy (E + Z) propèn-1-yl) 8-oxo 4-thia 1-azabicyclo /4.2.0./oct-2-èn-3-yl 2-carboxylate de 1,1-diméthyléthyle préparé selon les méthodes habituelles et 1,89 cm3 de thiéno /2,3-b/ pyridine et obtient après hydrolyse à l'acide formique 813 mg de produit attendu. /α/_{D} = 132,5α ± 2 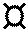 (c = 1% dans mélange H₂0-CH₃CN (1-1).

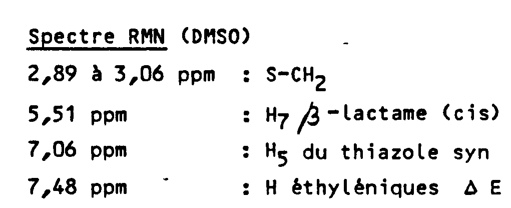

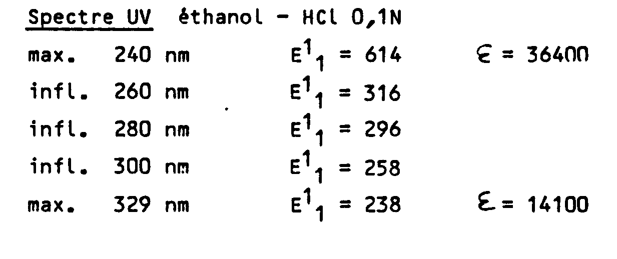

### Exemple 11 : Sel interne de 4-/3-/7-/2-(2-aminothiazol-4-yl) 2-difluorométhoxyimino acétamido/ 2-carboxy 8-oxo 4-thia 1-azabicyclo /4.2.0./ oct-2-èn-3-yl/ 2-(E)-propényl/ thiéno /3,2-b/ pyridinium (6S)(7S).

On opère comme à l'exemple 5 à partir de 300 mg de 7-/2-(2-tritylaminothiazol-4-yl) 2-(Z)-difluorométhoxyimino acétamido/ 3-/3-hydroxy (E) propén-1-yl 8-oxo 4-thia 1-azabicyclo /4.2.0./ oct-2-èn-3yl 2-carboxylate de 1,1-diméthyléthyle et de 210 mg de thiéno /3,2-b/ pyridine. Après traitement à l'acide formique puis à l'acide trifluoroacétique, on obtient 117 mg de produit que l'on reprend par 0,5 cm3 d'acétonitrile et 0.5 cm3 de carbonate de triéthylamine, chromatographie sur silice, élue par un mélange acétonitrile à 5% d'eau puis 10% et enfin 15% d'eau. On obtient 43 mg de produit attendu.

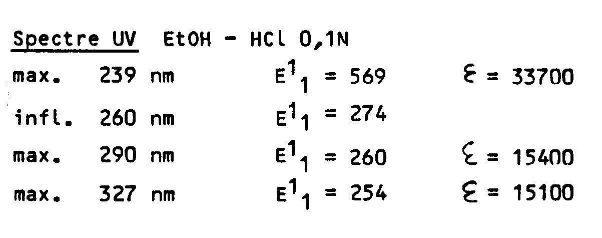

### Exemple 12 : Trifluorométhane sulfonate de 7-/3-/7-/2-(2-aminothiazol-4-yl)2-(propényloxyimino) acétamido/ 2-carboxy 8-oxo 4-thia 1-azabicyclo /4.2.0/ oct-2-èn-3-yl/ 2(E)-propényl thiéno /2,3-b/ pyridinium syn (6S)-(7S).

On opère comme à l'exemple 5 à partir de 160 mg de 7-/2-(2-trityl-aminothiazol-4-yl) 2-(Z)-propény- loxymino acétamido 3-(3-hydroxy (E) propényl) 8-oxo 4-thia 1-azabicyclo /4.2.0/ oct-2-èn-3-yl/ 2-carboxylate de 1,1-diméthyléthyle et de 2,2 cm3 d'une solution de thiénylpyridine (266 mg/5 cm3 de CH₂CI₂). Après hydrolyse à l'acide formique, on obtient 53 mg de produit attendu.

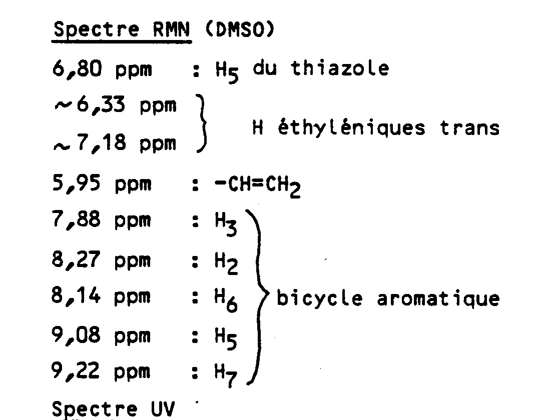

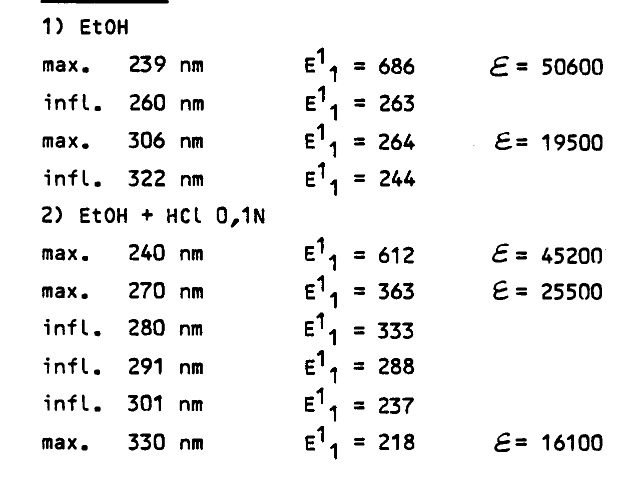

### Exemple 13: Acide 7-//2-(2-aminothiazol-4-yl) 2-méthoxyiminoacétyl/ amino/ 3-/3/5-(méthyl) 1,3,4-thiadiazol-2-yl/ thio/ 1-(E)-propényl/ 8-oxo 4-thia 1-azabicyclo /4.2.0./ oct-2-èn-2-carboxylique syn (6S) (7S).

### Stade A: 7-//(2-triphénylméthylaminothiazol-4-yl) 2-(méthoxyimino) acétylamino/ 3-/3-//5-(méthyl) 1,3,4-thiadiazol-2-yl/ thio/ 1-(E)-propényl/ 8-oxo 4-thia 1-azabicyclo /4.2.0./ oct-2-èn carboxylate de 1,1-diméthyléthyle syn (6S) (7S).

On refroidit à -70αC, 184,5 mg de l'isomère E obtenu au stade B de l'exemple 1 dans 4 cm3 de chlorure de méthylène anhydre, 120 mg d'iodure de tétrabutylammonium et 116 µl de 2,6-lutidine. On ajoute goutte à goutte 620 µl d'une solution (0,609 M/I) d'anhydride de trifluorométhane sulfonique dans du chlorure de méthylène. Après 10 minutes à -70αC, on laisse remonter la température à 20αC, évapore le solvant sous pression réduite et ajoute successivement en agitant 66,1 mg de 5-méthyle 1,3,4-thiadiazole, 2,5 cm3 de diméthylformamide et 104 mg de carbonate de potassium anhydre. On agite une demi-heure à 20-25 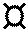 C et verse sur 10 cm3 d'acide chlorhydrique N et extrait à l'acétated'éthyle. On lave avec une solution aqueuse à 5% de bicarbonate de soude, puis à l'eau, sèche et évapore à sec sous pression réduite. On chromatographie le résidu sur silice, élue par un mélange acétate d'éthyle-chlorure de méthylène (15-85) et recueille 147 mg de produit attendu.

### Stade B : Acide 7-//2-(2-aminothiazol-4-yl) 2-méthoxyiminoacétyl/ amino/ 3-/3/5-(méthyl) 1,3,4-thiadiazol-2-yl/ thio/ 1-(E)-propényl/ 8-oxo 4-thia 1-azabicyclo /4.2.0./ oct-2-èn-2-carboxylique syn (6S) (7S).

On agite 20 minutes à températue ambiante 118 mg de produit obtenu ci-dessus et 2,3 cm3 d'acide trifluoroacétique. On ajoue goutte à goutte 25 cm3 d'éther anhydre, centrifuge, reprend le culot avec 5 cm3 d'éther 2 fois consécutives en centrifugeant à chaque fois puis sèche sous pression réduite. On reprend le produit dans 1,5 cm3 d'éthanol et 100 µl d'une solution (1M/I) de pyridine dans l'éthanol, centrifuge, reprend 2 fois le culot avec 1 cm3 d'éther en centrifugeant à chaque fois. Après séchage, on obtient 52 mg de produit attendu.

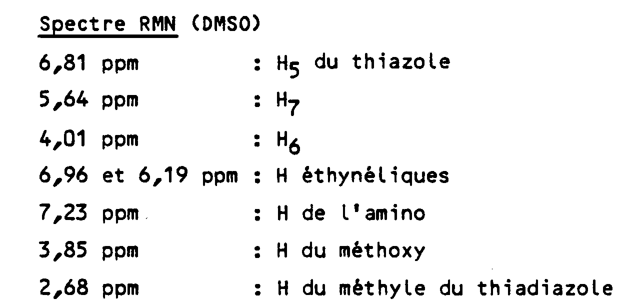

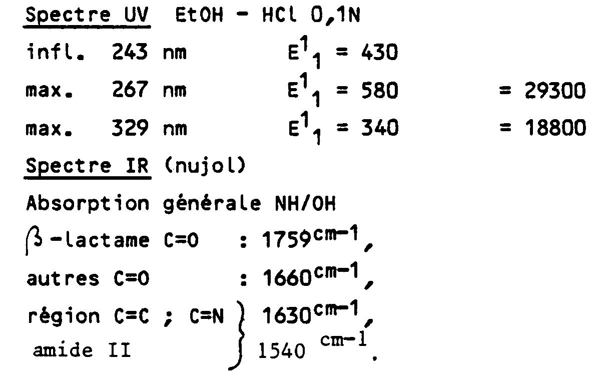

### Exemple 14 : Acide 7-/2-(2-aminothiazol-4-yl) 2-(Z)-(méthoxyimino) acétamido/ 3-/3-(1H-tétrazol-1-yl)1-(E)-propényl/8-oxo 4-thia 1-azabicyclo /4.2.0./ oct-2-èn-2-carboxylique (6S)(7S) et acide 7-/2-(2-aminothiazol-4-yl) 2-(Z)-(méthoxyimino) acétamido/ 3-/3-(2H-tétrazol-2-yl) 1-(E)-propényl/ 8-oxo 4-thia 1-azabicyclo /4.2.0./

### oct-2-èn-2-carboxylique (6S)(7S).

### Stade A : 7-/2-(2-tritylaminothiazol-4-yl) 2-(Z)-(méthoxyimino) acétamido/ 3-/3-(1H-tétrazol-1-yl) 1-(E)-propényl/ 8-oxo 4-thia 1-azabicyclo /4.2.0./ oct-2-èn-2-carboxylate de 1,1-diméthyléthyle et 7-/2-(2-tritylaminothiazol-4-yl) 2-(Z)-(méthoyximino) acétamido/ 3-/3-(2H-tétrazol-2-yl) 1-(E)-propényl/ 8-oxo 4-thia 1-azabicyclo /4.2.9./ oct-2-èn-2-carboxylate de 1,1-diméthyléthyle.

On dissout 230 mg de l'isomère obtenu au stade B de l'exemple 1, 149 mg d'iodure de tétrabutylammonium, 0,143 cm3 de 2,6-lutidine dans 5 cm3 de dichloroéthane. On refroidit à -70αC et ajoute goutte à goutte 1,7 cm3 d'une solution d'anhydride trifluorométhyl sulfonique (0,42 cm3 q.s.p. 10 cm3 CH₂CI₂). On laisse la température revenir à 20αC, évapore le solvant et ajoute 44 mg de 1 H- tétrazole, 131 mg de carbonate de potassium et 3 cm3 de diméthylformamide. Après 1 heure et 10 minutes d'agitation, on verse la solution dans l'eau contenant 1,5 cm3 d'acide chlorhydrique 2N et extrait à l'acétate d'éthyle. On lave la phase organique avec de l'eau contenant le thiosulfate 0,2 N en excès, sèche, concentre à sec et chromatographie le résidu sur silice, élue avec un mélange chlorure de méthylène-acétate d'éthyle d'abord (75-25) puis (50-50). Onisole 90 mg de l'isomère (position 2) et 99 mg de l'isomère (position 1).

### Stade B : Acide 7-/2-(2-aminothiazol-4-yl) 2-(Z)-(méthoxyimino) acétamido/ 3-/3-(1H-tétrazol-1-yl) 1-(E)-propényl/ 8-oxo 4-thia 1-azabicyclo /4.2.0./ oct-2-èn-2-carboxylique (6S)(7S).

On dissout les 99 mg de l'isomère (position 1) dans 1,2 cm3 d'acide formique 66%. On chauffe à 65 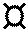 C pendant 2 heures, refroidit, dilue avec 1,5 cm3 d'eau, filtre, concentre à sec le filtrat, empâte le résidu dans l'eau, filtre, rince à l'eau, puis à l'éther et obtient 49 mg de produit attendu.

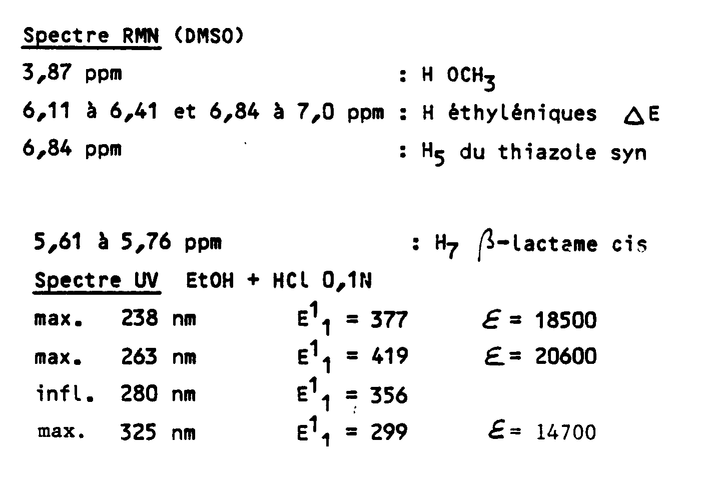

### Stade C : Acide 7-/2-(2-aminothiazol-4-yl) 2-(Z)-(méthoxyimino) acétamido/ 3-/3-(2H-tétrazol-2-yl) 1-(E)-propényl/ 8-oxo 4-thia 1-azabicyclo /4.2.0./ oct-2-èn-2-carboxylique (6S)(7S).

On opère comme au stade B à partir des 90 mg d'isomère (position 2) et obtient 31 mg de produit attendu.

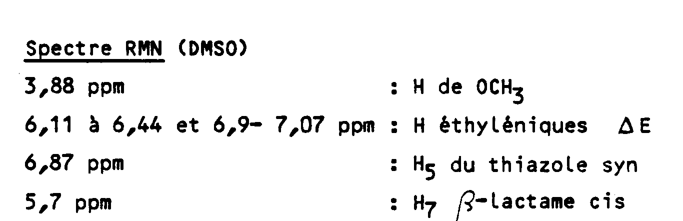

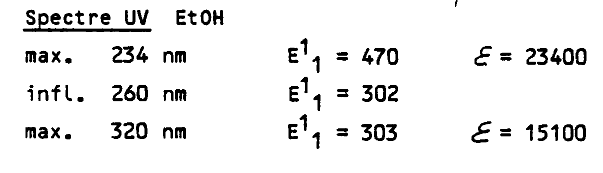

### Exemple 15 : Acide 7-/2-(2-aminothiazol-4-yl) 2-(méthoxyimino) acétamido/ 3-/3-(2-méthyl-1H-imidazol-1-yl) 1-(E)-propényl/8-oxo 4-thia 1-azabicyclo /4.2.0./ oct-2-èn-2-carboxylique syn (6S)(7S).

### Stade A : 7-/2-(2-tritylaminothiazol-4-yl) 2-(méthoxyimino) acétamido 3-/3-(2-méthylimidazol-1-yl) 1-(E)-propényl/ 8-oxo 4-thia 1-azabicyclo /4/2/0./ oct-2-èn-2-carboxylate de 1,1-diméthyléthyle syn (6S)(7S).

On opère comme au stade A de l'exemple 13 à partir de 62 mg de 2-méthylimidazole et obtient 82 mg de produit attendu.

### Stade B : Acide 7-/2-(2-aminothiazol-4-yl) 2-(méthoxyimino) acétamido/ 3-/3-(2-méthyl-1 H-imidazol-1-yl) 1-(E)-propényl/ 8-oxo 4-thia 1-azabicyclo /4.2.0./ oct-2-èn-2-carboxylique syn (6S)(7S).

On chauffe 3 heures à 60-65 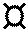 C, 80,5 mg de produit obtenu ci-dessus et 1 cm3 d'acide formique à 66%, évapore à sec à 30 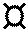 C sous pression réduite, reprend 2 fois consécutives le résidu avec 5 cm3 d'eau, évapore à sec. On reprend le résidu par 7 cm3 d'éther, agite une demi-heure, essore et obtient 41,5 mg de produit attendu.

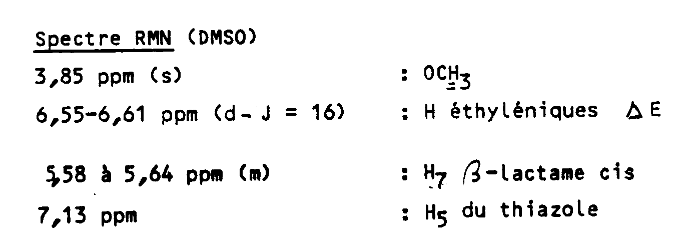

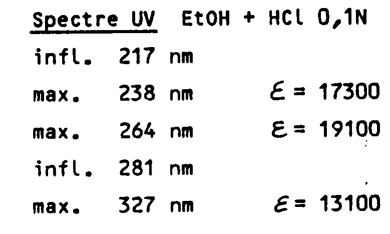

### Exemple 16 : Acide 7-/2-(2-aminothiazol-4-yl) 2-(méthoxyimino) acétamido/ 3-//(5-méthylthio) 1,3,4-thiadiazol-2-yl/ thio/ 1-(E)-propényl/8-oxo 4-thia 1-azabicyclo /4.2.0./ oct-2-èn-2-carboxylique syn (6S)(7S).

On opère comme à l'exemple 13 en utilisant le 2-mercapto 5-méthylthio 1,3,4-thiadiazole en éluant la chromatographie avec un mélange chlorure de méthylène-acétate d'éthyle (95-5) puis (9-1). Après déblocage des fonctions et purification, on obtient 69 mg de produit attendu.

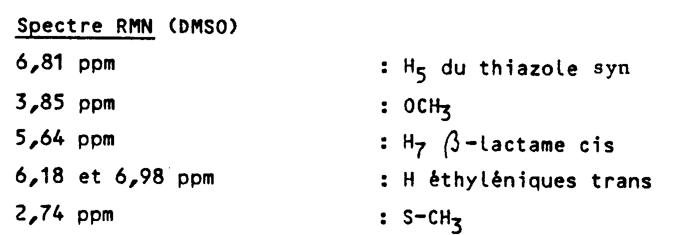

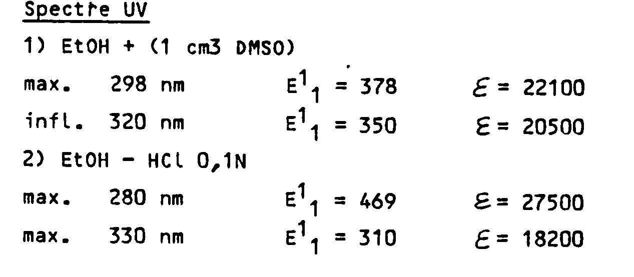

### Exemple 17 : Acide 7-/2-(2-aminothiazol-4-yl) 2-(méthoxyimino) acétamido/ 3-/3-/(5-amino) 1,3,4-thiadiazol-2-yl/ thio/ 1-(E)-propényl/8-oxo 4-thia 1-azabicyclo/4.2.0/oct-2-èn-2carboxylique syn (6S)(7S).

On opère comme à l'exemple 13 à partir de 184,5 mg de l'isomère E obtenu au stade B de l'exemple 1 et en utilisant 66,6 mg de 2-amino 5-mercapto 1,3,4-thiadiazole dans le diméthylformamide. On élue la chromatographie sur silice par un mélange chlorure de méthylène-acétate d'éthyle (1-1). on libère les fonctions bloquées par l'acide trifluoroacétique et obtient 60,5 mg de produit attendu.

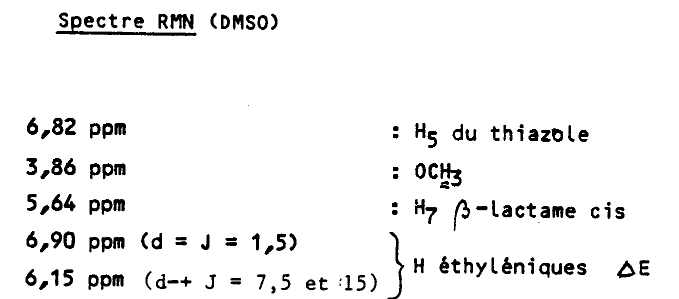

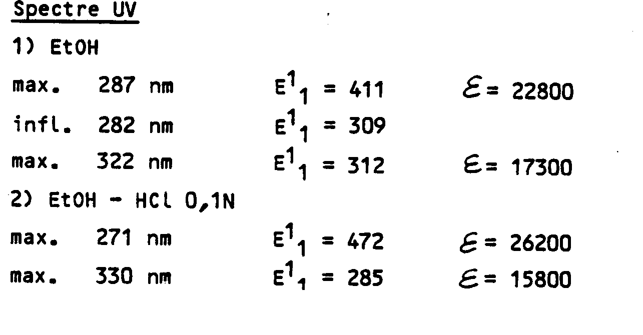

### Exemple 18 : Acide 7-/2-(2-aminothiazol-4-yl) 2-(méthoxyimino) acétamido/ 3-/3-/(9H-purin-6-yl) thio/ 1-(E)-propényl/ 8-oxo 4-thia 1-azabicyclo /4.2.0./ oct-2-èn-2-carboxylique syn (6S)(7S).

On opère comme à l'exemple 13 en utilisant 76 mg de 6-mercaptopurine. On chromatographie le produit sur silice en éluant d'abord par un mélange chlorure de méthylène-acétate d'éthyle (75-25) puis acétate d'éthyle seul et finalement avec un mélange chlorure de méthylène-méthanol (95-5). On effectue le déblocage et la purification et obtient 76 mg de produit attendu.

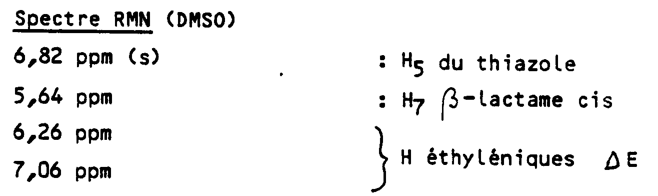

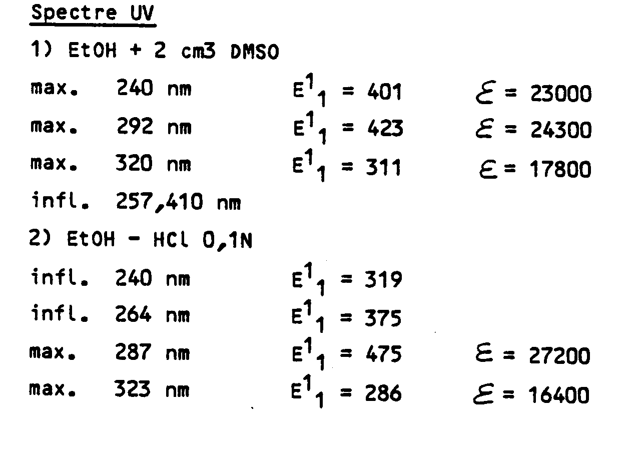

### Exemple 19 : Acide 7-/2-(2-aminothiazol-4-yl) 2-(méthoxyimino) acétamido 3-//3-(1,3,4-thiadiazol-2-yl) thio/ 1-(E)-propényl/8-oxo 4-thia 1-azabicyclo /4.2.0./ oct-2-èn-2-carboxylique syn (6S)(7S).

On opère comme indiqué à l'exemple 13 en utilisant 59,1 mg de 5-mercapto 1,3,4-thiadiazole et obtient 47,1 mq de produit attendu.

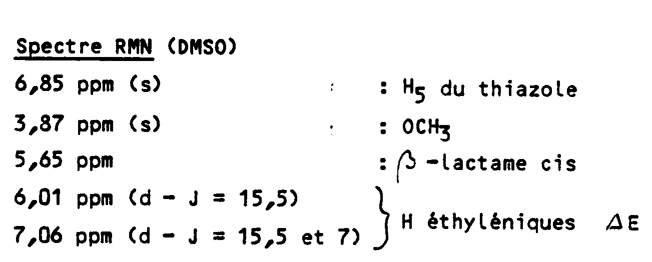

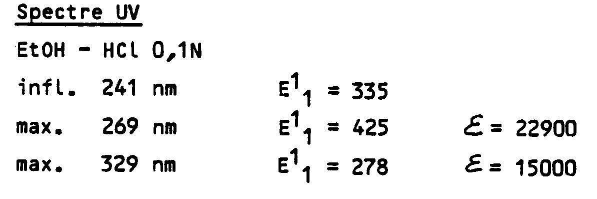

### Exemple 20 : Acide 7-/2-(2-aminothiazol-4-yl) 2-(méthoxyimino) acétamido 3-//3-(1,2,3-triazol-5-yl) thio/ 1-(E)-propényl/ 8-oxo 4-thia 1-azabicyclo /4.2.0./ oct-2-èn-2-carboxylique syn (6S)(7S).

On opère comme à l'exemple 13 en utilisant le thiolate de sodium, sans addition de carbonate de potassium. On effectue la chromatographie en éluant par un mélange chlorure de méthylène-acétate d'éthyle (75-25). Le déblocage des fonctions est effectué à l'aide de l'acide formique : 2 cm3 à 66% pour 149 mg de produit bloqué. On agite 3 heures à 60-65αC, chasse l'acide formique à 30 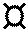 C sous pression réduite, reprend 2 fois à l'eau en évaporant à chaque fois. On triture le résidu à l'éther, essore et obtient 89 mg de produit que l'on dissout dans 6 cm3 de tétrahydrofuranne et 1 cm3 de méthanol. On introduit goutte à goutte cette solution dans 100 cm3 d'éther sous vive agitation, essore et obtient 69,5 mg de produit attendu.

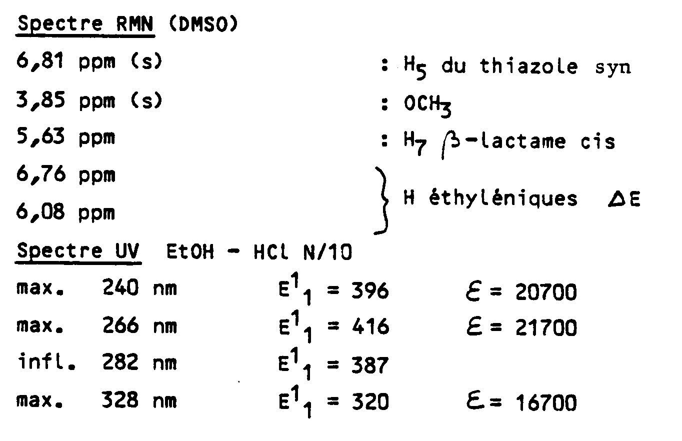

### Exemple 21 : 7-/2-(2-aminothiazol-4-yl) 2-(méthoxyimino) acétamido/ 3-/3-//4-méthyl 5-trifluorométhyl 4H-1,2,4-triazol-3-yl/ thio/ 1-(E)-propényl/ 8-oxo 4-thia 1-azabicyclo /4.2.0./ oct-2-èn-2-carboxylique syn (6S)-(7S).

On opère comme à l'exemple 13 en utilisant 90 mg de 4-méthyl 5-trifluorométhyle 4H-1,2,4-triazol-3-thione. On chromatographie sur silice, élue par un mélange chlorure de méthylène-acétate d'éthyle (75-25). Après traitement à l'acide formique à 66%, on obtient 78 mg de produit attendu.

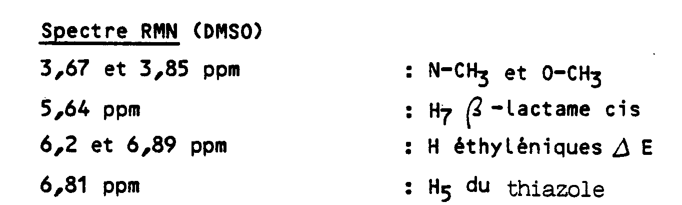

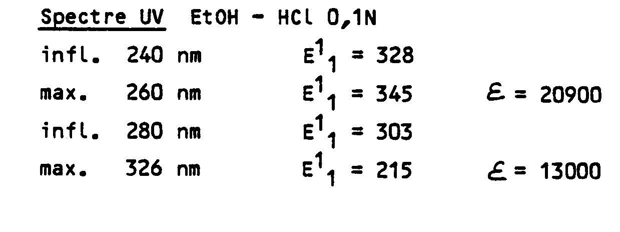

Exemple 22 : Acide 7-/2-(2-aminothiazol-4-yl) 2-(méthoxyimino) acétamido/ 3-/3-/(thiazol /5,4-b/ pyridin-2-yl)

### thio/ 1-(E)-propényl/8-oxo 4-thia 1-azabicyclo /4.2.0./ oct-2-èn-2-carboxylique syn (6S)(7S).

On opère comme à l'exemple 13 en utilisant 205 mg de 2-mercapto thiazolo /5,4-b/ pyridine. On chromatographie et élue par un mélange chlorure de méthylène-acétone (9-1). Le déblocage des fonctions est effectué à l'aide de l'acide formique sur 150 mg de produit obtenu et obtient 67 mg de produit brut. On chromatographie sur silice 45 mg de ce dernier, élue à l'acétate d'éthyle puis par un mélange acétone- acétate d'éthyle-eau(5-4-1). On obtient 17 mg de produit attendu.

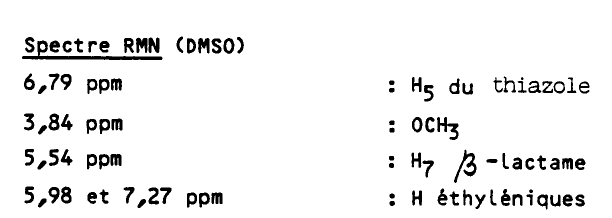

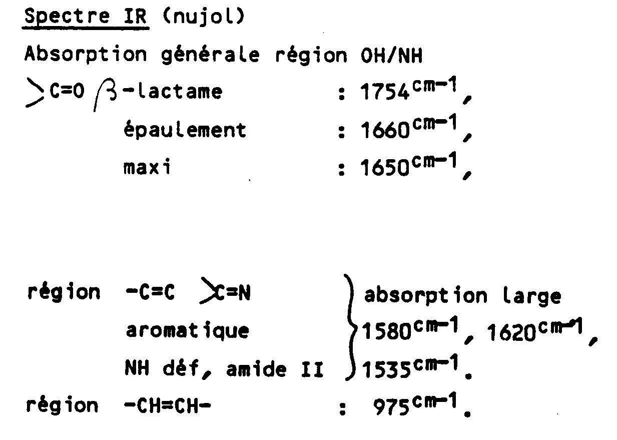

Exemple 23 : Acide 7-/2-(2-aminothiazol-4-yl) 2-(méthoxyimino) acétamido/ 3-/3-//1 H-1,2,4-triazol-3-yl/ thio/ 1-(E)-propényl/8-oxo 4-thia 1-azabicyclo/4.2.0./ oct-2-èn-2-carboxylique syn (6S)(7S).

On opère comme à l'exemple 21 en utilisant 50 mg de 1 H-1,2,4-triazol-3-thione, on obtient 62 mg de produit attendu.

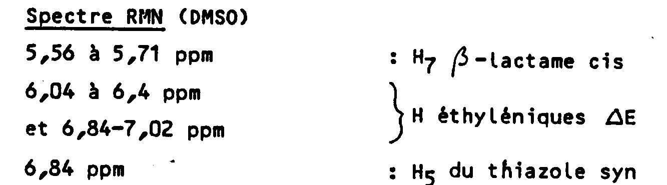

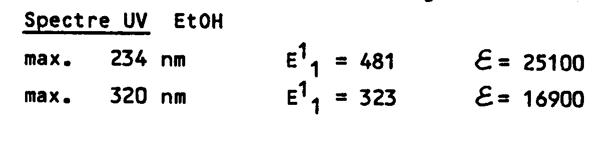

### Exemple 24 : Sel interne de 2-/3-/7-/2-(2-aminothiazol-4-yl) 2-(méthoxyimino) acétamido/ 2-carboxy 8-oxo 4-thia 1-azabicyclo /4.2.0./ oct-2-èn-3-yl/ 2-(E)-propényl/ isoquinoléinium syn (6S)(7S).

On opère comme à l'exemple 21 en partant de 148 mg de l'isomère E obtenu au stade B de l'exemple 1. Après addition de l'anhydride trifluorométhane sulfonique et distillation du solvant, on ajoute 5 cm3 d'acétonitrile et 52 µl d'isoquinoléine. Après 1 heure 15 minutes, on chasse l'acétonitrile, dissout le résidu dans l'acétate d'éthyle, lave à l'eau contenant du thiosulfate de sodium 0,2N en excès, lave àl'eau, sèche et concentre à sec. On chromatographie le résidu sur silice, élue avec un mélange chlorure de méthylène-méthanol (92-8) et isole 83 mg de produit intermédiaire bloqué. Après traitement à l'acide formique à 66%, filtration et évaporation du solvant, on reprend le résidu obtenu dans 1 cm3 d'acétonitrile et 1 cm3 d'une solution 1 M de triéthylamine et chromatographie sur silice, éluant : eau-acétonitrile (9-1) puis (85-15). On obtient 16,76 mg de produit attendu.

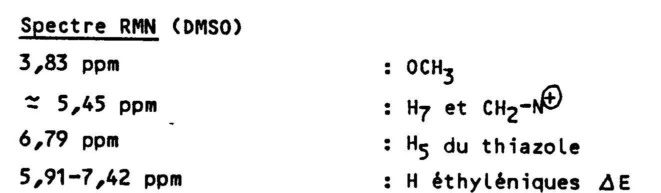

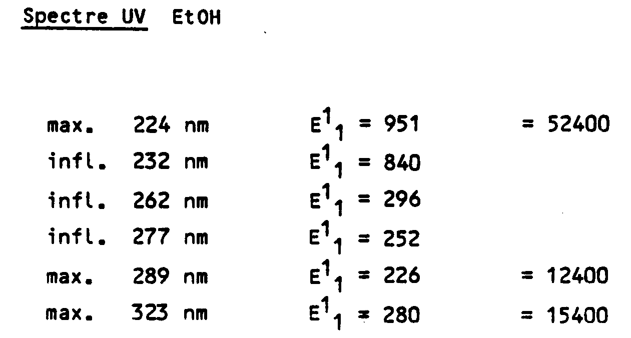

### Exemples 25 à 38 :

Les produits figurant dans les tableaux ci-après ont également été obtenus par le procédé de l'invention.

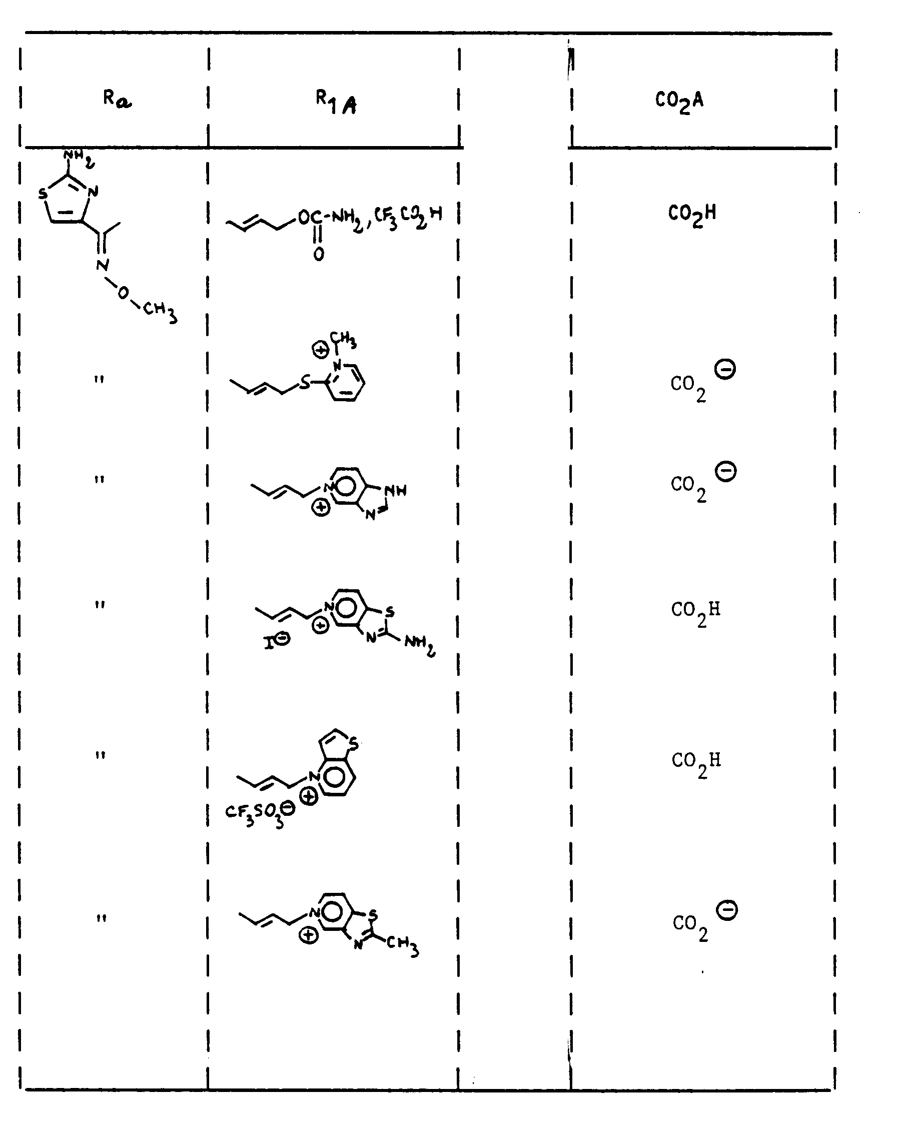

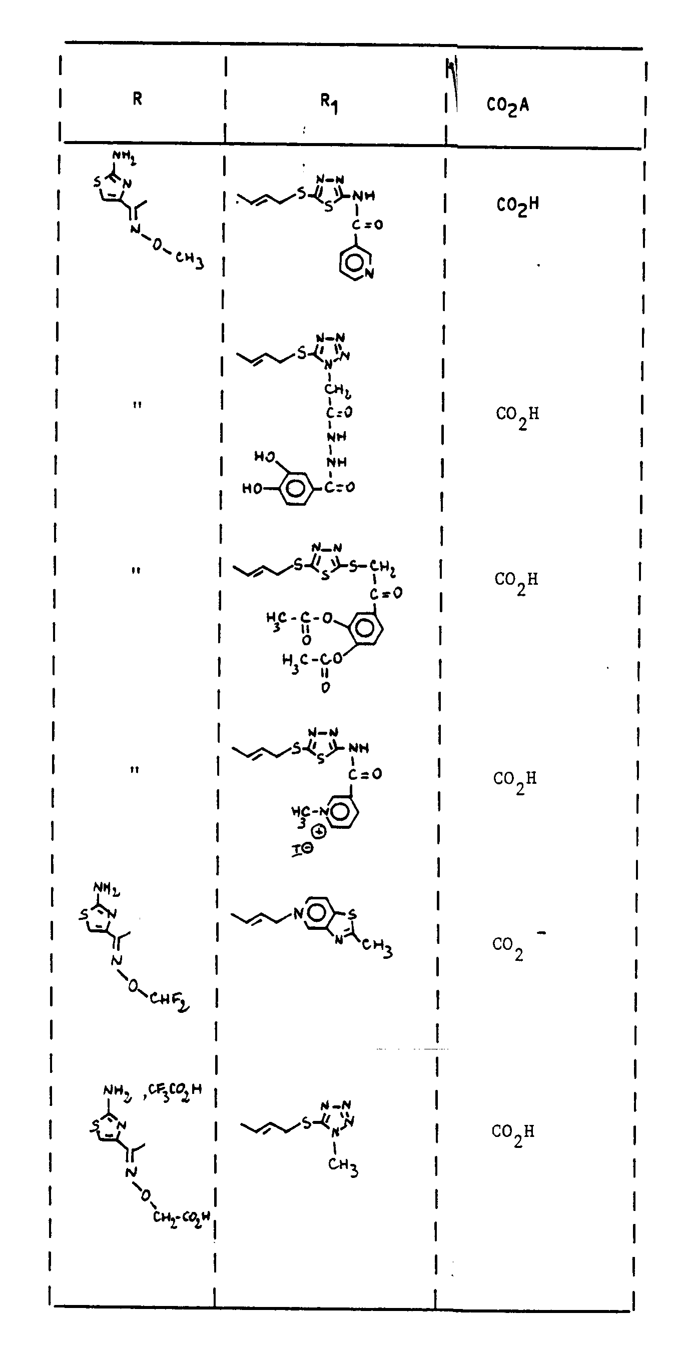

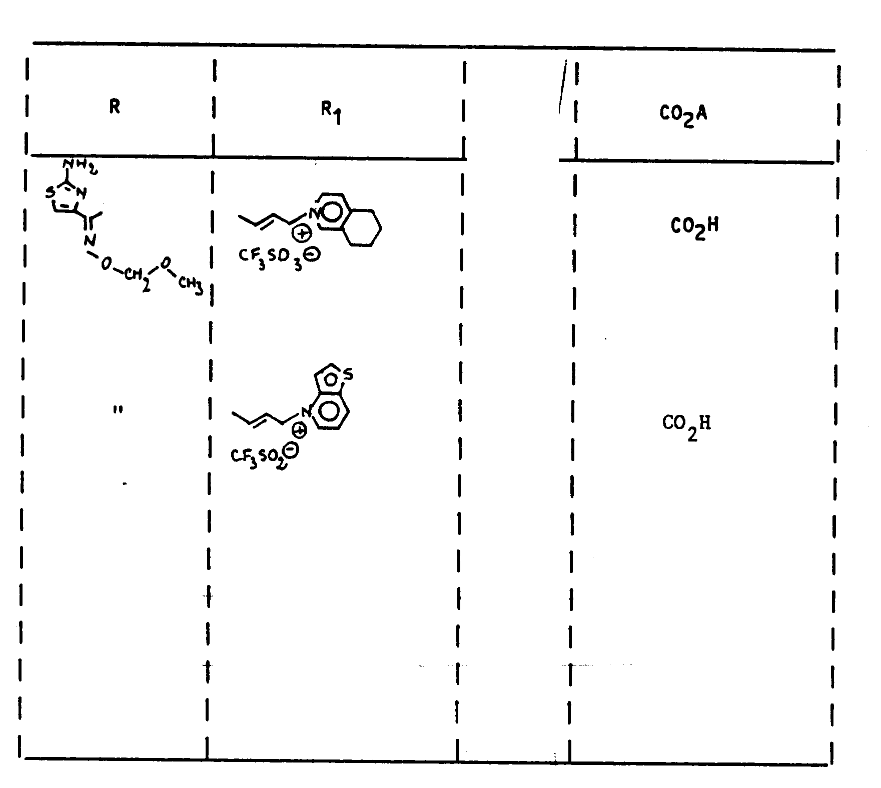

### Exemple 39 :

On a réalisé des préparations pour injections de formule :
- Sel interne de 7-/3-/7-//(2-amino-4-thiazolyl) 2-(Z)-(difluorométhoxy) imino/ acétyl/ amino/ 2-carboxy 8-oxo 4-thia 1-azabicyclo /4.2.0./ oct-2-èn-3-yl/ 2-(E)-propényl/ thiéno /2,3-b/ pyridinium (6S)(7S) ... 500mg
- Excipient aqueux stérile q.s.p.... 5 cm3.

### ETUDE PHARMACOLOGIQUE DES PRODUITS DE L'INVENTION

### Activité in vitro, méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre ou quarante-huit heures à l'étuve à 37αC, l'inhibition de la croissance est appréciée par transillumination ce qui permet de déterminer les concentrations minimales inhibitrices (C.M.I.) exprimées en µg/cm3.

Les résultats suivants sont obtenus :

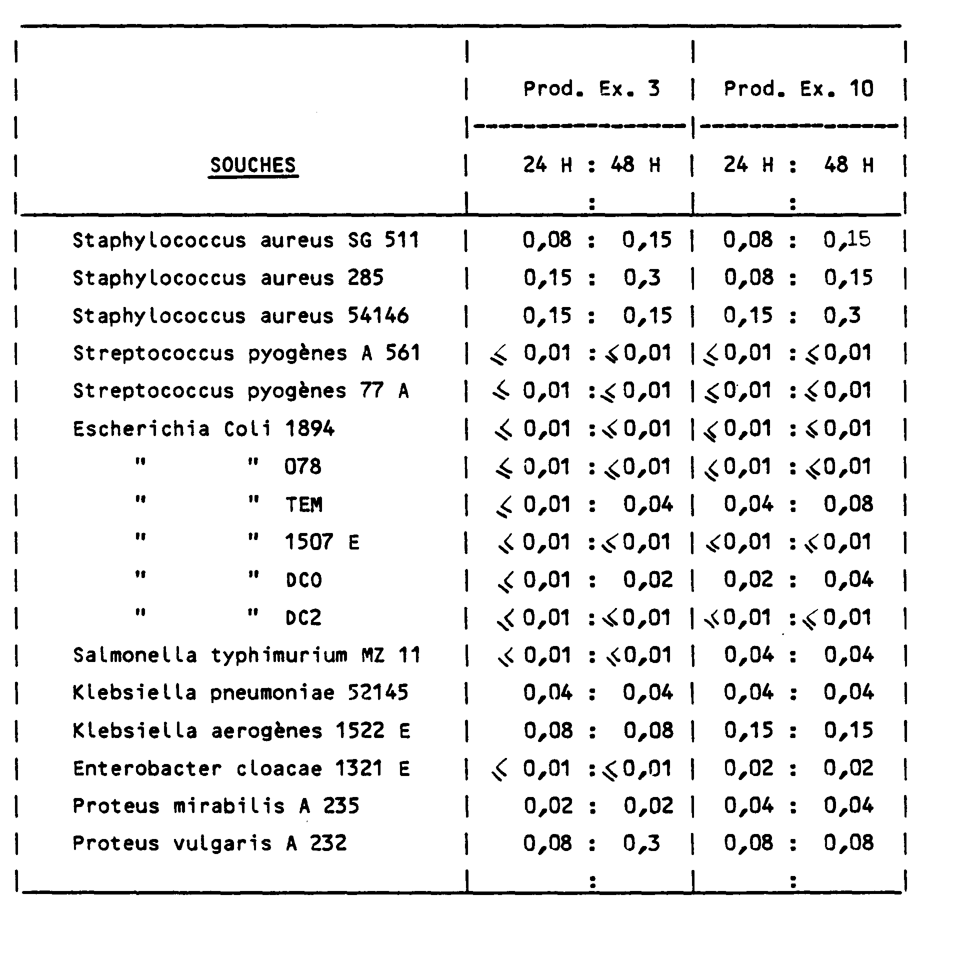

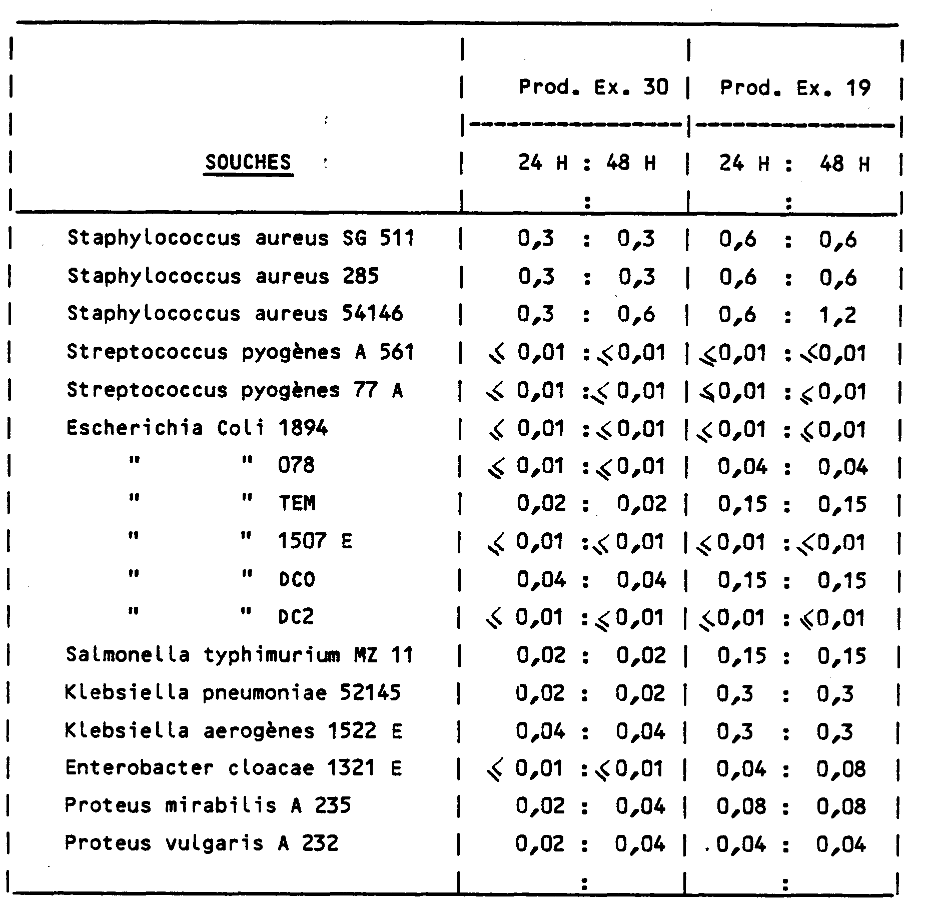

## Revendications (Revendications pour l'(les) Etat(s) contractant(s) suivant(s) : BE, CH, DE, FR, GB, IT, LI, LU, NL, SE)

1. Les produits répondant à la formule générale I 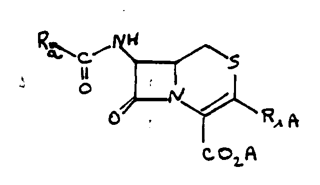 dans laquelle Ra représente un radical 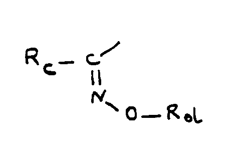 dans lequel Rc représente un radical 2-amino 4-thiazolyle, Rd représente:ou bien un atome d'hydrogène, ou bien un radical alkyle, alkényle, alkynyle ou cycloalkyle ayant au plus 6 atomes de carbone, ou un radical phényle, les dits radicaux alkyle, alkényle, alkynyle ou cycloalkyle étant éventuellement substitués par un radical choisi parmi les radicaux halogène, cyano, carbamoyle, nitro, amino, hydroxy, mercapto, oxo, carboxyle libre estérifié ou salifié, ou bien un radical difluoro méthyle, trifluorométhyle, méthoxyméthyle, 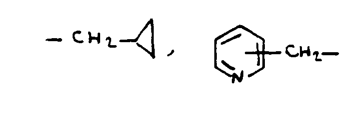 R_{lA} représente: - soit un radical 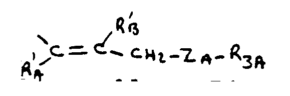 soit un radical 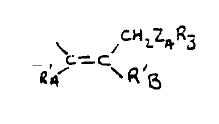 formules dans lesquelles R'A et R'B identiques ou différents représentent ou bien un atome d'hydrogène ou bien un radical alkyle ayant de 1 à 4 atomes de carbone, Z_{A} représente une simple liaison, un atome de soufre éventuellement oxydé ou un atome d'oxygène. R_{3A} représente ou bien un radical aryle carbocyclique ou hétérocyclique choisi parmi les radicaux phényle, diphényle, naphtyle, thiényle, furyle, pyrrolyle, imidazolyle, pyrazolyle, thiazolyle, isothiazolyle, oxazolyle, isoxazolyle, triazolyle, thiadiazolyle oxadiazolyle, tétrazolyle, pyridinyle, pyrimidinyle, pyrazinyle, pyridazinyle, thiazinyle oxazinyle, triazinyle, thiadiazinyle, oxadiazinyle, tétrazinyle, imidazolinyle, benzimidazolyle, benzothiazolyle, benzoxazole, ou bien à la condition que Z_{A} représente une simple liaison, un radical ammonium quaternaire choisi parmi les radicaux pyridinium, thienopyridinium, quinolinium, isoquinolinium, cyclopentylpyridinium, triméthylammonium, thiazolopyridinium, cyclohexenopyridinium, imidazopyridinium. Les radicaux R_{3A} pouvant être substitués par un ou plusieurs radicaux choisis parmi soit les radicaux alkyle ayant 1 à 4 atomes de carbone, ces radicaux alkyle étant eux-mêmes éventuellement substitués par un radical phényle ou thiényle, les radicaux phénoxy, méthoxy, éthoxycarbonyle, halogènes, hydroxy, carboxy libre estérifié ou salifié ou amino, soit les radicaux vinyle, allyle, butenyle, éthynyle, propargyle, phényle, tolyle, halogènes, amino, nitro, alkoxy ayant 1 à 4 atomes de carbone, méthylthio, hydroxy, mercapto, amino, carboxyle libre estérifié ou salifié ou carbamoyle, ou bien R_{3A} représente un radical acétyle, carbamoyle, alkoxycarbonyle, alkyle ou haloalkyle ayant 1 à 4 atomes de carbone, nitrile ou azido. R₄ représente un atome d'hydrogène ou un radical méthoxy. A représente un atome d'hydrogène, un équivalent de métal alcalin, alcalino-terreux, de magnésium, d'ammonium ou d'une base organique animée ou A représente un groupement ester ou CO₂A représente un groupement C0₂^{e.} n₂ représente un nombre entier égal à 0, 1 ou 2, ainsi que les sels des produits de formule (I) avec les acides minéraux ou organiques.

2. Les produits tels que définis à la revendication 1 et répondant à la formule I_{A} : 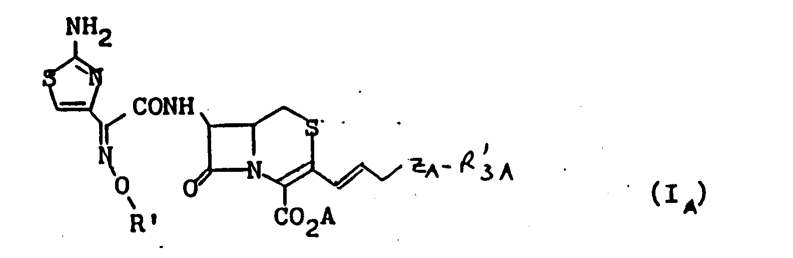 Isomère syn dans laquelle R' représente un atome d'hydrogène ou un radical alkyle, alkényle, alkynyle ou cycloalkyle ayant au plus 6 atomes de carbone, éventuellement substitué par un radical carboxy libre, estérifié ou salifié ou un radical difluorométhyle ou méthoxyméthyle, Zₐ représente une simple liaison et R'_{3A} représente un radical pyridinium, thiénopyridinium, thiazolopyridinium, cyclopentylpyridinium, cyclohexénopyridinium, triméthylammonium, ces radicaux pouvant être substitués par un radical choisi parmi les radicaux alkyle et amino.

3. Les produits de formule I dont les noms suivent :
- le sel interne de 7-/3-/7-//(2-amino 4-thiazolyl) 2-/Z-(difluorométhoxy) imino/ acétyl/ amino/ 2-carboxy 8-oxo 4-thia 1-azabicyclo /4.2.0./ oct-2-èn-3-yl/ 2-(E)-propényl/ thiéno /2,3-b/ pyridinium (6S)(7S) ;
- le sel interne de 5-/3-/7-//(2-amino 4-thiazolyl) (méthoxyimino) acétyl/ amino/ 2-carboxy 8-oxo 4-thia 1-azabicyclo /4.2.0./ oct-2-èn-3-yl/ 2-(E)-propényl/ 2-méthyl thiazolo /4,5-c/pyridinium syn (6S)(7S) ;
- le sel interne de 7-/3-/7-//(2-amino 4-thiazolyl) 2-(Z)-(méthoxyimino) acétyl/ amino/ 2-carboxy 8-oxo 4-thia 1-azabicyclo /4.2.0./ oct-2-èn-3-yl/ propèn-2-(E)-yl/ thiéno /2,3-b/ pyridinium (6S)(7S) ;
- l'acide 7-//(2-amino 4-thiazolyl) (méthoxyimino) acétyl/ amino/ 3-//3-(1,3,4-thiadiazol-2-yl) thio/ 1-(E)-propényl/ 8-oxo 4-thia 1-azabicyclo /4.2.0./ oct-2-èn-2-carboxylique syn (6S)(7S) ;
- le sel interne de 5-/3-/7-//(2-amino 4-thiazolyl) (difluorométhoxy) imino/ acétyl/ amino/ 2-carboxy 8-oxo 4-thia 1-azabicyclo /4.2.0./ oct-2-èn-3-yl/ 2-(E)-propényl/ 2-méthyl thiazolo /4,5-c/ pyridinium (6S)(7S) Z.

4. Procédé de préparation des produits de formule I telle que décrite à la revendication 1 caractérisé en ce que l'on traite un produit de formule VII : 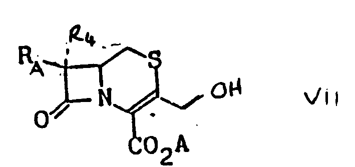 dans laquelle R₄ et A ont la signification indiquée à la revendication 1 et R_{A} représente soit un radical amino libre ou protégé par un groupement protecteur mono ou divalent, soit R_{A} représente le radical 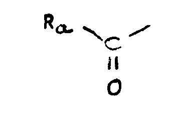 Rₐ ayant la signification indiquée à la revendication 1 soit par un agent d'halogénation soit par un agent de sulfonation, puis par la triphénylphosphine ou par un réactif de formule P(OAlk)₃ dans laquelle Alk représente un radical alkyle ayant de 1 à 4 atomes de carbone, pour obtenir ou bien un produit de formule VIII_{A} : 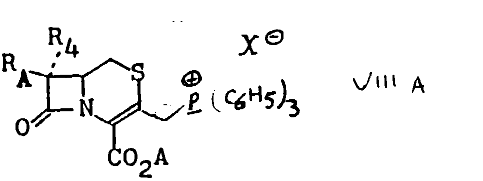 dans lequel R_{A}, R₄ et A ont la signification précédente et X⁻ représente le reste d'un anion, ou bien un produit de formule VIII'_{A} : 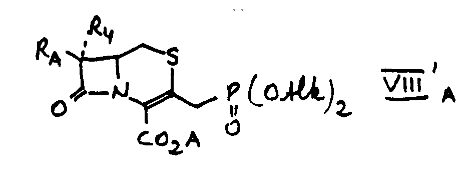 dans laquelle RA,R₄,A et Alk ont la signification précédente, produits de formules VillA ou VIII'_{A} que, si désiré l'on traite d'abord par une base forte puis par un halogènure d'alkyle de formule hal-R"_{A} dans laquelle hal représente un atome d'halogène et R"A a les valeurs de R'A différentes d'un atome d'hydrogène, pour obtenir les produits de formules VIII_{B} ou VIII'_{B} : 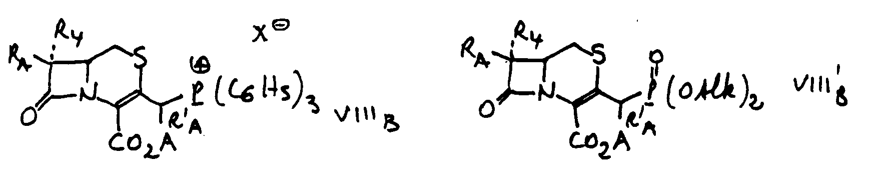 formules dans lesquelles R'A a la signification indiquée à la revendication 1 et produits de formules VIII_{B} ou VIII'_{B} que l'on traite par un produit de formule : 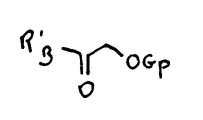 dans laquelle R'_{B} a la valeur indiquée à la revendication 1 et Gp représente un groupement protecteur du radical hydroxyle pour obtenir un produit de formule (IX) : 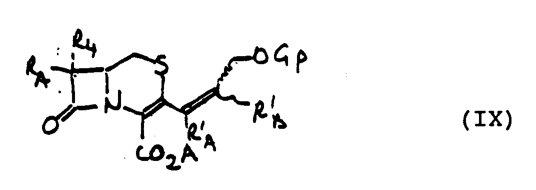 sous forme d'un mélange des isomères E et Z ou sous forme d'isomère E ou Z, produit de formule (IX), sur lequel on effectue les réactions suivantes dans un ordre quelconque :
a) coupure du groupement protecteur Gp ;
b) lorsque R_{A} représente un radical amino protégé par un groupement protecteur mono ou divalent, élimination de ce groupement protecteur et traitement du produit dans lequel R_{A} représente un radical amino par un acide de formule (IVₐ) :
RₐC0₂ H (IVₐ) formule dans laquelle Rₐ a la signification indiquée à la revendication 1 ou par un dérivé fonctionnel de cet acide.
c) Séparation des isomères E et Z ; pour obtenir un produit de formule (X) : 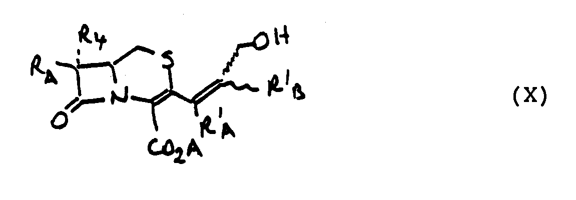 dans laquelle R_{A} a la signification indiquée à la revendication 1, produit de formule X sur lequel on fait agir soit un dérivé réactif du groupement R_{3A} pour obtenir un produit de formule XI_{A} : 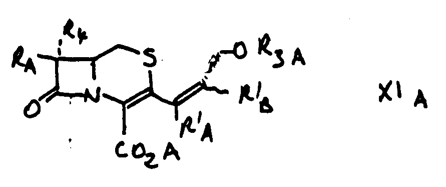 soit un dérivé d'un groupement d'activation du radical hydroxyle puis un dérivé ou un dérivé réactif de ce groupement R_{3A} pour obtenir un produit de formule XI_{B} : 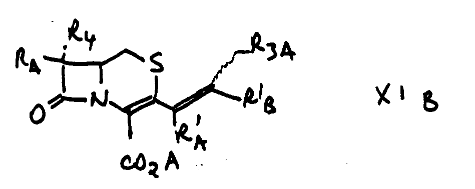 soit un dérivé d'un groupement d'activation du radical hydroxyle puis le produit de formule HS-R_{3A} ou un dérivé réactif de ce produit, pour obtenir un produit de formule Xl_{C} : 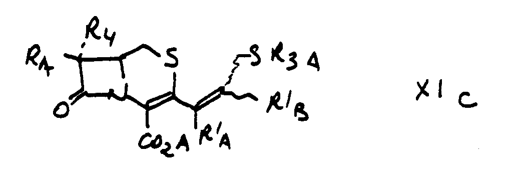 produits de formule XI_{A}, XI_{B} ou Xl_{C} que l'on soumet si nécessaire ou si désiré, à l'une quelconque ou à plusieurs des réactions suivantes, dans un ordre quelconque :
a) coupure par hydrolyse, hydrogénolyse ou par action de la thiourée de tout ou partie du ou des groupements protecteurs ;
b) estérification ou salification par une base du ou des groupements carboxy ou sulfo ;
c) salification par un acide du ou des groupements amino ;
d) dédoublement de la molécule pour obtenir un produit optiquement actif ;

5. A titre de médicaments, les produis de formule I telle que définie à la revendication 1, ainsi que leurs sels d'acides pharmaceutiquement acceptables.

6. A titre de médicaments, les produits de formule I_{A} telle que définie à l'une des revendications 2 et 3, ainsi que leurs sels d'acides pharmaceutiquement acceptables.

7. Compositions pharmaceutiques renfermant, à titre de principe actif, au moins un des médicaments selon l'une des revendications 5 ou 6.

## Revendications (Revendications pour l'(les) Etat(s) contractant(s) suivant(s) : AT)

1. Procédé de préparation des produits répondant à la formule générale (1) : 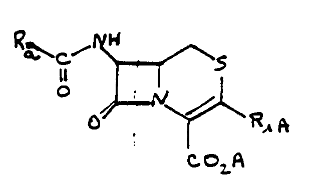 dans laquelle Ra représente un radical 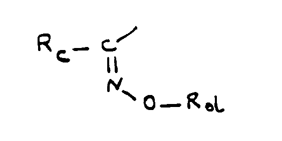 dans lequel Rc représente un radical 2-amino 4-thiazolyle, Rd représente:ou bien un atome d'hydrogène, ou bien un radical alkyle, alkényle, alkynyle ou cycloalkyle ayant au plus 6 atomes de carbone, ou un radical phényle, les dits radicaux alkyle, alkényle, alkynyle ou cycloalkyle étant éventuellement substitués par un radical choisi parmi les radicaux halogène, cyano, carbamoyle, nitro, amino, hydroxy, mercapto, oxo, carboxyle libre estérifié ou salifié, ou bien un radical difluoro méthyle, trifluorométhyle, méthoxyméthyle, 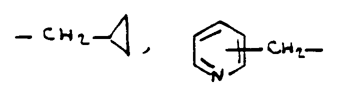 R_{lA} représente: - soit un radical 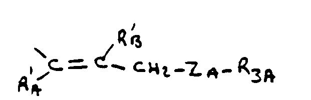 soit un radical 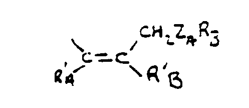 formules dans lesquelles R'_{A} et R'_{B} identiques ou différents représentent ou bien un atome d'hydrogène ou bien un radical alkyle ayant de 1 à 4 atomes de carbone, Z_{A} représente une simple liaison, un atome de soufre éventuellement oxydé ou un atome d'oxygène. R_{3A} représente ou bien un radical aryle carbocyclique ou hétérocyclique choisi parmi les radicaux phényle, diphényle, naphtyle, thiényle, furyle, pyrrolyle, imidazolyle, pyrazolyle, thiazolyle, isothiazolyle, oxazolyle, isoxazolyle, triazolyle, thiadiazolyle, oxadiazolyle, tétrazolyle, pyridinyle, pyrimidinyle, pyrazinyle, pyridazinyle, thiazinyle, oxazinyle, triazinyle, thiadiazinyle, oxadiazinyle, tétrazinyle, imidazolinyle, benzimidazolyle, benzothiazolyle, benzoxazole, ou bien à la condition que Z_{A} représente une simple liaison, un radical ammonium quaternaire choisi parmi les radicaux pyridinium, thienopyridinium, quinolinium, isoquinolinium, cyclopentylpyridinium, triméthylammonium, thiazolopyridinium, cyclohexenopyridinium, imidazopyridinium. Les radicaux R_{3A} pouvant être substitués par un ou plusieurs radicaux choisis parmi soit les radicaux alkyle ayant 1 à 4 atomes de carbone, ces radicaux alkyle étant eux-mêmes éventuellement substitués par un radical phényle ou thiényle, les radicaux phénoxy, méthoxy, éthoxycarbonyle, halogènes, hydroxy, carboxy libre estérifié ou salifié ou amino, soit les radicaux vinyle, allyle, butenyle, éthynyle, propargyle, phényle, tolyle, halogènes, amino, nitro, alkoxy ayant 1 à 4 atomes de carbone, méthylthio, hydroxy, mercapto, amino, carboxyle libre estérifié ou salifié ou carbamoyle, ou bien R_{3A} représente un radical acétyle, carbamoyle, alkoxycarbonyle, alkyle ou haloalkyle ayant 1 à 4 atomes de carbone, nitrile ou azido. R₄ représente un atome d'hydrogène ou un radical méthoxy. A représente un atome d'hydrogène, un équivalent de métal alcalin, alcalino-terreux, de magnésium, d'ammonium ou d'une base organique animée ou A représente un groupement ester ou CO₂A représente un groupement C0₂^{e.} n₂ représente un nombre entier égal à 0, 1 ou 2, ainsi que les sels des produits de formule (I) avec les acides minéraux ou organiques, caractérisé en ce que l'on traite un produit de formule (VII) : 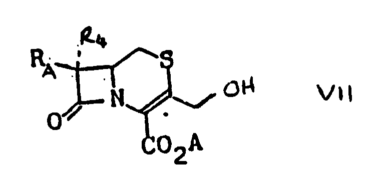 dans laquelle R₄ et A ont la signification indiquée précédemment et R_{A} représente soit un radical amino libre ou protégé par un groupement protecteur mono ou divalent, soit R_{A} représente le radical 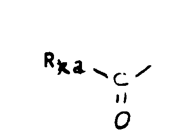 Rₐ ayant la signification indiquée précédemment soit par un agent d'halogénation soit par un agent de sulfonation, puis par la triphénylphosphine ou par un réactif de formule (P(OAlk)₃ dans laquelle Alk représente un radical alkyle ayant de 1 à 4 atomes de carbone, pour obtenir ou bien un produit de formule VIII_{A} : 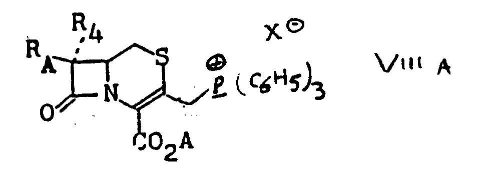 dans lequel R_{A}, R₄ et A ont la signification précédente et X^{⊖} représente le reste d'un anion, ou bien un produit de formule VIII'_{A} : 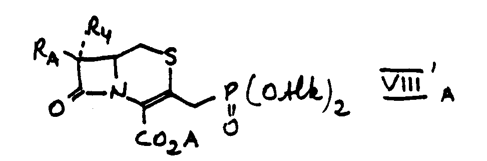 dans laquelle RA, R4 et Alk ont la signification précédente, produits de formules VillA ou VIII'_{A} que, si désiré l'on traite d'abord par une base forte puis par un halogènure d'alkyle de formule hal-R"_{A} dans laquelle hal représente un atome d'halogène et R"A a les valeurs de R'A différentes d'un atome d'hydrogène, pour obtenir les produits de formules VIII_{B} ou VIII'_{B} : 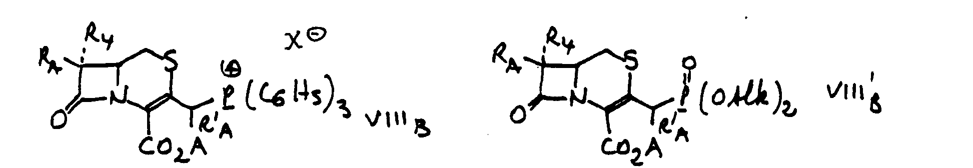 formules dans lesquelles R'A a la signification indiquée précédemment et produits de formules VIII_{B} ou VIII'_{B} que l'on traite par un produit de formule : 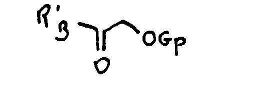 dans laquelle R'_{B} a la valeur indiquée à la revendication 1 et Gp représente un groupement protecteur du radical hydroxyle pour obtenir un produit de formule (IX) : 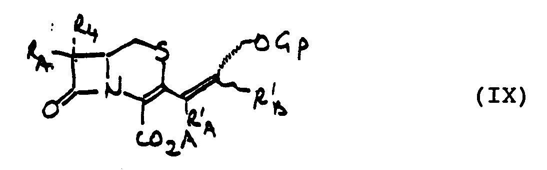 sous forme d'un mélange des isomères E et Z ou sous forme d'isomère E ou Z, produit de formule (IX), sur lequel on effectue les réactions suivantes dans un ordre quelconque :
a) coupure du groupement protecteur Gp ;
b) lorsque R_{A} représente un radical amino protégé par un groupement protecteur mono ou divalent, élimination de ce groupement protecteur et traitement du produit dans lequel R_{A} représente un radical amino par un acide de formule (IVₐ) : 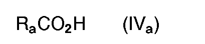 formule dans laquelle Rₐ a la signification indiquée précédemment ou par un dérivé fonctionnel de cet acide.
c) Séparation des isomères E et Z ; pour obtenir un produit de formule (X) : 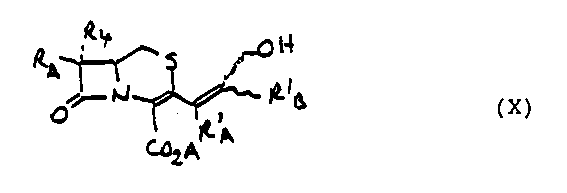 dans laquelle R_{A} a la signification indiquée à la revendication 1, produit de formule X sur lequel on fait agir soit un dérivé réactif du groupement R_{3A} pour obtenir un produit de formule XI_{A} : 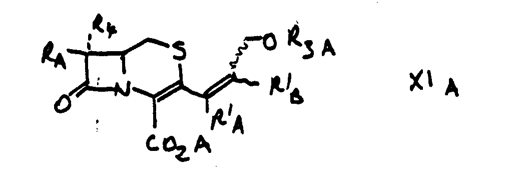 soit un dérivé d'un groupement d'activation du radical hydroxyle puis un dérivé ou un dérivé réactif d groupement R_{3A} pour obtenir un produit de formule XI_{B} : 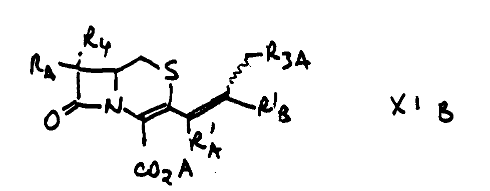 soit un dérivé d'un groupement d'activation du radical hydroxyle puis le produit de formule HS-R_{3A} ou un dérivé réactif de ce produit, pour obtenir un produit de formule Xl_{C} : 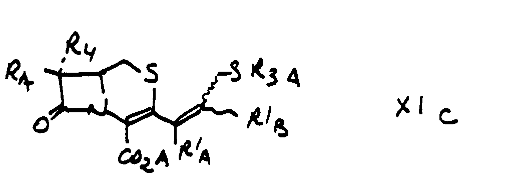 produits de formule XI_{A}, XI_{B} ou Xl_{C} que l'on soumet si nécessaire ou si désiré, à l'une quelconque ou à plusieurs des réactions suivantes, dans un ordre quelconque :
a) coupure par hydrolyse, hydrogénolyse ou par action de la thiourée de tout ou partie du ou des groupements protecteurs ;
b) estérification ou salification par une base du ou des groupements carboxy ou sulfo ;
c) salification par un acide du ou des groupements amino ;
d) dédoublement de la molécule pour obtenir un produit optiquement actif ;

2. Procédé selon la revendication 1, pour la préparation des produits répondant à la formule (I_{A}) : 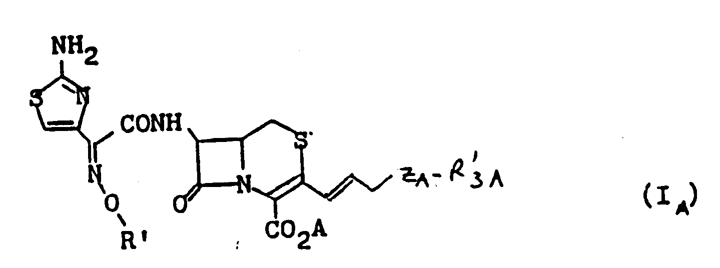 Isomère syn dans laquelle R' représente un atome d'hydrogène ou un radical alkyle, alkényle, alkynyle ou cycloalkyle ayant au plus 6 atomes de carbone, éventuellement substitué par un radical carboxy libre, estérifié ou salifié ou un radical difluorométhyle ou méthoxyméthyle, Zₐ représente une simple liaison et R'_{3A} représente un radical pyridinium, thiénopyridinium, thiazolopyridinium, cyclopentylpyridinium, cyclohexénopyridinium, triméthylammonium, ces radicaux pouvant être substitués par un radical choisi parmi les radicaux alkyle et amino. caractérisé en ce que l'on traité un produit de formule (VII) : 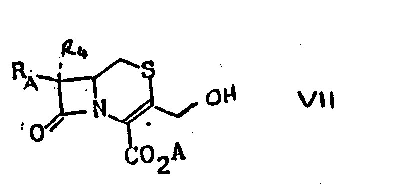 dans laquelle A a la signification indiquée à la revendication 1 et R_{c} représente soit un radical amino libre ou protégé par un groupement protecteur mono ou divalent, soit R_{c} représente le radical 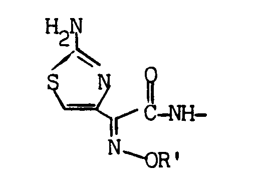 éventuellement protégé soit par un agent d'halogénation soit par un agent de sulfonation, puis par la triphénylphosphine ou par un réactif de formule P(OAlk)₃ dans laquelle Alk représente un radical alkyle ayant de 1 à 4 atomes de carbone, pour obtenir ou bien un produit de formule (VIII_{A}) : 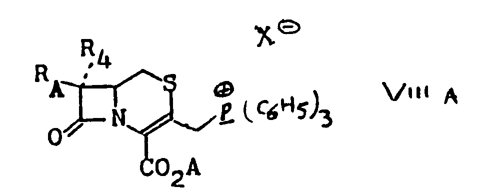 dans lequel R_{c} et A ont ta signification précédente et X⁻ représente le reste d'un anion, ou bien un produit de formule VIII'_{A} : 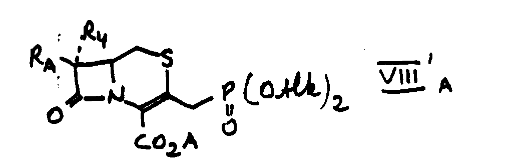 dans laquelle R_{c}, A et Alk ont la signification précédente, produits de formules VillA ou VIII'_{A} que l'on traite par un produit de formule : 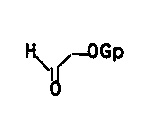 dans laquelle Gp représente un groupement protecteur du radical hydroxyle, pour obtenir un produit de formule IX' : 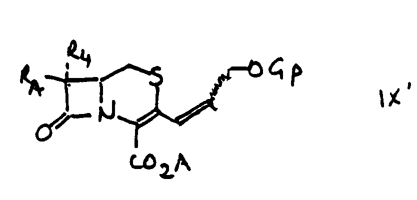 sous forme d'un mélange des isomères E et Z ou sous forme d'isomère E ou Z, produit de formule IX', sur lequel on effectue les réactions suivantes dans un ordre quelconque :
a) coupure du groupement protecteur Gp ;
b) lorsque R_{c} représente un radical amino protégé par un groupement protecteur mono ou divalent, élimination de ce groupement protecteur et traitement du produit dans lequel R_{c} représente un radical amino par un acide de formule IV'ₐ : 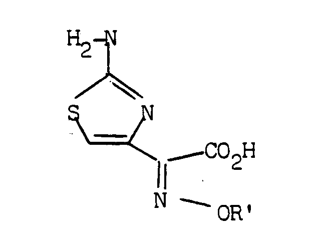 ou par un dérivé fonctionnel de cet acide,
c) séparation des isomères E et Z ; pour obtenir un produit de formule X' : 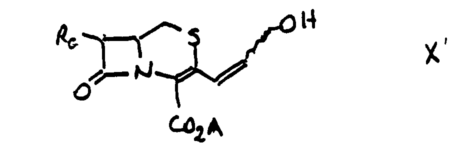 dans laquelle R_{c} a la signification indiquée à la revendication 1, produit de formule X' sur lequel on fait agir soit un dérivé réactif du groupement R'_{3A} pour obtenir un produit de formule XI'A : 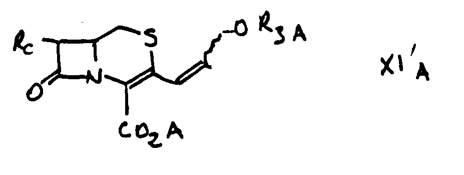 soit un dérivé d'un groupement d'activation du radical hydroxyle puis un dérivé ou un dérivé réactif de ce groupement R'_{3A} pour obtenir un produit de formule XI'_{B} : 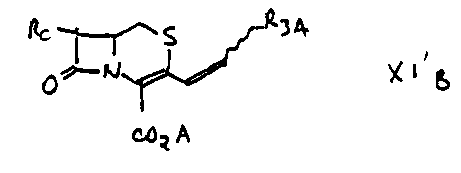 soit un dérivé d'un groupement d'activation du radical hydroxyle puis le produit de formule HS-R'_{3A} ou un dérivé réactif de ce produit, pour obtenir un produit de formule XI'ₑ : 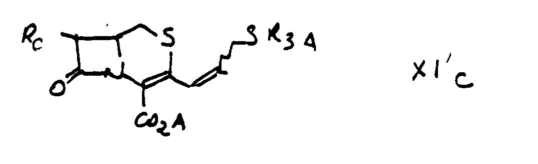 produits de formule XI'A, XI'_{B} ou Xl'_{c} que l'on soumet si nécessaire ou si désiré, à l'une quelconque ou à plusieurs des réactions suivantes, dans un ordre quelconque :
a) coupure par hydrolyse, hydrogénolyse ou par action de la thiourée de tout ou partie du ou des groupements protecteurs ;
b) estérification ou salification par une base du ou des groupements caboxy ou sulfo ;
c) salification par un acide du ou des groupements amino ;
d) dédoublement de la molécule pour obtenir un produit optiquement actif.

3. Procédé selon la revendication 2, caractérisé en ce que l'on utilise au départ un composé de formule VII ou un composé de formule IV'ₐ dans laquelle R' représente un atome d'hydrogène ou un radical méthyle ou isopropyle, éventuellement substitué par un radical carboxy, un radical difluorométhyle, un radical cyclobutyle ou cyclopropyle éventuellement substitué par un radical carboxy,

4. Procédé selon la revendication 2 ou 3, caractérisé en ce que l'on traite le produit de formule X' par un dérivé d'un groupement d'activation du radial hydroxyle puis un dérivé ou un dérivé réactif d'un radical pyridinium éventuellement substitué.

5. Procédé selon la revendication 1, caractérisé en ce que l'on prépare l'un quelconque des composés de formule I dont les noms suivent :
- le sel interne de 7-/3-/7-//(2-amino 4-thiazolyl) 2-/7-(difluorométhoxy) imino/ acétyl/ amino/ 2-carboxy 8-oxo 4-thia 1-azabicyclo /4.2.0./ oct-2-èn-3-yl/ 2-(E)-propényl/ thiéno /2,3-b/ pyridinium (6S)(7S) ;
- le sel interne de 5-/3-/7-//(2-amino 4-thiazolyl) (méthoxyimino) acétyl/ amino/ 2-carboxy 8-oxo 4-thia 1-azabicyclo /4.2.0./ oct-2-èn-3-yl/ 2-(E)-propényl/ 2-méthyl thiazolo /4,5-c/ puridinium syn (6S)(7S) ;
- le sel interne de 7-/3-/7-//(2-amino 4-thiazolyl) 2-(Z)-(méthoxyimino) acétyl/ amino/ 2-carboxy 8-oxo 4-thia 1-azabicyclo /4.2.0./ oct-2-èn-3-yl/ propèn-2-(E)-yl/ thiéno /2,3-b/ pyridinium (6S)(7S) ;
- l'acide 7-//(2-amino 4-thiazolyl) (méthoxyimino) acétyl/ amino/ 3-//3-(1,3,4-thiadiazol-2-yl) thio/ 1-(E)-propényl/ 8-oxo 4-thia 1-azabicyclo /4.2.0./ oct-2-èn-2-carboxylique syn (6S)(7S) ;
- le sel interne de 5-/3-/7-//(2-amino 4-thiazolyl) (difluorométhoxy) imino/ acétyl/ amino/ 2-carboxy 8-oxo 4-thia 1-azabicyclo /4.2.0./ oct-2-èn-3-yl/ 2-(E)-propényl/ 2-méthyl thiazolo /4,5-c/ pyridinium (6S)(7S) Z.

## Claims (Claims for the following Contracting State(s) : BE, CH, DE, FR, GB, IT, LI, LU, NL, SE)

1. The products corresponding to general formula I: 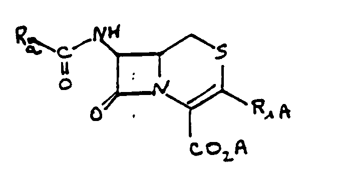 in which Ra represents a radical 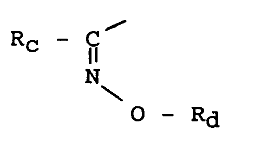 in which R_{c} represents a 2-amino-4-thiazolyl radical, R_{d} represents : either a hydrogen atom, or an alkyl, alkenyl, alkynyl or cycloalkyl radical having at most 6 carbon atoms, or a phenyl radical, the said alkyl, alkenyl, alkynyl or cycloalkyl radicals being optionally substituted by a radical chosen from the following radicals: halogen, cyano, carbamoyl, nitro, amino, hydroxy, mercapto, oxo, free, esterified or salified carboxyl, or a difluoromethyl, trifluoromethyl, methoxymethyl, 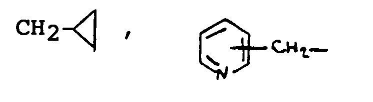 radical, R_{1A} represents: - either a 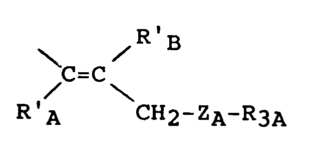 radical, or a 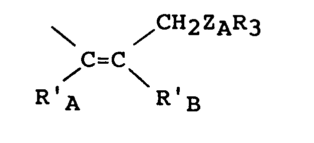 radical, in which formulae R'A and R'_{B}, identical or different represent either a hydrogen atom or an alkyl radical containing 1 to 4 carbon atoms, Z_{A} represents a single bond, an optionally oxidized sulphur atom or an oxygen atom, R₃A represents either a carbocyclic or heterocyclic aryl radical chosen from the following radicals: phenyl, diphenyl, naphthyl, thienyl, furyl, pyrrolyl, imadazolyl, pyrazolyl, thiazolyl, isothiazolyl, oxazolyl, isoxazolyl, triazolyl, thiadiazolyl, oxadiazolyl, tetrazolyl, pyridinyl, pyrimidinyl, pyrazinyl, pyridazinyl, thiazinyl, oxazinyl, triazinyl, thiadiazinyl, oxadiazinyl, tetrazinyl, imidazolinyl, benzimidazolyl, benzothiazolyl, benzoxazole, or on the condition that Z_{A} represents a single bond a quaternary ammonium radical chosen from the following radicals: pyridinium, thienopyridinium, quinolinium, isoquinolinium, cyclopentylpyridinium, trimethylammonium, thiazolopyridinium, cyclohexenopyridinium, imidazopyridinium, the R₃A radicals being able to be substituted by one or more radicals chosen from either the alkyl radicals having 1 to 4 carbon atoms, these alkyl radicals being themselves optionally substituted by a phenyl or thienyl radical, phenoxy, methoxy, ethoxycarbonyl, halogen, hydroxy, free, esterified or salified carboxy or amino radicals, or one of the following radicals: vinyl, allyl, butenyl, ethynyl, propargyl, phenyl, tolyl, halogen, amino, nitro, alkoxy having 1 to 4 carbon atoms, methylthio, hydroxy, mercapto, amino, free, esterified or salified carboxyl or carbamoyl, or R₃A represents one of the following radicals: acetyl, carbamoyl, alkoxycarbonyl, alkyl or haloalkyl having 1 to 4 carbon atoms, nitrile or azido, R₄ represents a hydrogen atom or a methoxy radical, A represents a hydrogen atom, an equivalent of an alkali metal, an alkaline-earth metal, magnesium, ammonium or an amino organic base or A represents an ester group or C0₂A represents a C0₂^{e} group, n₂ represents an integer equal to 0, 1 or 2, as well as the salts of the products of formula I with mineral or organic acids.

2. The products as defined in claim 1 and corresponding to formula I_{A}: 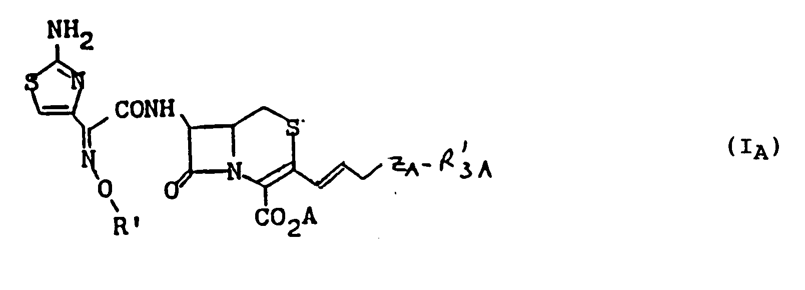 syn isomer in which R' represents a hydrogen atom or an alkyl, alkenyl, alkynyl or cycloalkyl radical having at most 6 carbon atoms, optionally substituted by a free, esterified or salified carboxy radical or a difluromethyl or methoxymethyl radical, Z_{A} represents a single bond and R'_{3A} represents one of the following radicals: pyridinium, thienopyridinium, thiazolopyridinium, cyclopentylpyridinium, cyclohexenopyridinium, trimethylammonium, these radicals being able to be substituted by a radical chosen from alkyl and amino radicals.

3. The products of formula I, the names of which follow:
- the internal salt of 7-[3-[7-[[(2-amino-4-thiazolyl)-2]-7-(difluoromethoxy)-imino]-acetyl]-amino]-2-carboxy-8-oxo-4-thia-1-azabicyclo-[4.2.0-[oct-2-en-3-yl-[2-(E)-propenyl]-thieno]-2,3-b]-pyridinium (6S)(7S);
- the internal salt of 5-[3-[7-[[(2-amino-4-thiazolyl)-(methoxyimino)-acetyl]-amino]-2-carboxy-8-oxo-4-thia-1-azabicyclo]-4.2.0]-oct-2-en-3-yl-[2-(E)-propenyl[-2-methyl-thiazolo]-4,5-c]-pyridinium syn (6S)(7S);
- the internal salt of 7-[3-[7-[[(2-amino-4-thiazolyl)-2-(Z)-(methoxyimino)-acetyl]-amino]-2-carboxy-8-oxo-4-thia-1-azabicyclo]-4.2.0]-oct-2-en-3-yl-[propen-2-(E)-yl-[thieno]-2,3-b]-pyridinium (6S)(7S);
- 7-[[(2-amino-4-thiazolyl)-(methoxyimino)-acetyl]-amino]-3-[[3-(1,3,4-thiadiazol-2-yl)-thio[-1-(E)-propenyl]-8-oxo-4-thia-1-azabicyclo]-4.2.0]-oct-2-en-2-carboxylic acid syn (6S)(7S);
- the internal salt of 5-[3-[7-[[(2-amino-4-thiazolyl)-(difluoromethoxy)-imino]-acetyl]-amino-2-carboxy-8-oxo-4-thia-1-azabicyclo-[4.2.0-[oct-2-en-3-yl-[2-(E)-propenyl]-2-methyl-thiazolo]-4,5-c]-pyridinium (6S)(7S).

4. Preparation process for the products of formula I as described in claim 1 characterized in that a product of formula VII: 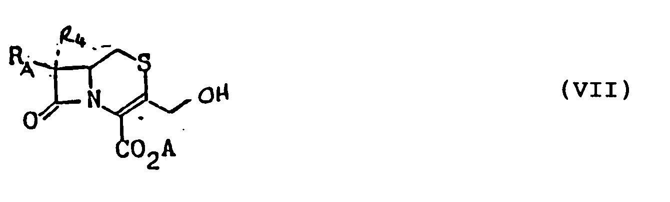 in which R₄ and A have the meaning indicated in claim 1 and R_{A} represents either an amino radical free or protected by a mono or divalent protector group, or R_{A} represents the 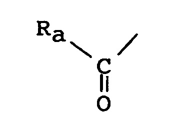 radical, Rₐ having the meaning indicated in claim 1, is treated either by a halogenation agent or by a sulphonation agent, then by triphenylphosphine or by a reagent of formula P(OAlk₃) in which Alk represents an alkyl radical having 1 to 4 carbon atoms, in order to obtain either a product of formula VIII_{A}: 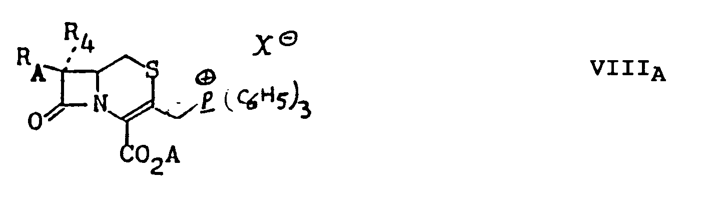 in which R_{A}, R₄ and A have the previous meaning and X^{⊖} represents the remainder of an anion, or a product of formula VIII'_{A}: 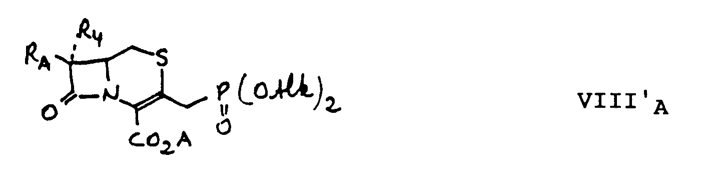 in which R_{A}, R₄, A and Alk have the previous meaning, which products of formulae VillA or VIII'_{A}, if desired, are first treated with a strong base then by an alkyl halide of formula hal-R"_{A} in which hal represents a halogen atom and R"_{A} has the values of R'A different to a hydrogen atom, in order to obtain the products of formulae VIII_{B} or VIII'_{B}: 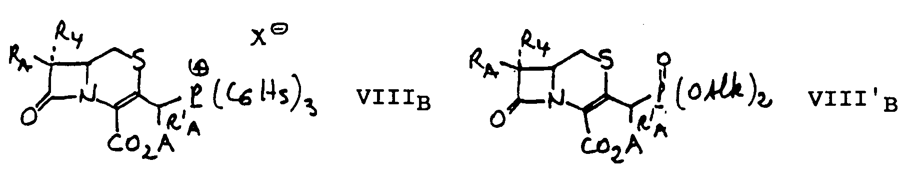 formulae in which R'A has the meaning indicated in claim 1 and which products of formulae VIII_{B} or VIII'_{B} are treated with a product of formula: 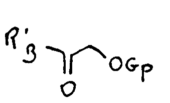 in which R'B has the value indicated in claim 1 and Gp represents a protector group of the hydroxyl radical, in order to obtain a product of formula IX: 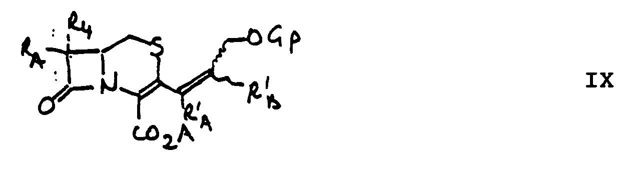 in the form of a mixture of E and Z isomers or in the form of E or Z isomer, on which product of formula IX the following reactions are carried out in any order:
a) detachment of the protector group Gp;
b) when R_{A} represents an amino group protected by a mono or divalent protector group, elimination of this protector group and treatment of the product in which R_{A} represents an amino radical by an acid of formula IV_{A}: 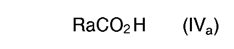 formula in which Ra has the meaning indicated in claim 1, or by a functional derivative of this acid;
c) separation of E and Z isomers; in order to obtain a product of formula X: 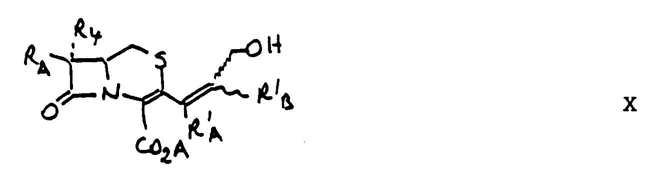 in which R_{A} has the meaning indicated in claim 1, on which product of formula X is reacted either a reactive derivative of the R_{3A} group in order to obtain a product of formula XI_{A}: 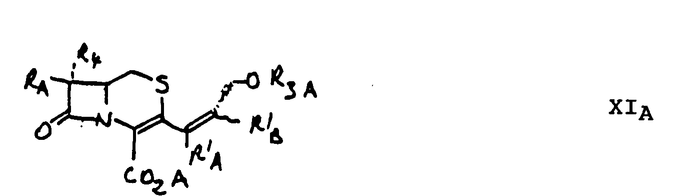 or a derivative of an activation group of the hydroxyl radical then a derivative or a reactive derivative of this R_{3A} group in order to obtain a product of formula XI_{B}: 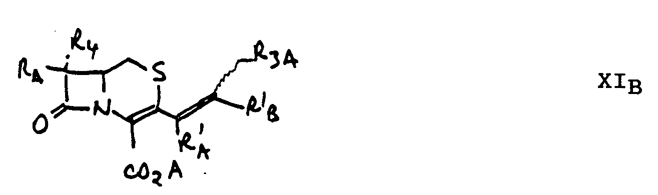 or a derivative of an activation group of the hydroxyl radical then the product of formula HS-R_{3A} or a reactive derivative of this product, in order to obtain a product of formula Xl_{C}: 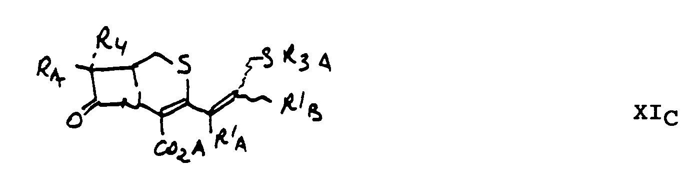 which products of formula XI_{A}, XI_{B} or Xlₑ are subjected, if necessary or if desired, to any one or to several of the following reactions, in any order:
a) detachment by hydrolysis, hydrogenolysis or by the action of thiourea of all or part of the protector group(s);
b) esterification or salification by a base of the carboxy or sulpho group(s);
c) salification by an acid of the amino group(s);
d) resolution of the molecule in order to obtain an optically active product.

5. As medicaments, the products of formula I as defined in claim 1, as well as their salts with pharmaceutically acceptable acids.

6. As medicaments, the products of formula I_{A} as defined in one of claims 2 and 3, as well as their salts with pharmaceutically acceptable acids.

7. Pharmaceutical compositions containing, as active ingredient, at least one of the medicaments according to one of claims 5 or 6.

## Claims (Claims for the following Contracting State(s) : AT)

1. Preparation process for the products corresponding to the general formula (I): 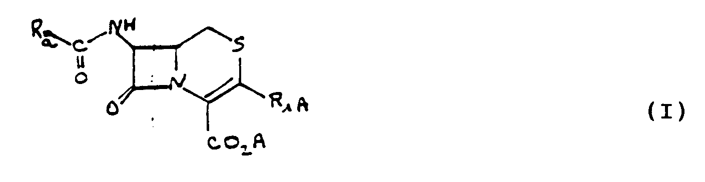 in which Ra represents a radical 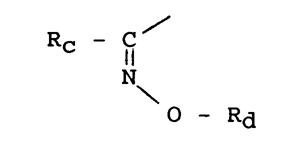 in which R_{c} represents a 2-amino-4-thiazolyl radical, R_{d} represents : either a hydrogen atom, or an alkyl, alkenyl, alkynyl or cycloalkyl radical having at most 6 carbon atoms, or a phenyl radical, the said alkyl, alkenyl, alkynyl or cycloalkyl radicals being optionally substituted by a radical chosen from the following radicals: halogen, cyano, carbamoyl, nitro, amino, hydroxy, mercapto, oxo, free, esterified or salified carboxyl, or a difluoromethyl, trifluoromethyl, methoxymethyl, 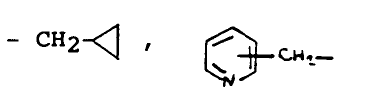 radical, R_{lA} represents: - either a 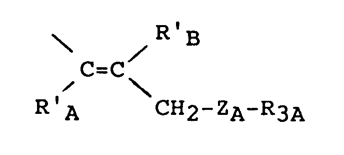 radical, or a 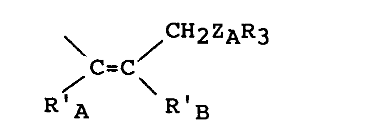 radical, in which formulae R'A and R'_{B}, identical or different represent either a hydrogen atom or an alkyl radical containing 1 to 4 carbon atoms, Z_{A} represents a single bond, an optionally oxidized sulphur atom or an oxygen atom, R₃A represents either a carbocyclic or heterocyclic aryl radical chosen from the following radicals: phenyl, diphenyl, naphthyl, thienyl, furyl, pyrrolyl, imadazolyl, pyrazolyl, thiazolyl, isothiazolyl, oxazolyl, isoxazolyl, triazolyl, thiadiazolyl, oxadiazolyl, tetrazolyl, pyridinyl, pyrimidinyl, pyrazinyl, pyridazinyl, thiazinyl, oxazinyl, triazinyl, thiadiazinyl, oxadiazinyl, tetrazinyl, imidazolinyl, benzimidazolyl, benzothiazolyl, benzoxazole, or on the condition that Z_{A} represents a single bond a quaternary ammonium radical chosen from the following radicals: pyridinium, thienopyridinium, quinolinium, isoquinolinium, cyclopentylpyridinium, trimethylammonium, thiazolopyridinium, cyclohexenopyridinium, imidazopyridinium, the R₃A radicals being able to be substituted by one or more radicals chosen from either the alkyl radicals having 1 to 4 carbon atoms, these alkyl radicals being themselves optionally substituted by a phenyl or thienyl radical, phenoxy, methoxy, ethoxycarbonyl, halogen, hydroxy, free, esterified or salified carboxy or amino radicals, or one of the following radicals: vinyl, allyl, butenyl, ethynyl, propargyl, phenyl, tolyl, halogen, amino, nitro, alkoxy having 1 to 4 carbon atoms, methylthio, hydroxy, mercapto, amino, free, esterified or salified carboxyl or carbamoyl, or R₃A represents one of the following radicals: acetyl, carbamoyl, alkoxycarbonyl, alkyl or haloalkyl having 1 to 4 carbon atoms, nitrile or azido, R₄ represents a hydrogen atom or a methoxy radical, A represents a hydrogen atom, an equivalent of an alkali metal, an alkaline-earth metal, magnesium, ammonium or an amino organic base or A represents an ester group or C0₂A represents a C0₂^{e} group, n₂ represents an integer equal to 0, 1 or 2, as well as the salts of the products of formula I with mineral or organic acids, characterized in that a product of formula (VII): 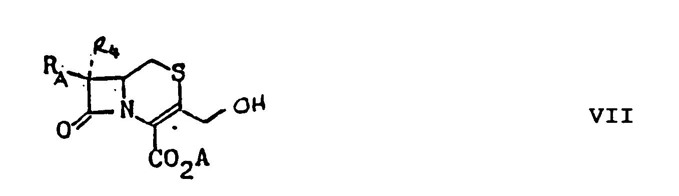 in which R₄ and A have the meaning indicated previously and R_{A} represents either an amino radical free or protected by a mono or divalent protector group, or R_{A} represents the 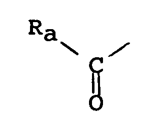 radical, Rₐ having the meaning indicated previously, is treated either by a halogenation agent or by a sulphonation agent, then by triphenylphosphine or by a reagent of formula P(OAlk)₃ in which Alk represents an alkyl radical having 1 to 4 carbon atoms, in order to obtain either a product of formula VIII_{A}: 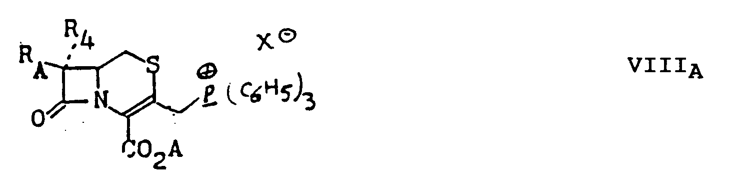 in which R_{A}, R₄ and A have the previous meaning and X^{e} represents the remainder of an anion, or a product of formula VIII'_{A}: 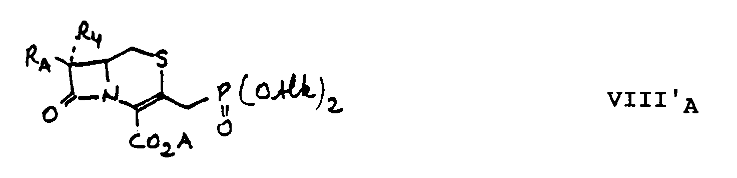 in which R_{A}, R₄, and Alk have the previous meaning, which products of formulae VillA or VIII'_{A}, if desired, are first treated with a strong base then by an alkyl halide of formula hal-R"A in which hal represents a halogen atom and R"A has the values of R'A different to a hydrogen atom, in order to obtain the products of formulae VIII_{B} or VIII'_{B}: 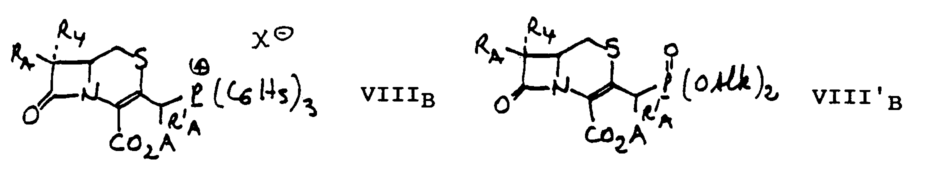 formulae in which R'A has the meaning indicated previously and which products of formulae VIII_{B} or VIII'_{B} are treated with a product of formula: 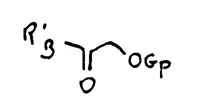 in which R'B has the value indicated previously and Gp represents a protector group of the hydroxyl radical, in order to obtain a product of formula IX: 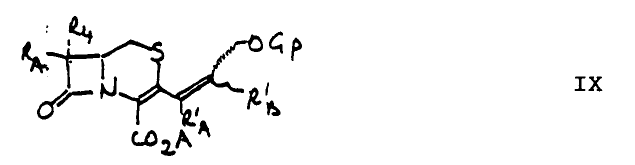 in the form of a mixture of E and Z isomers or in the form of E or Z isomer, on which product of formula IX the following reactions are carried out in any order:
a) detachment of the protector group Gp;
b) when R_{A} represents an amino group protected by a mono or divalent protector group, elimination of this protector group and treatment of the product in which R_{A} represents an amino radical by an acid of formula IVₐ: 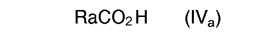 formula in which Ra has the meaning indicated previously, or by a functional derivative of this acid;
c) separation of E and Z isomers; in order to obtain a product of formula X: 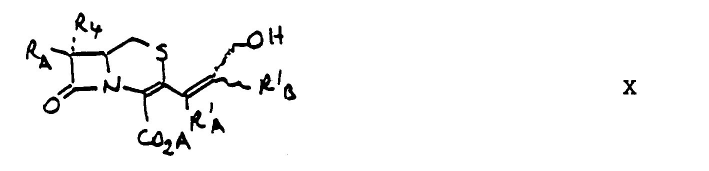 in which R_{A} has the meaning indicated previously, on which product of formula X is reacted either a reactive derivative of the R_{3A} group in order to obtain a product of formula XI_{A}: 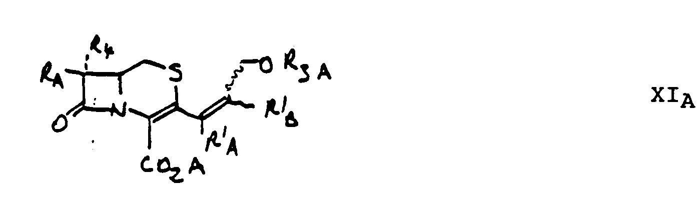 or a derivative of an activation group of the hydroxyl radical then a derivative or a reactive derivative of this R_{3A} group in order to obtain a product of formula XI_{B}: 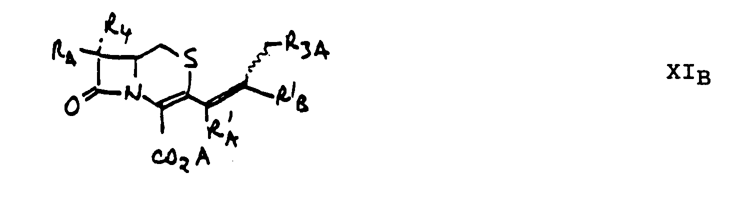 or a derivative of an activation group of the hydroxyl radical then the product of formula HS-R_{3A} or a reactive derivative of this product, in order to obtain a product of formula Xl_{C}: 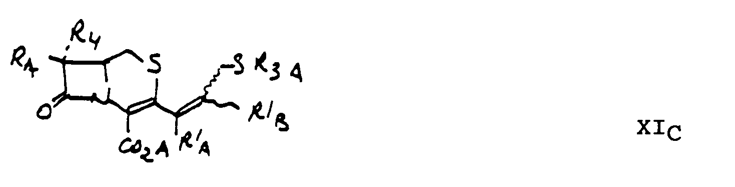 which products of formula XI_{A}, XI_{B} or Xlₑ are subjected, if necessary or if desired, to any one or to several of the following reactions, in any order:
a) detachment by hydrolysis, hydrogenolysis or by the action of thiourea of all or part of the protector group(s);
b) esterification or salification by a base of the carboxy or sulpho group(s);
c) salification by an acid of the amino group(s);
d) resolution of the molecule in order to obtain an optically active product.

. Process according to claim 1, for the preparation of products corresponding to formula (I_{A}): 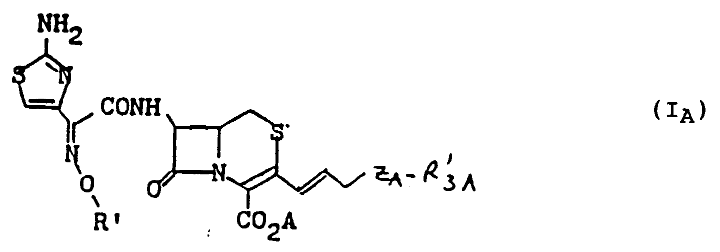 syn isomer in which R' represents a hydrogen atom or an alkyl, alkenyl, alkynyl or cycloalkyl radical having at most 6 carbon atoms, optionally substituted by a free, esterified or salified carboxy radical or a difluromethyl or methoxymethyl radical, Z_{A} represents a single bond and R'_{3A} represents one of the following radicals: pyridinium, thienopyridinium, thiazolopyridinium, cyclopentylpyridinium, cyclohexenopyridinium, trimethylammonium, these radicals being able to be substituted by a radical chosen from alkyl and amino radicals, characterized in that a product of formula (VII): 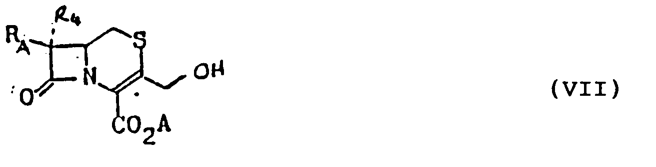 in which A has the meaning indicated in claim 1 and R_{c} represents either an amino radical free or protected by a mono or divalent protector group, or R_{c} represents the optionally protected radical: 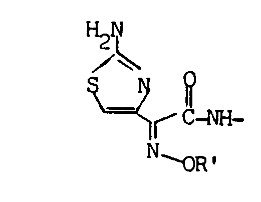 is treated either by a halogenation agent or by a sulphonation agent, then by triphenylphosphine or by a reagent of formula P(OAlk)₃ in which Alk represents an alkyl radical having 1 to 4 carbon atoms, in order to obtain either a product of formula (VIII_{A}): 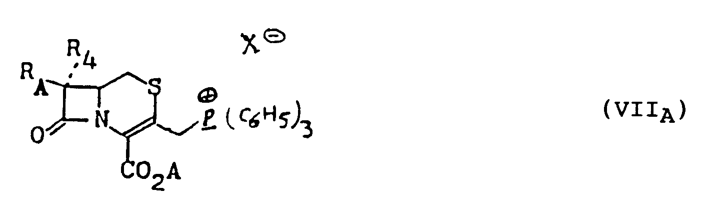 in which R_{c} and A have the previous meaning and X⁻ represents the remainder of an anion, or a product of formula VIII'_{A}: 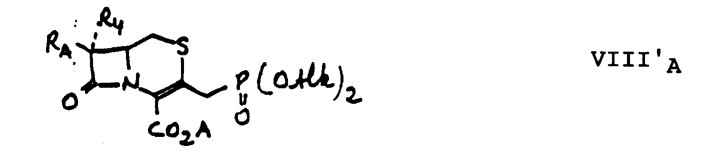 in which R_{c}, A and Alk have the previous meaning, which products of formulae VillA or VIII'_{A} are treated with a product of formula : 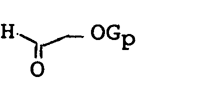 in which Gp represents a protector group of the hydroxyl radical, in order to obtain a product of formula IX': 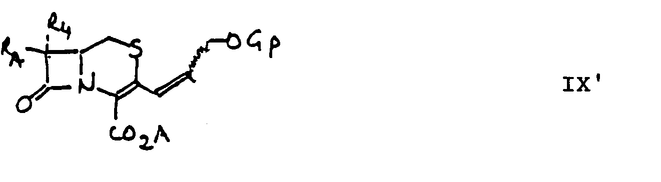 in the form of a mixture of E and Z isomers or in the form of E or Z isomers, on which product of formula IX' the following reactions are carried out, in any order:
a) detachment of the protector group Gp;
b) when R_{c} represents an amino radical protected by a mono or divalent protector group, elimination of this protector group and treatment of the product in which R_{c} represents an amino radical by an acid of formula IV'ₐ: 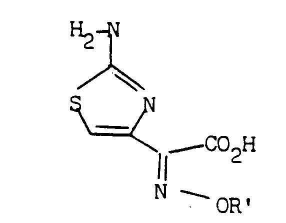 or by a functional derivative of this acid;
c) separation of E and Z isomers; in order to obtain a product of formula X': 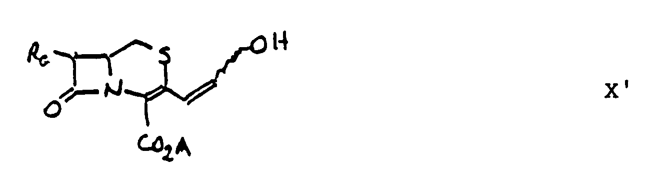 in which R_{c} has the meaning indicated in claim 1, on which product of formula X' is reacted either a reactive derivative of the R'_{3A} group in order to obtain a product of formula XI'_{A}: 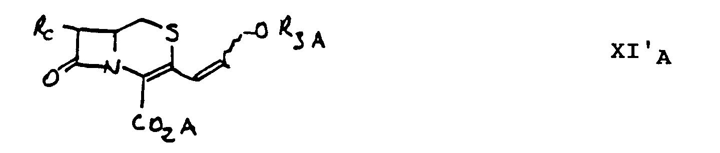 or a derivative of an activation group of the hydroxyl radical then a derivative or a reactive derivative of this R'_{3A} group in order to obtain a product of formula XI'_{B}: 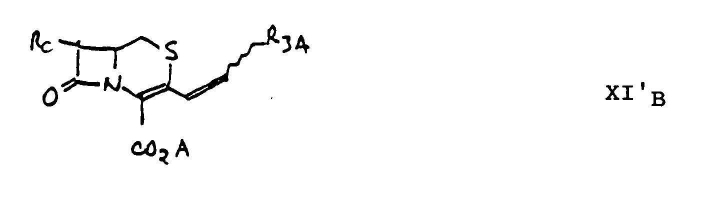 or a derivative of an activation group of the hydroxyl radical then the product of formula HS-R'_{3A} or a reactive derivative of this product, in order to obtain a product of formula XI'ₑ: 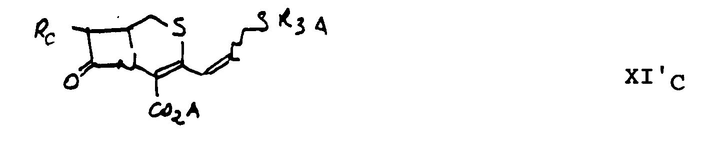 which products of formula XI'_{A}, XI'_{B} or Xl'_{c} are subjected, if necessary or if desired, to any one or to several of the following reactions, in any order:
a) detachment by hydrolysis, hydrogenolysis or by the action of thiourea of all or part of the protector group(s);
b) esterification or salification by a base of the carboxy or sulpho group(s);
c) salification by an acid of the amino group(s);
d) resolution of the molecule in order to obtain an optically active product.

3. Process according to claim 2, characterized in that a compound of formula VII or a compound of formula IV'ₐ isused at the start, in which R' represents a hydrogen atom or a methyl or isopropyl radical, optionally substituted by a carboxy radical, a difluoromethyl radical, a cyclobutyl or cyclopropyl radical, optionally substituted by a carboxy radical.

4. Process according to claim 2 pr 3, characterized in that the product of formula X' is treated with a derivative of an activation group of the hydroxyl radical, then with a derivative or a reactive derivative of an optionally substituted pyridinium radical.

5. Process according to claim 1, characterized in that any one of the compounds of formula 1 are prepared the names of which follow:
- the internal salt of 7-[3-[7-[[(2-amino-4-thiazolyl)-2]-7-(difluoromethoxy)-imino]-acetyl]-amino]-2-carboxy-8-oxo-4-thia-1-azabicyclo-[4.2.0-[oct-2-en-3-yl-[2-(E)-propenyl]-thieno]-2,3-b]-pyridinium (6S)(7S);
- the internal salt of 5-[3-[7-[[(2-amino-4-thiazolyl)-(methoxyimino)-acetyl]-amino]-2-carboxy-8-oxo-4-thia-1-azabicyclo]-4.2.0]-oct-2-en-3-yl-[2-(E)-propenyl[-2-methyl-thiazolo]-4,5-c]-pyridinium syn (6S)(7S);
- the internal salt of 7-[3-[7-[[(2-amino-4-thiazolyl)-2-(Z)-(methoxyimino)-acetyl]-amino]-2-carboxy-8-oxo-4-thia-1-azabicyclo]-4.2.0]-oct-2-en-3-yl-[propen-2-(E)-yl-[thieno]-2,3-b]-pyridinium (6S)(7S);
- 7-[[(2-amino-4-thiazolyl)-(methoxyimino)-acetyl]-amino]-3-[[3-(1,3,4-thiadiazol-2-yl)-thio[-1-(E)-propenyl]-8-oxo-4-thia-1-azabicyclo]-4.2.0]-oct-2-en-2-carboxylic acid syn (6S)(7S);
- the internal salt of 5-[3-[7-[[(2-amino-4-thiazolyl)-(difluoromethoxy)-imino]-acetyl]-amino-2-carboxy-8-oxo-4-thia-1-azabicyclo-[4.2.0-[oct-2-en-3-yl-[2-(E)-propenyl]-2-methyl-thiazolo]-4,5-c]-pyridinium (6S)(7S) Z.

## Patentansprüche (Patentansprüche für folgende(n) Vertragsstaat(en) : BE, CH, DE, FR, GB, IT, LI, LU, NL, SE)

1. Verbindungen der allgemeinen Formel I, 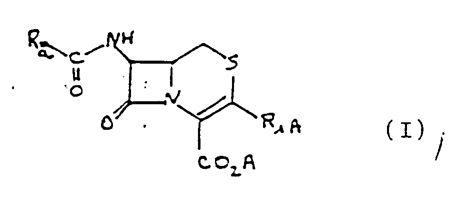 in der bedeuten:
Rₐ eine Gruppe 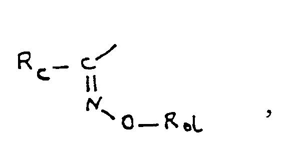 worin
R_{c} 2-Amino-4-thiazolyl und
R_{d} entweder ein Wasserstoffatom oder Alkyl, Alkenyl, Alkinyl oder Cycloalkyl mit höchstens 6 Kohlenstoffatomen oder eine Phenylgruppe, wobei die Alkyl-, Alkenyl-, Alkinyl- oder Cycloalkylgruppen gegebenenfalls mit einer unter Halogenen, Cyano, Carbamoyl, Nitro, Amino, Hydroxy, Mercapto, Oxo sowie freiem, verestertem oder in Salzform übergeführtem Carboxyl ausgewählten Gruppe substituiert sind oder Difluormethyl, Trifluormethyl, Methoxymethyl 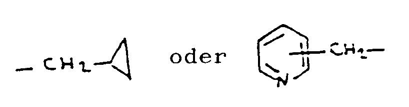 darstellen,
R_{1A} eine Gruppe der Formel 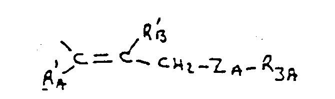 oder eine Gruppe der Formel 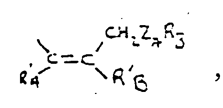 in denen R'_{A} und R'_{B}, die identisch oder verschieden sind, ein Wasserstoffatom oder Alkyl mit 1 bis 4 Kohlenstoffatomen und Z_{A} eine Einfachbindung, ein Schwefelatom, das gegebenenfalls oxidiert ist, oder ein Sauerstoffatom darstellen, wobei R_{3A} bedeutet: eine carbocyclische oder heterocyclische Arylgruppe, die ausgewählt ist unter Phenyl, Diphenyl, Naphthyl, Thienyl, Furyl, Pyrrolyl, Imidazolyl, Pyrazolyl, Thiazolyl, Isothiazolyl, Oxazolyl, Isoxazolyl, Triazolyl, Thiadiazolyl, Oxadiazolyl, Tetrazolyl, Pyridinyl, Pyrimidinyl, Pyrazinyl, Pyridazinyl, Thiazinyl, Oxazinyl, Triazinyl, Thiadiazinyl, Oxadiazinyl, Tetrazinyl, Imidazolinyl, Benzimidazolyl, Benzothiazolyl oder Benzoxazol, oder, wenn Z_{A} eine Einfachbindung darstellt, eine quaternäre Ammoniumgruppe, die ausgewählt ist unter Pyridinium, Thienopyridinium, Chinolinium, Isochinolinium, Cyclopentylpyridinium, Trimethylammonium, Thiazolopyridinium, Cyclohexenopyridinium und Imidazopyridinium, wobei die Gruppen R₃A mit einer oder mehreren Gruppen substituiert sein können, die ausgewählt sind unter Alkyl mit 1 bis 4 Kohlenstoffatomen, wobei diese Alkylgruppen ihrerseits gegebenenfalls mit Phenyl oder Thienyl substituiert sein können, Phenoxy, Methoxy, Ethoxycarbonyl, Halogenen, Hydroxy, freiem, verestertem oder in Salzform übergeführtem Carboxy, Amino, Vinyl, Allyl, Butenyl, Ethinyl, Propargyl, Phenyl, Tolyl, Halogenen, Amino, Nitro, Alkoxy mit 1 bis 4 Kohlenstoffatomen, Methylthio, Hydroxy, Mercapto, Amino, freiem, verestertem oder in Salzform übergeführtem Carboxy und Carbamoyl, oder R₃A Acetyl, Carbamoyl, Alkoxycarbonyl, Alkyl oder Halogenalkyl mit 1 bis 4 Kohlenstoffatomen, Nitril oder Azido bedeutet,
R₄ ein Wasserstoffatom oder Methoxy,
A ein Wasserstoffatom, ein Äquivalent eines Alkalimetalls, eines Erdalkalimetalls einschließlich Magnesium, Ammonium oder einer organischen Aminbase oder eine Estergruppe, oder C0₂A eine Gruppe C0₂-und
n₂ 1, 2 oder 3, sowie die Salze der Verbindungen der Formel I mit den anorganischen oder organischen Säuren.

2. Verbindungen nach Anspruch 1 der Formel I_{A}, 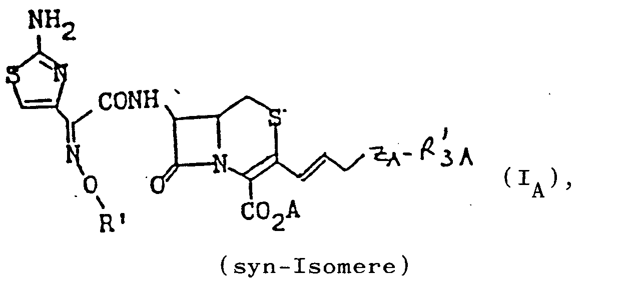 in der bedeuten:
R' ein Wasserstoffatom oder Alkyl, Alkenyl, Alkinyl oder Cycloalkyl mit maximal 6 Kohlenstoffatomen, die gegebenenfalls mit einer freien, veresterten oder in Salzform übergeführten Carboxygruppe, einer Difluormethylgruppe oder einer Methoxymethylgruppe substituiert sind,
Zₐ eine Einfachbindung und
R'_{3A} Pyridinium, Thienopyridinium, Thiazolopyridinium, Cyclopentylpyridinium, Cyclohexenopyridinium oder Trimethylammonium, die mit einer Alkyl- oder Aminogruppe substituiert sein können.

3. Die nachstehenden Verbindungen der Formel I:
Inneres Salz 7-[3-[7[2-Amino-4-thiazolyl-2-(Z)-difluormethoximinoacetamido]-2-carboxy-8-oxo-4-thia-1-azabicyclo-(4.2.0)-oct-2-en-3-yl]-2-(E)-propenyl]-thieno[2,3-b]pyridinium (6S)(7S);
inneres Salz 5-[3-[7-[2-Amino-4-thiazolylmethoximinoacetamido]-2-carboxy-8-oxo-4-thia-1-azabicyclo-(4.2.0)-oct-2-en-3-yl]-2-(E)-propenyl]-2-methylthiazolo[4,5-c]pyridinium syn (6S)(7S);
inneres Salz 7-[3-[7-[2-Amino-4-thiazolyl-2-(Z)-methoximinoacetamido]-2-carboxy-8-oxo-4-thia-1 - azabicyclo-(4.2.0)-oct-2-en-3-yl]-propen-2-(E)-yl]-thieno-[2,3-b]pyridinium (6S)(7S);
Säure 7-[2-Amino-4-thiazolylmethoximinoacetamido]-3-[[3-(1,3,4-thiadiazol-2-yl)-thio]-1-(E)-propenyl]-8-oxo-4-thia-1-azabicyclo-(4.2.0)-oct-2-en-2-carbonsäure syn (6S)(7S);
inneres Salz 5-[3-[7-[2-Amino-4-thiazolyldifluormethoximinoacetamido]-2-carboxy-8-oxo-4-thia-1-azabicyclo-(4.2.0)-oct-2-en-3-yl -2-(E)-propenyl]-2-methylthiazolo[4,5-c]pyridinium) (6S)(7S) Z.

4. Verfahren zur Herstellung der Verbindungen der Formel 1 nach Anspruch 1, gekennzeichnet durch Behandlung einer Verbindung der Formel VII, 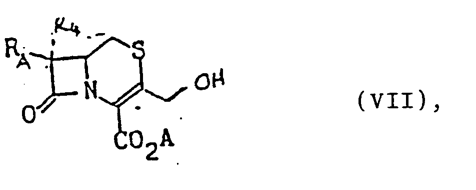 worin bedeuten:
R₄ und A dasselbe wie in Anspruch 1 und
R_{A} eine freie oder durch eine ein- oder zweiwertige Schutzgruppe geschützte Aminogruppe oder eine Gruppe 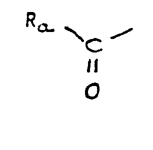 wobei Rₐ dasselbe wie in Anspruch 1 bedeutet, entweder mit einem Halogenierungsmittel oder mit einem Sulfonierungsmittel und anschließend mit Triphenylphosphin oder einem Reagens mit der Formel P(OAlk)₃, in der Alk eine Alkylgruppe mit 1 bis 4 Kohlenstoffatomen darstellt, unter Erhalt entweder einer Verbindung der Formel VIII_{A}, 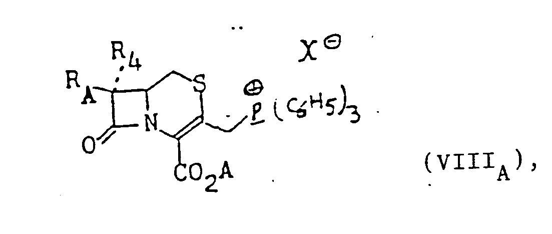 in der R_{A}, R₄ und A die obige Bedeutung haben und X⁻ den Rest eines Anions darstellt, bzw. einer Verbindung der Formel VIII'_{A}, 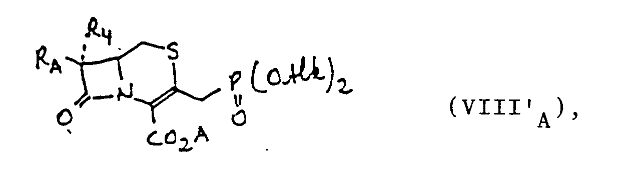 in der R_{A}, R₄, A und Alk die obige Bedeutung haben, und gewünschtenfalls anschließende Behandlung der Verbindungen der Formeln VIII_{A} oder VIII'_{A} zunächst mit einer starken Base und anschließend mit einem Alkylhalogenid der Formel hal-R"_{A}, in der hal ein Halogenatom und R"_{A} dasselbe wie R'_{A} mit Ausnahme von Wasserstoffatom bedeuten, unter Erhalt der Verbindungen der Formeln VIII_{B} oder VIII'_{B}, 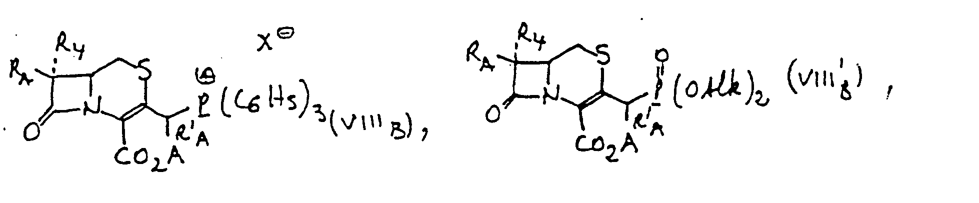 in denen R'_{A} dasselbe wie in Anspruch 1 bedeutet, und danach Behandlung der Verbindungen der Formeln VIII_{B} oder VIII'_{B} mit einer Verbindung der Formel 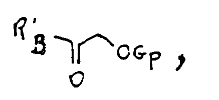 in der R'_{B} dasselbe wie in Anspruch 1 und Gp eine Hydroxy-Schutzgruppe darstellen, unter Erhalt einer Verbindung der Formel IX 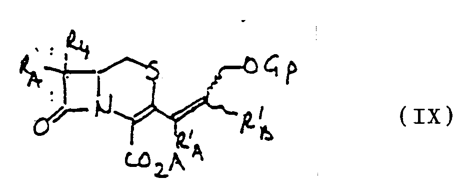 in Form eines Gemischs der E- und Z-Isomeren oder in Form des E- oder Z-Isomers, worauf die Verbindung der Formel IX folgenden Umsetzungen in beliebiger Reihenfolge unterzogen wird:
a) Abspaltung der Schutzgruppe Gp,
b) wenn R_{A} eine durch eine ein- oder zweiwertige Schutzgruppe geschützte Aminogruppe darstellt, Abspaltung dieser Schutzgruppe und Behandlung der Verbindung, in deren Formel R_{A} eine Aminogruppe darstellt, mit einer Säure der Formel IVa, 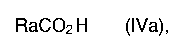 in der Ra dasselbe wie in Anspruch 1 bedeutet, oder mit einem funktionellen Derivat dieser Säure,
c) Trennung der E- und Z-Isomeren, unter Erhalt einer Verbindung der Formel X 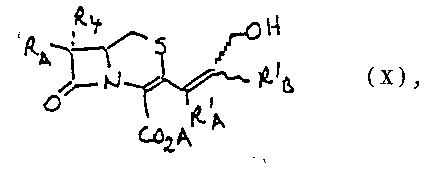 in der R_{A} dasselbe wie in Anspruch 1 bedeutet, und Umsetzung der Verbindung der Formel X entweder mit einem reaktiven Derivat der Grupper R_{3A} unter Erhalt einer Verbindung der Formel XI_{A} 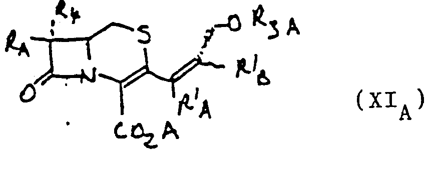 oder mit einem Derivat einer Hydroxy-Aktivierungsgruppe und dann mit einem Derivat oder einem reaktiven Derivat der Gruppe R_{3A} unter Erhalt einer Verbindung der Formel XI_{B} 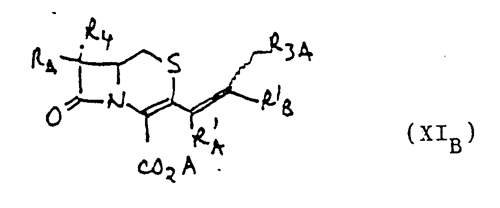 oder mit einem Derivat einer Hydroxy-Aktivierungsgruppe und dann der Verbindung der Formel HS-R_{3A} oder einem reaktiven Derivat dieser Verbindung unter Erhalt einer Verbindung der Formel Xl_{C} 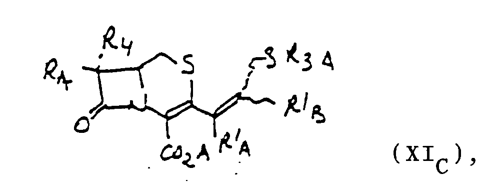 worauf die Verbindungen der Formeln XI_{A}, XI_{B} oder Xl_{C} erforderlichenfalls oder gewünschtenfalls einer oder mehreren der folgenden Reaktionen in beliebiger Reihenfolge unterzogen werden:
a) Abspaltung aller oder eines Teils des bzw. der Schutzgruppen durch Hydrolyse, Hydrogenolyse oder durch Einwirkung von Thioharnstoff;
b) Veresterung der Carboxy- oder Sulfogruppe(n) oder Überführung mit einer Base in entsprechende Salze;
c) Überführung der Aminogruppen mit einer Säure in entsprechende Salze;
d) Spaltung, um ein optisch aktives Produkt zu erhalten.

5. Die Verbindungen der Formel 1 nach Anspruch 1 sowie ihre Salze mit pharmazeutisch akzeptablen Säuren als Arzneimittel.

6. Die Verbindungen der Formel I_{A} nach Anspruch 2 oder 3 sowie ihre Salze mit pharmazeutisch akzeptablen Säuren als Arzneimittel.

7. Pharmazeutische Zusammensetzungen, die als Wirkstoff mindestens ein Arzneimittel nach Anspruch 5 oder 6 enthalten.

## Patentansprüche (Patentansprüche für folgende(n) Vertragsstaat(en) : AT)

1. Verfahren zur Herstellung der Verbindungen der allgemeinen Formel I, 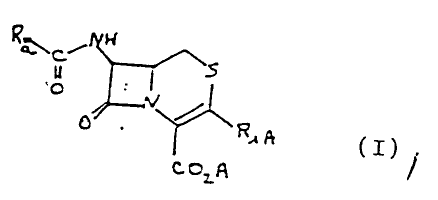 in der bedeuten:
Rₐ eine Gruppe 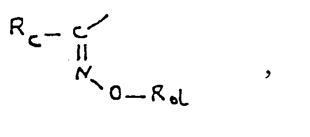 worin
R_{c} 2-Amino-4-thiazolyl und
R_{d} entweder ein Wasserstoffatom oder Alkyl, Alkenyl, Alkinyl oder Cycloalkyl mit höchstens 6 Kohlenstoffatomen oder eine Phenylgruppe, wobei die Alkyl-, Alkenyl-, Alkinyl- oder Cycloalkylgruppen gegebenenfalls mit einer unter Halogenen, Cyano, Carbamoyl, Nitro, Amino, Hydroxy, Mercapto, Oxo sowie freiem, verestertem oder in Salzform übergeführtem Carboxyl ausgewählten Gruppe substituiert sind oder Difluormethyl, Trifluormethyl, Methoxymethyl 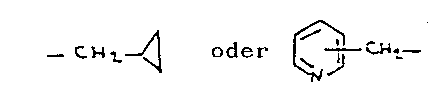 darstellen,
R_{1A} eine Gruppe der Formel 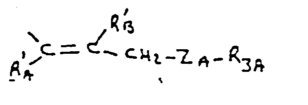 oder eine Gruppe der Formel 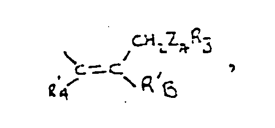 in denen R'_{A} und R'_{B}, die identisch oder verschieden sind, ein Wasserstoffatom oder Alkyl mit 1 bis 4 Kohlenstoffatomen und Z_{A} eine Einfachbindung, ein Schwefelatom, das gegebenenfalls oxidiert ist, oder ein Sauerstoffatom darstellen, wobei R_{3A} bedeutet: eine carbocyclische oder heterocyclische Arylgruppe, die ausgewählt ist unter Phenyl, Diphenyl, Naphthyl, Thienyl, Furyl, Pyrrolyl, Imidazolyl, Pyrazolyl, Thiazolyl, Isothiazolyl, Oxazolyl, Isoxazolyl, Triazolyl, Thiadiazolyl, Oxadiazolyl, Tetrazolyl, Pyridinyl, Pyrimidinyl, Pyrazinyl, Pyridazinyl, Thiazinyl, Oxazinyl, Triazinyl, Thiadiazinyl, Oxadiazinyl, Tetrazinyl, Imidazolinyl, Benzimidazolyl, Benzothiazolyl oder Benzoxazol, oder, wenn Z_{A} eine Einfachbindung darstellt, eine quaternäre Ammoniumgruppe, die ausgewählt ist unter Pyridinium, Thienopyridinium, Chinolinium, Isochinolinium, Cyclopentylpyridinium, Trimethylammonium, Thiazolopyridinium, Cyclohexenopyridinium und Imidazopyridinium, wobei die Gruppen R₃A mit einer oder mehreren Gruppen substituiert sein können, die ausgewählt sind unter Alkyl mit 1 bis 4 Kohlenstoffatomen, wobei diese Alkylgruppen ihrerseits gegebenenfalls mit Phenyl oder Thienyl substituiert sein können, Phenoxy, Methoxy, Ethoxycarbonyl, Halogenen, Hydroxy, freiem, verestertem oder in Salzform übergeführtem Carboxy, Amino, Vinyl, Allyl, Butenyl, Ethinyl, Propargyl, Phenyl, Tolyl, Halogenen, Amino, Nitro, Alkoxy mit 1 bis 4 Kohlenstoffatomen, Methylthio, Hydroxy, Mercapto, Amino, freiem, verestertem oder in Salzform übergeführtem Carboxy und Carbamoyl, oder R₃A Acetyl, Carbamoyl, Alkoxycarbonyl, Alkyl oder Halogenalkyl mit 1 bis 4 Kohlenstoffatomen, Nitril oder Azido bedeutet,
R₄ ein Wasserstoffatom oder Methoxy,
A ein Wasserstoffatom, ein Äquivalent eines Alkalimetalls, eines Erdalkalimetalls einschließlich Magnesium, Ammonium oder einer organischen Aminbase oder eine Estergruppe, oder C0₂A eine Gruppe C0₂-, und
n₂ 1, 2 oder 3, sowie der Salze der Verbindungen der Formel I mit den anorganischen oder organischen Säuren, gekennzeichnet durch Behandlung einer Verbindung der Formel VII, 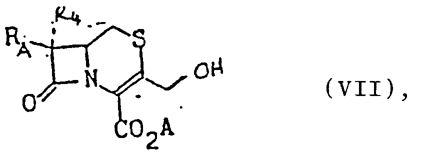 worin bedeuten:
R₄ und A dasselbe wie in Anspruch 1 und
R_{A} eine freie oder durch eine ein- oder zweiwertige Schutzgruppe geschützte Aminogruppe oder eine Gruppe 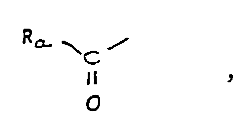 wobei Rₐ dasselbe wie in Anspruch 1 bedeutet, entweder mit einem Halogenierungsmittel oder mit einem Sulfonierungsmittel und anschließend mit Triphenylphosphin oder einem Reagens mit der Formel P(OAlk)₃, in der Alk eine Alkylgruppe mit 1 bis 4 Kohlenstoffatomen darstellt, unter Erhalt entweder einer Verbindung der Formel VIII_{A}, 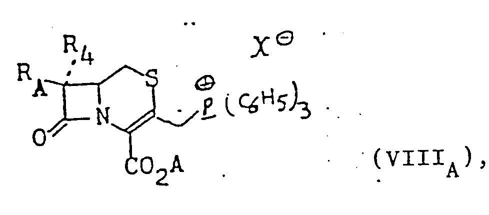 in der R_{A}, R₄ und A die obige Bedeutung haben und
X⁻ den Rest eines Anions darstellt, bzw. einer Verbindung der Formel VIII'_{A}, 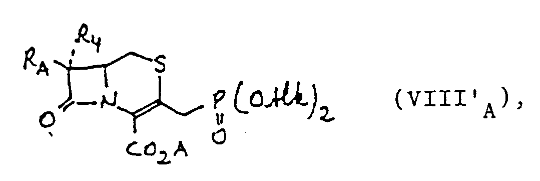 in der R_{A}, R₄, A und Alk die obige Bedeutung haben, und gewünschtenfalls anschließende Behandlung der Verbindungen der Formeln VIII_{A} oder VIII'_{A} zunächst mit einer starken Base und anschließend mit einem Alkylhalogenid der Formel hal-R"_{A}, in der hal ein Halogenatom und R"_{A} dasselbe wie R'_{A} mit Ausnahme von Wasserstoffatom bedeuten, unter Erhalt der Verbindungen der Formeln VIII_{B} oder VIII'_{B}, 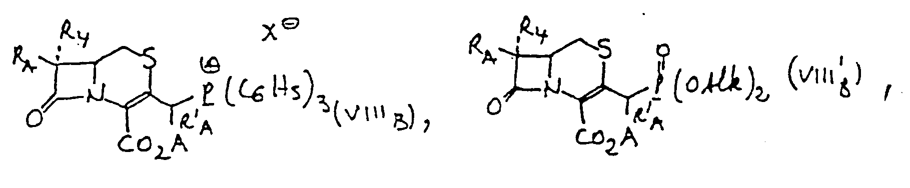 in denen R'_{A} dasselbe wie in Anspruch 1 bedeutet, und danach Behandlung der Verbindungen der Formeln VIII_{B} oder VIII'_{B} mit einer Verbindung der Formel 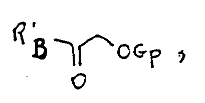 in der R'_{B} dasselbe wie in Anspruch 1 und Gp eine Hydroxy-Schutzgruppe darstellen, unter Erhalt einer Verbindung der Formel IX 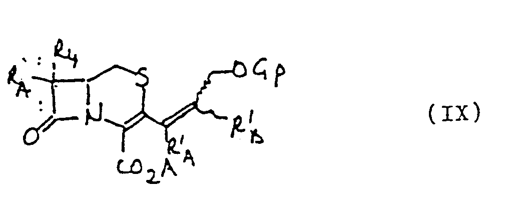 in Form eines Gemischs der E- und Z-Isomeren oder in Form des E- oder Z-Isomers, worauf die Verbindung der Formel IX folgenden Umsetzungen in beliebiger Reihenfolge unterzogen wird:
a) Abspaltung der Schutzgruppe Gp,
b) wenn R_{A} eine durch eine ein- oder zweiwertige Schutzgruppe geschützte Aminogruppe darstellt, Abspaltung dieser Schutzgruppe und Behandlung der Verbindung, in deren Formel R_{A} eine Aminogruppe darstellt, mit einer Säure der Formel IVa, 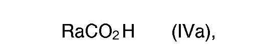 in der Ra dasselbe wie in Anspruch 1 bedeutet, oder mit einem funktionellen Derivat dieser Säure,
c) Trennung der E- und Z-Isomeren, unter Erhalt einer Verbindung der Formel X 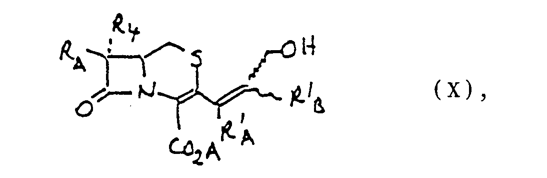 in der R_{A} dasselbe wie in Anspruch 1 bedeutet, und Umsetzung der Verbindung der Formel X entweder mit einem reaktiven Derivat der Grupper R_{3A} unter Erhalt einer Verbindung der Formel XI_{A} 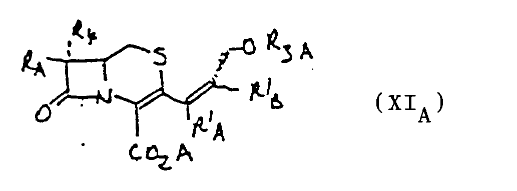 oder mit einem Derivat einer Hydroxy-Aktivierungsgruppe und dann mit einem Derivat oder einem reaktiven Derivat der Gruppe R_{3A} unter Erhalt einer Verbindung der Formel XI_{B} 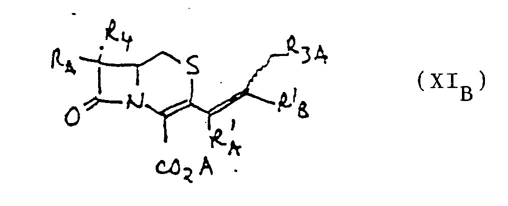 oder mit einem Derivat einer Hydroxy-Aktivierungsgruppe und dann der Verbindung der Formel HS-R_{3A} oder einem reaktiven Derivat dieser Verbindung unter Erhalt einer Verbindung der Formel Xl_{C} 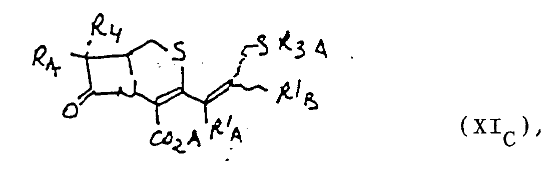 worauf die Verbindungen der Formeln XI_{A}, XI_{B} oder Xl_{C} erforderlichenfalls oder gewünschtenfalls einer oder mehreren der folgenden Reaktionen in beliebiger Reihenfolge unterzogen werden:
a) Abspaltung aller oder eines Teils des bzw. der Schutzgruppen durch Hydrolyse, Hydrogenolyse oder durch Einwirkung von Thioharnstoff;
b) Veresterung der Carboxy- oder Sulfogruppe(n) oder Überführung in entsprechende Salze mit einer Base;
c) Überführung der Aminogruppen mit einer Säure in entsprechende Salze;
d) Spaltung, um ein optisch aktives Produkt zu erhalten.

2. Verfahren nach Anspruch 1 zur Herstellung der Verbindungen der Formel I_{A}, 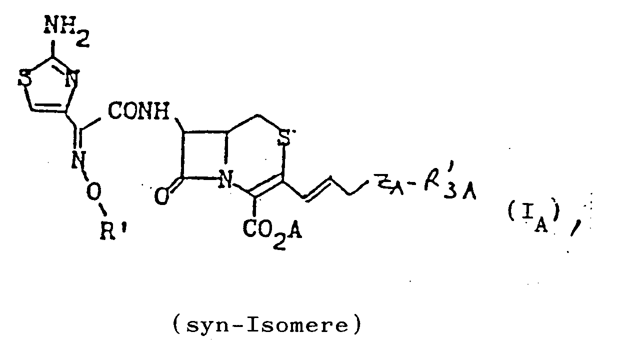 in der bedeuten:
R' ein Wasserstoffatom oder Alkyl, Alkenyl, Alkinyl oder Cycloalkyl mit maximal 6 Kohlenstoffatomen, die gegebenenfalls mit einer freien, veresterten oder in Salzform übergeführten Carboxygruppe, einer Difluormethylgruppe oder einer Methoxymethylgruppe substituiert sind,
Zₐ eine Einfachbindung und
R'_{3A} Pyridinium, Thienopyridinium, Thiazolopyridinium, Cyclopentylpyridinium, Cyclohexenopyridinium oder Trimethylammonium, die mit einer Alkyl- oder Aminogruppe substituiert sein können, gekennzeichnet durch Behandlung einer Verbindung der Formel VII, 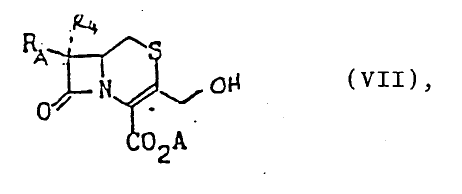 in der A dasselbe wie in Anspruch 1 darstellt und wobei R_{c} entweder eine freie oder durch eine einwertige oder zweiwertige Schutzgruppe geschützte Aminogruppe oder die gegebenenfalls mit einer Schutzgruppe geschützte Gruppe 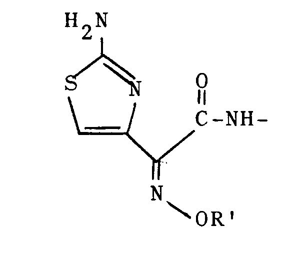 bedeutet, entweder mit einem Halogenierungsmittel oder mit einem Sulfonierungsmittel und anschließend mit Triphenylphosphin oder einem Reagens der Formel P(OAlk)₃, in der Alk eine Alkylgruppe mit 1 bis 4 Kohlenstoffatomen darstellt, unter Erhalt einer Verbindung der Formel VIII_{A}, 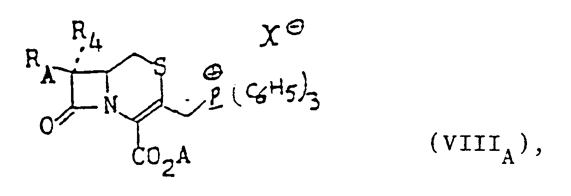 in der R_{c} und A die obige Bedeutung haben und X⁻ den Rest eines Anions darstellt, oder einer Verbindung der Formel VIII'_{A}, 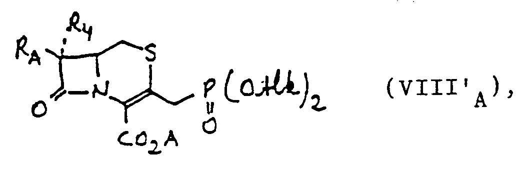 in der R_{c}, A und Alk die obige Bedeutung haben, Behandlung der Verbindungen der Formeln VIII_{A} oder VIII'_{A} mit einer Verbindung der Formel 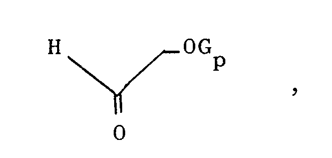 in der Gp eine Hydroxy-Schutzgruppe darstellt, unter Erhalt einer Verbindung Formel IX' 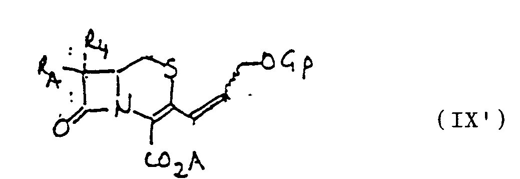 in Form eines Gemischs der E- und Z-Isomeren oder in Form des E- oder Z-Isomers, worauf die Verbindung der Formel IX' folgenden Umsetzungen in beliebiger Reihenfolge unterzogen wird:
a) Abspaltung der Schutzgruppe Gp;
b) wenn R_{A} eine durch eine ein- oder zweiwertige Schutzgruppe geschützte Aminogruppe darstellt, Abspaltung dieser Schutzgruppe und Behandlung der Verbindung, in deren Formel R_{c} eine Aminogruppe darstellt, mit einer Säure der Formel IV'a 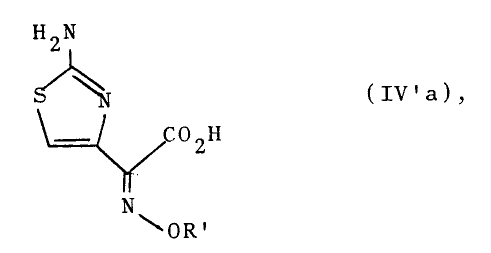 oder mit einem funktionellen Derivat dieser Säure;
c) Trennung der E- und Z-Isomeren unter Erhalt einer Verbindung der Formel X' 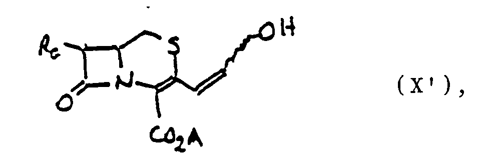 in der R_{c} dasselbe wie in Anspruch 1 bedeutet, und Umsetzung der Verbindung der Formel X' entweder mit einem reaktiven Derivat der Gruppe R'_{3A} unter Erhalt einer Verbindung der Formel XI'_{A} 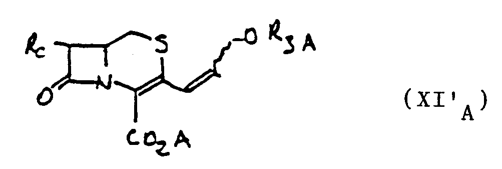 oder mit einem Derivat einer Hydroxy-Aktivierungsgruppe und dann mit einem Derivat oder einem reaktiven Derivat der Gruppe R'_{3A} unter Erhalt einer Verbindung der Formel XI'_{B} 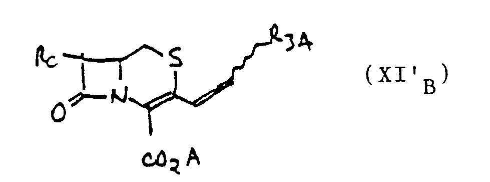 oder mit einem Derivat einer Hydroxy-Aktivierungsgruppe und dann der Verbindung der Formel HS-R'_{3A} oder einem reaktiven Derivat dieser Verbindung unter Erhalt einer Verbindung der Formel XI'ₑ 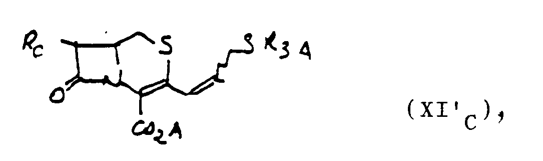 worauf die Verbindungen der Formeln XI'_{A}, XI'_{B} oder XI'α erforderlichenfalls oder gewünschtenfalls einer oder mehreren der folgenden Reaktionen in beliebiger Reihenfolge unterzogen werden:
a) Abspaltung aller oder eines Teils des bzw. der Schutzgruppen durch Hydrolyse, Hydrogenolyse oder durch Einwirkung von Thioharnstoff,
b) Veresterung der Carboxy- oder Sulfogruppe(n) oder Überführung mit einer Base in entsprechende Salze;
c) Überführung der Aminogruppen mit einer Säure in entsprechende Salze;
d) Spaltung, um ein optisch aktives Produkt zu erhalten.

3. Verfahren nach Anspruch 2, dadurch gekennzeichnet, daß anfangs eine Verbindung der Formel VII oder eine Verbindung der Formel IV'a eingesetzt werden, worin R' ein Wasserstoffatom oder gegebenenfalls mit Carboxy substituiertes Methyl oder Isopropyl, Difluormethyl, Cyclobutyl oder gegebenenfalls mit Carboxy substituiertes Cyclopropyl bedeutet.

4. Verfahren nach Anspruch 2 oder 3, dadurch gekennzeichnet, daß die Verbindung der Formel X' mit einem Derivat einer Hydroxy-Aktivierungsgruppe und anschließend mit einem Derivat oder einem reaktiven Derivat einer gegebenenfalls substituierten Pyridiniumgruppe behandelt wird.

5. Verfahren nach Anspruch 1, gekennzeichnet durch Herstellung einer der nachstehenden Verbindungen der Formel I:
Inneres Salz 7-[3-[7-[2-Amino-4-thiazolyl-2-(Z)-difluormethoximinoacetamido]-2-carboxy-8-oxo-4-thia-1-azabicyclo-(4.2.0)-oct-2-en-3-yl]-2-(E)-propenyl]-thieno[2,3-b]pyridinium (6S)(7S);
inneres Salz 5-[3-[7-[2-Amino-4-thiazolylmethoximinoacetamido]-2-carboxy-8-oxo-4-thia-1-azabicyclo-(4.2.0)-oct-2-en-3-yl]-2-(E)-propenyl]-2-methylthiazolo[4,5-c]pyridinium syn (6S)(7S);
inneres Salz 7-[3-[7-[2-Amino-4-thiazolyl-2-(Z)-methoximinoacetamido]-2-carboxy-8-oxo-4-thia-1 - azabicyclo-(4.2.0)-oct-2-en-3-yl]-propen-2-(E)-yl]-thieno-[2,3-b]pyridinium (6S)(7S);
Säure 7-[2-Amino-4-thiazolylmethoximinoacetamido]-3-[[3-(1,3,4-thiaiazol-2-yl)-thio]-1-(E)-propenyl]-8-oxo-4-thia-1-azabicyclo-(4.2.0)-oct-2-en-2-carbonsäure syn (6S)(7S);
inneres Salz 5-[3-[7-[2-Amino-4-thiazolyldifluormethoximinoacetamido]-2-carboxy-8-oxo-4-thia-1-azabicyclo-(4.2.0)-oct-2-en-3-yl -2-(E)-propenyl]-2-methylthiazolo[4,5-c]pyridinium) (6S)(7S) Z.
